# EUROPEAN PATENT SPECIFICATION

(11) **EP 4 452 412 B1**
(45) Date of publication and mention of the grant of the patent: **29.10.2025**
(21) Application number: 22843301.7
(22) Date of filing: 21.12.2022
(51) Int. Cl.: A61P 31/04, C07D 498/22, A61K 31/445

(54) **RIFABUTIN ANALOGS FOR THE TREATMENT OF DISEASE**
RIFABUTIN-ANALOGA ZUR BEHANDLUNG VON KRANKHEITEN
ANALOGUES DE RIFABUTINE POUR LE TRAITEMENT D'UNE MALADIE

(30) Priority: 22.12.2021 EP 21217179
(43) Date of publication of application: 30.10.2024
(73) Proprietor: BioVersys AG, 4057 Basel (CH)
(72) Inventor: ANTRAYGUES, Kevin, 59120 Loos (FR); BOUROTTE, Marilyne, 59840 Perenchies (FR); DALE, Glenn E., 4053 Basel (CH); DEFERT, Olivier, 59000 Lille (FR); GITZINGER, Marc, 4057 Basel (CH); LOCIURO, Sergio, 4058 Basel (CH); MAINGOT, Mathieu, 59800 Lille (FR); TREBOSC, Vincent, 68680 Kembs-Loechlé (FR); WILLAND, Nicolas, 59800 Lille (FR)
(74) Representative: Latscha Schöllhorn Partner AG
(86) International application number: PCT/EP2022/087273
(87) International publication number: WO 2023/118319

(56) References cited:
- KUNIN C M: "ANTIMICROBIAL ACTIVITY OF RIFABUTIN", CLINICAL INFECTIOUS DISEASES, THE UNIVERSITY OF CHICAGO PRESS, CHICAGO, IL, US, vol. 22, no. SUPPL. 01, 1 April 1996 (1996-04-01), pages S03 - S14, XP000600925, ISSN: 1058-4838
- TREBOSC VINCENT ET AL: "Rifabutin for infusion (BV100) for the treatment of severe carbapenem-resistant Acinetobacter baumannii infections", DRUG DISCOVERY TODAY, ELSEVIER, AMSTERDAM, NL, vol. 26, no. 9, 6 July 2021 (2021-07-06), pages 2099 - 2104, XP086789772, ISSN: 1359-6446, [retrieved on 20210706], DOI: 10.1016/J.DRUDIS.2021.07.001

## Description

The present disclosure relates to compounds and pharmaceutical compositions comprising the same for the treatment, amelioration and/or prevention of disease. In some embodiments, the disease is a bacterial infection, preferably a bacterial infection caused by one or more bacterium belonging to the species *A. baumannii,* and/or *S. aureus,* and/or a genus of non-tuberculous *Mycobacteria* (NTM), preferably *M. abscessus.*

### RELATED ART

Rifamycins such as rifabutin are known antibiotics with activity against a broad spectrum of pathogens such as *Clostridium spp., Enterococcus spp., Hemophilus spp., Legionella spp., Mycobacterium spp.* (tuberculous and non-tuberculous Mycobacteria), *Neisseria spp., Staphylococcus spp., Streptococcus spp., Listeria monocytogenes, Moraxella catarrhalis, Bacillus spp., Bacteroides spp., Gardnerella vaginalis, Lactobacillus spp., Mobiluncus spp., Helicobacter pylori, Campylobacter jejuni, Chlamydia trachomatis* and *Toxoplasma gondii* (Kunin, Clin. Infect. Dis., 1996; Farr and Mandell, Med. Clin. North. Am., 1982; Thomsberry et al., Rev. Infect. Dis., 1983; Hoover et al., Diagn. Microbiol. Infect. Dis., 1993; Kerry et al., J. Antimicrob. Chemother., 1975).

Rifabutin has been recently shown to have potent *in vitro* and *in vivo* activity against *Mycobacterium abscessus* (Aziz et al., Antimicrob. Agents Chemother., 2017; Dick et al., Antimicrob. Agents Chemother., 2020) and *Acinetobacter baumannii* (Luna et al., Nat. Microbiol., 2020; Trebosc et al., Drug Discov. Today, 2021; Trebosc et al., J. Antimicrob. Chemother., 2020). However, *A. baumannii* can show reduced rifabutin sensitivity through mechanisms such as mutation of the RNA polymerase β-subunit (RpoB) gene or expression of the ADP ribosyl transferase (Arr) enzyme (Trebosc et al., J. Antimicrob. Chemother., 2020). The Arr enzyme ribosylates rifamycins at the C23 carbon, and mutations of the RpoB subunit limits the ability of rifabutin to interact with and inhibit the RpoB subunit. Moreover, *M. abscessus* endogenously encodes the gene coding for the Arr enzyme, making it naturally less sensitive to treatment with rifabutin (Schäfle et al., Antimicrob. Agents Chemother., 2021). Accordingly, there is a need for more effective rifamycins for the treatment of bacterial infections such as *M. abscessus* and *A. baumannii* infections.

### SUMMARY OF THE INVENTION

In one aspect, the present disclosure provides a compound of the Formula I or a pharmaceutically acceptable salt, tautomer, solvate, hydrate or enantiomer thereof: 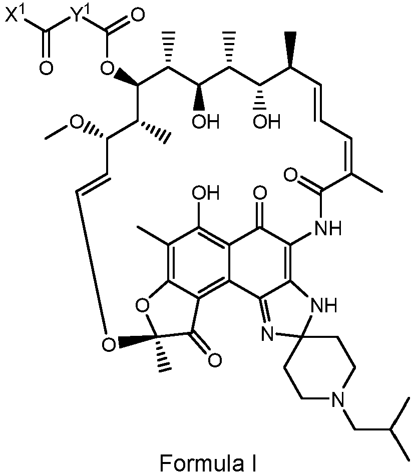 wherein:
Y¹ is (CR¹R²)ₙ, wherein n is an integer from 1 to 6 and R¹ and R² are independently, at each occurrence, selected from the group consisting of hydrogen, -C₁-C₆ alkyl, and -C₁-C₆ alkoxy, wherein each alkyl is optionally substituted with one or more halogen or -C₃-C₆ cycloalkyl, and wherein each cycloalkyl is further optionally substituted with one or more halogen;
X¹ is -OR¹¹ or -NR⁵¹R⁵²;
R¹¹ is independently -C₁-C₆ alkyl, -C₁-C₆ alkylene-R^{12A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, or 5-10 membered heteroaryl, wherein each alkyl, alkylene, alkenyl, alkynyl, cycloalkyl, cycloalkenyl, heterocycloalkyl, aryl or heteroaryl is optionally substituted with one or more R¹²;
R¹² and R^{12A} are each independently -C₁-C₆ alkyl, -C₁-C₆ alkylene-R^{16A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, halogen, cyano, oxo, - OR¹³, -CO₂R¹³, or -NR¹⁴R¹⁵, wherein each alkyl, alkylene, alkenyl, alkynyl, cycloalkyl, cycloalkenyl, heterocycloalkyl, aryl, heteroaryl and alkoxy is optionally substituted with one or more R¹⁶;
R¹³, R¹⁴ and R¹⁵ are each independently hydrogen, -C₁-C₆ alkyl, -C₁-C₆ alkylene-R^{20A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, - S(O)₂(C₁-C₆ alkyl), or -S(O)₂NR¹⁸R¹⁹, wherein each alkyl, alkylene, alkenyl, alkynyl, cycloalkyl, cycloalkenyl, heterocycloalkyl, aryl, heteroaryl and alkoxy is optionally substituted with one or more R²⁰;
R¹⁶ and R^{16A} are each independently -C₁-C₆ alkyl, -C₁-C₆ alkylene-R^{20A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, halogen, cyano, oxo, - OR¹⁷, -S(O)₂(C₁-C₆ alkyl), or -S(O)₂NR¹⁸R¹⁹, wherein each alkyl, alkylene, alkenyl, alkynyl, cycloalkyl, cycloalkenyl, heterocycloalkyl, aryl, heteroaryl and alkoxy is optionally substituted with one or more R²⁰;
R¹⁷, R¹⁸ and R¹⁹ are each independently hydrogen, halogen, -OH, -C₁-C₆ alkyl, -C₁-C₆ alkylene-R^{23A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, -S(O)₂(C₁-C₆ alkyl), or S(O)₂NR²¹R²², wherein each alkyl, alkylene, alkenyl, alkynyl, cycloalkyl, cycloalkenyl, heterocycloalkyl, aryl, heteroaryl and alkoxy is optionally substituted with one or more R²³;
R²⁰ and R^{20A} are each independently halogen, -OH, -C₁-C₆ alkyl, -C₁-C₆ alkylene-R^{23A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, halogen, cyano, oxo, -S(O)₂(C₁-C₆ alkyl), or S(O)₂NR²¹R²², wherein each alkyl, alkylene, alkenyl, alkynyl, cycloalkyl, cycloalkenyl, heterocycloalkyl, aryl, heteroaryl and alkoxy is optionally substituted with one or more R²³;
R²¹ and R²² are each independently hydrogen or -C₁-C₆ alkyl; and
R²³ and R^{23A} are each independently -C₁-C₆ alkyl, -C₁-C₆ alkoxy, -C₁-C₆ haloalkyl, halogen, or cyano;
R⁵¹ and R⁵² are each independently hydrogen, -C₁-C₆ alkyl, -C₁-C₆ alkylene-R^{53A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, or 5-10 membered heteroaryl, wherein each alkyl, alkylene, alkenyl, alkynyl, cycloalkyl, cycloalkenyl, heterocycloalkyl, aryl or heteroaryl is optionally substituted with one or more R⁵³; or R⁵¹ and R⁵², together with the nitrogen atom to which they are attached, combine to form a 3-15 membered heterocycloalkyl or 5-15 membered heteroaryl, wherein the heterocycloalkyl or heteroaryl are optionally substituted with one or more R⁵³;
R⁵³ and R^{53A} are each independently -C₁-C₆ alkyl, -C₁-C₆ alkylene-R^{57A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, halogen, cyano, oxo, - OR⁵⁴, -C(O)R⁵⁴, -CO₂R⁵⁴, or -NR⁵⁵R⁵⁶, wherein each alkyl, alkylene, alkenyl, alkynyl, cycloalkyl, cycloalkenyl, heterocycloalkyl, aryl, heteroaryl and alkoxy is optionally substituted with one or more R⁵⁷;
R⁵⁴, R⁵⁵ and R⁵⁶ are each independently hydrogen, -C₁-C₆ alkyl, -C₁-C₆ alkylene-R^{61A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, - CO₂R⁵⁸, -S(O)₂(C₁-C₆ alkyl), -S(O)₂(C₆-C₁₀ aryl), or -S(O)₂NR⁵⁹R⁶⁰, wherein each alkyl, alkylene, alkenyl, alkynyl, cycloalkyl, cycloalkenyl, heterocycloalkyl, aryl, heteroaryl and alkoxy is optionally substituted with one or more R⁶¹;
R⁵⁷ and R^{57A} are each independently -C₁-C₆ alkyl, -C₁-C₆ alkylene-R^{61A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, halogen, cyano, oxo, - OR⁵⁸, NR⁵⁹R⁶⁰, -S(O)₂(C₁-C₆ alkyl), or -S(O)₂NR⁵⁹R⁶⁰, wherein each alkyl, alkylene, alkenyl, alkynyl, cycloalkyl, cycloalkenyl, heterocycloalkyl, aryl, heteroaryl and alkoxy is optionally substituted with one or more R⁶¹;
R⁵⁸, R⁵⁹ and R⁶⁰ are each independently hydrogen, -C₁-C₆ alkyl, -C₁-C₆ alkylene-R⁶⁴, - C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, - S(O)₂(C₁-C₆ alkyl), or S(O)₂NR⁶²R⁶³, wherein each alkyl, alkylene, alkenyl, alkynyl, cycloalkyl, cycloalkenyl, heterocycloalkyl, aryl, heteroaryl and alkoxy is optionally substituted with one or more R⁶⁴;
R⁶¹ and R^{61A} are each independently halogen, -OH, -C₁-C₆ alkyl, -C₁-C₆ alkylene-R^{64A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, halogen, cyano, oxo, -S(O)₂(C₁-C₆ alkyl), or S(O)₂NR⁶²R⁶³, wherein each alkyl, alkylene, alkenyl, alkynyl, cycloalkyl, cycloalkenyl, heterocycloalkyl, aryl, heteroaryl and alkoxy is optionally substituted with one or more R⁶⁴; and
R⁶² and R⁶³ are each independently hydrogen and -C₁-C₆ alkyl; and
R⁶⁴ and R^{64A} are each independently -C₁-C₆ alkyl, -C₁-C₆ alkoxy, -C₁-C₆ haloalkyl, phenyl, halogen, or cyano.

In one aspect, the present disclosure provides a pharmaceutical composition comprising a compound as described herein (preferably a compound of Formula **I),** or a pharmaceutically acceptable salt thereof, and a pharmaceutically acceptable carrier. **In** some preferred embodiments, the pharmaceutical composition is effective for treating a bacterial infection. **In** some preferred embodiments, the bacterial infection is caused by one or more bacterium belonging to the *genus Acinetobacter, Staphylococcus, and*/*or Mycobacteria.* **In** some preferred embodiments, the bacterial infection is caused by one or more bacterium belonging to the species *A. baumannii,* and/or *S. aureus,* and/or a genus of non-tuberculous *Mycobacteria, preferably M. abscessus.* **In** preferred embodiments, the bacterial infection is caused by one or more bacterium belonging to a genus of non-tuberculous *Mycobacteria,* preferably *M. abscessus.* **In** some embodiments, the infection is caused by one or more bacterium belonging to the genus *Acinetobacter* and/or *Staphylococcus,* preferably *A. baumannii* and/or S. *aureus.*

In one aspect, the present disclosure provides a compound as described herein (preferably a compound of Formula I), or a pharmaceutically acceptable salt thereof, for use in a method for treating a bacterial infection. In some preferred embodiments, the bacterial infection is caused by one or more bacterium belonging to the genus *Acinetobacter, Staphylococcus,* and/or *Mycobacteria.* In some preferred embodiments, the bacterial infection is caused by one or more bacterium belonging to the species *A. baumannii,* and/or *S. aureus,* and/or a genus of non-tuberculous *Mycobacteria,* preferably *M. abscessus.* In preferred embodiments, the bacterial infection is caused by one or more bacterium belonging to a genus of non-tuberculous *Mycobacteria,* preferably *M. abscessus.* In some embodiments, the infection is caused by one or more bacterium belonging to the genus *Acinetobacter* and/or *Staphylococcus,* preferably *A. baumannii* and/or S. *aureus.*

In one aspect, the present disclosure provides a use of a compound as described herein (preferably a compound of Formula I), or a pharmaceutically acceptable salt thereof, or pharmaceutical composition comprising a compound as described herein in the manufacture of a medicament for treating a bacterial infection. In some preferred embodiments, the bacterial infection is caused by one or more bacterium belonging to the genus *Acinetobacter, Staphylococcus,* and/or *Mycobacteria.* In some preferred embodiments, the bacterial infection is caused by one or more bacterium belonging to the species *A. baumannii,* and/or *S. aureus,* and/or a genus of non-tuberculous *Mycobacteria,* preferably *M. abscessus.* In preferred embodiments, the bacterial infection is caused by one or more bacterium belonging to a genus of non-tuberculous *Mycobacteria,* preferably *M. abscessus.* In some embodiments, the infection is caused by one or more bacterium belonging to the genus *Acinetobacter* and/or *Staphylococcus,* preferably *A. baumannii* and/or S. *aureus.*

In one aspect, the present disclosure provides a method of treating a bacterial infection in a subject in need thereof, comprising administering to the subject a therapeutically effective amount of a compound as described herein (preferably a compound of Formula I) or a pharmaceutically acceptable salt thereof. In some preferred embodiments, the bacterial infection is caused by one or more bacterium belonging to the genus *Acinetobacter, Staphylococcus,* and/or *Mycobacteria.* In some preferred embodiments, the bacterial infection is caused by one or more bacterium belonging to the species *A. baumannii,* and/or *S. aureus,* and/or a genus of non-tuberculous *Mycobacteria,* preferably *M. abscessus.* In preferred embodiments, the bacterial infection is caused by one or more bacterium belonging to a genus of non-tuberculous *Mycobacteria,* preferably *M. abscessus.* In some embodiments, the infection is caused by one or more bacterium belonging to the genus *Acinetobacter* and/or *Staphylococcus,* preferably *A. baumannii* and/or S. *aureus.*

The present disclosure provides rifabutin analogs that are modified at the C25 acetate and pharmaceutical compositions thereof. The inventive compounds exhibit broad antibacterial activity against a wide array of bacterial species, and thus maintain the broad antibacterial activity characteristic of the rifamycin class of antibiotics. In addition, the compounds showed enhanced and unexpected activity against various strains of *M. abscessus* and *A. baumannii* compared with currently available antibiotics, including rifamycins such as rifabutin and rifampicin. In preferred embodiments the inventive compounds are particularly active against strains that harbor resistance mutations making the strains resistant to current antibiotics. In preferred embodiments said resistance mechanisms are mutations in the RpoB gene and/or expression of the Arr enzyme. Additional features and advantages of the present technology will be apparent to one of skill in the art upon reading the Detailed Description, below.

### DETAILED DESCRIPTION

The present disclosure provides analogs of rifabutin that are effective in treating bacterial infections, preferably bacterial infections caused by one or more bacterium belonging to a genus of non-tuberculous *Mycobacteria,* preferably *M. abscessus.*

The details of the technology are set forth in the accompanying description below. Although methods and materials similar or equivalent to those described herein can be used in the practice or testing of the present technology, illustrative methods and materials are now described. Other features, objects, and advantages of the disclosure will be apparent from the description and from the claims. In the specification and the appended claims, the singular forms also include the plural unless the context clearly dictates otherwise. Unless defined otherwise, all technical and scientific terms used herein have the same meaning as commonly understood by one of ordinary skill in the art to which this technology belongs.

### Definitions

Unless defined otherwise, all technical and scientific terms used herein have the same meanings as commonly understood by one of ordinary skill in the art to which this disclosure belongs.

The articles "a" and "an" are used in this disclosure to refer to one or more than one (i.e., to at least one) of the grammatical object of the article. By way of example, "an element" means one element or more than one element.

The term "and/or" is used in this disclosure to mean either "and" or "or" unless indicated otherwise.

The term "optionally substituted" is understood to mean that a given chemical moiety (e.g. an alkyl group) can (but is not required to) be bonded other substituents (e.g. heteroatoms). For instance, an alkyl group that is optionally substituted can be a fully saturated alkyl chain (i.e. a pure hydrocarbon). Alternatively, the same optionally substituted alkyl group can have substituents different from hydrogen. For instance, it can, at any point along the chain be bounded to a halogen atom, a hydroxyl group, or any other substituent described herein. Thus, the term "optionally substituted" means that a given chemical moiety has the potential to contain other functional groups, but does not necessarily have any further functional groups.

The term "aryl" refers to cyclic, aromatic hydrocarbon groups that have 1 to 2 aromatic rings, including monocyclic or bicyclic groups such as phenyl, biphenyl or naphthyl. A -C₆-C₁₀ aryl group contains between 6 and 10 carbon atoms. When containing two aromatic rings (bicyclic, etc.), the aromatic rings of the aryl group may be joined at a single point (e.g., biphenyl), or fused (e.g., naphthyl). The aryl group may be optionally substituted by one or more substituents, e.g., 1 to 5 substituents, at any point of attachment. Exemplary substituents include, but are not limited to, -H, -halogen, -O-C₁-C₆ alkyl, -C₁-C₆ alkyl, -OC₂-C₆ alkenyl, - OC₂-C₆ alkynyl, -C₂-C₆ alkenyl, -C₂-C₆ alkynyl, -OH, -OP(O)(OH)₂, -OC(O)C₁-C₆ alkyl, - C(O)C₁-C₆ alkyl, -OC(O)OC₁-C₆ alkyl, -NH₂, -NH(C₁-C₆ alkyl), -N(C₁-C₆ alkyl)₂, -S(O)₂-C₁-C₆ alkyl, -S(O)NHC₁-C₆ alkyl, and -S(O)N(C₁-C₆ alkyl)₂. The substituents can themselves be optionally substituted. Furthermore, when containing two fused rings, the aryl groups herein defined may have an unsaturated or partially saturated ring fused with a fully saturated ring. Exemplary ring systems of these aryl groups include indanyl, indenyl, tetrahydronaphthalenyl, and tetrahydrobenzoannulenyl.

Unless otherwise specifically defined, "heteroaryl" means a monovalent monocyclic or polycyclic aromatic radical of 5 to 24 ring atoms or a polycyclic aromatic radical, containing one or more ring heteroatoms selected from N, S, P, and O, the remaining ring atoms being C. A 5-15 membered heteroaryl group contains between 5 and 15 atoms, whereas a 5-10 membered heteroaryl group contains between 5 and 10 atoms. Heteroaryl as herein defined also means a bicyclic heteroaromatic group wherein the heteroatom is selected from N, S, P, and O, preferably N, S, and O, more preferably N and O. The aromatic radical is optionally substituted independently with one or more substituents described herein. Examples include, but are not limited to, furyl, thienyl, pyrrolyl, pyridyl, pyrazolyl, pyrimidinyl, imidazolyl, isoxazolyl, oxazolyl, oxadiazolyl, pyrazinyl, indolyl, thiophen-2-yl, quinolyl, benzopyranyl, isothiazolyl, thiazolyl, thiadiazole, indazole, benzimidazolyl, thieno[3,2-b]thiophene, triazolyl, triazinyl, imidazo[1,2-b]pyrazolyl, furo[2,3-c]pyridinyl, imidazo[1,2-a]pyridinyl, indazolyl, pyrrolo[2,3-c]pyridinyl, pyrrolo[3,2-c]pyridinyl, pyrazolo[3,4-c]pyridinyl, thieno[3,2-c]pyridinyl, thieno[2,3-c]pyridinyl, thieno[2,3-b]pyridinyl, benzothiazolyl, indolyl, indolinyl, indolinonyl, dihydrobenzothiophenyl, dihydrobenzofuranyl, benzofuran, chromanyl, thiochromanyl, tetrahydroquinolinyl, dihydrobenzothiazine, dihydrobenzoxanyl, quinolinyl, isoquinolinyl, 1,6-naphthyridinyl, benzo[de]isoquinolinyl, pyrido[4,3-b][1,6]naphthyridinyl, thieno[2,3-b]pyrazinyl, quinazolinyl, tetrazolo[1,5-a]pyridinyl, [1,2,4]triazolo[4,3-a]pyridinyl, isoindolyl, pyrrolo[2,3-b]pyridinyl, pyrrolo[3,4-b]pyridinyl, pyrrolo[3,2-b]pyridinyl, imidazo[5,4-b]pyridinyl, pyrrolo[1,2-a]pyrimidinyl, tetrahydro pyrrolo[1,2-a]pyrimidinyl, 3,4-dihydro-2H-1λ2-pyrrolo[2,1-b]pyrimidine, dibenzo[b,d]thiophene, pyridin-2-one, furo[3,2-c]pyridinyl, furo[2,3-c]pyridinyl, 1H-pyrido[3,4-b][1,4]thiazinyl, benzooxazolyl, benzoisoxazolyl, furo[2,3-b]pyridinyl, benzothiophenyl, 1,5-naphthyridinyl, furo[3,2-b]pyridine, [1,2,4]triazolo[1,5-a]pyridinyl, benzo [1,2,3]triazolyl, imidazo[1,2-a]pyrimidinyl, [1,2,4]triazolo[4,3-b]pyridazinyl, benzo[c][1,2,5]thiadiazolyl, benzo[c][1,2,5]oxadiazole, 1,3-dihydro-2H-benzo[d]imidazol-2-one, 3,4-dihydro-2H-pyrazolo[1,5-b][1,2]oxazinyl, 4,5,6,7-tetrahydropyrazolo[1,5-a]pyridinyl, thiazolo[5,4-d]thiazolyl, imidazo[2,1-b][1,3,4]thiadiazolyl, thieno[2,3-b]pyrrolyl, 3H-indolyl, and derivatives thereof.

The term "alkyl" refers to a straight or branched chain saturated hydrocarbon. -C₁-C₆ alkyl groups contain 1 to 6 carbon atoms. Examples of a -C₁-C₆ alkyl group include, but are not limited to, methyl, ethyl, propyl, butyl, pentyl, isopropyl, isobutyl, sec-butyl and tert-butyl, isopentyl and neopentyl.

The term "alkenyl" means an aliphatic hydrocarbon group containing a carbon-carbon double bond and which may be straight or branched. A -C₂-C₆ alkenyl group is an alkenyl group containing between 2 and 6 carbon atoms. Some alkenyl groups have 2 to about 4 carbon atoms in the chain. Branched means that one or more lower alkyl groups such as methyl, ethyl, or propyl are attached to a linear alkenyl chain. Exemplary alkenyl groups include ethenyl, propenyl, n-butenyl, and i-butenyl. As used herein, the term "alkenyl" includes groups that contain a carbon-carbon double bond anywhere within the carbon chain.

The term "alkynyl" means an aliphatic hydrocarbon group containing a carbon-carbon triple bond and which may be straight or branched. A -C₂-C₆ alkynyl group is an alkynyl group containing between 2 and 6 carbon atoms. Some alkynyl groups have 2 to about 4 carbon atoms in the chain. Branched means that one or more lower alkyl groups such as methyl, ethyl, or propyl are attached to a linear alkynyl chain. Exemplary alkynyl groups include ethynyl, propynyl, n-butynyl, 2-butynyl, 3-methylbutynyl, and n-pentynyl. As used herein, the term "alkynyl" includes groups that contain a carbon-carbon triple bond anywhere within the carbon chain.

The terms "alkylene" or "alkylenyl," as used herein, refer to a straight or branched hydrocarbon chain bi-radical derived from alkyl, as defined herein, wherein one hydrogen of said alkyl is cleaved off generating the second radical of said alkylene. Examples of alkylene are, by way of illustration, -CH₂-, -CH₂-CH₂-, -CH(CH₃)-, -CH₂-CH₂-CH₂-, -CH(CH₃)-CH₂-, or -CH(CH₂CH₃)-.

The term "cycloalkyl" means monocyclic or polycyclic saturated carbon rings containing 3-18 carbon atoms. A -C₃-C₆ cycloalkyl contains between 3 and 6 carbon atoms. Examples of cycloalkyl groups include, without limitations, cyclopropyl, cyclobutyl, cyclopentyl, cyclohexyl, cycloheptanyl, cyclooctanyl, norboranyl, norborenyl, bicyclo[2.2.2]octanyl, or bicyclo[2.2.2]octenyl. A -C₃-C₈ cycloalkyl is a cycloalkyl group containing between 3 and 8 carbon atoms. A cycloalkyl group can be fused (e.g., decalin) or bridged (e.g., norbornane).

The term "cycloalkenyl" means monocyclic, non-aromatic unsaturated carbon rings containing 5-18 carbon atoms. Examples of cycloalkenyl groups include, without limitation, cyclopentenyl, cyclohexenyl, cycloheptenyl, cyclooctenyl, and norborenyl. A -C₅-C₈ cycloalkenyl is a cycloalkenyl group containing between 5 and 8 carbon atoms.

The terms "heterocyclyl" or "heterocycloalkyl" or "heterocycle" refer to monocyclic or polycyclic 3 to 24-membered ring system containing carbon and heteroatoms taken from oxygen, nitrogen, or sulfur (preferably oxygen and nitrogen) and wherein at least one ring does not comprise delocalized π electrons (aromaticity) shared among the ring carbon or heteroatoms. A 3-15 membered heterocycloalkyl group contains between 3 and 15 atoms, and a 3-10 membered heterocycloalkyl group contains between 3 and 10 atoms. Heterocyclyl rings include, but are not limited to, oxetanyl, azetadinyl, tetrahydrofuranyl, pyrrolidinyl, oxazolinyl, oxazolidinyl, thiazolinyl, thiazolidinyl, pyranyl, thiopyranyl, tetrahydropyranyl, dioxalinyl, piperidinyl, morpholinyl, thiomorpholinyl, thiomorpholinyl S-oxide, thiomorpholinyl S-dioxide, piperazinyl, azepinyl, oxepinyl, diazepinyl, tropanyl, and homotropanyl.

A heterocyclyl or heterocycloalkyl ring can also be fused or bridged, e.g., can be a bicyclic or tricyclic ring. Furthermore, when containing two or more fused rings, the heterocycloalkyl groups herein defined can have an unsaturated or partially saturated ring fused with an aromatic or heteroaromatic ring or fused aromatic or heteroaromatic ring system. Exemplary ring systems of such heterocyle-aryl or heterocyle-heteroaryl groups include indolinyl, indolinonyl, dihydrobenzothiophenyl, dihydrobenzofuran, chromanyl, thiochromanyl, tetrahydroquinolinyl, dihydrobenzothiazine, 3,4-dihydro-1H--isoquinolinyl, 2,3-dihydrobenzofuran, indolinyl, indolyl, and dihydrobenzoxanyl.

A heterocyclyl or heterocycloalkyl ring can also be a spirocyclic heterocycle or spiroheterocycle. As used herein a spirocyclic heterocycle or spiroheterocycle is understood to mean a bicyclic or multicyclic ring system in which at least two rings are connected through a single atom, and wherein at least one of the rings is a heterocycle (e.g., at least one of the rings is furanyl, morpholinyl, or piperadinyl). One or both of the rings in a spiroheterocycle can be can be fused to one or more additional carbocyclic, heterocyclic, aromatic, or heteroaromatic ring to form, e.g., a tricyclic ring system in which two of the rings are connected through a single atom.

As used herein, the term "halo" or "halogen" means fluoro (F), chloro (Cl), bromo (Br), or iodo (I).

As used herein, the term "haloalkyl" refers to an alkyl group substituted with one or more (preferably 1 to 6, more preferably 1 to 3) halogen atoms which are selected independently from fluoro, chloro, bromo and iodo, and are preferably all fluoro atoms. It will be understood that the maximum number of halogen atoms is limited by the number of available attachment sites and, thus, depends on the number of carbon atoms comprising the alkyl moiety of the haloalkyl group. "Haloalkyl" may, e.g., refer to CF₃, CHF₂, CH₂F, CF₂CH₃, CH₂CF₃, CH₂CHF₂, CH₂CF₂CH₃, CH₂CF₂CF₃, or CH(CF₃)₂. Very preferred "haloalkyl" as substituents for the inventive compounds are CF₃, CHF₂, and CH₂CF₃, and again further preferred are CF₃ and CHF₂.

The term "carbonyl" refers to a functional group composing a carbon atom double-bonded to an oxygen atom. It can be abbreviated herein as "oxo", as C(O), or as C=O.

"C₁-C₆-alkoxy", as used herein, refers to a "substituted hydroxyl" of the formula (-OR'), wherein R' is an optionally substituted -C₁-C₆ alkyl, as defined herein, and the oxygen moiety is directly attached to the parent molecule, and thus the term "C₁-C₆ alkoxy", as used herein, refers to straight chain or branched -C₁-C₆ alkoxy which may be, for example, methoxy, ethoxy, n-propoxy, isopropoxy, n-butoxy, isobutoxy, sec-butoxy, tert-butoxy, straight or branched pentoxy, straight or branched hexyloxy. Preferred are -C₁-C₄ alkoxy and -C₁-C3 alkoxy.

The disclosure also includes pharmaceutical compositions comprising an effective amount of a disclosed compound and a pharmaceutically acceptable carrier. Representative "pharmaceutically acceptable salts" include, e.g., water-soluble and water-insoluble salts, such as the acetate, amsonate (4,4-diaminostilbene-2,2-disulfonate), benzenesulfonate, benzonate, bicarbonate, bisulfate, bitartrate, borate, bromide, butyrate, calcium, calcium edetate, camsylate, carbonate, chloride, citrate, clavulariate, dihydrochloride, edetate, edisylate, estolate, esylate, fumarate, gluceptate, gluconate, glutamate, glycollylarsanilate, hexafluorophosphate, hexylresorcinate, hydrabamine, hydrobromide, hydrochloride, hydroxynaphthoate, hydroiodide, sethionate, lactate, lactobionate, laurate, magnesium, malate, maleate, mandelate, mesylate, methylbromide, methylnitrate, methylsulfate, mucate, napsylate, nitrate, N-methylglucamine ammonium salt, 3-hydroxy-2-naphthoate, oleate, oxalate, palmitate, pamoate (1,1-methene-bis-2-hydroxy-3-naphthoate, einbonate), pantothenate, phosphate/diphosphate, picrate, polygalacturonate, propionate, p-toluenesulfonate, salicylate, stearate, subacetate, succinate, sulfate, sulfosalicylate, suramate, tannate, tartrate, teoclate, tosylate, triethiodide, and valerate salts.

The term "stereoisomers" refers to the set of compounds which have the same number and type of atoms and share the same bond connectivity between those atoms, but differ in three-dimensional structure. The term "stereoisomer" refers to any member of this set of compounds.

The term "diastereomers" refers to the set of stereoisomers which cannot be made superimposable by rotation around single bonds. For example, cis- and trans-double bonds, *endo-* and exo-substitution on bicyclic ring systems, and compounds containing multiple stereogenic centers with different relative configurations are considered to be diastereomers. The term "diastereomer" refers to any member of this set of compounds. In some examples presented, the synthetic route may produce a single diastereomer or a mixture of diastereomers. In some cases these diastereomers were separated and in other cases a wavy bond is used to indicate the structural element where configuration is variable.

The term "enantiomers" refers to a pair of stereoisomers which are non-superimposable mirror images of one another. The term "enantiomer" refers to a single member of this pair of stereoisomers. The term "racemic" refers to a 1:1 mixture of a pair of enantiomers.

The term "tautomers" refers to a set of compounds that have the same number and type of atoms, but differ in bond connectivity and are in equilibrium with one another. A "tautomer" is a single member of this set of compounds. Typically a single tautomer is drawn but it is understood that this single structure is meant to represent all possible tautomers that might exist. Examples include enol-ketone tautomerism. When a ketone is drawn it is understood that both the enol and ketone forms are part of the present disclosure.

An "effective amount" when used in connection with a compound is an amount effective for treating or preventing a disease in a subject as described herein.

The term "carrier", as used in this disclosure, encompasses carriers, excipients, and diluents and means a material, composition or vehicle, such as a liquid or solid filler, diluent, excipient, solvent or encapsulating material, involved in carrying or transporting a pharmaceutical agent from one organ, or portion of the body, to another organ, or portion of the body of a subject.

The term "treating" with regard to a subject, refers to improving at least one symptom of the subject's disorder. Treating includes curing, improving, or at least partially ameliorating the disorder.

The term "disorder" is used in this disclosure to mean, and is used interchangeably with, the terms disease, condition, or illness, unless otherwise indicated.

The term "administer", "administering", or "administration" as used in this disclosure refers to either directly administering a disclosed compound or pharmaceutically acceptable salt of the disclosed compound or a composition to a subject, or administering a prodrug derivative or analog of the compound or pharmaceutically acceptable salt of the compound or composition to the subject, which can form an equivalent amount of active compound within the subject's body.

The term "solvate" refers to a complex of variable stoichiometry formed by a solute and solvent. Such solvents for the purpose of the disclosure may not interfere with the biological activity of the solute. Examples of suitable solvents include, but are not limited to, water, MeOH, EtOH, and AcOH. Solvates wherein water is the solvent molecule are typically referred to as "hydrates." Hydrates include compositions containing stoichiometric amounts of water, as well as compositions containing variable amounts of water.

A "patient" or "subject" is a mammal, e.g., a human, mouse, rat, guinea pig, dog, cat, horse, cow, pig, or non-human primate, such as a monkey, chimpanzee, baboon or rhesus.

As used herein, the "Arr" enzyme refers to the ADP ribosyl transferase enzyme. In preferred embodiments the Arr enzyme ribosylates rifamycins and rifamycin derivatives at the C23 carbon. In some embodiments, compounds of Formula I can be used to treat bacterial infections caused by bacteria expressing or overexpressing Arr.

As used herein, "RpoB" refers to the RNA polymerase β-subunit, preferably a bacterial RNA polymerase β-subunit. In some embodiments, compounds of Formula I can be used to treat bacterial infections caused by bacteria having a resistance mutation in the RpoB gene.

### Compounds of the Invention

In one aspect, the present invention provides a compound of the Formula I or a pharmaceutically acceptable salt, tautomer, solvate, hydrate or enantiomer thereof: 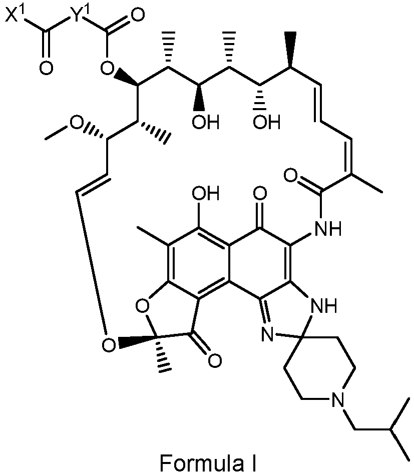 wherein:
Y¹ is (CR¹R²)ₙ, wherein n is an integer from 1 to 6 and R¹ and R² are independently, at each occurrence, selected from the group consisting of hydrogen, -C₁-C₆ alkyl, and -C₁-C₆ alkoxy, wherein each alkyl is optionally substituted with one or more halogen or -C₃-C₆ cycloalkyl, and wherein each cycloalkyl is further optionally substituted with one or more halogen;
X¹ is -OR¹¹ or -NR⁵¹R⁵²;
R¹¹ is independently -C₁-C₆ alkyl, -C₁-C₆ alkylene-R^{12A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, or 5-10 membered heteroaryl, wherein each alkyl, alkylene, alkenyl, alkynyl, cycloalkyl, cycloalkenyl, heterocycloalkyl, aryl or heteroaryl is optionally substituted with one or more R¹²;
R¹² and R^{12A} are each independently -C₁-C₆ alkyl, -C₁-C₆ alkylene-R^{16A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, halogen, cyano, oxo, - OR¹³, -CO₂R¹³, or -NR¹⁴R¹⁵, wherein each alkyl, alkylene, alkenyl, alkynyl, cycloalkyl, cycloalkenyl, heterocycloalkyl, aryl, heteroaryl and alkoxy is optionally substituted with one or more R¹⁶;
R¹³, R¹⁴ and R¹⁵ are each independently hydrogen, -C₁-C₆ alkyl, -C₁-C₆ alkylene-R^{20A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, - S(O)₂(C₁-C₆ alkyl), or -S(O)₂NR¹⁸R¹⁹, wherein each alkyl, alkylene, alkenyl, alkynyl, cycloalkyl, cycloalkenyl, heterocycloalkyl, aryl, heteroaryl and alkoxy is optionally substituted with one or more R²⁰;
R¹⁶ and R^{16A} are each independently -C₁-C₆ alkyl, -C₁-C₆ alkylene-R^{20A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, halogen, cyano, oxo, - OR¹⁷, -S(O)₂(C₁-C₆ alkyl), or -S(O)₂NR¹⁸R¹⁹, wherein each alkyl, alkylene, alkenyl, alkynyl, cycloalkyl, cycloalkenyl, heterocycloalkyl, aryl, heteroaryl and alkoxy is optionally substituted with one or more R²⁰;
R¹⁷, R¹⁸ and R¹⁹ are each independently hydrogen, halogen, -OH, -C₁-C₆ alkyl, -C₁-C₆ alkylene-R^{23A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, -S(O)₂(C₁-C₆ alkyl), or S(O)₂NR²¹R²², wherein each alkyl, alkylene, alkenyl, alkynyl, cycloalkyl, cycloalkenyl, heterocycloalkyl, aryl, heteroaryl and alkoxy is optionally substituted with one or more R²³;
R²⁰ and R^{20A} are each independently halogen, -OH, -C₁-C₆ alkyl, -C₁-C₆ alkylene-R^{23A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, halogen, cyano, oxo, -S(O)₂(C₁-C₆ alkyl), or S(O)₂NR²¹R²², wherein each alkyl, alkylene, alkenyl, alkynyl, cycloalkyl, cycloalkenyl, heterocycloalkyl, aryl, heteroaryl and alkoxy is optionally substituted with one or more R²³;
R²¹ and R²² are each independently hydrogen or -C₁-C₆ alkyl; and
R²³ and R^{23A} are each independently -C₁-C₆ alkyl, -C₁-C₆ alkoxy, -C₁-C₆ haloalkyl, halogen, or cyano;
R⁵¹ and R⁵² are each independently hydrogen, -C₁-C₆ alkyl, -C₁-C₆ alkylene-R^{53A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, or 5-10 membered heteroaryl, wherein each alkyl, alkylene, alkenyl, alkynyl, cycloalkyl, cycloalkenyl, heterocycloalkyl, aryl or heteroaryl is optionally substituted with one or more R⁵³; or R⁵¹ and R⁵², together with the nitrogen atom to which they are attached, combine to form a 3-15 membered heterocycloalkyl or 5-15 membered heteroaryl, wherein the heterocycloalkyl or heteroaryl are optionally substituted with one or more R⁵³;
R⁵³ and R^{53A} are each independently -C₁-C₆ alkyl, -C₁-C₆ alkylene-R^{57A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, halogen, cyano, oxo, - OR⁵⁴, -C(O)R⁵⁴, -CO₂R⁵⁴, or -NR⁵⁵R⁵⁶, wherein each alkyl, alkylene, alkenyl, alkynyl, cycloalkyl, cycloalkenyl, heterocycloalkyl, aryl, heteroaryl and alkoxy is optionally substituted with one or more R⁵⁷;
R⁵⁴, R⁵⁵ and R⁵⁶ are each independently hydrogen, -C₁-C₆ alkyl, -C₁-C₆ alkylene-R^{61A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, - CO₂R⁵⁸, -S(O)₂(C₁-C₆ alkyl), -S(O)₂(C₆-C₁₀ aryl), or -S(O)₂NR⁵⁹R⁶⁰, wherein each alkyl, alkylene, alkenyl, alkynyl, cycloalkyl, cycloalkenyl, heterocycloalkyl, aryl, heteroaryl and alkoxy is optionally substituted with one or more R⁶¹;
R⁵⁷ and R^{57A} are each independently -C₁-C₆ alkyl, -C₁-C₆ alkylene-R^{61A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, halogen, cyano, oxo, - OR⁵⁸, NR⁵⁹R⁶⁰, -S(O)₂(C₁-C₆ alkyl), or -S(O)₂NR⁵⁹R⁶⁰, wherein each alkyl, alkylene, alkenyl, alkynyl, cycloalkyl, cycloalkenyl, heterocycloalkyl, aryl, heteroaryl and alkoxy is optionally substituted with one or more R⁶¹;
R⁵⁸, R⁵⁹ and R⁶⁰ are each independently hydrogen, -C₁-C₆ alkyl, -C₁-C₆ alkylene-R⁶⁴, - C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, - S(O)₂(C₁-C₆ alkyl), or S(O)₂NR⁶²R⁶³, wherein each alkyl, alkylene, alkenyl, alkynyl, cycloalkyl, cycloalkenyl, heterocycloalkyl, aryl, heteroaryl and alkoxy is optionally substituted with one or more R⁶⁴;
R⁶¹ and R^{61A} are each independently halogen, -OH, -C₁-C₆ alkyl, -C₁-C₆ alkylene-R^{64A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, halogen, cyano, oxo, -S(O)₂(C₁-C₆ alkyl), or S(O)₂NR⁶²R⁶³, wherein each alkyl, alkylene, alkenyl, alkynyl, cycloalkyl, cycloalkenyl, heterocycloalkyl, aryl, heteroaryl and alkoxy is optionally substituted with one or more R⁶⁴; and
R⁶² and R⁶³ are each independently hydrogen and -C₁-C₆ alkyl; and
R⁶⁴ and R^{64A} are each independently -C₁-C₆ alkyl, -C₁-C₆ alkoxy, -C₁-C₆ haloalkyl, phenyl, halogen, or cyano.

In some embodiments, Y¹ is (CR¹R²)ₙ, wherein n is an integer from 1 to 5 and R¹ and R² are independently, at each occurrence, selected from hydrogen, -C₁-C₄ alkyl, and -C₁-C₄ alkoxy, wherein each alkyl is optionally substituted with one or more halogen or -C₃-C₆ cycloalkyl, and wherein each cycloalkyl is further optionally substituted with one or more halogens.

In some embodiments, Y¹ is (CR¹R²)ₙ, wherein n is an integer from 1 to 4 and R¹ and R² are independently, at each occurrence, selected from hydrogen, -C₁-C₄ alkyl, and -C₁-C₄ alkoxy, wherein each alkyl is optionally substituted with one or more halogen or -C₃-C₆ cycloalkyl, and wherein each cycloalkyl is further optionally substituted with one or more halogens.

In some embodiments, Y¹ is (CR¹R²)ₙ, wherein n is an integer from 1 to 3 and R¹ and R² are independently, at each occurrence, selected from hydrogen, -C₁-C₄ alkyl, and -C₁-C₄ alkoxy, wherein each alkyl is optionally substituted with one or more halogen or -C₃-C₆ cycloalkyl, and wherein each cycloalkyl is further optionally substituted with one or more halogens.

In some embodiments, Y¹ is (CR¹R²)ₙ, wherein n is an integer from 1 to 3 and R¹ and R² are independently, at each occurrence, selected from hydrogen, -C₁-C₂ alkyl, and -C₁-C₂ alkoxy, wherein each alkyl is optionally substituted with one or more fluorine, chlorine, or -C₃-C₆ cycloalkyl, and wherein each cycloalkyl is further optionally substituted with one or more fluorine or chlorine.

In some embodiments, Y¹ is (CR¹R²)ₙ, wherein n is an integer from 1 to 3 and R¹ and R² are independently, at each occurrence, selected from hydrogen, and -C₁-C₂ alkyl, wherein each alkyl is optionally substituted with one or more fluorine, chlorine, or -C₃-C₆ cycloalkyl, and wherein each cycloalkyl is further optionally substituted with one or more fluorine or chlorine.

In some embodiments, Y¹ is (CR¹R²)ₙ, wherein n is an integer from 1 to 3 and R¹ and R² are independently, at each occurrence, selected from hydrogen and -C₁-C₂ alkyl, wherein each alkyl is optionally substituted with one -C₃-C₆ cycloalkyl, wherein said cycloalkyl is optionally substituted with one to three, preferably one or two halogens selected from fluorine and chlorine; or wherein each alkyl is optionally substituted with one to three, preferably one or two halogens selected from fluorine and chlorine.

In some embodiments, Y¹ is (CR¹R²)ₙ, wherein n is an integer from 1 to 3 and R¹ and R² are independently, at each occurrence, selected from -H, methyl, and ethyl; wherein each methyl and ethyl is optionally substituted with one or more fluorine or chlorine.

In some embodiments, Y¹ is (CR¹R²)ₙ, wherein n is an integer from 1 to 3 and R¹ and R² are independently, at each occurrence, selected from -H, methyl, and ethyl.

In some embodiments, the compound is of the Formula I-A: 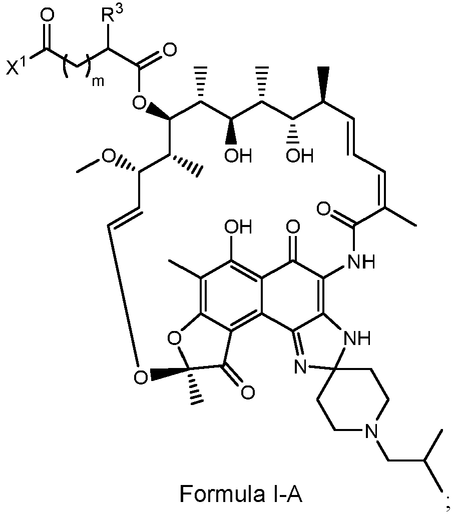 wherein
X¹ is as defined in Formula I above or in subsequent embodiments herein;
m is an integer between 0 and 4, preferably between 0 and 3, yet more preferably between 0 and 2; and
R³ is selected from -H and -C₁-C₆ alkyl, preferably -H and -C₁-C₃ alkyl, more preferably -H, methyl and ethyl.

In one embodiment, m is an integer from 0 to 3; preferably wherein m is an integer from 0 to 2; and wherein R³ is selected from -C₁-C₆ alkyl, preferably -C₁-C₄ alkyl, more preferably - C₁-C₃ alkyl, and yet more preferably methyl. In one embodiment, m is an integer from 0 to 3; preferably wherein m is an integer from 0 to 2; and wherein R³ is hydrogen.

In some embodiments, the compound is of the Formula I-B, Formula I-C, Formula I-D, Formula I-E, Formula I-F, or Formula I-G: 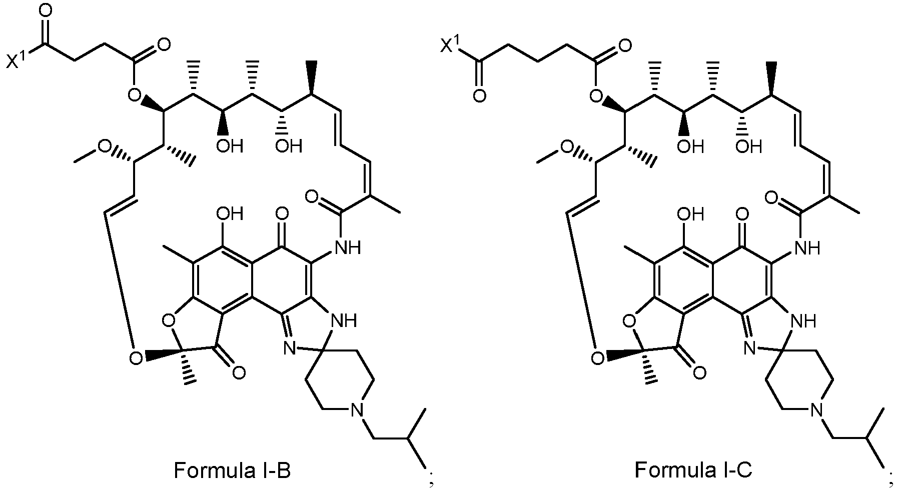 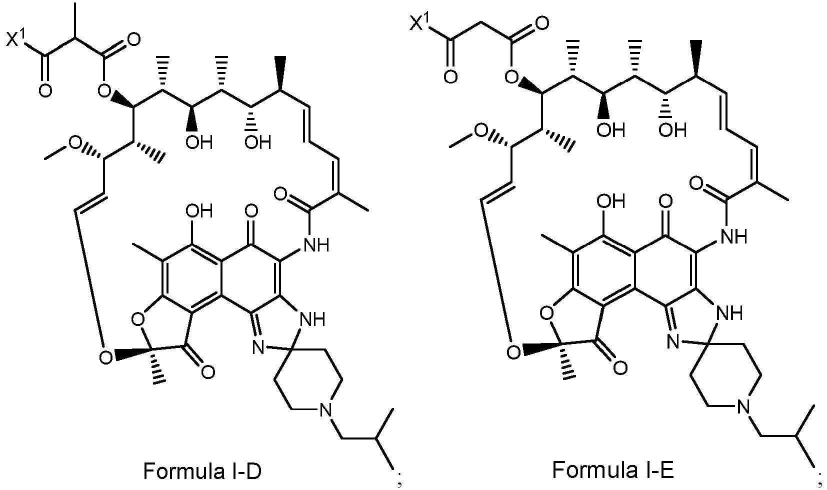 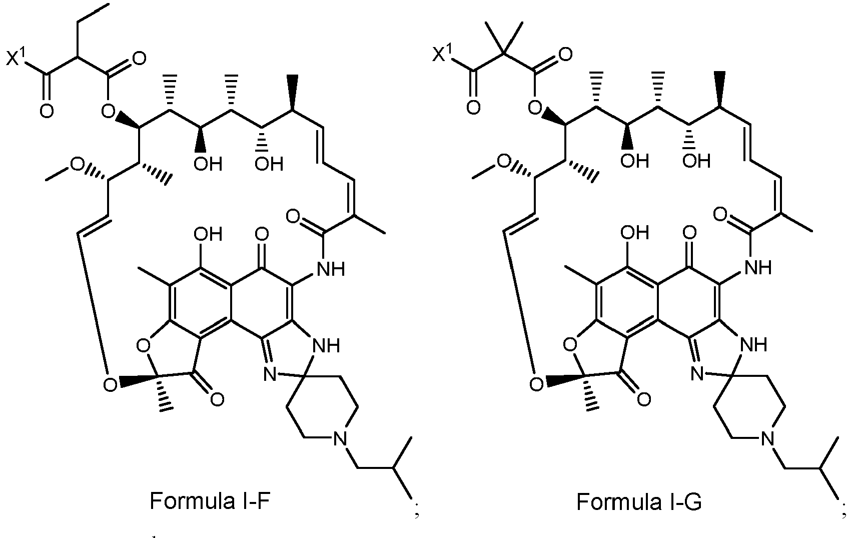 wherein X¹ is as defined for Formula I.
In some preferred embodiments of any of Formula I, Formula I-A, Formula I-B, Formula I-C, Formula I-D, Formula I-E, Formula I-F, or Formula I-G,
R¹¹ is independently -C₁-C₆ alkyl, -C₁-C₆ alkylene-R^{12A}, -C₃-C₆ cycloalkyl, -C₅-C₈ cycloalkenyl, 3-10 membered heterocycloalkyl, -C₆-C₁₀ aryl, or 5-10 membered heteroaryl, wherein each alkylene, cycloalkyl, cycloalkenyl, heterocycloalkyl, aryl or heteroaryl is optionally substituted with one or more R¹²;
R¹² and R^{12A} are each independently -C₁-C₆ alkyl, -C₁-C₆ alkylene-R^{16A}, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, halogen, cyano, oxo, -OR¹³, -CO₂R¹³, or -NR¹⁴R¹⁵, wherein each alkyl, alkylene, cycloalkyl, cycloalkenyl, heterocycloalkyl, aryl, heteroaryl and alkoxy is optionally substituted with one or more R¹⁶;
R¹³, R¹⁴ and R¹⁵ are each independently hydrogen or -C₁-C₆ alkyl; and
R¹⁶ and R^{16A} are each independently -C₁-C₆ alkyl;
R⁵¹ and R⁵² are each independently hydrogen, -C₁-C₆ alkyl, -C₁-C₆ alkylene-R^{53A}, -C₃-C₆ cycloalkyl, -C₅-C₈ cycloalkenyl, 3-10 membered heterocycloalkyl, -C₆-C₁₀ aryl, or 5-10 membered heteroaryl, wherein each alkyl, alkylene, alkenyl, alkynyl, cycloalkyl, cycloalkenyl, heterocycloalkyl, aryl or heteroaryl is optionally substituted with one or more R⁵³; or R⁵¹ and R⁵², together with the nitrogen atom to which they are attached, combine to form a 3-15 membered heterocycloalkyl or 5-15 membered heteroaryl, wherein the heterocycloalkyl or heteroaryl are optionally substituted with one or more R⁵³;
R⁵³ and R^{53A} are each independently -C₁-C₆ alkyl, -C₁-C₆ alkylene-R^{57A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, halogen, cyano, oxo, - OR⁵⁴, -C(O)R⁵⁴, -CO₂R⁵⁴, or -NR⁵⁵R⁵⁶, wherein each alkyl, alkylene, alkenyl, alkynyl, cycloalkyl, cycloalkenyl, heterocycloalkyl, aryl, heteroaryl and alkoxy is optionally substituted with one or more R⁵⁷;
R⁵⁴, R⁵⁵ and R⁵⁶ are each independently hydrogen, -C₁-C₆ alkyl, -C₁-C₆ alkylene-R^{61A}, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, -CO₂R⁵⁸, -S(O)₂(C₆-C₁₀ aryl), - S(O)₂(C₁-C₆ alkyl), or -S(O)₂NR⁵⁹R⁶⁰, wherein each alkyl, alkylene, cycloalkyl, cycloalkenyl, heterocycloalkyl, aryl, heteroaryl and alkoxy is optionally substituted with one or more R⁶¹;
R⁵⁷ and R^{57A} are each independently -C₁-C₆ alkyl, -C₁-C₆ alkylene-R^{61A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, halogen, cyano, oxo, - OR⁵⁸, NR⁵⁹R⁶⁰, -S(O)₂(C₁-C₆ alkyl), or -S(O)₂NR⁵⁹R⁶⁰, wherein each alkyl, alkylene, alkenyl, alkynyl, cycloalkyl, cycloalkenyl, heterocycloalkyl, aryl, heteroaryl and alkoxy is optionally substituted with one or more R⁶¹;
R⁵⁸, R⁵⁹ and R⁶⁰ are each independently hydrogen, -C₁-C₆ alkyl, -C₁-C₆ alkylene-R^{64A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, or -C₁-C₆ haloalkyl, wherein each alkyl, alkylene, alkenyl, alkynyl, cycloalkyl, cycloalkenyl, heterocycloalkyl, aryl, heteroaryl and alkoxy is optionally substituted with one or more R⁶⁴; and
R⁶¹, R^{61A}, R⁶⁴ and R^{64A} are each independently -OH, halogen, -CN, -C₁-C₆ alkyl, -C₁-C₆ alkoxy, -C₁-C₆ haloalkyl, or phenyl.

In some embodiments, R¹¹ is independently -C₁-C₆ alkylene-R^{12A}, -C₃-C₆ cycloalkyl, - C₅-C₈ cycloalkenyl, 3-10 membered heterocycloalkyl, -C₆-C₁₀ aryl, or 5-10 membered heteroaryl, wherein each alkylene, cycloalkyl, cycloalkenyl, heterocycloalkyl, aryl or heteroaryl is optionally substituted with one or more R¹²;
R¹² and R^{12A} are each independently -C₁-C₆ alkyl, -C₁-C₆ alkylene-R^{16A}, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, halogen, cyano, oxo, -OR¹³, -CO₂R¹³, or -NR¹⁴R¹⁵, wherein each alkyl, alkylene, cycloalkyl, cycloalkenyl, heterocycloalkyl, aryl, heteroaryl and alkoxy is optionally substituted with one or more R¹⁶;
R¹³, R¹⁴ and R¹⁵ are each independently hydrogen or -C₁-C₆ alkyl; and
R¹⁶ and R^{16A} are each independently -C₁-C₆ alkyl;
R⁵¹ and R⁵² are each independently hydrogen, -C₁-C₆ alkyl, -C₁-C₆ alkylene-R^{53A}, -C₃-C₆ cycloalkyl, -C₅-C₈ cycloalkenyl, 3-10 membered heterocycloalkyl, -C₆-C₁₀ aryl, or 5-10 membered heteroaryl, wherein each alkyl, alkylene, cycloalkyl, cycloalkenyl, heterocycloalkyl, aryl or heteroaryl is optionally substituted with one or more R⁵³; or R⁵¹ and R⁵², together with the nitrogen atom to which they are attached, combine to form a 3-15 membered heterocycloalkyl or 5-15 membered heteroaryl, wherein the heterocycloalkyl or heteroaryl are optionally substituted with one or more R⁵³;
R⁵³ and R^{53A} are each independently -C₁-C₆ alkyl, -C₁-C₆ alkylene-R^{57A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, halogen, cyano, oxo, - OR⁵⁴, -C(O)R⁵⁴, -CO₂R⁵⁴, or -NR⁵⁵R⁵⁶, wherein each alkyl, alkylene, alkenyl, alkynyl, cycloalkyl, cycloalkenyl, heterocycloalkyl, aryl, heteroaryl and alkoxy is optionally substituted with one or more R⁵⁷;
R⁵⁴, R⁵⁵ and R⁵⁶ are each independently hydrogen, -C₁-C₆ alkyl, -C₁-C₆ alkylene-R^{61A}, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, -CO₂R⁵⁸, -S(O)₂(C₆-C₁₀ aryl), - S(O)₂(C₁-C₆ alkyl), or -S(O)₂NR⁵⁹R⁶⁰, wherein each alkyl, alkylene, cycloalkyl, cycloalkenyl, heterocycloalkyl, aryl, heteroaryl and alkoxy is optionally substituted with one or more R⁶¹;
R⁵⁷ and R^{57A} are each independently -C₁-C₆ alkyl, -C₁-C₆ alkylene-R^{61A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, halogen, cyano, oxo, - OR⁵⁸, NR⁵⁹R⁶⁰, -S(O)₂(C₁-C₆ alkyl), or -S(O)₂NR⁵⁹R⁶⁰, wherein each alkyl, alkylene, alkenyl, alkynyl, cycloalkyl, cycloalkenyl, heterocycloalkyl, aryl, heteroaryl and alkoxy is optionally substituted with one or more R⁶¹;
R⁵⁸, R⁵⁹ and R⁶⁰ are each independently hydrogen, -C₁-C₆ alkyl, -C₁-C₆ alkylene-R^{64A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, or -C₁-C₆ haloalkyl, wherein each alkyl, alkylene, alkenyl, alkynyl, cycloalkyl, cycloalkenyl, heterocycloalkyl, aryl, heteroaryl and alkoxy is optionally substituted with one or more R⁶⁴; and
R⁶¹, R^{61A}, R⁶⁴ and R^{64A} are each independently -C₁-C₆ alkyl or phenyl.

In one embodiment, R¹¹ is independently -C₁-C₆ alkylene-R^{12A}, -C₅-C₆ cycloalkyl, -C₅-C₆ cycloalkenyl, 3-10 membered heterocycloalkyl, -C₆-C₁₀ aryl, or 5-10 membered heteroaryl, wherein each alkylene, cycloalkyl, cycloalkenyl, heterocycloalkyl, aryl or heteroaryl is optionally substituted with one or more R¹²;
R¹² is independently -C₁-C₆ alkyl, -C₁-C₆ alkylene-R^{16A}, -C₅-C₆ cycloalkyl, -C₅-C₆ cycloalkenyl, 3-10 membered heterocycloalkyl, -C₆-C₁₀ aryl, 5-10 membered heteroaryl, -C₁-C₂ alkoxy, -C₁-C₂ haloalkyl, halogen, cyano, oxo, -OR¹³, -CO₂R¹³, or -NR¹⁴R¹⁵, wherein each alkyl, alkylene, cycloalkyl, cycloalkenyl, heterocycloalkyl, aryl, heteroaryl and alkoxy is optionally substituted with one or more R¹⁶;
R^{12A} is independently -C₁-C₆ alkylene-R^{16A}, -C₅-C₆ cycloalkyl, -C₅-C₆ cycloalkenyl, 3-10 membered heterocycloalkyl, -C₆-C₁₀ aryl, 5-10 membered heteroaryl, -C₁-C₂ haloalkyl, halogen, cyano, oxo, -OR¹³, -CO₂R¹³, or -NR¹⁴R¹⁵, wherein each alkyl, alkylene, cycloalkyl, cycloalkenyl, heterocycloalkyl, aryl and heteroaryl is optionally substituted with one or more R¹⁶;
R¹³, R¹⁴ and R¹⁵ are each independently hydrogen or -C₁-C₄ alkyl; and
R¹⁶ and R^{16A} are each independently -C₁-C₄ alkyl;
R⁵¹ and R⁵² are each independently hydrogen, -C₁-C₆ alkyl, -C₁-C₆ alkylene-R^{53A}, -C₃-C₆ cycloalkyl, -C₅-C₈ cycloalkenyl, 3-10 membered heterocycloalkyl, -C₆-C₁₀ aryl, or 5-10 membered heteroaryl, wherein each alkyl, alkylene, cycloalkyl, cycloalkenyl, heterocycloalkyl, aryl or heteroaryl is optionally substituted with one or more R⁵³; or R⁵¹ and R⁵², together with the nitrogen atom to which they are attached, combine to form a 3-15 membered heterocycloalkyl or 5-15 membered heteroaryl, wherein the heterocycloalkyl or heteroaryl are optionally substituted with one or more R⁵³;
R⁵³ is independently -C₁-C₆ alkyl, -C₁-C₆ alkylene-R^{57A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, halogen, cyano, oxo, -OR⁵⁴, -C(O)R⁵⁴, -CO₂R⁵⁴, or -NR⁵⁵R⁵⁶, wherein each alkyl, alkylene, alkenyl, alkynyl, cycloalkyl, cycloalkenyl, heterocycloalkyl, aryl, heteroaryl and alkoxy is optionally substituted with one or more R⁵⁷;
R^{53A} is independently -C₁-C₆ alkylene-R^{57A}, -C₅-C₆ cycloalkyl, -C₅-C₆ cycloalkenyl, 3-10 membered heterocycloalkyl, -C₆-C₁₀ aryl, 5-10 membered heteroaryl, -C₁-C₆ alkoxy, -C₁-C₂ haloalkyl, halogen, cyano, oxo, -OR⁵⁴, -CO₂R⁵⁴, or -NR⁵⁵R⁵⁶, wherein eachalkylene, cycloalkyl, cycloalkenyl, heterocycloalkyl, aryl, heteroaryl and alkoxy is optionally substituted with one or more R⁵⁷;
R⁵⁴, R⁵⁵ and R⁵⁶ are each independently hydrogen, -C₁-C₆ alkyl, -C₁-C₆ alkylene-R^{61A}, -C₅-C₆ cycloalkyl, 3-8 membered heterocycloalkyl, -C₆-C₁₀ aryl, 5-6 membered heteroaryl, -C₁ haloalkyl, -CO₂R⁵⁸, -S(O)₂(C₆-C₁₀ aryl),, wherein each alkyl, alkylene, cycloalkyl, heterocycloalkyl, aryl or heteroaryl is optionally substituted with one or more R⁶¹;
R⁵⁷ is independently -C₁-C₆ alkyl, -C₁-C₆ alkylene-R^{61A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, halogen, cyano, oxo, -OR⁵⁸, NR⁵⁹R⁶⁰, - S(O)₂(C₁-C₆ alkyl), or -S(O)₂NR⁵⁹R⁶⁰, wherein each alkyl, alkylene, alkenyl, alkynyl, cycloalkyl, cycloalkenyl, heterocycloalkyl, aryl, heteroaryl and alkoxy is optionally substituted with one or more R⁶¹;
R^{57A} is independently -C₁-C₆ alkyl, -C₁-C₆ alkylene-R^{61A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, halogen, cyano, oxo, -OR⁵⁸, NR⁵⁹R⁶⁰, -S(O)₂(C₁-C₆ alkyl), or -S(O)₂NR⁵⁹R⁶⁰, wherein each alkyl, alkylene, alkenyl, alkynyl, cycloalkyl, cycloalkenyl, heterocycloalkyl, aryl, heteroaryl and alkoxy is optionally substituted with one or more R⁶¹;
R⁵⁸, R⁵⁹ and R⁶⁰ are each independently hydrogen, -C₁-C₆ alkyl, -C₁-C₆ alkylene-R^{64A}, -C₃-C₆ cycloalkyl, 3-10 membered heterocycloalkyl, -C₆-C₁₀ aryl, 5-10 membered heteroaryl, -C₁-C₆ alkoxy, or -C₁-C₆ haloalkyl, wherein each alkyl, alkylene, cycloalkyl, cycloalkenyl, heterocycloalkyl, aryl, heteroaryl and alkoxy is optionally substituted with one or more R⁶⁴; and
R⁶¹, R^{61A}, R⁶⁴ and R^{64A} are each independently -C₁-C₆ alkyl or phenyl.

In some embodiments, R¹¹ is independently -C₁-C₆ alkylene-R^{12A}, -C₅-C₆ cycloalkyl, - C₅-C₆ cycloalkenyl, 3-8 membered heterocycloalkyl, phenyl, or 5-6 membered heteroaryl, wherein each alkylene, cycloalkyl, cycloalkenyl, heterocycloalkyl, aryl or heteroaryl is optionally substituted with one or more R¹²;
R¹² is independently -C₁-C₆ alkyl, -C₁-C₆ alkylene-R^{16A}, -C₅-C₆ cycloalkyl, -C₅-C₆ cycloalkenyl, 3-8 membered heterocycloalkyl, phenyl, 5-6 membered heteroaryl, -C₁ haloalkyl, halogen, cyano, oxo, -OR¹³, -CO₂R¹³, or -NR¹⁴R¹⁵, wherein each alkyl, alkylene, cycloalkyl, cycloalkenyl, heterocycloalkyl, phenyl and heteroaryl is optionally substituted with one or more R¹⁶;
R^{12A} is independently -C₅-C₆ cycloalkyl, -C₅-C₆ cycloalkenyl, 3-8 membered heterocycloalkyl, phenyl, 5-6 membered heteroaryl, -C₁ haloalkyl, halogen, cyano, oxo, -OR¹³, -CO₂R¹³, or -NR¹⁴R¹⁵, wherein each cycloalkyl, cycloalkenyl, heterocycloalkyl, phenyl and heteroaryl is optionally substituted with one or more R¹⁶;
R¹³, R¹⁴ and R¹⁵ are each independently hydrogen or -C₁-C₄ alkyl; and
R¹⁶ and R^{16A} are each independently -C₁-C₄ alkyl;
R⁵¹ and R⁵² are each independently hydrogen, -C₁-C₆ alkyl, -C₁-C₆ alkylene-R^{53A}, -C₅-C₆ cycloalkyl, -C₅-C₆ cycloalkenyl, 3-8 membered heterocycloalkyl, phenyl, or 5-6 membered heteroaryl, wherein each alkyl, alkylene, cycloalkyl, cycloalkenyl, heterocycloalkyl, phenyl or heteroaryl is optionally substituted with one or more R⁵³; or R⁵¹ and R⁵², together with the nitrogen atom to which they are attached, combine to form a 3-8 membered heterocycloalkyl or 5-6 membered heteroaryl, wherein the heterocycloalkyl or heteroaryl are optionally substituted with one or more R⁵³;
R⁵³ is independently -C₁-C₆ alkyl, -C₁-C₆ alkylene-R^{57A}, -C₃-C₆ cycloalkyl, -C₅-C₆ cycloalkenyl, 3-8 membered heterocycloalkyl, phenyl, 5-6 membered heteroaryl, -C₁-C₂ alkoxy, -C₁-C₂ haloalkyl, halogen, cyano, oxo, -OR⁵⁴, -C(O)R⁵⁴, -CO₂R⁵⁴, or -NR⁵⁵R⁵⁶, wherein each alkyl, alkylene, cycloalkyl, cycloalkenyl, heterocycloalkyl, aryl, heteroaryl and alkoxy is optionally substituted with one or more R⁵⁷;
R^{53A} is independently -C₁-C₆ alkylene-R^{57A}, -C₅-C₆ cycloalkyl, 3-8 membered heterocycloalkyl, phenyl, 5-6 membered heteroaryl, -C₁-C₄ alkoxy, -C₁-C₂ haloalkyl, halogen, cyano, oxo, -OR⁵⁴, -CO₂R⁵⁴, or -NR⁵⁵R⁵⁶, wherein each alkylene, cycloalkyl, heterocycloalkyl, aryl, heteroaryl and alkoxy is optionally substituted with one or more R⁵⁷;
R⁵⁴, R⁵⁵ and R⁵⁶ are each independently hydrogen, -C₁-C₆ alkyl, -C₁-C₆ alkylene-R^{61A}, -C₅-C₆ cycloalkyl, 3-6 membered heterocycloalkyl, -C₆-C₁₀ aryl, 5-6 membered heteroaryl, -C₁ haloalkyl, -CO₂R⁵⁸, -S(O)₂(C₆-C₁₀ aryl), wherein each alkyl, alkylene, cycloalkyl, heterocycloalkyl, aryl or heteroaryl is optionally substituted with one or more R⁶¹;
R⁵⁷ is independently -C₁-C₆ alkyl, -C₁-C₆ alkylene-R^{61A}, -C₃-C₆ cycloalkyl, 3-8 membered heterocycloalkyl, phenyl, 5-6 membered heteroaryl, -C₁-C₂ alkoxy, -C₁-C₂ haloalkyl, halogen, cyano, oxo, -OR⁵⁸, NR⁵⁹R⁶⁰, -S(O)₂(C₁-C₆ alkyl), or -S(O)₂NR⁵⁹R⁶⁰, wherein each alkyl, alkylene, cycloalkyl, heterocycloalkyl, phenyl, heteroaryl and alkoxy is optionally substituted with one or more R⁶¹;
R^{57A} is independently -C₁-C₆ alkyl, -C₁-C₆ alkylene-R^{61A}, -C₃-C₆ cycloalkyl, 3-10 membered heterocycloalkyl, phenyl, 5-6 membered heteroaryl, -C₁-C₂ alkoxy, -C₁-C₂ haloalkyl, halogen, cyano, oxo, -OR⁵⁸, NR⁵⁹R⁶⁰, -S(O)₂(C₁-C₆ alkyl), or -S(O)₂NR⁵⁹R⁶⁰, wherein each alkyl, alkylene, cycloalkyl, heterocycloalkyl, phenyl, heteroaryl and alkoxy is optionally substituted with one or more R⁶¹;
R⁵⁸, R⁵⁹ and R⁶⁰ are each independently hydrogen, -C₁-C₆ alkyl, -C₁-C₆ alkylene-R^{64A}, -C₃-C₆ cycloalkyl, 5-6 membered heterocycloalkyl, phenyl, 5-6 membered heteroaryl, -C₁-C₂ alkoxy or -C₁-C₂ haloalkyl, wherein each alkyl, alkylene, cycloalkyl, heterocycloalkyl, phenyl, heteroaryl and alkoxy is optionally substituted with one or more R⁶⁴; and
R⁶¹, R^{61A}, R⁶⁴ and R^{64A} are each independently -C₁-C₆ alkyl or phenyl.

In one embodiment, R¹¹ is independently -C₁-C₆ alkylene-R^{12A}, -C₅-C₆ cycloalkyl, 3-6 membered heterocycloalkyl, phenyl, or 5-6 membered heteroaryl, wherein each alkylene, cycloalkyl, cycloalkenyl, heterocycloalkyl, aryl or heteroaryl is optionally substituted with one or more R¹²;
R¹² is independently -C₁-C₆ alkyl, -C₁-C₆ alkylene-R^{16A}, -C₅-C₆ cycloalkyl, 3-6 membered heterocycloalkyl, phenyl, 5-6 membered heteroaryl, -C₁ haloalkyl, halogen, cyano, oxo, -OR¹³, -CO₂R¹³, or -NR¹⁴R¹⁵, wherein each alkyl, alkylene, cycloalkyl, heterocycloalkyl, phenyl and heteroaryl is optionally substituted with one or more R¹⁶;
R^{12A} is independently -C₅-C₆ cycloalkyl, 3-6 membered heterocycloalkyl, phenyl, 5-6 membered heteroaryl, -C₁ haloalkyl, halogen, cyano, oxo, -OR¹³, -CO₂R¹³, or -NR¹⁴R¹⁵ wherein each cycloalkyl, heterocycloalkyl, phenyl and heteroaryl is optionally substituted with one or more R¹⁶;
R¹³, R¹⁴ and R¹⁵ are each independently hydrogen or -C₁-C₄ alkyl; and
R¹⁶ and R^{16A} are each independently -C₁-C₄ alkyl;
R⁵¹ and R⁵² are each independently hydrogen, -C₁-C₆ alkyl, -C₁-C₆ alkylene-R^{53A}, -C₅-C₆ cycloalkyl, 3-8 membered heterocycloalkyl, phenyl, or 5-6 membered heteroaryl, wherein each alkyl, alkylene, cycloalkyl, heterocycloalkyl, phenyl or heteroaryl is optionally substituted with one or more R⁵³; or R⁵¹ and R⁵², together with the nitrogen atom to which they are attached, combine to form a 3-8 membered heterocycloalkyl or 5-6 membered heteroaryl, wherein the heterocycloalkyl or heteroaryl are optionally substituted with one or more R⁵³;
R⁵³ is independently -C₁-C₆ alkyl, -C₁-C₆ alkylene-R^{57A}, -C₃-C₆ cycloalkyl, 3-8 membered heterocycloalkyl, phenyl, 5-6 membered heteroaryl, -C₁-C₂ alkoxy, -C₁-C₂ haloalkyl, halogen, cyano, oxo, -OR⁵⁴, -C(O)R⁵⁴, -CO₂R⁵⁴, or -NR⁵⁵R⁵⁶, wherein each alkyl, alkylene, cycloalkyl, heterocycloalkyl, phenyl, heteroaryl and alkoxy is optionally substituted with one or more R⁵⁷;
R^{53A} is independently -C₁-C₆ alkylene-R^{57A}, -C₅-C₆ cycloalkyl, 3-8 membered heterocycloalkyl, phenyl, 5-6 membered heteroaryl, -C₁-C₄ alkoxy, -C₁-C₂ haloalkyl, halogen, cyano, oxo, -OR⁵⁴, -CO₂R⁵⁴, or -NR⁵⁵R⁵⁶, wherein each alkylene, cycloalkyl, heterocycloalkyl, aryl, heteroaryl and alkoxy is optionally substituted with one or more R⁵⁷;
R⁵⁴, R⁵⁵ and R⁵⁶ are each independently hydrogen, -C₁-C₆ alkyl, -C₁-C₆ alkylene-R^{61A}, -C₅-C₆ cycloalkyl, 3-6 membered heterocycloalkyl, phenyl, 5-6 membered heteroaryl, -C₁ haloalkyl, -CO₂R⁵⁸, -S(O)₂(C₆-C₁₀ aryl), wherein each alkyl, alkylene, cycloalkyl, heterocycloalkyl, phenyl or heteroaryl is optionally substituted with one or more R⁶¹;
R⁵⁷ is independently -C₁-C₆ alkyl, -C₁-C₆ alkylene-R^{61A}, -C₃-C₆ cycloalkyl, 5-6 membered heterocycloalkyl, phenyl, 5-6 membered heteroaryl, -C₁-C₂ alkoxy, -C₁-C₂ haloalkyl, halogen, cyano, oxo, -OR⁵⁸, NR⁵⁹R⁶⁰, -S(O)₂(C₁-C₆ alkyl), or -S(O)₂NR⁵⁹R⁶⁰, wherein each alkyl, alkylene, cycloalkyl, heterocycloalkyl, phenyl, heteroaryl and alkoxy is optionally substituted with one or more R⁶¹;
R^{57A} is independently -C₁-C₆ alkyl, -C₁-C₆ alkylene-R^{61A}, -C₃-C₆ cycloalkyl, 3-8 membered heterocycloalkyl, phenyl, 5-6 membered heteroaryl, -C₁-C₂ alkoxy, -C₁-C₂ haloalkyl, halogen, cyano, oxo, -OR⁵⁸, NR⁵⁹R⁶⁰, wherein each alkyl, alkylene, cycloalkyl, heterocycloalkyl, phenyl, heteroaryl and alkoxy is optionally substituted with one or more R⁶¹;
R⁵⁸, R⁵⁹ and R⁶⁰ are each independently hydrogen, -C₁-C₆ alkyl, -C₃-C₆ cycloalkyl, 5-6 membered heterocycloalkyl, phenyl, 5-6 membered heteroaryl, -C₁-C₂ alkoxy or -C₁-C₂ haloalkyl, wherein each alkyl, alkylene, cycloalkyl, heterocycloalkyl, phenyl, heteroaryl and alkoxy is optionally substituted with one or more R⁶⁴; and
R⁶¹, R^{61A}, R⁶⁴ and R^{64A} are each independently -C₁-C₆ alkyl or phenyl.

In some embodiments, R¹¹ is independently -C₁-C₆ alkylene-R^{12A}, -C₅-C₆ cycloalkyl, 3-6 membered heterocycloalkyl, phenyl, or 5-6 membered heteroaryl, wherein each alkylene, cycloalkyl, heterocycloalkyl, phenyl or heteroaryl is optionally substituted with one or more R¹²;
R¹² is independently -C₁-C₆ alkyl, -C₁ haloalkyl, halogen, cyano, oxo, -OR¹³, -CO₂R¹³, or -NR¹⁴R¹⁵;
R^{12A} is independently -C₅-C₆ cycloalkyl, 5-6 membered heterocycloalkyl, phenyl, 5-6 membered heteroaryl, -OR¹³, -CO₂R¹³, or -NR¹⁴R¹⁵, wherein each cycloalkyl, heterocycloalkyl, phenyl or heteroaryl is optionally substituted with one or more R¹⁶;
R¹³, R¹⁴ and R¹⁵ are each independently hydrogen or -C₁-C₄ alkyl; and
R¹⁶ is -C₁-C₄ alkyl;
R⁵¹ and R⁵² are each independently hydrogen, -C₁-C₆ alkyl, -C₁-C₆ alkylene-R^{53A}, -C₅-C₆ cycloalkyl, 3-8 membered heterocycloalkyl, phenyl, or 5-6 membered heteroaryl, wherein preferably at least one of R⁵¹ and R⁵² is hydrogen, wherein each alkyl, alkylene, cycloalkyl, heterocycloalkyl, phenyl or heteroaryl is optionally substituted with one or more R⁵³; or R⁵¹ and R⁵², together with the nitrogen atom to which they are attached, combine to form a 3-8 membered heterocycloalkyl or 5-6 membered heteroaryl, wherein the heterocycloalkyl or heteroaryl are optionally substituted with one or more R⁵³;
R⁵³ is independently -C₁-C₆ alkyl, -C₁-C₆ alkylene-R^{57A}, -C₃-C₆ cycloalkyl, 3-8 membered heterocycloalkyl, phenyl, 5-6 membered heteroaryl, -C₁-C₂ alkoxy, -C₁-C₂ haloalkyl, halogen, cyano, oxo, -OR⁵⁴, -C(O)R⁵⁴, -CO₂R⁵⁴, or -NR⁵⁵R⁵⁶, wherein each alkyl, alkylene, cycloalkyl, heterocycloalkyl, phenyl, heteroaryl and alkoxy is optionally substituted with one or more R⁵⁷;
R^{53A} is independently -C₁-C₆ alkylene-R^{57A}, -C₅-C₆ cycloalkyl, 3-6 membered heterocycloalkyl, phenyl, 5-6 membered heteroaryl, -C₁-C₄ alkoxy, -C₁-C₂ haloalkyl, halogen, cyano, oxo, -OR⁵⁴, -CO₂R⁵⁴, or -NR⁵⁵R⁵⁶, wherein each alkylene, cycloalkyl, heterocycloalkyl, aryl, heteroaryl and alkoxy is optionally substituted with one or more R⁵⁷;
R⁵⁴, R⁵⁵ and R⁵⁶ are each independently hydrogen, -C₁-C₆ alkyl, -C₁-C₆ alkylene-R^{61A}, -C₅-C₆ cycloalkyl, 3-6 membered heterocycloalkyl, phenyl, 5-6 membered heteroaryl, -C₁ haloalkyl, -CO₂R⁵⁸, -S(O)₂(C₆-C₁₀ aryl), wherein each alkyl, alkylene, cycloalkyl, heterocycloalkyl, phenyl or heteroaryl is optionally substituted with one or more R⁶¹;
R⁵⁷ is independently -C₁-C₆ alkyl, -C₁-C₆ alkylene-R^{61A}, -C₃-C₆ cycloalkyl, 5-6 membered heterocycloalkyl, phenyl, 5-6 membered heteroaryl, -C₁-C₂ alkoxy, -C₁-C₂ haloalkyl, halogen, cyano, oxo, -OR⁵⁸, NR⁵⁹R⁶⁰, -S(O)₂(C₁-C₆ alkyl), or -S(O)₂NR⁵⁹R⁶⁰, wherein each alkyl, alkylene, cycloalkyl, heterocycloalkyl, phenyl, heteroaryl and alkoxy is optionally substituted with one or more R⁶¹;
R^{57A} is independently -C₁-C₆ alkyl, -C₃-C₆ cycloalkyl, 3-6 membered heterocycloalkyl, phenyl, 5-6 membered heteroaryl, -C₁-C₂ alkoxy, -C₁-C₂ haloalkyl, halogen, cyano, oxo, -OR⁵⁸, NR⁵⁹R⁶⁰, wherein each alkyl, cycloalkyl, heterocycloalkyl, phenyl, heteroaryl and alkoxy is optionally substituted with one or more R⁶¹;
R⁵⁸, R⁵⁹ and R⁶⁰ are each independently hydrogen, -C₁-C₆ alkyl, -C₃-C₆ cycloalkyl, phenyl, wherein each alkyl, cycloalkyl and phenyl is optionally substituted with one or more R⁶⁴; and
R⁶¹, R^{61A} and R⁶⁴ are each independently -C₁-C₆ alkyl or phenyl.

In one embodiment, R¹¹ is independently -C₁-C₆ alkylene-R^{12A}, -C₅-C₆ cycloalkyl, 5-6 membered heterocycloalkyl, or phenyl, wherein each alkylene, cycloalkyl, heterocycloalkyl or phenyl is optionally substituted with one or more R¹²;
R¹² is independently -C₁-C₆ alkyl, -C₁ haloalkyl, oxo, -OR¹³, -CO₂R¹³, or -NR¹⁴R¹⁵
R^{12A} is independently -C₅-C₆ cycloalkyl, 5-6 membered heterocycloalkyl, phenyl, - OR¹³, -CO₂R¹³, or -NR¹⁴R¹⁵, wherein each cycloalkyl, heterocycloalkyl or phenyl is optionally substituted with one or more R¹⁶;
R¹³, R¹⁴ and R¹⁵ are each independently hydrogen or -C₁-C₄ alkyl; and
R¹⁶ is -C₁-C₄ alkyl;
R⁵¹ and R⁵² are each independently hydrogen, -C₁-C₆ alkyl, -C₁-C₆ alkylene-R^{53A}, -C₅-C₆ cycloalkyl, 3-8 membered heterocycloalkyl or phenyl, wherein preferably at least one of R⁵¹ and R⁵² is hydrogen, wherein each alkyl, alkylene, cycloalkyl, heterocycloalkyl or phenyl is optionally substituted with one or more R⁵³; or R⁵¹ and R⁵², together with the nitrogen atom to which they are attached, combine to form a 3-6 membered heterocycloalkyl or 5-6 membered heteroaryl, wherein the heterocycloalkyl or heteroaryl are optionally substituted with one or more R⁵³;
R⁵³ is independently -C₁-C₆ alkyl, -C₁-C₆ alkylene-R^{57A}, -C₃-C₆ cycloalkyl, 3-8 membered heterocycloalkyl or phenyl, -C₁-C₂ alkoxy, -C₁-C₂ haloalkyl, halogen, cyano, oxo, - OR⁵⁴, -C(O)R⁵⁴, -CO₂R⁵⁴, or -NR⁵⁵R⁵⁶, wherein each alkyl, alkylene, cycloalkyl, heterocycloalkyl, phenyl and alkoxy is optionally substituted with one or more R⁵⁷;
R^{53A} is independently -C₁-C₆ alkylene-R ^{57A}, -C₅-C₆ cycloalkyl, 3-6 membered heterocycloalkyl, phenyl, 5-6 membered heteroaryl, -C₁-C₄ alkoxy, -C₁-C₂ haloalkyl, halogen, cyano, oxo, -OR⁵⁴, -CO₂R⁵⁴, or -NR⁵⁵R⁵⁶, wherein each alkylene, cycloalkyl, heterocycloalkyl, phenyl, heteroaryl and alkoxy is optionally substituted with one or more R⁵⁷;
R⁵⁴, R⁵⁵ and R⁵⁶ are each independently hydrogen, -C₁-C₆ alkyl, -C₁-C₆ alkylene-R^{61A}, -C₅-C₆ cycloalkyl, 3-6 membered heterocycloalkyl, phenyl, -C₁ haloalkyl, -CO₂R⁵⁸, -S(O)₂(C₆-C₁₀ aryl), wherein each alkyl, alkylene, cycloalkyl, heterocycloalkyl and phenyl is optionally substituted with one or more R⁶¹;
R⁵⁷ is independently -C₁-C₆ alkyl, -C₁-C₆ alkylene-R^{61A}, -C₃-C₆ cycloalkyl, 5-6 membered heterocycloalkyl, phenyl, -C₁-C₂ alkoxy, -C₁-C₂ haloalkyl, halogen, cyano, oxo, - OR⁵⁸, NR⁵⁹R⁶⁰, -S(O)₂(C₁-C₆ alkyl), or -S(O)₂NR⁵⁹R⁶⁰, wherein each alkyl, alkylene, cycloalkyl, heterocycloalkyl, phenyl and alkoxy is optionally substituted with one or more R⁶¹;
R^{57A} is independently -C₁-C₆ alkyl, -C₃-C₆ cycloalkyl, 3-6 membered heterocycloalkyl, phenyl, -C₁-C₂ alkoxy, -C₁-C₂ haloalkyl, halogen, cyano, oxo, -OR⁵⁸, NR⁵⁹R⁶⁰, wherein each alkyl, cycloalkyl, heterocycloalkyl, phenyl and alkoxy is optionally substituted with one or more R⁶¹;
R⁵⁸, R⁵⁹ and R⁶⁰ are each independently hydrogen, -C₁-C₆ alkyl, -C₃-C₆ cycloalkyl, phenyl, wherein each alkyl, cycloalkyl and phenyl is optionally substituted with one or more R⁶⁴; and
R⁶¹, R^{61A} and R⁶⁴ are each independently -C₁-C₆ alkyl or phenyl.

In some embodiments, R¹¹ is independently -C₁-C₆ alkylene-R^{12A}, -C₅-C₆ cycloalkyl, 5-6 membered heterocycloalkyl, or phenyl, wherein each alkylene, cycloalkyl, heterocycloalkyl or phenyl is optionally substituted with one or more R¹²;
R¹² is independently -C₁-C₂ alkyl;
R^{12A} is independently -C₅-C₆ cycloalkyl, 5-6 membered heterocycloalkyl, phenyl, - OR¹³, -CO₂R¹³, or -NR¹⁴R¹⁵, wherein each cycloalkyl, heterocycloalkyl or phenyl is optionally substituted with one or more R¹⁶;
R¹³, R¹⁴ and R¹⁵ are each independently hydrogen or -C₁-C₂ alkyl; and
R¹⁶ is -C₁-C₂ alkyl;
R⁵¹ and R⁵² are each independently hydrogen, -C₁-C₆ alkyl, -C₁-C₆ alkylene-R^{53A}, -C₅-C₆ cycloalkyl, 5-6 membered heterocycloalkyl or phenyl, wherein preferably at least one of R⁵¹ and R⁵² is hydrogen, wherein each alkyl, alkylene, cycloalkyl, heterocycloalkyl or phenyl is optionally substituted with one or more R⁵³; or R⁵¹ and R⁵², together with the nitrogen atom to which they are attached, combine to form a 5-6 membered heterocycloalkyl, wherein the heterocycloalkyl is optionally substituted with one or more R⁵³;
R⁵³ is independently -C₁-C₆ alkyl, -C₁-C₆ alkylene-R^{57A}, -C₃-C₆ cycloalkyl, 3-6 membered heterocycloalkyl or phenyl, -C₁ alkoxy, -C₁ haloalkyl, halogen, cyano, oxo, -OR⁵⁴, -C(O)R⁵⁴, -CO₂R⁵⁴, or -NR⁵⁵R⁵⁶, wherein each alkyl, alkylene, cycloalkyl, heterocycloalkyl and phenyl is optionally substituted with one or more R⁵⁷;
R^{53A} is independently -C₁-C₄ alkylene-R^{57A}, -C₅-C₆ cycloalkyl, 3-6 membered heterocycloalkyl, phenyl, -C₁-C₄ alkoxy, -C₁ haloalkyl, halogen, cyano, oxo, -OR⁵⁴, -CO₂R⁵⁴, or -NR⁵⁵R⁵⁶, wherein each alkylene, cycloalkyl, heterocycloalkyl and phenyl is optionally substituted with one or more R⁵⁷;
R⁵⁴, R⁵⁵ and R⁵⁶ are each independently hydrogen, -C₁-C₆ alkyl, -C₁-C₆ alkylene-R^{61A}, -C₅-C₆ cycloalkyl, 3-6 membered heterocycloalkyl, phenyl, -C₁ haloalkyl, -CO₂R⁵⁸, -S(O)₂(C₆-C₁₀ aryl), wherein each alkyl, alkylene, cycloalkyl, heterocycloalkyl and phenyl is optionally substituted with one or more R⁶¹;
R⁵⁷ is independently -C₁-C₆ alkyl, -C₁-C₂ alkylene-R^{61A}, -C₁ alkoxy, -C₁ haloalkyl, halogen, cyano, oxo, -OR⁵⁸, NR⁵⁹R⁶⁰, -S(O)₂(C-C₂ alkyl), or -S(O)₂NR⁵⁹R⁶⁰, wherein each alkyl and alkylene is optionally substituted with one or more R⁶¹;
R^{57A} is independently -C₁-C₆ alkyl, -C₅-C₆ cycloalkyl, 3-6 membered heterocycloalkyl, phenyl, -C₁ alkoxy, -C₁ haloalkyl, -OR⁵⁸, NR⁵⁹R⁶⁰, wherein each alkyl, cycloalkyl, heterocycloalkyl and phenyl is optionally substituted with one or more R⁶¹;
R⁵⁸, R⁵⁹ and R⁶⁰ are each independently hydrogen, -C₁-C₄ alkyl and phenyl, wherein each alkyl and phenyl is optionally substituted with one or more R⁶⁴; wherein R⁶⁴ is independently -C₁-C₆ alkyl; and
R⁶¹ and R^{61A} are each independently -C₁-C₆ alkyl or phenyl.

In one embodiment, R¹¹ is independently -C₁-C₆ alkylene-R^{12A} or 5-6 membered heterocycloalkyl, wherein each alkylene and heterocycloalkyl is optionally substituted with one or more R¹²;
R¹² is independently -C₁-C₂ alkyl;
R^{12A} is independently 5-6 membered heterocycloalkyl, phenyl, -OR¹³, -CO₂R¹³, or - NR¹⁴R¹⁵, wherein each heterocycloalkyl or phenyl is optionally substituted with one or more R¹⁶;
R¹³, R¹⁴ and R¹⁵ are each independently hydrogen or -C₁-C₂ alkyl; and
R¹⁶ is -C₁-C₂ alkyl;
R⁵¹ and R⁵² are each independently hydrogen, -C₁-C₆ alkyl, -C₁-C₆ alkylene-R^{53A}, -C₅-C₆ cycloalkyl, 5-6 membered heterocycloalkyl or phenyl, wherein preferably at least one of R⁵¹ and R⁵² is hydrogen, wherein each alkyl, alkylene, cycloalkyl, heterocycloalkyl or phenyl is optionally substituted with one or more R⁵³; or R⁵¹ and R⁵², together with the nitrogen atom to which they are attached, combine to form a 5-6 membered heterocycloalkyl, wherein the heterocycloalkyl is optionally substituted with one or more R⁵³;
R⁵³ is independently -C₁-C₆ alkyl, -C₁-C₆ alkylene-R^{57A}, -C₃-C₆ cycloalkyl, 3-6 membered heterocycloalkyl or phenyl, oxo, -OR⁵⁴, -C(O)R⁵⁴, -CO₂R⁵⁴, or -NR⁵⁵R⁵⁶, wherein each alkyl, alkylene, cycloalkyl, heterocycloalkyl and phenyl is optionally substituted with one or more R⁵⁷;
R^{53A} is independently -C₅-C₆ cycloalkyl, 3-6 membered heterocycloalkyl, phenyl, -C₁-C₄ alkoxy, oxo, -CO₂R⁵⁴, or -NR⁵⁵R⁵⁶, wherein each cycloalkyl, heterocycloalkyl and phenyl is optionally substituted with one or more R⁵⁷;
R⁵⁴, R⁵⁵ and R⁵⁶ are each independently hydrogen, -C₁-C₆ alkyl, -C₁-C₆ alkylene-R^{61A}, -C₅-C₆ cycloalkyl, 3-6 membered heterocycloalkyl, phenyl, -CO₂R⁵⁸, -S(O)₂(phenyl), wherein each alkyl, alkylene, cycloalkyl, heterocycloalkyl and phenyl is optionally substituted with one or more R⁶¹;
R⁵⁷ is independently -C₁-C₆ alkyl, -C₁-C₂ alkylene-R^{61A}, oxo, -OR⁵⁸, NR⁵⁹R⁶⁰, - S(O)₂(C₁-C₂ alkyl), or -S(O)₂NR⁵⁹R⁶⁰, wherein each alkyl and alkylene is optionally substituted with one or more R⁶¹;
R^{57A} is independently -C₁-C₄ alkyl, -C₅-C₆ cycloalkyl, 3-6 membered heterocycloalkyl, phenyl, -C₁ alkoxy, -C₁ haloalkyl, -OR⁵⁸, NR⁵⁹R⁶⁰, wherein each alkyl, cycloalkyl, heterocycloalkyl and phenyl is optionally substituted with one or more R⁶¹;
R⁵⁸, R⁵⁹ and R⁶⁰ are each independently hydrogen, -C₁-C₄ alkyl and phenyl, wherein each alkyl and phenyl is optionally substituted with one or more R⁶⁴; wherein R⁶⁴ is independently -C₁-C₆ alkyl; and
R⁶¹ and R^{61A} are each independently -C₁-C₄ alkyl or phenyl.
In some embodiments, R¹¹ is independently -C₁-C₄ alkylene-R^{12A} or 5-6 membered heterocycloalkyl, wherein each alkylene or heterocycloalkyl is optionally substituted with one or more R¹²;
R¹² is independently -C₁-C₂ alkyl;
R^{12A} is independently 5-6 membered heterocycloalkyl, phenyl, -OR¹³, -CO₂R¹³, or - NR¹⁴R¹⁵, wherein each heterocycloalkyl or phenyl is optionally substituted with one or more R¹⁶;
R¹³, R¹⁴ and R¹⁵ are each independently hydrogen or -C₁-C₂ alkyl; and
R¹⁶ is -C₁-C₂ alkyl;
R⁵¹ and R⁵² are each independently hydrogen, -C₁-C₆ alkyl, -C₁-C₆ alkylene-R^{53A}, -C₅-C₆ cycloalkyl, 5-6 membered heterocycloalkyl or phenyl, wherein preferably at least one of R⁵¹ and R⁵² is hydrogen, wherein each alkyl, alkylene, cycloalkyl, heterocycloalkyl or phenyl is optionally substituted with one or more R⁵³; or R⁵¹ and R⁵², together with the nitrogen atom to which they are attached, combine to form a 5-6 membered heterocycloalkyl, wherein the heterocycloalkyl is optionally substituted with one or more R⁵³;
R⁵³ is independently -C₁-C₆ alkyl, -C₁-C₆ alkylene-R^{57A}, -C₃-C₆ cycloalkyl, 5-6 membered heterocycloalkyl or phenyl, oxo, -OR⁵⁴, -C(O)R⁵⁴, -CO₂R⁵⁴, or -NR⁵⁵R⁵⁶, wherein each alkyl, alkylene, cycloalkyl, heterocycloalkyl and phenyl is optionally substituted with one or more R⁵⁷;
R^{53A} is independently -C₅-C₆ cycloalkyl, 5-6 membered heterocycloalkyl, phenyl, -C₁-C₄ alkoxy, oxo, -CO₂R⁵⁴, or -NR⁵⁵R⁵⁶, wherein each cycloalkyl, heterocycloalkyl and phenyl is optionally substituted with one or more R⁵⁷;
R⁵⁴, R⁵⁵ and R⁵⁶ are each independently hydrogen, -C₁-C₆ alkyl, -C₁-C₆ alkylene-R^{61A}, phenyl, -CO₂R⁵⁸, -S(O)₂(phenyl), wherein each alkyl, alkylene and phenyl is optionally substituted with one or more R⁶¹;
R⁵⁷ is independently -C₁-C₆ alkyl, oxo, NR⁵⁹R⁶⁰, -S(O)₂(C-C₂ alkyl), or - S(O)₂NR⁵⁹R⁶⁰, wherein each alkyl and alkylene is optionally substituted with one or more R⁶¹;
R^{57A} is independently -C₁-C₄ alkyl, -C₅-C₆ cycloalkyl, 3-6 membered heterocycloalkyl, phenyl, -OR⁵⁸, NR⁵⁹R⁶⁰, wherein each alkyl, cycloalkyl, heterocycloalkyl and phenyl is optionally substituted with one or more R⁶¹;
R⁵⁸, R⁵⁹ and R⁶⁰ are each independently hydrogen and -C₁-C₂ alkyl; and
R⁶¹ and R^{61A} are each independently -C₁-C₂ alkyl or phenyl.

In one embodiment, R¹¹ is independently -C₁-C₄ alkylene-R^{12A} or 5-6 membered heterocycloalkyl, wherein each alkylene or heterocycloalkyl is optionally substituted with one or more R¹²;
R¹² is independently -C₁-C₂ alkyl;
R^{12A} is independently 5-6 membered heterocycloalkyl, phenyl, -CO₂R¹³, or -NR¹⁴R¹⁵, wherein each heterocycloalkyl or phenyl is optionally substituted with one or more R¹⁶; and wherein said 5-6 membered heterocycloalkyl does not comprise heteroatoms other than O and N, and wherein preferably said 5-6 membered heterocycloalkyl contains 1 to 4 heteroatoms, preferably 1 to 3, further preferably one or two heteroatoms selected from O and N, and wherein again further preferably said 5-6 membered heterocycloalkyl is selected from pyrrolidine, piperidine, piperazine or morpholine;
R¹³, R¹⁴ and R¹⁵ are each independently hydrogen or -C₁-C₂ alkyl; and
R¹⁶ is -C₁-C₂ alkyl;
R⁵¹ and R⁵² are each independently hydrogen, -C₁-C₆ alkyl, -C₁-C₄ alkylene-R^{53A}, -C₅-C₆ cycloalkyl, 5-6 membered heterocycloalkyl or phenyl, wherein preferably at least one of R⁵¹ and R⁵² is hydrogen, wherein each cycloalkyl, heterocycloalkyl or phenyl is optionally substituted with one or more R⁵³; or R⁵¹ and R⁵², together with the nitrogen atom to which they are attached, combine to form a 5-6 membered heterocycloalkyl, wherein the heterocycloalkyl is optionally substituted with one or more R⁵³;
R⁵³ is independently -C₁-C₄ alkyl, -C₁-C₄ alkylene-R^{57A}, -C₅-C₆ cycloalkyl, 5-6 membered heterocycloalkyl or phenyl, -OR⁵⁴, -C(O)R⁵⁴, -CO₂R⁵⁴, or -NR⁵⁵R⁵⁶, wherein each alkyl, alkylene, cycloalkyl, heterocycloalkyl and phenyl is optionally substituted with one or more R^{S7;}
R^{53A} is independently -C₅-C₆ cycloalkyl, 5-6 membered heterocycloalkyl, phenyl, -C₁-C₄ alkoxy, -CO₂R⁵⁴, or -NR⁵⁵R⁵⁶, wherein each cycloalkyl, heterocycloalkyl and phenyl is optionally substituted with one or more R⁵⁷;
R⁵⁴, R⁵⁵ and R⁵⁶ are each independently hydrogen, -C₁-C₄ alkyl, -C₁-C₄ alkylene-R^{61A}, phenyl, -CO₂R⁵⁸, -S(O)₂(phenyl), wherein phenyl is optionally substituted with one or more R⁶¹;
R⁵⁷ is independently -C₁-C₄ alkyl, oxo, NR⁵⁹R⁶⁰, -S(O)₂(C-C₂ alkyl), or - S(O)₂NR⁵⁹R^{6o};
R^{57A} is independently -C₁-C₄ alkyl, -C₅-C₆ cycloalkyl, 5-6 membered heterocycloalkyl, phenyl, wherein each cycloalkyl, heterocycloalkyl and phenyl is optionally substituted with one or more R⁶¹;
R⁵⁸, R⁵⁹ and R⁶⁰ are each independently hydrogen or -C₁-C₂ alkyl;
R⁶¹is independently -C₁-C₂ alkyl; and
R^{61A} is independently phenyl.

In one embodiment, R¹¹ is independently -C₁-C₄ alkylene-R^{12A} or 5-6 membered heterocycloalkyl, wherein each alkylene or heterocycloalkyl is optionally substituted with one or more R¹²;
R¹² is independently -C₁-C₂ alkyl;
R^{12A} is independently 5-6 membered heterocycloalkyl, phenyl, -CO₂R¹³, or -NR¹⁴R¹⁵, wherein each heterocycloalkyl or phenyl is optionally substituted with one or more R¹⁶; and wherein said 5-6 membered heterocycloalkyl contains 1 to 4 heteroatoms, preferably 1 to 3, further preferably one or two heteroatoms selected from O and N, and wherein again further preferably said 5-6 membered heterocycloalkyl is selected from pyrrolidine, piperidine, piperazine or morpholine;
R¹³, R¹⁴ and R¹⁵ are each independently hydrogen or -C₁-C₂ alkyl; and
R¹⁶ is -C₁-C₂ alkyl;
R⁵¹ and R⁵² are each independently hydrogen, -C₁-C₄ alkyl, -C₁-C₄ alkylene-R^{53A}, -C₅-C₆ cycloalkyl, 5-6 membered heterocycloalkyl or phenyl, wherein preferably at least one of R⁵¹ and R⁵² is hydrogen, wherein each cycloalkyl, heterocycloalkyl or phenyl is optionally substituted with one or more R⁵³; or R⁵¹ and R⁵², together with the nitrogen atom to which they are attached, combine to form a 5-6 membered heterocycloalkyl, wherein the heterocycloalkyl is optionally substituted with one or more R⁵³;
R⁵³ is independently -C₁-C₄ alkyl, -C₁-C₃ alkylene-R^{57A}, -C₅-C₆ cycloalkyl, 5-6 membered heterocycloalkyl or phenyl, -OR⁵⁴, -C(O)R⁵⁴, -CO₂R⁵⁴, or -NR⁵⁵R⁵⁶, wherein each cycloalkyl, heterocycloalkyl and phenyl is optionally substituted with one or more R⁵⁷;
R^{53A} is independently -C₅-C₆ cycloalkyl, 5-6 membered heterocycloalkyl, phenyl, -C₁-C₂ alkoxy, -CO₂R⁵⁴, or -NR⁵⁵R⁵⁶, wherein each cycloalkyl, heterocycloalkyl and phenyl is optionally substituted with one or more R⁵⁷;
R⁵⁴, R⁵⁵ and R⁵⁶ are each independently hydrogen, -C₁-C₂ alkyl, -C₁-C₂ alkylene-R^{61A}, phenyl, -CO₂R⁵⁸, -S(O)₂(phenyl), wherein phenyl is optionally substituted with one or more R⁶¹;
R⁵⁷ is independently -C₁-C₂ alkyl, oxo, NR⁵⁹R⁶⁰, or -S(O)₂NR⁵⁹R⁶⁰;
R^{57A} is independently -C₁-C₂ alkyl, -C₅-C₆ cycloalkyl, 5-6 membered heterocycloalkyl, phenyl, wherein each cycloalkyl, heterocycloalkyl and phenyl is optionally substituted with one or more R⁶¹;
R⁵⁸, R⁵⁹ and R⁶⁰ are each independently hydrogen or -C₁-C₂ alkyl;
R⁶¹is independently -C₁-C₂ alkyl;
R^{61A} is independently phenyl; and
wherein said 5-6 membered heterocycloalkyl contains 1 to 4 heteroatoms, preferably 1 to 3, further preferably one or two heteroatoms, selected from O and N, and wherein again further preferably said 5-6 membered heterocycloalkyl is selected from pyrrolidine, piperidine, piperazine or morpholine.

In one embodiment, R¹¹ is independently -C₁-C₄ alkylene-R^{12A} or 5-6 membered heterocycloalkyl, wherein each alkylene or heterocycloalkyl is optionally substituted with one or more R¹²;
R¹² is independently -C₁-C₂ alkyl;
R^{12A} is independently 5-6 membered heterocycloalkyl, phenyl, -CO₂R¹³, or -NR¹⁴R¹⁵, wherein each heterocycloalkyl or phenyl is optionally substituted with one or more R¹⁶; and wherein said 5-6 membered heterocycloalkyl contains one or two heteroatoms selected from O and N, and wherein preferably said 5-6 membered heterocycloalkyl is selected from pyrrolidine, piperidine, piperazine or morpholine;
R¹³, R¹⁴ and R¹⁵ are each independently hydrogen or -C₁-C₂ alkyl; and
R¹⁶ is -C₁-C₂ alkyl;
R⁵¹ and R⁵² are each independently hydrogen, -C₁-C₄ alkyl, -C₁-C₄ alkylene-R^{53A}, -C₅-C₆ cycloalkyl, 5-6 membered heterocycloalkyl or phenyl, wherein preferably at least one of R⁵¹ and R⁵² is hydrogen, wherein each cycloalkyl, heterocycloalkyl or phenyl is optionally substituted with one or more R⁵³; or R⁵¹ and R⁵², together with the nitrogen atom to which they are attached, combine to form a 5-6 membered heterocycloalkyl, wherein the heterocycloalkyl is optionally substituted with one or more R⁵³;
R⁵³ is independently -C₁-C₄ alkyl, -C₁-C₃ alkylene-R^{57A}, -C₅-C₆ cycloalkyl, 5-6 membered heterocycloalkyl or phenyl, -OR⁵⁴, -C(O)R⁵⁴, -CO₂R⁵⁴, or -NR⁵⁵R⁵⁶, wherein each cycloalkyl, heterocycloalkyl and phenyl is optionally substituted with one or more R⁵⁷;
R^{53A} is independently -C₅-C₆ cycloalkyl, 5-6 membered heterocycloalkyl, phenyl, -C₁-C₂ alkoxy, -CO₂R⁵⁴, or -NR⁵⁵R⁵⁶, wherein each cycloalkyl, heterocycloalkyl and phenyl is optionally substituted with one or more R⁵⁷;
R⁵⁴, R⁵⁵ and R⁵⁶ are each independently hydrogen, -C₁-C₂ alkyl, -C₁-C₂ alkylene-R^{61A}, phenyl, -CO₂R⁵⁸, -S(O)₂(phenyl), wherein phenyl is optionally substituted with one or more R⁶¹;
R⁵⁷ is independently -C₁-C₂ alkyl, oxo, NR⁵⁹R⁶⁰, or -S(O)₂NR⁵⁹R⁶⁰;
R^{57A} is independently -C₁-C₂ alkyl, 5-6 membered heterocycloalkyl, wherein each heterocycloalkyl is optionally substituted with one or more R⁶¹;
R⁵⁸, R⁵⁹ and R⁶⁰ are each independently hydrogen or -C₁-C₂ alkyl;
R⁶¹is independently -C₁-C₂ alkyl;
R^{61A} is independently phenyl; and
wherein said 5-6 membered heterocycloalkyl contains 1 to 4 heteroatoms, preferably 1 to 3, further preferably one or two heteroatoms, selected from O and N, and wherein again further preferably said 5-6 membered heterocycloalkyl is selected from pyrrolidine, piperidine, piperazine or morpholine.

In one embodiment, R¹¹ is independently -C₁-C₄ alkylene-R^{12A} or 5-6 membered heterocycloalkyl, wherein each alkylene or heterocycloalkyl is optionally substituted with one or more R¹²;
R¹² is independently -C₁-C₂ alkyl;
R^{12A} is independently 5-6 membered heterocycloalkyl, phenyl, -CO₂R¹³, or -NR¹⁴R¹⁵, wherein each heterocycloalkyl or phenyl is optionally substituted with one or more R¹⁶; and wherein said 5-6 membered heterocycloalkyl is selected from pyrrolidine, piperidine, piperazine or morpholine, and wherein preferably said 5-6 membered heterocycloalkyl is pyrrolidine;
R¹³, R¹⁴ and R¹⁵ are each independently hydrogen or -C₁-C₂ alkyl; and
R¹⁶ is -C₁-C₂ alkyl,
R⁵¹ and R⁵² are each independently hydrogen, -C₁-C₄ alkyl, -C₁-C₄ alkylene-R^{53A}, -C₅-C₆ cycloalkyl, 5-6 membered heterocycloalkyl or phenyl, wherein preferably at least one of R⁵¹ and R⁵² is hydrogen, wherein each cycloalkyl, heterocycloalkyl or phenyl is optionally substituted with one or more R⁵³; or R⁵¹ and R⁵², together with the nitrogen atom to which they are attached, combine to form a 5-6 membered heterocycloalkyl, wherein the heterocycloalkyl is optionally substituted with one or more R⁵³;
R⁵³ is independently -C₁-C₄ alkyl, -C₁-C₃ alkylene-R^{57A}, -C₅-C₆ cycloalkyl, 5-6 membered heterocycloalkyl or phenyl, -OR⁵⁴, -C(O)R⁵⁴, -CO₂R⁵⁴, or -NR⁵⁵R⁵⁶, wherein each cycloalkyl, heterocycloalkyl and phenyl is optionally substituted with one or more R⁵⁷;
R^{53A} is independently -C₅-C₆ cycloalkyl, 5-6 membered heterocycloalkyl, phenyl, -C₁-C₂ alkoxy, -CO₂R⁵⁴, or -NR⁵⁵R⁵⁶, wherein each cycloalkyl, heterocycloalkyl and phenyl is optionally substituted with one or more R⁵⁷;
R⁵⁴, R⁵⁵ and R⁵⁶ are each independently hydrogen, -C₁-C₂ alkyl, -C₁-C₂ alkylene-R^{61A}, phenyl, -CO₂R⁵⁸, -S(O)₂(phenyl), wherein phenyl is optionally substituted with one or more R⁶¹;
R⁵⁷ is independently -C₁-C₂ alkyl, oxo, NR⁵⁹R⁶⁰, or -S(O)₂NR⁵⁹R⁶⁰;
R^{57A} is independently -C₁-C₂ alkyl, 5-6 membered heterocycloalkyl, wherein each heterocycloalkyl is optionally substituted with one or more R⁶¹;
R⁵⁸, R⁵⁹ and R⁶⁰ are each independently hydrogen or -C₁-C₂ alkyl;
R⁶¹is independently -C₁-C₂ alkyl;
R^{61A} is independently phenyl; and
wherein said 5-6 membered heterocycloalkyl contains 1 to 4 heteroatoms, preferably 1 to 3, further preferably one or two heteroatoms, selected from O and N, and wherein again further preferably said 5-6 membered heterocycloalkyl is selected from pyrrolidine, piperidine, piperazine or morpholine.

In one aspect, the present invention provides a compound of the Formula I or a pharmaceutically acceptable salt, tautomer, solvate, hydrate or enantiomer thereof: 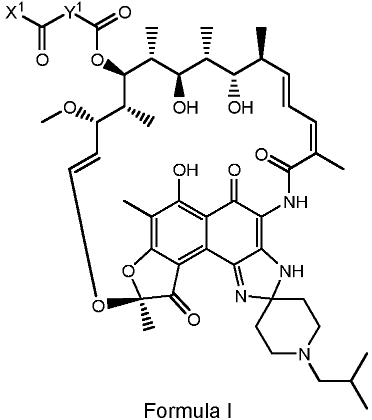 wherein:
Y¹ is (CR¹R²)ₙ, wherein n is an integer from 1 to 6 and R¹ and R² are independently, at each occurrence, selected from the group consisting of hydrogen, -C₁-C₆ alkyl, and -C₁-C₆ alkoxy, wherein each alkyl is optionally substituted with one or more halogen or -C₃-C₆ cycloalkyl, and wherein each cycloalkyl is further optionally substituted with one or more halogen;
X¹ is -OR¹¹ or -NR⁵¹R⁵²;
R¹¹ is independently -C₁-C₆ alkyl, -C₃-C₆ cycloalkyl, -C₁-C₆ alkylene-C₃-C₆ cycloalkyl, 3-10 membered heterocycloalkyl, -C₁-C₆ alkylene-3-10 membered heterocycloalkyl, -C₆-C₁₀ aryl, -C₁-C₆ alkylene-C₆-C₁₀ aryl, 5-10 membered heteroaryl, or -C₁-C₆ alkylene-5-10 membered heteroaryl; wherein each alkyl, cycloalkyl, heterocycloalkyl, aryl or heteroaryl is optionally substituted with one or more R¹²;
R¹² is independently -C₁-C₆ alkyl, -C₁-C₆ alkoxy, -C₁-C₆ haloalkyl, halogen, cyano, oxo, -OR¹³, -CO₂R¹³, or -NR¹⁴R¹⁵;
R¹³, R¹⁴ and R¹⁵ are each independently hydrogen, -C₁-C₆ alkyl, or -C₆-C₁₀ aryl;
R⁵¹ and R⁵² are each independently hydrogen, -C₁-C₆ alkyl, -C₃-C₆ cycloalkyl, -C₁-C₆ alkylene-C₃-C₆ cycloalkyl, 3-10 membered heterocycloalkyl, -C₁-C₆ alkylene-3-10 membered heterocycloalkyl, -C₆-C₁₀ aryl, -C₁-C₆ alkylene-C₆-C₁₀ aryl, 5-10 membered heteroaryl, or -C₁-C₆ alkylene-5-10 membered heteroaryl;
wherein each alkyl, cycloalkyl, heterocycloalkyl, aryl or heteroaryl is optionally substituted with one or more R⁵³; or R⁵¹ and R⁵², together with the nitrogen atom to which they are attached, combine to form a 3-15 membered heterocycloalkyl or 5-15 membered heteroaryl, wherein the heterocycloalkyl or heteroaryl are optionally substituted with one or more R⁷³;
R⁵³ is independently -C₁-C₆ alkyl, -C₃-C₆ cycloalkyl, -C₁-C₆ alkylene-C₃-C₆ cycloalkyl, -C₆-C₁₀ aryl, -C₁-C₆ alkylene-C₆-C₁₀ aryl, 3-10 membered heterocycloalkyl, -C₁-C₆ alkylene-3-10 membered heterocycloalkyl, 5-10 membered heteroaryl, -C₁-C₆ alkylene-5-10 membered heteroaryl, -C₁-C₆ alkoxy, -C₁-C₆ haloalkyl, halogen, cyano, oxo, -OR⁵⁴, -C(O)R⁵⁴, -CO₂R⁵⁴, or -NR⁵⁵R⁵⁶, wherein each alkyl, cycloalkyl, heterocycloalkyl, aryl, and heteroaryl is optionally substituted with one or more R⁵⁷;
R⁵⁴ is independently hydrogen or -C₁-C₆ alkyl;
R⁵⁵ and R⁵⁶ are each independently hydrogen, -C₁-C₆ alkyl, -C₃-C₆ cycloalkyl, 3-10 membered heterocycloalkyl, -C₆-C₁₀ aryl, 5-10 membered heteroaryl, -C₁-C₆ haloalkyl, - CO₂C₁-C₆ alkyl, -S(O)₂(C₁-C₆ alkyl), or -S(O)₂(C₆-C10 aryl), wherein each alkyl is optionally substituted with one or more phenyl;
R⁵⁷ is -C₁-C₆ alkyl;
R⁷³ is independently selected from -C₁-C₆ alkyl, -C₃-C₆ cycloalkyl, -C₁-C₆ alkylene-C₃-C₆ cycloalkyl, -C₆-C₁₀ aryl, -C₁-C₆ alkylene-C₆-C₁₀ aryl, 3-10 membered heterocycloalkyl, -C₁-C₆ alkylene-3-10 membered heterocycloalkyl, 5-10 membered heteroaryl, -C₁-C₆ alkylene-5-10 membered heteroaryl, -NR⁷⁵R⁷⁶, -OR⁷⁴, or oxo;
wherein each alkylene is optionally substituted with one or more oxo or -OH;
wherein each alkyl, cycloalkyl, aryl, heterocycloalkyl, or heteroaryl is optionally substituted with one or more -C₁-C₆ alkyl, -C₁-C₆ haloalkyl, halogen, -NH₂, -NH(C₁-C₆ alkyl), -N(C₁-C₆ alkyl)₂, -OH, -OCH₃, -OCH₂CH₂OH, -SO₂NH₂, phenyl, or oxo;
R⁷⁵ and R⁷⁶ are each independently -H, -C₁-C₆ alkyl, -CO₂C₁-C₆ alkyl, -SO₂phenyl, - C₆-C₁₀ aryl, or 5- to 6-membered heteroaryl, wherein each -C₆-C₁₀ aryl or 5- to 6-membered heteroaryl is optionally substituted with one or more -C₁-C₆ alkyl, halogen -OC₁-C₆ alkyl, or - CN; and
R⁷⁴ is independently -H, -C₁-C₆ alkyl, -C₆-C₁₀ aryl or -C₅-C₆ cycloalkyl, wherein said cycloalkyl is optionally substituted by one or more -C₁-C₆ alkyl.

In some embodiments, R¹¹ is selected from -C₁-C₄ alkyl, 5 to 6 membered heterocycloalkyl, or -C₁-C₂alkylene-5 to 6 membered heterocycloalkyl; wherein each alkyl is optionally substituted with one or more R¹² and wherein each 5 to 6 membered heterocycloalkyl is optionally substituted with one or more -C₁-C₄ alkyl;
wherein R¹² is independently selected from phenyl, -CO₂R¹³, and -NR¹⁴R¹⁵;
wherein R¹³, R¹⁴ and R¹⁵ are each independently -C₁-C₂ alkyl;
R⁵¹ and R⁵² are each independently hydrogen, -C₁-C₆ alkyl, -C₃-C₆ cycloalkyl, -C₁-C₆alkylene-C₃-C₆ cycloalkyl, 3 to 10 membered heterocycloalkyl, -C₁-C₆alkylene-3 to 10 membered heterocycloalkyl, -C₆-C₁₀ aryl, or -C₁-C₆alkylene-C₆-C₁₀ aryl;
wherein each alkyl and cycloalkyl is optionally substituted with one or more R⁵³;
wherein each heterocycloalkyl is optionally substituted with one or more -C₁-C₆ alkyl or -C₁-C₆alkylene-phenyl;
wherein each aryl is optionally substituted with one or more -C₁-C₆ alkyl or -C₁-C₆alkylene-5 to 6-membered heterocycloalkyl, and wherein said 5 to 6-membered heterocycloalkyl is optionally substituted with one or more -C₁-C₆ alkyl;
R⁵³ is independently -OR⁵⁴, -CO₂R⁵⁴, or -NR⁵⁵R⁵⁶;
R⁵⁴ is -C₁-C₄ alkyl; and
R⁵⁵ and R⁵⁶ are each independently -H, -C₁-C₄ alkyl, phenyl, or -C₁-C₆alkylene-phenyl; or
R⁵¹ and R⁵², together with the nitrogen atom to which they are attached, combine to form a 3-15 membered heterocycloalkyl, wherein said heterocycloalkyl is optionally fused to one or more additional aryl or heteroaryl rings or said heterocycloalkyl is optionally connected at a single atom to another cycloalkyl or heterocycloalkyl to form a 10- to 15-membered spirocyclic heterocycle, or said heterocycloalkyl optionally comprises a C₂ bridging alkylene; and wherein said 3-15 membered heterocycloalkyl is optionally substituted with one or more, preferably one or two, R⁷³;
R⁷³ is independently selected from -C₁-C₆ alkyl, -C₃-C₆ cycloalkyl, -C₁-C₆ alkylene-C₃-C₆ cycloalkyl, -C₆-C₁₀ aryl, -C₁-C₆ alkylene-C₆-C₁₀ aryl, 3-10 membered heterocycloalkyl, -C₁-C₆ alkylene-3-10 membered heterocycloalkyl, 5-10 membered heteroaryl, -C₁-C₆ alkylene-5-10 membered heteroaryl, -NR⁷⁵R⁷⁶, -OR⁷⁴, or oxo;
wherein each alkylene is optionally substituted with one or more oxo or -OH;
wherein each alkyl, cycloalkyl, aryl, heterocycloalkyl, or heteroaryl is optionally substituted with one or more -C₁-C₆ alkyl, -C₁-C₆ haloalkyl, halogen, -NH₂, -NH(C₁-C₆ alkyl), -N(C₁-C₆ alkyl)₂, -OH, -OCH₃, -OCH₂CH₂OH, -SO₂NH₂, phenyl, or oxo;
R⁷⁵ and R⁷⁶ are each independently -H, -C₁-C₆ alkyl, -CO₂C₁-C₆ alkyl, -SO₂phenyl, - C₆-C₁₀ aryl, or 5- to 6-membered heteroaryl, wherein each -C₆-C₁₀ aryl or 5- to 6-membered heteroaryl is optionally substituted with one or more -C₁-C₆ alkyl, halogen -OC₁-C₆ alkyl, or - CN; and
R⁷⁴ is independently -H, -C₁-C₆ alkyl, -C₆-C₁₀ aryl or -C₅-C₆ cycloalkyl, wherein said cycloalkyl is optionally substituted by one or more -C₁-C₆ alkyl.

In some embodiments, R¹¹ is selected from -C₁-C₄ alkyl, 5 to 6 membered heterocycloalkyl, or -C₁-C₂alkylene-5 to 6 membered heterocycloalkyl; wherein each alkyl is optionally substituted with one or more R¹² and wherein each 5 to 6 membered heterocycloalkyl does not comprise heteroatoms other than O and N and wherein each 5 to 6 membered heterocycloalkyl is further optionally substituted with one or more -C₁-C₂ alkyl;
wherein R¹² is independently selected from phenyl, -CO₂R¹³, and -NR¹⁴R¹⁵;
wherein R¹³, R¹⁴ and R¹⁵ are each independently -C₁-C₂ alkyl;
R⁵¹ and R⁵² are each independently hydrogen, -C₁-C₄ alkyl, -C₃-C₆ cycloalkyl, -C₁-C₄alkylene-C₃-C₆ cycloalkyl, 3 to 10 membered heterocycloalkyl, -C₁-C₄alkylene-3 to 10 membered heterocycloalkyl, -C₆-C₁₀ aryl, or -C₁-C₄alkylene-C₆-C₁₀ aryl;
wherein each alkyl and cycloalkyl is optionally substituted with one or more R⁵³;
wherein each heterocycloalkyl is optionally substituted with one or more -C₁-C₆ alkyl or -C₁-C₄alkylene-phenyl;
wherein each aryl is optionally substituted with one or more -C₁-C₆ alkyl or -C₁-C₄alkylene-5 to 6-membered heterocycloalkyl, and wherein said 5 to 6-membered heterocycloalkyl is optionally substituted with one or more -C₁-C₄ alkyl;
R⁵³ is independently -OR⁵⁴, -CO₂R⁵⁴, or -NR⁵⁵R⁵⁶;
R⁵⁴ is -C₁-C₄ alkyl; and
R⁵⁵ and R⁵⁶ are each independently -H, -C₁-C₄ alkyl, phenyl, or -C₁-C₄alkylene-phenyl; or
R⁵¹ and R⁵², together with the nitrogen atom to which they are attached, combine to form a 3-15 membered heterocycloalkyl, wherein said heterocycloalkyl is optionally fused to one or more additional aryl or heteroaryl rings or said heterocycloalkyl is optionally connected at a single atom to another cycloalkyl or heterocycloalkyl to form a 10- to 15-membered spirocyclic heterocycle, or said heterocycloalkyl optionally comprises a C₂ bridging alkylene; and wherein said 3-15 membered heterocycloalkyl is optionally substituted with one or more, preferably one or two, R⁷³;
R⁷³ is independently selected from -C₁-C₆ alkyl, -C₃-C₆ cycloalkyl, -C₁-C₆ alkylene-C₃-C₆ cycloalkyl, -C₆-C₁₀ aryl, -C₁-C₆ alkylene-C₆-C₁₀ aryl, -C(O)-C₆-C₁₀ aryl, 3-10 membered heterocycloalkyl, -C₁-C₆ alkylene-3-10 membered heterocycloalkyl, 5-10 membered heteroaryl, -C₁-C₆ alkylene-5-10 membered heteroaryl, -CH(OH)-5-10 membered heteroaryl, - NR⁷⁵R⁷⁶, -OR⁷⁴, or oxo;
wherein said -C₁-C₆ alkyl is optionally substituted with one or more -NH₂, -NH(C₁-C₆ alkyl), -N(C₁-C₆ alkyl)₂, -OH, -OCH₂CH₂OH, or phenyl;
wherein each aryl is optionally substituted with one or more -C₁-C₆ alkyl, -C₁-C₆ haloalkyl, phenyl, halogen, -OH, -OCH₃, or -SO₂NH₂;
wherein each heterocycloalkyl is optionally substituted with one or more -C₁-C₆ alkyl or oxo;
wherein each heteroaryl is optionally substituted with one or more oxo, -C₁-C₆ alkyl, - C₁-C₆ haloalkyl, or halogen;
R⁷⁵ and R⁷⁶ are each independently -H, -C₁-C₆ alkyl, -CO₂C₁-C₆ alkyl, -SO₂phenyl, - C₆-C₁₀ aryl, or 5- to 6-membered heteroaryl, wherein each -C₆-C₁₀ aryl or 5- to 6-membered heteroaryl is optionally substituted with one or more -C₁-C₆ alkyl, halogen -OC₁-C₆ alkyl, or - CN; and
R⁷⁴ is independently -H, -C₆-C₁₀ aryl or -C₅-C₆ cycloalkyl, wherein said cycloalkyl is optionally substituted by one or more -C₁-C₆ alkyl.

In some embodiments, R¹¹ is selected from -C₁-C₄ alkyl, 5 to 6 membered heterocycloalkyl, or -C₁-C₂alkylene-5 to 6 membered heterocycloalkyl; wherein each alkyl is optionally substituted with one or more R¹², preferably with exactly one R¹², and wherein each 5 to 6 membered heterocycloalkyl is selected from pyrrolidine, piperidine, piperazine, and morpholine, and wherein each 5 to 6 membered heterocycloalkyl is further optionally substituted with one or more -C₁-C₂ alkyl;
wherein R¹² is independently selected from phenyl, -CO₂R¹³, and -NR¹⁴R¹⁵;
   wherein R¹³, R¹⁴ and R¹⁵ are each independently -C₁-C₂ alkyl;
R⁵¹ and R⁵² are each independently hydrogen, -C₁-C₄ alkyl, -C₅-C₆ cycloalkyl, -C₁-C₄alkylene-C₅-C₆ cycloalkyl, 5 to 6 membered heterocycloalkyl, -C₁-C₄alkylene-5 to 6 membered heterocycloalkyl, -C₆ aryl, or -C₁-C₄alkylene-C₆ aryl;
wherein each alkyl and cycloalkyl is optionally substituted with one or more R⁵³;
wherein each heterocycloalkyl is optionally substituted with one or more -C₁-C₄ alkyl or -C₁-C₄alkylene-phenyl;
wherein each -C₆ aryl is optionally substituted with one or more -C₁-C₄ alkyl or -C₁-C₄alkylene-5 to 6-membered heterocycloalkyl, and wherein said 5 to 6-membered heterocycloalkyl is optionally substituted with one or more -C₁-C₄ alkyl;
R⁵³ is independently -OR⁵⁴, -CO₂R⁵⁴, or -NR⁵⁵R⁵⁶;
R⁵⁴ is -C₁-C₄ alkyl; and
R⁵⁵ and R⁵⁶ are each independently -H, -C₁-C₄ alkyl, phenyl, or -C₁-C₄alkylene-phenyl; or
R⁵¹ and R⁵², together with the nitrogen atom to which they are attached, combine to form a 3-15 membered heterocycloalkyl, wherein said heterocycloalkyl is optionally fused to one or more additional aryl or heteroaryl rings or said heterocycloalkyl is optionally connected at a single atom to another cycloalkyl or heterocycloalkyl to form a 10- to 15-membered spirocyclic heterocycle, or said heterocycloalkyl optionally comprises a C₂ bridging alkylene; and wherein said 3-15 membered heterocycloalkyl is optionally substituted with one or more, preferably one or two, R⁷³;
R⁷³ is independently selected from -C₁-C₄ alkyl, -C₃-C₆ cycloalkyl, -C₁-C₄ alkylene-C₃-C₆ cycloalkyl, -C₆-C₁₀ aryl, -C₁-C₄ alkylene-C₆-C₁₀ aryl, -C(O)-C₆-C₁₀ aryl, 3-10 membered heterocycloalkyl, -C₁-C₄ alkylene-3-10 membered heterocycloalkyl, 5-10 membered heteroaryl, -C₁-C₄ alkylene-5-10 membered heteroaryl, -CH(OH)-5-10 membered heteroaryl, - NR⁷⁵R⁷⁶, -OR⁷⁴, or oxo;
wherein said -C₁-C₄ alkyl is optionally substituted with one or more -NH₂, -NH(C₁-C₄ alkyl), -N(C₁-C₄ alkyl)₂, -OH, -OCH₂CH₂OH, or phenyl;
wherein each aryl is optionally substituted with one or more -C₁-C₄ alkyl, -C₁-C₄ haloalkyl, phenyl, halogen, -OH, -OCH₃, or -SO₂NH₂;
wherein each heterocycloalkyl is optionally substituted with one or more -C₁-C₄ alkyl or oxo;
wherein each heteroaryl is optionally substituted with one or more oxo, -C₁-C₄ alkyl, - C₁-C₄ haloalkyl, or halogen;
R⁷⁵ and R⁷⁶ are each independently -H, -C₁-C₄ alkyl, -CO₂C₁-C₄ alkyl, -SO₂phenyl, - C₆-C₁₀ aryl, or 5- to 6-membered heteroaryl, wherein each -C₆-C₁₀ aryl or 5- to 6-membered heteroaryl is optionally substituted with one or more -C₁-C₄ alkyl, halogen -OC₁-C₄ alkyl, or - CN; and
   R⁷⁴ is independently -H, -C₆-C₁₀ aryl or -C₅-C₆ cycloalkyl, wherein said cycloalkyl is optionally substituted by one or more -C₁-C₄ alkyl.

In some embodiments, R¹¹ is selected from -C₁-C₄ alkyl, 5 to 6 membered heterocycloalkyl, or -C₁-C₂alkylene-5 to 6 membered heterocycloalkyl; wherein each alkyl is optionally substituted with one or more R¹², preferably with exactly one R¹², and wherein each 5 to 6 membered heterocycloalkyl is selected from pyrrolidine and piperidine, and wherein each 5 to 6 membered heterocycloalkyl is further optionally substituted with one or more -C₁-C₂ alkyl;
wherein R¹² is independently selected from phenyl, -CO₂R¹³, and -NR¹⁴R¹⁵;
wherein R¹³, R¹⁴ and R¹⁵ are each independently -C₁-C₂ alkyl;
R⁵¹ and R⁵² are each independently hydrogen, -C₁-C₄ alkyl, -C₅-C₆ cycloalkyl, -C₁-C₃alkylene-C₅-C₆ cycloalkyl, 5 to 6 membered heterocycloalkyl, -C₁-C₃alkylene-5 to 6 membered heterocycloalkyl, -C₆ aryl, or -C₁-C₃alkylene-C₆ aryl;
wherein each alkyl and cycloalkyl is optionally substituted with one or more R⁵³;
wherein each heterocycloalkyl is optionally substituted with one or more -C₁-C₂ alkyl or -C₁-C₂alkylene-phenyl;
wherein each -C₆ aryl is optionally substituted with one or more -C₁-C₂ alkyl or -C₁-C₄alkylene-5 to 6-membered heterocycloalkyl, and wherein said 5 to 6-membered heterocycloalkyl is optionally substituted with one or more -C₁-C₂ alkyl;
R⁵³ is independently -OR⁵⁴, -CO₂R⁵⁴, or -NR⁵⁵R⁵⁶;
R⁵⁴ is -C₁-C₂ alkyl; and
R⁵⁵ and R⁵⁶ are each independently -H, -C₁-C₂ alkyl, phenyl, or -C₁-C₂alkylene-phenyl; or
R⁵¹ and R⁵², together with the nitrogen atom to which they are attached, combine to form a 3-15 membered heterocycloalkyl, wherein said heterocycloalkyl is optionally fused to one or more additional aryl or heteroaryl rings or said heterocycloalkyl is optionally connected at a single atom to another cycloalkyl or heterocycloalkyl to form a 10- to 15-membered spirocyclic heterocycle, or said heterocycloalkyl optionally comprises a C₂ bridging alkylene; and wherein said 3-15 membered heterocycloalkyl is optionally substituted with one or more, preferably one or two, R⁷³;
R⁷³ is independently selected from -C₁-C₄ alkyl, -C₃-C₆ cycloalkyl, -C₁-C₄ alkylene-C₃-C₆ cycloalkyl, -C₆-C₁₀ aryl, -C₁-C₄ alkylene-C₆-C₁₀ aryl, -C(O)-C₆-C₁₀ aryl, 3-10 membered heterocycloalkyl, -C₁-C₄ alkylene-3-10 membered heterocycloalkyl, 5-10 membered heteroaryl, -C₁-C₄ alkylene-5-10 membered heteroaryl, -CH(OH)-5-10 membered heteroaryl, - NR⁷⁵R⁷⁶, -OR⁷⁴, or oxo;
wherein said -C₁-C₄ alkyl is optionally substituted with one or more -N(C₁-C₄ alkyl)₂, - OH, -OCH₂CH₂OH, or phenyl;
wherein each aryl is optionally substituted with one or more -C₁-C₄ alkyl, -C₁-C₄ haloalkyl, phenyl, halogen, -OCH₃, or -SO₂NH₂;
wherein each heterocycloalkyl is optionally substituted with one or more -C₁-C₄ alkyl or oxo;
wherein each heteroaryl is optionally substituted with one or more oxo, -C₁-C₄ alkyl, - C₁-C₄ haloalkyl, or halogen;
R⁷⁵ and R⁷⁶ are each independently -H, -C₁-C₄ alkyl, -CO₂C₁-C₄ alkyl, -SO₂phenyl, - C₆-C₁₀ aryl, or 5- to 6-membered heteroaryl, wherein each -C₆-C₁₀ aryl or 5- to 6-membered heteroaryl is optionally substituted with one or more -C₁-C₄ alkyl, halogen -OC₁-C₄ alkyl, or - CN; and
R⁷⁴ is independently -H, -C₆-C₁₀ aryl or -C₅-C₆ cycloalkyl, wherein said cycloalkyl is optionally substituted by one or more -C₁-C₄ alkyl.

In some embodiments, R¹¹ is selected from -C₁-C₄ alkyl, 5 to 6 membered heterocycloalkyl, -C₁-C₂alkylene-5 to 6 membered heterocycloalkyl, or -C₁-C₂alkylene-phenyl; wherein each alkyl is optionally substituted with one or more R¹², preferably with exactly one R¹², and wherein each 5 to 6 membered heterocycloalkyl is selected from pyrrolidine and piperidine, and wherein each 5 to 6 membered heterocycloalkyl is further optionally substituted with one or more -C₁-C₂ alkyl;
wherein R¹² is independently selected from -CO₂R¹³, and -NR¹⁴R¹⁵;
wherein R¹³, R¹⁴ and R¹⁵ are each independently -C₁-C₂ alkyl;
R⁵¹ and R⁵² are each independently hydrogen, -C₁-C₄ alkyl, -C₅-C₆ cycloalkyl, -C₁-C₃alkylene-C₅-C₆ cycloalkyl, 5 to 6 membered heterocycloalkyl, -C₁-C₃alkylene-5 to 6 membered heterocycloalkyl, -C₆ aryl, or -C₁-C₃alkylene-C₆ aryl;
wherein each alkyl and cycloalkyl is optionally substituted with one or more R⁵³;
wherein each heterocycloalkyl is selected from pyrrolidine, piperidine, piperazine, and morpholine, and wherein each heterocycle is optionally substituted with one or more -C₁-C₂ alkyl or -C₁-C₂alkylene-phenyl;
wherein each -C₆ aryl is optionally substituted with one or more -C₁-C₂ alkyl or -C₁-C₄alkylene-5 to 6-membered heterocycloalkyl, and wherein said 5 to 6-membered heterocycloalkyl is optionally substituted with one or more -C₁-C₂ alkyl;
R⁵³ is independently -OR⁵⁴, -CO₂R⁵⁴, or -NR⁵⁵R⁵⁶;
R⁵⁴ is -C₁-C₂ alkyl; and
R⁵⁵ and R⁵⁶ are each independently -H, -C₁-C₂ alkyl, phenyl, or -C₁-C₂alkylene-phenyl; or
R⁵¹ and R⁵², together with the nitrogen atom to which they are attached, combine to form a 3-15 membered heterocycloalkyl, wherein said heterocycloalkyl is optionally fused to one or more additional aryl or heteroaryl rings or said heterocycloalkyl is optionally connected at a single atom to another cycloalkyl or heterocycloalkyl to form a 10- to 15-membered spirocyclic heterocycle, or said heterocycloalkyl optionally comprises a C₂ bridging alkylene; and wherein said 3-15 membered heterocycloalkyl is optionally substituted with one or more, preferably one or two, R⁷³;
R⁷³ is independently selected from -C₁-C₄ alkyl, -C₃-C₆ cycloalkyl, -C₁-C₃ alkylene-C₃-C₆ cycloalkyl, -C₆-C₁₀ aryl, -C₁-C₃ alkylene-C₆-C₁₀ aryl, -C(O)-C₆-C₁₀ aryl, 3-10 membered heterocycloalkyl, -C₁-C₃ alkylene-3-10 membered heterocycloalkyl, 5-10 membered heteroaryl, -C₁-C₃ alkylene-5-10 membered heteroaryl, -CH(OH)-5-10 membered heteroaryl, - NR⁷⁵R⁷⁶, -OR⁷⁴, or oxo;
wherein said -C₁-C₄ alkyl is optionally substituted with one or more -N(C₁-C₂ alkyl)₂, - OH, -OCH₂CH₂OH, or phenyl;
wherein each aryl is optionally substituted with one or more -C₁-C₂ alkyl, -C₁-C₂ haloalkyl, phenyl, halogen, -OCH₃, or -SO₂NH₂;
wherein each heterocycloalkyl is optionally substituted with one or more -C₁-C₂ alkyl or oxo;
wherein each heteroaryl is optionally substituted with one or more oxo, -C₁-C₂ alkyl, - C₁-C₂ haloalkyl, or halogen;
R⁷⁵ and R⁷⁶ are each independently -H, -C₁-C₂ alkyl, -CO₂C₁-C₄ alkyl, -SO₂phenyl, - C₆-C₁₀ aryl, or 5- to 6-membered heteroaryl, wherein each -C₆-C₁₀ aryl or 5- to 6-membered heteroaryl is optionally substituted with one or more -C₁-C₂ alkyl, halogen -OC₁-C₂ alkyl, or - CN; and
R⁷⁴ is independently -H, -C₆-C₁₀ aryl or -C₅-C₆ cycloalkyl, wherein said cycloalkyl is optionally substituted by one or more -C₁-C₂ alkyl.

In some embodiments, R¹¹ is selected from -C₁-C₄ alkyl, 5 to 6 membered heterocycloalkyl, -C₁-C₂alkylene-5 to 6 membered heterocycloalkyl, or -C₁-C₂alkylene-phenyl; wherein each alkyl is optionally substituted with one or more R¹², preferably with exactly one R¹², and wherein each 5 to 6 membered heterocycloalkyl is selected from pyrrolidine and piperidine, and wherein each 5 to 6 membered heterocycloalkyl is further optionally substituted with one or more -C₁-C₂ alkyl;
wherein R¹² is independently selected from -CO₂R¹³, and -NR¹⁴R¹⁵;
wherein R¹³, R¹⁴ and R¹⁵ are each independently -C₁-C₂ alkyl;
R⁵¹ and R⁵² are each independently hydrogen, -C₁-C₄ alkyl, -C₅-C₆ cycloalkyl, -C₁-C₃alkylene-C₅-C₆ cycloalkyl, 5 to 6 membered heterocycloalkyl, -C₁-C₃alkylene-5 to 6 membered heterocycloalkyl, -C₆ aryl, or -C₁-C₃alkylene-C₆ aryl;
wherein each alkyl and cycloalkyl is optionally substituted with one or more R⁵³;
wherein each heterocycloalkyl is selected from pyrrolidine, piperidine, piperazine, and morpholine, and wherein each heterocycle is optionally substituted with one or more -C₁-C₂ alkyl or -C₁-C₂alkylene-phenyl;
wherein each -C₆ aryl is optionally substituted with one or more -C₁-C₂ alkyl or -C₁-C₄alkylene-5 to 6-membered heterocycloalkyl, wherein said heterocycloalkyl is selected from pyrrolidine, piperidine, piperazine, and morpholine, and wherein said 5 to 6-membered heterocycloalkyl is optionally substituted with one or more -C₁-C₂ alkyl;
R⁵³ is independently -OR⁵⁴, -CO₂R⁵⁴, or -NR⁵⁵R⁵⁶;
R⁵⁴ is -C₁-C₂ alkyl; and
R⁵⁵ and R⁵⁶ are each independently -H, -C₁-C₂ alkyl, phenyl, or -C₁-C₂alkylene-phenyl; or
R⁵¹ and R⁵², together with the nitrogen atom to which they are attached, combine to form a membered heterocycloalkyl, wherein said heterocycloalkyl is selected from azetidine, pyrrolidine, imidazolidine, piperidine, piperazine, morpholine, or 1,4-diazepane, wherein said azetidine, pyrrolidine, imidazolidine, piperidine, piperazine, morpholine, or 1,4-diazepane is optionally fused to one or more additional aryl or heteroaryl rings or said heterocycloalkyl is optionally connected at a single atom to another cycloalkyl or heterocycloalkyl to form a 10- to 15-membered spirocyclic heterocycle, or said heterocycle is piperidine optionally comprising a C₂ bridging alkylene; and wherein said heterocycloalkyl is further optionally substituted with one or more, preferably one or two, R⁷³;
R⁷³ is independently selected from -C₁-C₄ alkyl, -C₃-C₆ cycloalkyl, -C₁-C₃ alkylene-C₃-C₆ cycloalkyl, -C₆-C₁₀ aryl, -C₁-C₃ alkylene-C₆-C₁₀ aryl, -C(O)-C₆-C₁₀ aryl, 3-10 membered heterocycloalkyl, -C₁-C₃ alkylene-3-10 membered heterocycloalkyl, 5-10 membered heteroaryl, -C₁-C₃ alkylene-5-10 membered heteroaryl, -CH(OH)-5-10 membered heteroaryl, - NR⁷⁵R⁷⁶, -OR⁷⁴, or oxo;
wherein said -C₁-C₄ alkyl is optionally substituted with one or more -N(C₁-C₂ alkyl)₂, - OH, -OCH₂CH₂OH, or phenyl;
wherein each aryl is optionally substituted with one or more -C₁-C₂ alkyl, -CF₃, phenyl, halogen, -OCH₃, or -SO₂NH₂;
wherein each heterocycloalkyl is optionally substituted with one or more -C₁-C₂ alkyl or oxo;
wherein each heteroaryl is optionally substituted with one or more oxo, -C₁-C₂ alkyl, - CF₃, or halogen;
R⁷⁵ and R⁷⁶ are each independently -H, -C₁-C₂ alkyl, -CO₂C₁-C₄ alkyl, -SO₂phenyl, - C₆-C₁₀ aryl, or 5- to 6-membered heteroaryl, wherein each -C₆-C₁₀ aryl or 5- to 6-membered heteroaryl is optionally substituted with one or more -C₁-C₂ alkyl, halogen -OC₁-C₂ alkyl, or - CN; and
R⁷⁴ is independently -H, -C₆-C₁₀ aryl or -C₅-C₆ cycloalkyl, wherein said cycloalkyl is optionally substituted by one or more -C₁-C₂ alkyl.

In some embodiments, R¹¹ is selected from -C₁-C₄ alkyl, 5 to 6 membered heterocycloalkyl, -C₁-C₂alkylene-5 to 6 membered heterocycloalkyl, or -C₁-C₂alkylene-phenyl; wherein each alkyl is optionally substituted with one or more R¹², preferably with exactly one R¹², and wherein each 5 to 6 membered heterocycloalkyl is selected from pyrrolidine and piperidine, and wherein each 5 to 6 membered heterocycloalkyl is further optionally substituted with one or more -C₁-C₂ alkyl;
wherein R¹² is independently selected from -CO₂R¹³, and -NR¹⁴R¹⁵;
wherein R¹³, R¹⁴ and R¹⁵ are each independently -C₁-C₂ alkyl;
R⁵¹ and R⁵² are each independently hydrogen, -C₁-C₄ alkyl, -C₅-C₆ cycloalkyl, -C₁-C₃alkylene-C₅-C₆ cycloalkyl, 5 to 6 membered heterocycloalkyl, -C₁-C₃alkylene-5 to 6 membered heterocycloalkyl, -C₆ aryl, or -C₁-C₃alkylene-C₆ aryl;
wherein each alkyl and cycloalkyl is optionally substituted with one or more R⁵³;
wherein each heterocycloalkyl is selected from pyrrolidine, piperidine, and piperazine, and wherein each heterocycle is optionally substituted with one or more -C₁-C₂ alkyl or -C₁-C₂alkylene-phenyl;
wherein each -C₆ aryl is optionally substituted with one or more -C₁-C₂ alkyl or -C₁-C₄alkylene-5 to 6-membered heterocycloalkyl, wherein said heterocycloalkyl is selected from piperidine and piperazine, and wherein said 5 to 6-membered heterocycloalkyl is optionally substituted with one or more -C₁-C₂ alkyl;
R⁵³ is independently -OR⁵⁴, -CO₂R⁵⁴, or -NR⁵⁵R⁵⁶;
R⁵⁴ is -C₁-C₂ alkyl; and
R⁵⁵ and R⁵⁶ are each independently -H, -C₁-C₂ alkyl, phenyl, or -C₁-C₂alkylene-phenyl; or
R⁵¹ and R⁵², together with the nitrogen atom to which they are attached, combine to form a membered heterocycloalkyl, wherein said heterocycloalkyl is selected from azetidine, pyrrolidine, imidazolidine, piperidine, piperazine, morpholine, or 1,4-diazepane, wherein said azetidine, pyrrolidine, imidazolidine, piperidine, piperazine, morpholine, or 1,4-diazepane is optionally fused to one or more additional aryl or heteroaryl rings or said heterocycloalkyl is optionally connected at a single atom to another cycloalkyl or heterocycloalkyl to form a 10- to 15-membered spirocyclic heterocycle, or said heterocycle is piperidine optionally comprising a C₂ bridging alkylene; and wherein said heterocycloalkyl is further optionally substituted with one or more, preferably one or two, R⁷³, and wherein when said heterocycloalkyl is azetidine, said azetidine is substituted at the 3-position and when said heterocycloalkyl is piperidine or piperazine, said piperidine or piperazine is substituted at the 4-position;
R⁷³ is independently selected from -C₁-C₄ alkyl, -C₃-C₆ cycloalkyl, -C₁-C₃ alkylene-C₃-C₆ cycloalkyl, -C₆-C₁₀ aryl, -C₁-C₃ alkylene-C₆-C₁₀ aryl, -C(O)-C₆-C₁₀ aryl, 3-10 membered heterocycloalkyl, -C₁-C₃ alkylene-3-10 membered heterocycloalkyl, 5-10 membered heteroaryl, -C₁-C₃ alkylene-5-10 membered heteroaryl, -CH(OH)-5-10 membered heteroaryl, - NR⁷⁵R⁷⁶, -OR⁷⁴, or oxo;
wherein said -C₁-C₄ alkyl is optionally substituted with one or more -N(C₁-C₂ alkyl)₂, - OH, -OCH₂CH₂OH, or phenyl;
wherein each aryl is optionally substituted with one or more -C₁-C₂ alkyl, -CF₃, phenyl, halogen, -OCH₃, or -SO₂NH₂;
wherein each heterocycloalkyl is optionally substituted with one or more -C₁-C₂ alkyl or oxo;
wherein each heteroaryl is optionally substituted with one or more oxo, -C₁-C₂ alkyl, - CF₃, or halogen;
R⁷⁵ and R⁷⁶ are each independently -H, -C₁-C₂ alkyl, -CO₂C₁-C₄ alkyl, -SO₂phenyl, - C₆-C₁₀ aryl, or 5- to 6-membered heteroaryl, wherein each -C₆-C₁₀ aryl or 5- to 6-membered heteroaryl is optionally substituted with one or more -C₁-C₂ alkyl, halogen -OC₁-C₂ alkyl, or - CN; and
R⁷⁴ is independently -H, -C₆-C₁₀ aryl or -C₅-C₆ cycloalkyl, wherein said cycloalkyl is optionally substituted by one or more -C₁-C₂ alkyl.
In some embodiments, R¹¹ is selected from -C₁-C₄ alkyl, 5 to 6 membered heterocycloalkyl, -C₁-C₂alkylene-5 to 6 membered heterocycloalkyl, or -C₁-C₂alkylene-phenyl; wherein each alkyl is optionally substituted with one or more R¹², preferably with exactly one R¹², and wherein each 5 to 6 membered heterocycloalkyl is selected from pyrrolidine and piperidine, and wherein each 5 to 6 membered heterocycloalkyl is further optionally substituted with one or more -C₁-C₂ alkyl;
wherein R¹² is independently selected from -CO₂R¹³, and -NR¹⁴R¹⁵;
wherein R¹³, R¹⁴ and R¹⁵ are each independently -C₁-C₂ alkyl;
R⁵¹ is hydrogen, and R⁵² is independently hydrogen, -C₁-C₄ alkyl, -C₅-C₆ cycloalkyl, - C₁-C₃alkylene-C₅-C₆ cycloalkyl, 5 to 6 membered heterocycloalkyl, -C₁-C₃alkylene-5 to 6 membered heterocycloalkyl, -C₆ aryl, or -C₁-C₃alkylene-C₆ aryl;
wherein each alkyl and cycloalkyl is optionally substituted with one or more R⁵³;
wherein each heterocycloalkyl is selected from pyrrolidine, piperidine, and piperazine, and wherein each heterocycle is optionally substituted with one or more -C₁-C₂ alkyl or -C₁-C₂alkylene-phenyl;
wherein each -C₆ aryl is optionally substituted with one or more -C₁-C₂ alkyl or -C₁-C₄alkylene-5 to 6-membered heterocycloalkyl, wherein said heterocycloalkyl is selected from piperidine and piperazine, and wherein said 5 to 6-membered heterocycloalkyl is optionally substituted with one or more -C₁-C₂ alkyl;
R⁵³ is independently -OR⁵⁴, -CO₂R⁵⁴, or -NR⁵⁵R⁵⁶;
R⁵⁴ is -C₁-C₂ alkyl; and
R⁵⁵ and R⁵⁶ are each independently -H, -C₁-C₂ alkyl, phenyl, or -C₁-C₂alkylene-phenyl; or
R⁵¹ and R⁵², together with the nitrogen atom to which they are attached, combine to form a membered heterocycloalkyl, wherein said heterocycloalkyl is selected from azetidine, pyrrolidine, imidazolidine, piperidine, piperazine, morpholine, or 1,4-diazepane, wherein said azetidine, pyrrolidine, imidazolidine, piperidine, piperazine, morpholine, or 1,4-diazepane is optionally fused to one or more additional aryl or heteroaryl rings or said heterocycloalkyl is optionally connected at a single atom to another cycloalkyl or heterocycloalkyl to form a 10- to 15-membered spirocyclic heterocycle; and wherein said heterocycloalkyl is further optionally substituted with one or more, preferably one or two, R⁷³, and wherein when said heterocycloalkyl is azetidine, said azetidine is substituted at the 3-position and when said heterocycloalkyl is piperidine or piperazine, said piperidine or piperazine is substituted at the 4-position;
R⁷³ is independently selected from -C₁-C₄ alkyl, -C₃-C₆ cycloalkyl, -C₁-C₃ alkylene-C₃-C₆ cycloalkyl, -C₆-C₁₀ aryl, -C₁-C₃ alkylene-C₆-C₁₀ aryl, -C(O)-C₆-C₁₀ aryl, 3-10 membered heterocycloalkyl, -C₁-C₃ alkylene-3-10 membered heterocycloalkyl, 5-10 membered heteroaryl, -C₁-C₃ alkylene-5-10 membered heteroaryl, -CH(OH)-5-10 membered heteroaryl, - NR⁷⁵R⁷⁶, -OR⁷⁴, or oxo;
wherein said -C₁-C₄ alkyl is optionally substituted with one or more -N(C₁-C₂ alkyl)₂, - OH, -OCH₂CH₂OH, or phenyl;
wherein each aryl is optionally substituted with one or more -C₁-C₂ alkyl, -CF₃, phenyl, halogen, -OCH₃, or -SO₂NH₂;
wherein each heterocycloalkyl is optionally substituted with one or more -C₁-C₂ alkyl or oxo;
wherein each heteroaryl is optionally substituted with one or more oxo, -C₁-C₂ alkyl, - CF₃, or halogen;
R⁷⁵ and R⁷⁶ are each independently -H, -C₁-C₂ alkyl, -CO₂C₁-C₄ alkyl, -SO₂phenyl, - C₆-C₁₀ aryl, or 5- to 6-membered heteroaryl, wherein each -C₆-C₁₀ aryl or 5- to 6-membered heteroaryl is optionally substituted with one or more -C₁-C₂ alkyl, halogen -OC₁-C₂ alkyl, or - CN; and
R⁷⁴ is independently -H, -C₆-C₁₀ aryl or -C₅-C₆ cycloalkyl, wherein said cycloalkyl is optionally substituted by one or more -C₁-C₂ alkyl.

In some embodiments, R¹¹ is selected from -C₁-C₄ alkyl, 5 to 6 membered heterocycloalkyl, -C₁-C₂alkylene-5 to 6 membered heterocycloalkyl, or -C₁-C₂alkylene-phenyl; wherein each alkyl is optionally substituted with one or more R¹², preferably with exactly one R¹², and wherein each 5 to 6 membered heterocycloalkyl is selected from pyrrolidine and piperidine, and wherein each 5 to 6 membered heterocycloalkyl is further optionally substituted with one or more -C₁-C₂ alkyl;
wherein R¹² is independently selected from -CO₂R¹³, and -NR¹⁴R¹⁵;
wherein R¹³, R¹⁴ and R¹⁵ are each independently -C₁-C₂ alkyl;
R⁵¹ is hydrogen, and R⁵² is independently hydrogen, -C₁-C₄ alkyl, -C₅-C₆ cycloalkyl, - C₁-C₃alkylene-C₅-C₆ cycloalkyl, 5 to 6 membered heterocycloalkyl, -C₁-C₃alkylene-5 to 6 membered heterocycloalkyl, -C₆ aryl, or -C₁-C₃alkylene-C₆ aryl;
wherein each alkyl and cycloalkyl is optionally substituted with one or more R⁵³;
wherein each heterocycloalkyl is selected from pyrrolidine, piperidine, and piperazine, and wherein each heterocycle is optionally substituted with one or more -C₁-C₂ alkyl or -C₁-C₂alkylene-phenyl;
wherein each -C₆ aryl is optionally substituted with one or more -C₁-C₂ alkyl or -C₁-C₄alkylene-5 to 6-membered heterocycloalkyl, wherein said heterocycloalkyl is selected from piperidine and piperazine, and wherein said 5 to 6-membered heterocycloalkyl is optionally substituted with one or more -C₁-C₂ alkyl;
R⁵³ is independently -OR⁵⁴, -CO₂R⁵⁴, or -NR⁵⁵R⁵⁶;
R⁵⁴ is -C₁-C₂ alkyl; and
R⁵⁵ and R⁵⁶ are each independently -H, -C₁-C₂ alkyl, phenyl, or -C₁-C₂alkylene-phenyl; or
R⁵¹ and R⁵², together with the nitrogen atom to which they are attached, combine to form a membered heterocycloalkyl, wherein said heterocycloalkyl is selected from azetidine, pyrrolidine, imidazolidine, piperidine, piperazine, morpholine, or 1,4-diazepane, wherein said heterocycloalkyl is further optionally substituted with one or more, preferably one or two, R⁷³;
R⁷³ is independently selected from -C₁-C₄ alkyl, -C₃-C₆ cycloalkyl, -C₁-C₃ alkylene-C₃-C₆ cycloalkyl, -C₆-C₁₀ aryl, -C₁-C₃ alkylene-C₆-C₁₀ aryl, -C(O)-C₆-C₁₀ aryl, 3-10 membered heterocycloalkyl, -C₁-C₃ alkylene-3-10 membered heterocycloalkyl, 5-10 membered heteroaryl, -C₁-C₃ alkylene-5-10 membered heteroaryl, -CH(OH)-5-10 membered heteroaryl, - NR⁷⁵R⁷⁶, -OR⁷⁴, or oxo;
wherein said -C₁-C₄ alkyl is optionally substituted with one or more -N(C₁-C₂ alkyl)₂, - OH, -OCH₂CH₂OH, or phenyl;
wherein each aryl is optionally substituted with one or more -C₁-C₂ alkyl, -CF₃, phenyl, halogen, -OCH₃, or -SO₂NH₂;
wherein each heterocycloalkyl is optionally substituted with one or more -C₁-C₂ alkyl or oxo;
wherein each heteroaryl is optionally substituted with one or more oxo, -C₁-C₂ alkyl, - CF₃, or halogen;
R⁷⁵ and R⁷⁶ are each independently -H, -C₁-C₂ alkyl, -CO₂C₁-C₄ alkyl, -SO₂phenyl, - C₆-C₁₀ aryl, or 5- to 6-membered heteroaryl, wherein each -C₆-C₁₀ aryl or 5- to 6-membered heteroaryl is optionally substituted with one or more -C₁-C₂ alkyl, halogen -OC₁-C₂ alkyl, or - CN; and
R⁷⁴ is independently -H, -C₆-C₁₀ aryl or -C₅-C₆ cycloalkyl, wherein said cycloalkyl is optionally substituted by one or more -C₁-C₂ alkyl.
In some embodiments, X¹ is -OR¹¹.

In some embodiments, the compound is of the Formula I-B1, Formula I-C1, Formula I-D1, Formula I-E1, I-F1, or I-G1: 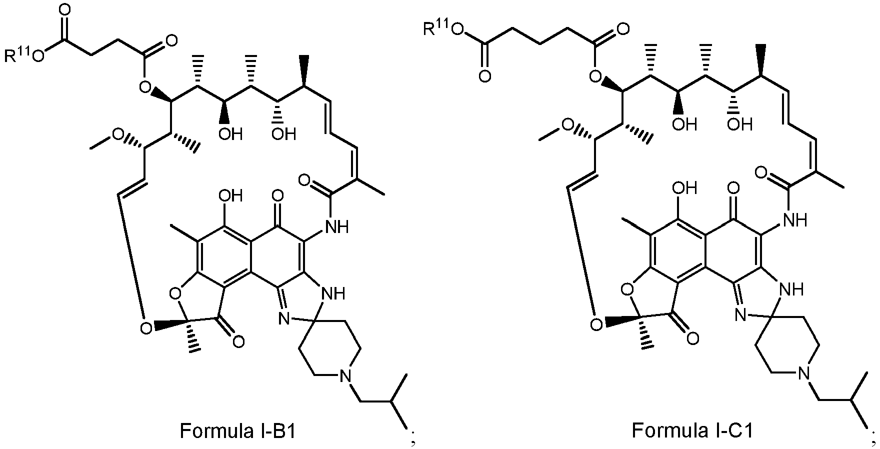 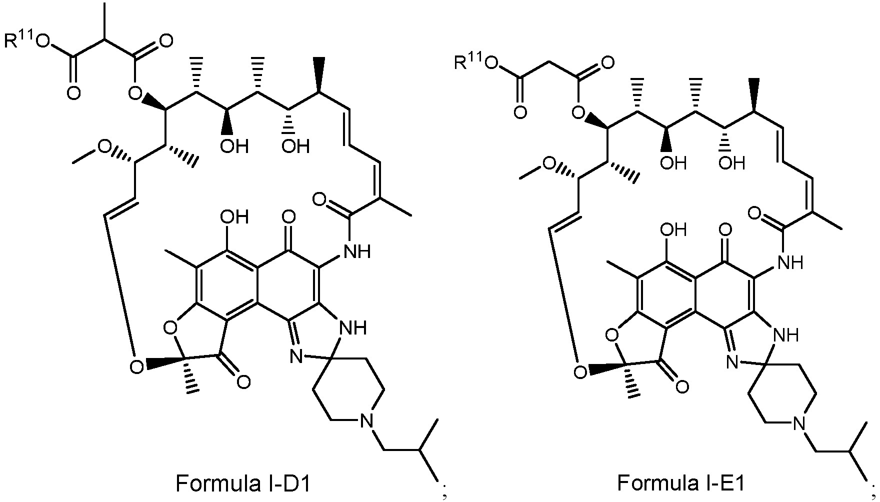 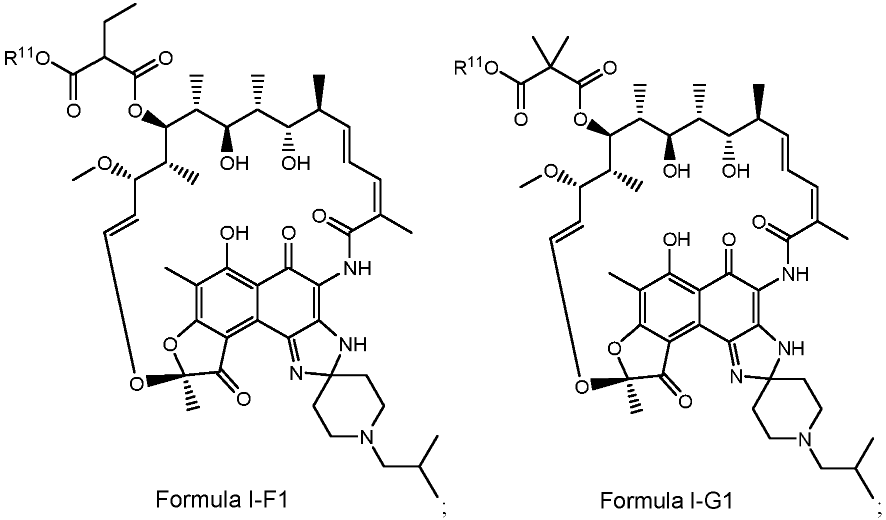 wherein R¹¹ is as defined in Formula I.

In some embodiments of any of Formula I, Formula I-A, Formula I-B, Formula I-C, Formula I-D, Formula I-E, Formula I-F, Formula I-G, Formula I-A1, Formula I-B1, Formula I-C1, Formula I-D1, Formula I-E1, Formula I-F1, or Formula I-G1,
R¹¹ is -C₁-C₆ alkyl, -C₂-C₆ alkenyl, or -C₂-C₆ alkynyl, wherein each alkyl, alkenyl, or alkynyl is optionally substituted with one or more, preferably one or two, further preferably with exactly one R¹²; preferably wherein R¹¹ is -C₁-C₄ alkyl, -C₂-C₄ alkenyl, or -C₂-C₄ alkynyl, wherein each alkyl, alkenyl, or alkynyl is optionally substituted with one or more, preferably one or two, further preferably with exactly one R¹²; more preferably wherein R¹¹ is -C₁-C₃ alkyl, -C₂-C₃ alkenyl, or -C₂-C₃ alkynyl, wherein each alkyl, alkenyl, or alkynyl is optionally substituted with one or more, preferably one or two, further preferably with exactly one R¹²; yet more preferably wherein R¹¹ is -C₁-C₂ alkyl, -C₂ alkenyl, or -C₂ alkynyl, wherein each alkyl, alkenyl, or alkynyl is optionally substituted with one or more, preferably one or two, further preferably with exactly one R¹².

In some embodiments, R¹¹ is -C₁-C₆ alkyl optionally substituted with one or more, preferably one or two, further preferably with exactly one R¹²; preferably wherein R¹¹ is -C₁-C₄ alkyl optionally substituted with one or more, preferably one or two, further preferably with exactly one R¹²; more preferably wherein R¹¹ is -C₁-C₃ alkyl optionally substituted with one or more, preferably one or two, further preferably with exactly one R¹²; yet more preferably wherein R¹¹ is -C₁-C₂ alkyl optionally substituted with one or more, preferably one or two, further preferably with exactly one R¹².

In some embodiments, R¹¹ is -C₁-C₄ alkyl, preferably -C₁-C₃ alkyl, or 3-10 membered heterocycloalkyl, wherein each alkyl or heterocycloalkyl is optionally substituted with one or more R¹²; wherein R¹² is independently -C₁-C₆ alkyl, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, halogen, cyano, oxo, -OR¹³, -CO₂R¹³, or -NR¹⁴R¹⁵, wherein each alkyl, alkenyl, alkynyl, cycloalkyl, cycloalkenyl, heterocycloalkyl, aryl or heteroaryl is optionally substituted with one or more R¹⁶.

In some embodiments, R¹¹ is -C₁-C₄ alkyl, preferably -C₁-C₃ alkyl, or 3-10 membered heterocycloalkyl, wherein each alkyl or heterocycloalkyl is optionally substituted with one or more R¹²; wherein R¹² is independently -C₁-C₆ alkyl, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, halogen, cyano, oxo, -OR¹³, -CO₂R¹³, or -NR¹⁴R¹⁵, wherein each alkyl, alkenyl, alkynyl, cycloalkyl, cycloalkenyl, heterocycloalkyl, aryl or heteroaryl is optionally substituted with one or more, preferably one or two, preferably exactly one R¹⁶; wherein R¹³, R¹⁴ and R¹⁵ are each independently hydrogen, -C₁-C₆ alkyl, -C₃-C₆ cycloalkyl, -C₅-C₈ cycloalkenyl, 3-10 membered heterocycloalkyl, -C₆-C₁₀ aryl, or 5-10 membered heteroaryl, wherein each cycloalkyl, cycloalkenyl, heterocycloalkyl, aryl or heteroaryl is optionally substituted with one or more, preferably one or two, further preferably with exactly one R²⁰; wherein R¹⁶ and R²⁰ are independently selected from -C₁-C₄ alkyl, halogen, cyano, -C₁-C₄ alkoxy, or -C₁-C₂ haloalkyl.

In some embodiments, R¹¹ is -C₁-C₄ alkyl, preferably -C₁-C₃ alkyl, or 3-6 membered heterocycloalkyl, wherein each alkyl is optionally substituted with one or more R¹² and each heterocycloalkyl is optionally substituted with one or more -C₁-C₄ alkyl; wherein R¹² is independently -C₃-C₆ cycloalkyl, -C₅-C₈ cycloalkenyl, 3-6 membered heterocycloalkyl, phenyl, 5-10 membered heteroaryl, -C₁-C₄ alkoxy, -C₁-C₄ haloalkyl, halogen, -OR¹³, -CO₂R¹³, or - NR¹⁴R¹⁵, wherein each cycloalkyl, cycloalkenyl, heterocycloalkyl, phenyl or heteroaryl is optionally substituted with one or more, preferably one or two, preferably exactly one R¹⁶; wherein R¹³, R¹⁴ and R¹⁵ are each independently hydrogen, or -C₁-C₄ alkyl; and wherein R¹⁶ is independently selected from -C₁-C₄ alkyl, halogen, cyano, -C₁-C₄ alkoxy, or -C₁-C₂ haloalkyl.

In some embodiments, R¹¹ is -C₁-C₄ alkyl, preferably -C₁-C₃ alkyl, or 3-6 membered heterocycloalkyl, wherein each alkyl is optionally substituted with one or more R¹² and each heterocycloalkyl is optionally substituted with one or more -C₁-C₄ alkyl; wherein R¹² is independently 3-6 membered heterocycloalkyl, phenyl, -OR¹³, -CO₂R¹³, or -NR¹⁴R¹⁵, wherein each heterocycloalkyl or phenyl is optionally substituted with one or more, preferably one or two, preferably exactly one R¹⁶; wherein R¹³, R¹⁴ and R¹⁵ are each independently hydrogen or -C₁-C₄ alkyl; and wherein R¹⁶ is independently -C₁-C₄ alkyl.

In some embodiments, R¹¹ is -C₁-C₄ alkyl, preferably -C₁-C₃ alkyl, or 5-6 membered heterocycloalkyl, wherein each alkyl is optionally substituted with one or more R¹² and each heterocycloalkyl is optionally substituted with one or more -C₁-C₂ alkyl; wherein R¹² is independently 5-6 membered heterocycloalkyl, phenyl, -CO₂R¹³, or -NR¹⁴R¹⁵, wherein each heterocycloalkyl or phenyl is optionally substituted with exactly one R¹⁶; wherein R¹³, R¹⁴ and R¹⁵ are each independently hydrogen or -C₁-C₂ alkyl; and wherein R¹⁶ is independently -C₁-C₂ alkyl, preferably methyl.

In some embodiments, R¹¹ is -C₁-C₄ alkyl, preferably -C₁-C₃ alkyl, or 5-6 membered heterocycloalkyl comprising one or two heteroatoms selected from N and O, wherein each alkyl is optionally substituted with one or more R¹² and each heterocycloalkyl is optionally substituted with one or more -C₁-C₂ alkyl; wherein R¹² is independently 5-6 membered heterocycloalkyl, phenyl, -CO₂R¹³, or -NR¹⁴R¹⁵, wherein each heterocycloalkyl or phenyl is optionally substituted with exactly one R¹⁶; wherein said heterocycloalkyl does not comprise heteroatoms other than O and N, preferably wherein said 5-6 membered heterocycloalkyl contains 1 to 4 heteroatoms, preferably 1 to 3, further preferably 1 or 2 heteroatoms selected from O and N; wherein R¹³, R¹⁴ and R¹⁵ are each independently -C₁-C₂ alkyl; and wherein R¹⁶ is independently -C₁-C₂ alkyl, preferably methyl.

In some embodiments, R¹¹ is -C₁-C₄ alkyl, preferably -C₁-C₃ alkyl, or 5-6 membered heterocycloalkyl selected from piperidine, piperazine, and pyrrolidine, wherein each alkyl is optionally substituted with one or more R¹² and each heterocycloalkyl is optionally substituted with one or more -C₁-C₂ alkyl; wherein R¹² is independently 5-6 membered heterocycloalkyl, phenyl, -CO₂R¹³, or -NR¹⁴R¹⁵, wherein each heterocycloalkyl or phenyl is optionally substituted with exactly one R¹⁶; wherein said heterocycloalkyl does not comprise heteroatoms other than O and N, preferably wherein said 5-6 membered heterocycloalkyl is selected from pyrrolidine, piperazine, or morpholine; wherein R¹³, R¹⁴ and R¹⁵ are each independently -C₁-C₂ alkyl; and wherein R¹⁶ is independently -C₁-C₂ alkyl, preferably methyl.

In some embodiments, R¹¹ is -C₁-C₄ alkyl, preferably -C₁-C₃ alkyl, or heterocycle selected from piperidine and pyrrolidine, wherein each alkyl is optionally substituted with one or more R¹² and each piperidine or pyrrolidine is optionally substituted with one methyl; wherein R¹² is independently 5-6 membered heterocycloalkyl, phenyl, -CO₂R¹³, or -NR¹⁴R¹⁵, wherein each heterocycloalkyl or phenyl is optionally substituted with exactly one R¹⁶; wherein said heterocycloalkyl does not comprise heteroatoms other than O and N, preferably wherein said 5-6 membered heterocycloalkyl is selected from pyrrolidine, piperidine, piperazine, or morpholine, more preferably wherein said 5-6 membered heterocycle is selected from pyrrolidine and piperidine; wherein R¹³, R¹⁴ and R¹⁵ are each independently -C₁-C₂ alkyl; and wherein R¹⁶ is independently -C₁-C₂ alkyl, preferably methyl.

In some embodiments, R¹¹ is selected from -C₁-C₄ alkyl, 5 to 6 membered heterocycloalkyl, or -C₁-C₂alkylene-5 to 6 membered heterocycloalkyl; wherein each alkyl is optionally substituted with one or more R¹² and wherein each 5 to 6 membered heterocycloalkyl is optionally substituted with one or more -C₁-C₄ alkyl;
wherein R¹² is independently selected from phenyl, -CO₂R¹³, and -NR¹⁴R¹⁵; and
wherein R¹³, R¹⁴ and R¹⁵ are each independently -C₁-C₂ alkyl.

In some embodiments, R¹¹ is selected from -C₁-C₄ alkyl, 5 to 6 membered heterocycloalkyl, or -C₁-C₂alkylene-5 to 6 membered heterocycloalkyl; wherein each alkyl is optionally substituted with one or more R¹² and wherein each 5 to 6 membered heterocycloalkyl does not comprise heteroatoms other than O and N and wherein each 5 to 6 membered heterocycloalkyl is further optionally substituted with one or more -C₁-C₂ alkyl;
wherein R¹² is independently selected from phenyl, -CO₂R¹³, and -NR¹⁴R¹⁵;
wherein R¹³, R¹⁴ and R¹⁵ are each independently -C₁-C₂ alkyl.

In some embodiments, R¹¹ is selected from -C₁-C₄ alkyl, 5 to 6 membered heterocycloalkyl, or -C₁-C₂alkylene-5 to 6 membered heterocycloalkyl; wherein each alkyl is optionally substituted with one or more R¹², preferably with exactly one R¹², and wherein each 5 to 6 membered heterocycloalkyl is selected from pyrrolidine, piperidine, piperazine, and morpholine, and wherein each 5 to 6 membered heterocycloalkyl is further optionally substituted with one or more -C₁-C₂ alkyl;
wherein R¹² is independently selected from phenyl, -CO₂R¹³, and -NR¹⁴R¹⁵;
wherein R¹³, R¹⁴ and R¹⁵ are each independently -C₁-C₂ alkyl.

In some embodiments, R¹¹ is selected from -C₁-C₄ alkyl, 5 to 6 membered heterocycloalkyl, or -C₁-C₂alkylene-5 to 6 membered heterocycloalkyl; wherein each alkyl is optionally substituted with one or more R¹², preferably with exactly one R¹², and wherein each 5 to 6 membered heterocycloalkyl is selected from pyrrolidine and piperidine, and wherein each 5 to 6 membered heterocycloalkyl is further optionally substituted with one or more -C₁-C₂ alkyl;
wherein R¹² is independently selected from phenyl, -CO₂R¹³, and -NR¹⁴R¹⁵;
wherein R¹³, R¹⁴ and R¹⁵ are each independently -C₁-C₂ alkyl.

In some embodiments, R¹¹ is selected from -C₁-C₄ alkyl, 5 to 6 membered heterocycloalkyl, -C₁-C₂alkylene-5 to 6 membered heterocycloalkyl, or -C₁-C₂alkylene-phenyl; wherein each alkyl is optionally substituted with one or more R¹², preferably with exactly one R¹², and wherein each 5 to 6 membered heterocycloalkyl is selected from pyrrolidine and piperidine, and wherein each 5 to 6 membered heterocycloalkyl is further optionally substituted with one or more -C₁-C₂ alkyl;
wherein R¹² is independently selected from -CO₂R¹³, and -NR¹⁴R¹⁵;
wherein R¹³, R¹⁴ and R¹⁵ are each independently -C₁-C₂ alkyl.

In some embodiments, X¹ is -NR⁵¹NR⁵².

In some embodiments, the compound is of the Formula I-B2, Formula I-C2, Formula I-D2, Formula I-E2, Formula I-F2, or Formula I-G2: 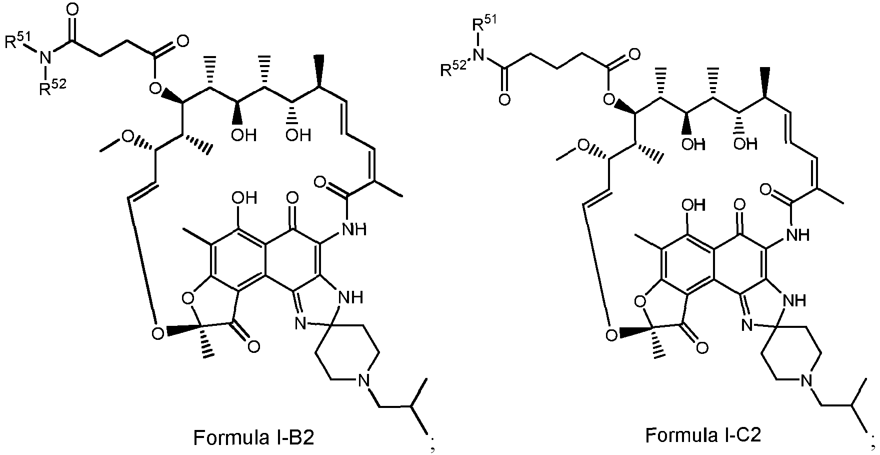 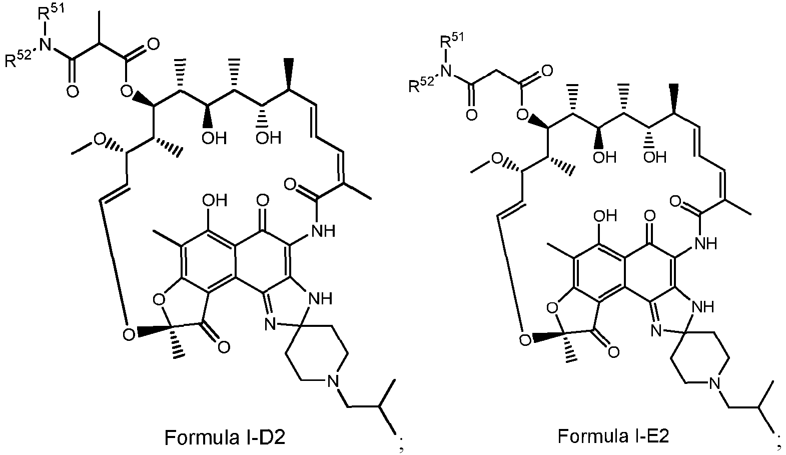 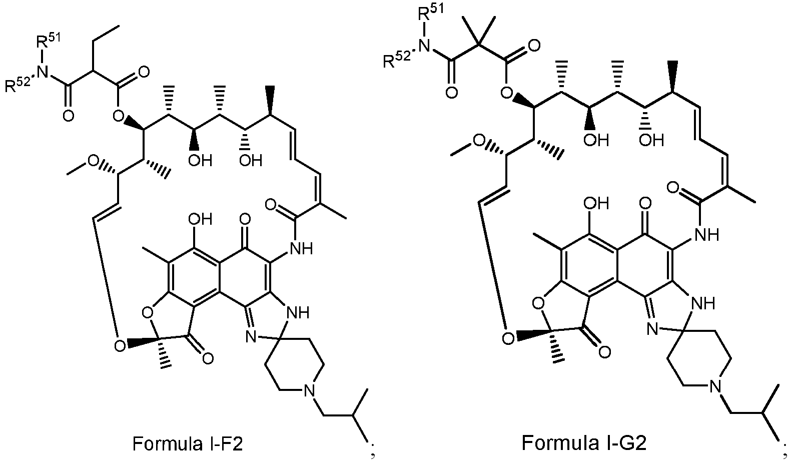 wherein R⁵¹ and R⁵² are as defined in Formula I.

In some embodiments of any of Formula I, Formula I-A, Formula I-B, Formula I-C, Formula I-D, Formula I-E, Formula I-F, Formula I-G, Formula I-A2, Formula I-B2, Formula I-C2, Formula I-D2, Formula I-E2, Formula I-F2, or Formula I-G2, R⁵¹ and R⁵² are each independently hydrogen, -C₁-C₆ alkyl, preferably -C₁-C₄ alkyl, more preferably -C₁-C₃ alkyl, 3-10 membered heterocycloalkyl, or -C₆-C₁₀ aryl, wherein each alkyl, heterocycloalkyl, or aryl is optionally substituted with one or more R⁵³; or
R⁵¹ and R⁵², together with the nitrogen atom to which they are attached, combine to form a 3-15 membered heterocycloalkyl or 5-15 membered heteroaryl, wherein the heterocycloalkyl or heteroaryl are optionally substituted with one or more R⁵³.

In some embodiments, R⁵¹ and R⁵² are each independently hydrogen, -C₁-C₆ alkyl, preferably -C₁-C₄ alkyl, more preferably -C₁-C₃ alkyl, 3-10 membered heterocycloalkyl, or - C₆-C₁₀ aryl, wherein each alkyl, heterocycloalkyl, or aryl is optionally substituted with one or more R⁵³; or
R⁵¹ and R⁵², together with the nitrogen atom to which they are attached, combine to form a 3-15 membered heterocycloalkyl or 5-15 membered heteroaryl, wherein the heterocycloalkyl or heteroaryl are optionally substituted with one or more R⁵³;
wherein R⁵³ is independently hydrogen, -C₁-C₆ alkyl, -C₂-C₆ alkenyl, -C₂-C₆ alkynyl, - C₃-C₆ cycloalkyl, -C₅-C₈ cycloalkenyl, 3-10 membered heterocycloalkyl, -C₆-C₁₀ aryl, 5-10 membered heteroaryl, halogen, cyano, oxo, -OR⁵⁴, -CO₂R⁵⁴, or -NR⁵⁵R⁵⁶, wherein each alkyl, alkenyl, alkynyl, cycloalkyl, cycloalkenyl, heterocycloalkyl, aryl, and heteroaryl is optionally substituted with one or more R⁵⁷.

In some embodiments, R⁵¹ and R⁵² are each independently hydrogen, -C₁-C₆ alkyl, preferably -C₁-C₄ alkyl, more preferably -C₁-C₃ alkyl, 3-10 membered heterocycloalkyl, or - C₆-C₁₀ aryl, wherein each alkyl, heterocycloalkyl, or aryl is optionally substituted with one or more R⁵³; or
R⁵¹ and R⁵², together with the nitrogen atom to which they are attached, combine to form a 3-15 membered heterocycloalkyl or 5-15 membered heteroaryl, wherein the heterocycloalkyl or heteroaryl are optionally substituted with one or more R⁵³;
wherein R⁵³ is independently -C₁-C₆ alkyl, -C₃-C₆ cycloalkyl, 3-10 membered heterocycloalkyl, -C₆-C₁₀ aryl, 5-10 membered heteroaryl, -OR⁵⁴, -CO₂R⁵⁴, or -NR⁵⁵R⁵⁶, wherein each alkyl, cycloalkyl, cycloalkenyl, heterocycloalkyl, aryl, or heteroaryl is optionally substituted with one or more R⁵⁷.

In some embodiments, R⁵¹ is hydrogen, or R⁵¹ and R⁵², together with the nitrogen atom to which they are attached, combine to form a 3-15 membered heterocycloalkyl or 5-15 membered heteroaryl, wherein the heterocycloalkyl or heteroaryl are optionally substituted with one or more R⁵⁷.

In some embodiments, R⁵¹ is hydrogen, and R⁵² is independently hydrogen, -C₁-C₆ alkyl, preferably -C₁-C₄ alkyl, more preferably -C₁-C₃ alkyl, 3-10 membered heterocycloalkyl, or phenyl, wherein each alkyl, heterocycloalkyl, or phenyl is optionally substituted with one or more R⁵³; or
R⁵¹ and R⁵², together with the nitrogen atom to which they are attached, combine to form a 3-15 membered heterocycloalkyl or 5-15 membered heteroaryl, wherein the heterocycloalkyl or heteroaryl are optionally substituted with one or more R⁵³;
wherein R⁵³ is independently -C₁-C₆ alkyl, -C₃-C₆ cycloalkyl, 3-10 membered heterocycloalkyl, phenyl, 5-10 membered heteroaryl, -OR⁵⁴, -CO₂R⁵⁴, or -NR⁵⁵R⁵⁶, wherein each alkyl, cycloalkyl, heterocycloalkyl, phenyl or heteroaryl is optionally substituted with one or more R⁵⁷;
wherein R⁵⁴, R⁵⁵ and R⁵⁶ are each independently hydrogen, -C₁-C₆ alkyl, or phenyl, wherein said -C₁-C₆ alkyl is optionally substituted with one or more R⁶¹; or wherein R⁵⁵ and R⁵⁶ combine to form a 5-6 membered heterocycloalkyl optionally substituted with one or more R⁵⁷; and R⁵⁷. is -C₁-C₆ alkyl, oxo, -NR⁵⁹R⁶⁰, or SO₂NH₂, wherein said -C₁-C₆ alkyl is optionally substituted with one or more R⁶¹.

In some embodiments, R⁵¹ is hydrogen, and R⁵² is independently hydrogen, -C₁-C₆ alkyl, preferably -C₁-C₄ alkyl, more preferably -C₁-C₃ alkyl, 5-6 membered heterocycloalkyl, or phenyl, wherein each alkyl, heterocycloalkyl, or phenyl is optionally substituted with one or more, preferably one or two, preferably exactly one R⁵³; or
R⁵¹ and R⁵², together with the nitrogen atom to which they are attached, combine to form a 5-6 membered heterocycloalkyl or 5-6 membered heteroaryl, wherein the heterocycloalkyl or heteroaryl are optionally substituted with one or more, preferably one or two, preferably exactly one R⁵³;
wherein R⁵³ is independently -C₁-C₆ alkyl, preferably -C₁-C₄ alkyl, more preferably - C₁-C₃ alkyl, -C₅-C₆ cycloalkyl, 5-6 membered heterocycloalkyl, phenyl, 5-6 membered heteroaryl, -OR⁵⁴, -CO₂R⁵⁴, or -NR⁵⁵R⁵⁶, wherein each alkyl, cycloalkyl, heterocycloalkyl, phenyl or heteroaryl is optionally substituted with one or more, preferably one or two, more preferably exactly one R⁵⁷;
wherein R⁵⁴, R⁵⁵ and R⁵⁶ are each independently hydrogen, -C₁-C₆ alkyl, preferably - C₁-C₄ alkyl, more preferably -C₁-C₃ alkyl, or phenyl, wherein said -C₁-C₆ alkyl is optionally substituted with one or more R⁶¹; or wherein R⁵⁵ and R⁵⁶ combine to form a 5-6 membered heterocycloalkyl optionally substituted with one or more R⁵⁷; and R⁵⁷. is -C₁-C₆ alkyl, oxo, - NR⁵⁹R⁶⁰, SO₂NH₂, wherein said -C₁-C₆ alkyl is optionally substituted with one or more R⁶¹.

In some embodiments, R⁵¹ is hydrogen, and R⁵² is independently hydrogen, -C₁-C₆ alkyl, preferably -C₁-C₄ alkyl, more preferably -C₁-C₃ alkyl, 5-6 membered heterocycloalkyl wherein the heterocycloalkyl comprises no more than 1 to 4, preferably one to 3, more preferably one or two heteroatoms selected from O and N, or phenyl, wherein each alkyl, heterocycloalkyl, or phenyl is optionally substituted with one or more, preferably one or two, preferably exactly one R⁵³; or
R⁵¹ and R⁵², together with the nitrogen atom to which they are attached, combine to form a 5-6 membered heterocycloalkyl, wherein the heterocycloalkyl comprises no more than 1 to 4, preferably one to 3, more preferably one or two heteroatoms selected from O and N, and is optionally substituted with one or more, preferably one or two, preferably exactly one R⁵³;
wherein R⁵³ is independently -C₁-C₆ alkyl, preferably -C₁-C₄ alkyl, more preferably - C₁-C₃ alkyl, -C₅-C₆ cycloalkyl, 5-6 membered heterocycloalkyl wherein the heterocycloalkyl comprises no more than 1 to 4, preferably one to 3, more preferably one or two heteroatoms selected from O and N, phenyl, -OR⁵⁴, -CO₂R⁵⁴, or -NR⁵⁵R⁵⁶, wherein each alkyl, cylcloalkyl, heterocycloalkyl, or phenyl is optionally substituted with one or more, preferably one or two, more preferably exactly one R⁵⁷;
wherein R⁵⁴, R⁵⁵ and R⁵⁶ are each independently hydrogen, -C₁-C₆ alkyl, preferably - C₁-C₄ alkyl, more preferably -C₁-C₃ alkyl, or phenyl, wherein said -C₁-C₆ alkyl is optionally substituted with one or more R⁶¹; or wherein R⁵⁵ and R⁵⁶ combine to form a 5-6 membered heterocycloalkyl optionally substituted with one or more R⁵⁷; and R⁵⁷ is -C₁-C₆ alkyl, oxo, - NR⁵⁹R⁶⁰, or SO₂NH₂, wherein said -C₁-C₆ alkyl is optionally substituted with one or more R⁶¹.

In some embodiments, R⁵¹ is hydrogen, and R⁵² is independently hydrogen, -C₁-C₆ alkyl, preferably -C₁-C₄ alkyl, more preferably -C₁-C₃ alkyl, piperidine, piperazine, morpholine, or phenyl, wherein each alkyl, piperidine, piperazine, morpholine, or phenyl is optionally substituted with one or more, preferably one or two, preferably exactly one R⁵³; or
R⁵¹ and R⁵², together with the nitrogen atom to which they are attached, combine to form a piperidine, piperazine, or morpholine, wherein the piperidine, piperazine, or morpholine is optionally substituted with one or more, preferably one or two, preferably exactly one R⁵³;
wherein R⁵³ is independently -C₁-C₆ alkyl, preferably -C₁-C₄ alkyl, more preferably - C₁-C₃ alkyl, cyclohexane, piperidine, piperazine, morpholine, phenyl, -OR⁵⁴, -CO₂R⁵⁴, or - NR⁵⁵R⁵⁶, wherein each cyclohexane, piperidine, piperazine, morpholine, or phenyl is optionally substituted with one or more, preferably one or two, more preferably exactly one R⁵⁷;
wherein R⁵⁴, R⁵⁵ and R⁵⁶ are each independently hydrogen, -C₁-C₆ alkyl, preferably - C₁-C₄ alkyl, more preferably -C₁-C₃ alkyl, or phenyl; or wherein R⁵⁵ and R⁵⁶ combine to form a 5-6 membered heterocycloalkyl substituted with oxo and methyl; and R⁵⁷ is -C₁-C₆ alkyl, oxo, -NR⁵⁹R⁶⁰, SO₂NH₂, wherein said -C₁-C₆ alkyl is optionally substituted with one or more R⁶¹; wherein R⁵⁹ and R⁶⁰ are independently hydrogen or -C₁-C₃ alkyl optionally substituted by R⁶⁴; R⁶¹ is 6-membered heterocycloalkyl, and R⁶² is -C₁-C₃ alkyl, preferably -C₁-C₂ alkyl, preferably methyl; and R⁶⁴ is methyl or phenyl.

In some embodiments, R⁵¹ is hydrogen, and R⁵² is independently hydrogen, -C₁-C₆ alkyl, preferably -C₁-C₄ alkyl, more preferably -C₁-C₃ alkyl, piperidine, piperazine, morpholine, or phenyl, wherein each alkyl, piperidine, piperazine, morpholine, or phenyl is optionally substituted with one or more, preferably one or two, preferably exactly one R⁵³; or
R⁵¹ and R⁵², together with the nitrogen atom to which they are attached, combine to form a piperidine, piperazine, or morpholine, wherein the piperidine, piperazine, or morpholine is optionally substituted with one or more, preferably one or two, preferably exactly one R⁵³;
wherein R⁵³ is independently -C₁-C₆ alkyl, preferably -C₁-C₄ alkyl, more preferably - C₁-C₃ alkyl, cyclohexane, piperidine, piperazine, morpholine, phenyl, -OR⁵⁴, -CO₂R⁵⁴, or - NR⁵⁵R⁵⁶, wherein each cyclohexane, piperidine, piperazine, morpholine, phenyl or heteroaryl is optionally substituted with one or more, preferably one or two, more preferably exactly one R⁵⁷;
wherein R⁵⁴, R⁵⁵ and R⁵⁶ are each independently hydrogen, -C₁-C₆ alkyl, preferably - C₁-C₄ alkyl, more preferably -C₁-C₃ alkyl, or phenyl, wherein said -C₁-C₆ alkyl is optionally substituted with one or more phenyl; and R⁵⁷ is -C₁-C₆ alkyl, oxo, -NR⁵⁹R⁶⁰, or SO₂NH₂.

In some embodiments, R⁵¹ is hydrogen; and R⁵² is 3-10 membered heterocycloalkyl.

In some embodiments, R⁵¹ is hydrogen, and R⁵² is 5-6 membered heterocycloalkyl optionally substituted by one or more, preferably one or two, preferably exactly one methyl.

In some embodiments, R⁵¹ is hydrogen, and R⁵² is piperidine, piperazine, or morpholine optionally substituted by one or more, preferably one or two, preferably exactly one methyl.

In some embodiments, R⁵¹ is hydrogen; and R⁵² is an optionally substituted -C₃-C₆ cycloalkyl; preferably an optionally substituted -C₅-C₆ cycloalkyl.

In some embodiments, R⁵¹ is hydrogen, and R⁵² is cyclohexane optionally substituted by one or more, preferably one or two, preferably exactly one R⁵³, wherein R⁵³ is NR⁵⁵R⁵⁶, and R⁵⁵ and R⁵⁶ are each independently hydrogen, -C₁-C₆ alkyl, preferably methyl, wherein each - C₁-C₆ alkyl is optionally substituted by phenyl.

In some embodiments, R⁵¹ and R⁵² are both independently -C₆-C₁₀ aryl, preferably wherein R⁵¹ and R⁵² are both independently phenyl.

In some embodiments, R⁵¹ and R⁵² are each independently hydrogen, -C₁-C₆ alkyl, -C₃-C₆ cycloalkyl, -C₁-C₆alkylene-C₃-C₆ cycloalkyl, 3 to 10 membered heterocycloalkyl, -C₁-C₆alkylene-3 to 10 membered heterocycloalkyl, -C₆-C₁₀ aryl, or -C₁-C₆alkylene-C₆-C₁₀ aryl;
wherein each alkyl and cycloalkyl is optionally substituted with one or more R⁵³;
wherein each heterocycloalkyl is optionally substituted with one or more -C₁-C₆ alkyl or -C₁-C₆alkylene-phenyl;
wherein each aryl is optionally substituted with one or more -C₁-C₆ alkyl or -C₁-C₆alkylene-5 to 6-membered heterocycloalkyl, and wherein said 5 to 6-membered heterocycloalkyl is optionally substituted with one or more -C₁-C₆ alkyl;
R⁵³ is independently -OR⁵⁴, -CO₂R⁵⁴, or -NR⁵⁵R⁵⁶;
R⁵⁴ is -C₁-C₄ alkyl; and
R⁵⁵ and R⁵⁶ are each independently -H, -C₁-C₄ alkyl, phenyl, or -C₁-C₆alkylene-phenyl.

In some embodiments, R⁵¹ and R⁵² are each independently hydrogen, -C₁-C₄ alkyl, -C₃-C₆ cycloalkyl, -C₁-C₄alkylene-C₃-C₆ cycloalkyl, 3 to 10 membered heterocycloalkyl, -C₁-C₄alkylene-3 to 10 membered heterocycloalkyl, -C₆-C₁₀ aryl, or -C₁-C₄alkylene-C₆-C₁₀ aryl;
wherein each alkyl and cycloalkyl is optionally substituted with one or more R⁵³;
wherein each heterocycloalkyl is optionally substituted with one or more -C₁-C₆ alkyl or -C₁-C₄alkylene-phenyl;
wherein each aryl is optionally substituted with one or more -C₁-C₆ alkyl or -C₁-C₄alkylene-5 to 6-membered heterocycloalkyl, and wherein said 5 to 6-membered heterocycloalkyl is optionally substituted with one or more -C₁-C₄ alkyl;
R⁵³ is independently -OR⁵⁴, -CO₂R⁵⁴, or -NR⁵⁵R⁵⁶;
R⁵⁴ is -C₁-C₄ alkyl; and
R⁵⁵ and R⁵⁶ are each independently -H, -C₁-C₄ alkyl, phenyl, or -C₁-C₄alkylene-phenyl.

In some embodiments, R⁵¹ and R⁵² are each independently hydrogen, -C₁-C₄ alkyl, -C₅-C₆ cycloalkyl, -C₁-C₄alkylene-C₅-C₆ cycloalkyl, 5 to 6 membered heterocycloalkyl, -C₁-C₄alkylene-5 to 6 membered heterocycloalkyl, -C₆ aryl, or -C₁-C₄alkylene-C₆ aryl;
wherein each alkyl and cycloalkyl is optionally substituted with one or more R⁵³;
wherein each heterocycloalkyl is optionally substituted with one or more -C₁-C₄ alkyl or -C₁-C₄alkylene-phenyl;
wherein each -C₆ aryl is optionally substituted with one or more -C₁-C₄ alkyl or -C₁-C₄alkylene-5 to 6-membered heterocycloalkyl, and wherein said 5 to 6-membered heterocycloalkyl is optionally substituted with one or more -C₁-C₄ alkyl;
R⁵³ is independently -OR⁵⁴, -CO₂R⁵⁴, or -NR⁵⁵R⁵⁶;
R⁵⁴ is -C₁-C₄ alkyl; and
R⁵⁵ and R⁵⁶ are each independently -H, -C₁-C₄ alkyl, phenyl, or -C₁-C₄alkylene-phenyl.

In some embodiments, R⁵¹ and R⁵² are each independently hydrogen, -C₁-C₄ alkyl, -C₅-C₆ cycloalkyl, -C₁-C₃alkylene-C₅-C₆ cycloalkyl, 5 to 6 membered heterocycloalkyl, -C₁-C₃alkylene-5 to 6 membered heterocycloalkyl, -C₆ aryl, or -C₁-C₃alkylene-C₆ aryl;
wherein each alkyl and cycloalkyl is optionally substituted with one or more R⁵³;
wherein each heterocycloalkyl is optionally substituted with one or more -C₁-C₂ alkyl or -C₁-C₂alkylene-phenyl;
wherein each -C₆ aryl is optionally substituted with one or more -C₁-C₂ alkyl or -C₁-C₄alkylene-5 to 6-membered heterocycloalkyl, and wherein said 5 to 6-membered heterocycloalkyl is optionally substituted with one or more -C₁-C₂ alkyl;
R⁵³ is independently -OR⁵⁴, -CO₂R⁵⁴, or -NR⁵⁵R⁵⁶;
R⁵⁴ is -C₁-C₂ alkyl; and
R⁵⁵ and R⁵⁶ are each independently -H, -C₁-C₂ alkyl, phenyl, or -C₁-C₂alkylene-phenyl.

In some embodiments, R⁵¹ and R⁵² are each independently hydrogen, -C₁-C₄ alkyl, -C₅-C₆ cycloalkyl, -C₁-C₃alkylene-C₅-C₆ cycloalkyl, 5 to 6 membered heterocycloalkyl, -C₁-C₃alkylene-5 to 6 membered heterocycloalkyl, -C₆ aryl, or -C₁-C₃alkylene-C₆ aryl;
wherein each alkyl and cycloalkyl is optionally substituted with one or more R⁵³;
wherein each heterocycloalkyl is selected from pyrrolidine, piperidine, piperazine, and morpholine, and wherein each heterocycle is optionally substituted with one or more -C₁-C₂ alkyl or -C₁-C₂alkylene-phenyl;
wherein each -C₆ aryl is optionally substituted with one or more -C₁-C₂ alkyl or -C₁-C₄alkylene-5 to 6-membered heterocycloalkyl, and wherein said 5 to 6-membered heterocycloalkyl is optionally substituted with one or more -C₁-C₂ alkyl;
R⁵³ is independently -OR⁵⁴, -CO₂R⁵⁴, or -NR⁵⁵R⁵⁶;
R⁵⁴ is -C₁-C₂ alkyl; and
R⁵⁵ and R⁵⁶ are each independently -H, -C₁-C₂ alkyl, phenyl, or -C₁-C₂alkylene-phenyl.

In some embodiments, R⁵¹ and R⁵² are each independently hydrogen, -C₁-C₄ alkyl, -C₅-C₆ cycloalkyl, -C₁-C₃alkylene-C₅-C₆ cycloalkyl, 5 to 6 membered heterocycloalkyl, -C₁-C₃alkylene-5 to 6 membered heterocycloalkyl, -C₆ aryl, or -C₁-C₃alkylene-C₆ aryl;
wherein each alkyl and cycloalkyl is optionally substituted with one or more R⁵³;
wherein each heterocycloalkyl is selected from pyrrolidine, piperidine, piperazine, and morpholine, and wherein each heterocycle is optionally substituted with one or more -C₁-C₂ alkyl or -C₁-C₂alkylene-phenyl;
wherein each -C₆ aryl is optionally substituted with one or more -C₁-C₂ alkyl or -C₁-C₄alkylene-5 to 6-membered heterocycloalkyl, wherein said heterocycloalkyl is selected from pyrrolidine, piperidine, piperazine, and morpholine, and wherein said 5 to 6-membered heterocycloalkyl is optionally substituted with one or more -C₁-C₂ alkyl;
R⁵³ is independently -OR⁵⁴, -CO₂R⁵⁴, or -NR⁵⁵R⁵⁶;
R⁵⁴ is -C₁-C₂ alkyl; and
R⁵⁵ and R⁵⁶ are each independently -H, -C₁-C₂ alkyl, phenyl, or -C₁-C₂alkylene-phenyl.

In some embodiments, R⁵¹ and R⁵² are each independently hydrogen, -C₁-C₄ alkyl, -C₅-C₆ cycloalkyl, -C₁-C₃alkylene-C₅-C₆ cycloalkyl, 5 to 6 membered heterocycloalkyl, -C₁-C₃alkylene-5 to 6 membered heterocycloalkyl, -C₆ aryl, or -C₁-C₃alkylene-C₆ aryl;
wherein each alkyl and cycloalkyl is optionally substituted with one or more R⁵³;
wherein each heterocycloalkyl is selected from pyrrolidine, piperidine, and piperazine, and wherein each heterocycle is optionally substituted with one or more -C₁-C₂ alkyl or -C₁-C₂alkylene-phenyl;
wherein each -C₆ aryl is optionally substituted with one or more -C₁-C₂ alkyl or -C₁-C₄alkylene-5 to 6-membered heterocycloalkyl, wherein said heterocycloalkyl is selected from piperidine and piperazine, and wherein said 5 to 6-membered heterocycloalkyl is optionally substituted with one or more -C₁-C₂ alkyl;
R⁵³ is independently -OR⁵⁴, -CO₂R⁵⁴, or -NR⁵⁵R⁵⁶;
R⁵⁴ is -C₁-C₂ alkyl; and
R⁵⁵ and R⁵⁶ are each independently -H, -C₁-C₂ alkyl, phenyl, or -C₁-C₂alkylene-phenyl.

In some embodiments, R⁵¹ is hydrogen, and R⁵² is independently hydrogen, -C₁-C₄ alkyl, -C₅-C₆ cycloalkyl, -C₁-C₃alkylene-C₅-C₆ cycloalkyl, 5 to 6 membered heterocycloalkyl, -C₁-C₃alkylene-5 to 6 membered heterocycloalkyl, -C₆ aryl, or -C₁-C₃alkylene-C₆ aryl;
wherein each alkyl and cycloalkyl is optionally substituted with one or more R⁵³;
wherein each heterocycloalkyl is selected from pyrrolidine, piperidine, and piperazine, and wherein each heterocycle is optionally substituted with one or more -C₁-C₂ alkyl or -C₁-C₂alkylene-phenyl;
wherein each -C₆ aryl is optionally substituted with one or more -C₁-C₂ alkyl or -C₁-C₄alkylene-5 to 6-membered heterocycloalkyl, wherein said heterocycloalkyl is selected from piperidine and piperazine, and wherein said 5 to 6-membered heterocycloalkyl is optionally substituted with one or more -C₁-C₂ alkyl;
R⁵³ is independently -OR⁵⁴, -CO₂R⁵⁴, or -NR⁵⁵R⁵⁶;
R⁵⁴ is -C₁-C₂ alkyl; and
R⁵⁵ and R⁵⁶ are each independently -H, -C₁-C₂ alkyl, phenyl, or -C₁-C₂alkylene-phenyl.

In some embodiments, R⁵¹ and R⁵² combine to form an optionally substituted 3-10 membered heterocycloalkyl; preferably an optionally substituted 5-6 membered heterocycloalkyl.

In some embodiments, R⁵¹ and R⁵², together with the nitrogen atom to which they are attached, combine to form a 5-6 membered heterocycloalkyl, wherein the heterocycloalkyl is optionally substituted with one or more R⁵³.

In some embodiments, R⁵¹ and R⁵², together with the nitrogen atom to which they are attached, combine to form a 5-6 membered heterocycloalkyl, wherein the heterocycloalkyl comprises one to four, preferably one to three, more preferably tone to two heteroatoms selected from O and N, and is optionally substituted with one or more R⁵³.

In some embodiments, R⁵¹ and R⁵², together with the nitrogen atom to which they are attached, combine to form a piperidine, piperazine, or morpholine, wherein in the piperidine, piperazine, or morpholine is optionally substituted with one or more R⁵³.

In some embodiments, R⁵¹ and R⁵², together with the nitrogen atom to which they are attached, combine to form a piperidine, piperazine, or morpholine, wherein in the piperidine, piperazine, or morpholine is optionally substituted with one or more R⁵³; wherein R⁵³ is independently -C₁-C₆ alkyl, (preferably -C₁-C₃ alkyl, preferably methyl), phenyl, or NR⁵⁹R⁶⁰, wherein said phenyl is optionally substituted by -SO₂NH₂,
wherein R⁵⁹ and R⁶⁰ are independently methyl or phenyl, or R⁵⁹ and R⁶⁰ combine to form a piperazine substituted by oxo and methyl.

In some embodiments, R⁵¹ is hydrogen; and R⁵² is optionally substituted -C₆-C₁₀ aryl; preferably wherein the optionally substituted aryl is optionally substituted phenyl.

In some embodiments, R⁵¹ is hydrogen, and R⁵² is phenyl; wherein said phenyl is optionally substituted by -C₁-C₆ alkyl, preferably -C₁-C₃ alkyl; wherein said -C₁-C₆ alkyl is optionally substituted by piperidine or piperazine, and wherein each piperidine or piperazine is optionally substituted with methyl.

In some embodiments, X¹ is -NR⁵¹R⁵², wherein R⁵¹ and R⁵² are each independently hydrogen, -C₁-C₆ alkyl, -C₁-C₆ alkylene-R^{53A}, -C₅-C₆ cycloalkyl, 5-6 membered heterocycloalkyl or phenyl, wherein preferably at least one of R⁵¹ and R⁵² is hydrogen, wherein each alkyl, alkylene, cycloalkyl, heterocycloalkyl or phenyl is optionally substituted with one or more, preferably one or two, R⁵³; or R⁵¹ and R⁵², together with the nitrogen atom to which they are attached, combine to form a 3-15 membered heterocycloalkyl, wherein said heterocycloalkyl is optionally substituted with one or more, preferably one or two, R⁵³.

In some preferred embodiments, X¹ is -NR⁵¹R⁵², wherein R⁵¹ and R⁵², together with the nitrogen atom to which they are attached, combine to form a heterocycloalkyl, wherein said heterocycloalkyl is optionally substituted with one or more R⁵³.

In some embodiments, X¹ is -NR⁵¹R⁵², wherein R⁵¹ and R⁵², together with the nitrogen atom to which they are attached, combine to form a 3-15 membered heterocycloalkyl, wherein said heterocycloalkyl is optionally substituted with one or more, preferably one or two, R⁵³; or wherein said heterocycle is fused to one or more additional aryl or heteroaryl rings to form a 10- to 15-membered heteroaryl, wherein said 10- to 15-membered heteroaryl is optionally substituted with one or more, preferably one or two, R⁵³; or wherein said heterocycle is connected at a single atom to another cycloalkyl or heterocycloalkyl to form a 10- to 15-membered spirocyclic heterocycle; and wherein said spirocyclic is optionally substituted with one or more, preferably one or two, R⁵³.

In some embodiments, X¹ is -NR⁵¹R⁵², wherein R⁵¹ and R⁵², together with the nitrogen atom to which they are attached, combine to form a 3-15 membered heterocycloalkyl, wherein said heterocycloalkyl is optionally substituted with one or more R⁵³.

In some embodiments, X¹ is -NR⁵¹R⁵², wherein R⁵¹ and R⁵², together with the nitrogen atom to which they are attached, combine to form a 3-15 membered heterocycloalkyl, wherein said heterocycloalkyl is fused to one or more additional aryl or heteroaryl rings to form a 10-to 15-membered heteroaryl, wherein said 10- to 15-membered heteroaryl is optionally substituted with one or more R⁵³.

In some embodiments, X¹ is -NR⁵¹R⁵², wherein R⁵¹ and R⁵², together with the nitrogen atom to which they are attached, combine to form a 3-15 membered heterocycloalkyl, wherein said heterocycloalkyl is connected at a single atom to another cycloalkyl or heterocycloalkyl to form a 10- to 15-membered spirocyclic heterocycle; and wherein said spirocyclic is optionally substituted with one or more R⁵³.

In some embodiments, X¹ is -NR⁵¹R⁵², wherein R⁵¹ and R⁵², together with the nitrogen atom to which they are attached, combine to form a 3-15 membered heterocycloalkyl, wherein said 3- to 15-membered heterocycloalkyl is a piperidine, morpholine, 1,4-diazepane, piperazine, or an azetidine. In another embodiment, said heterocycloalkyl is a piperidine. In another embodiment, said heterocycloalkyl is a morpholine. In another embodiment, said heterocycloalkyl is a 1,4-diazepane. In another embodiment, said heterocycloalkyl is a piperazine. In another embodiment, said heterocycloalkyl is an azetidine,

In some preferred embodiments, X¹ is -NR⁵¹R⁵², wherein R⁵¹ and R⁵², together with the nitrogen atom to which they are attached, combine to form a heterocycloalkyl, wherein (i) said heterocycloalkyl is a piperidine, morpholine, 1,4-diazepane, piperazine, or an azetidine, wherein said piperidine, morpholine, 1,4-diazepane, piperazine or azetidine is optionally substituted with one or more R⁵³; or (ii) wherein said heterocycle is a piperidine fused to one or more additional aryl or heteroaryl rings to form a 10- to 15-membered heteroaryl, wherein said 10- to 15-membered heteroaryl is optionally substituted with one or more R⁵³; or (iii) wherein said heterocycle is a piperidine connected at a single atom to another cycloalkyl or heterocycloalkyl to form a 10- to 15-membered spirocyclic heterocycle; and wherein said spirocyclic is optionally substituted with one or more R⁵³.

In some preferred embodiments, X¹ is -NR⁵¹R⁵², wherein R⁵¹ and R⁵², together with the nitrogen atom to which they are attached, combine to form a heterocycloalkyl, wherein said heterocycloalkyl is a piperidine, morpholine, 1,4-diazepane, piperazine, or an azetidine, wherein said piperidine, morpholine, 1,4-diazepane, piperazine or azetidine is optionally substituted with one or more R⁵³; or wherein said wherein said piperidine, morpholine, 1,4-diazepane, piperazine or azetidine is fused to one or more additional aryl or heteroaryl rings to form a 10- to 15-membered heteroaryl, wherein said 10- to 15-membered heteroaryl is optionally substituted with one or more R⁵³; or wherein said piperidine, morpholine, 1,4-diazepane, piperazine or azetidine is connected at a single atom to another cycloalkyl or heterocycloalkyl to form a 10- to 15-membered spirocyclic heterocycle; and wherein said spirocyclic is optionally substituted with one or more R⁵³.

In another embodiment, X¹ is -NR⁵¹R⁵², wherein R⁵¹ and R⁵², together with the nitrogen atom to which they are attached, combine to form a heterocycloalkyl, wherein said heterocycloalkyl is a piperidine, morpholine, 1,4-diazepane, piperazine, or an azetidine, wherein said heterocycloalkyl is optionally substituted with one or more R⁵³. In another embodiment, said heterocycloalkyl is a piperidine, morpholine, 1,4-diazepane, piperazine, or an azetidine, wherein said heterocycle is fused to one or more additional aryl or heteroaryl rings to form a 10- to 15-membered heteroaryl, wherein said 10- to 15-membered heteroaryl is optionally substituted with one or more R⁵³. In another embodiment, said heterocycloalkyl is a piperidine, morpholine, 1,4-diazepane, piperazine, or an azetidine, and wherein said heterocycle is connected at a single atom to another cycloalkyl or heterocycloalkyl to form a 10- to 15-membered spirocyclic heterocycle; and wherein said spirocyclic is optionally substituted with one or more R⁵³.

In another embodiment, X¹ is -NR⁵¹R⁵², wherein R⁵¹ and R⁵², together with the nitrogen atom to which they are attached, combine to form a heterocycloalkyl, wherein said heterocycloalkyl is a piperidine, wherein said piperidine is optionally substituted with one or more R⁵³. In another embodiment, said heterocycloalkyl is a piperidine, wherein said piperidine is fused to one or more additional aryl or heteroaryl rings to form a 10- to 15-membered heteroaryl, wherein said 10- to 15-membered heteroaryl is optionally substituted with one or more R⁵³. In another embodiment, said heterocycloalkyl is a piperidine, and wherein said piperidine is connected at a single atom to another cycloalkyl or heterocycloalkyl to form a 10-to 15-membered spirocyclic heterocycle; and wherein said spirocyclic is optionally substituted with one or more R⁵³.

In another embodiment, X¹ is -NR⁵¹R⁵², wherein R⁵¹ and R⁵², together with the nitrogen atom to which they are attached, combine to form a heterocycloalkyl, wherein said heterocycloalkyl is a morpholine, wherein said morpholine is optionally substituted with one or more R⁵³. In another embodiment, said heterocycloalkyl is a morpholine, wherein said morpholine is fused to one or more additional aryl or heteroaryl rings to form a 10- to 15-membered heteroaryl, wherein said 10- to 15-membered heteroaryl is optionally substituted with one or more R⁵³. In another embodiment, said heterocycloalkyl is a morpholine, and wherein said morpholine is connected at a single atom to another cycloalkyl or heterocycloalkyl to form a 10- to 15-membered spirocyclic heterocycle; and wherein said spirocyclic is optionally substituted with one or more R⁵³.

In another embodiment, X¹ is -NR⁵¹R⁵², wherein R⁵¹ and R⁵², together with the nitrogen atom to which they are attached, combine to form a heterocycloalkyl, wherein said heterocycloalkyl is a 1,4-diazepane, wherein said 1,4-diazepane is optionally substituted with one or more R⁵³. In another embodiment, said heterocycloalkyl is a 1,4-diazepane, wherein said 1,4-diazepane is fused to one or more additional aryl or heteroaryl rings to form a 10- to 15-membered heteroaryl, wherein said 10- to 15-membered heteroaryl is optionally substituted with one or more R⁵³. In another embodiment, said heterocycloalkyl is a 1,4-diazepane, and wherein said 1,4-diazepane is connected at a single atom to another cycloalkyl or heterocycloalkyl to form a 10- to 15-membered spirocyclic heterocycle; and wherein said spirocyclic is optionally substituted with one or more R⁵³.

In another embodiment, X¹ is -NR⁵¹R⁵², wherein R⁵¹ and R⁵², together with the nitrogen atom to which they are attached, combine to form a heterocycloalkyl, wherein said heterocycloalkyl is a piperazine, wherein said piperazine is optionally substituted with one or more R⁵³. In another embodiment, said heterocycloalkyl is a piperazine, wherein said piperazine is fused to one or more additional aryl or heteroaryl rings to form a 10- to 15-membered heteroaryl, wherein said 10- to 15-membered heteroaryl is optionally substituted with one or more R⁵³. In another embodiment, said heterocycloalkyl is a piperazine, and wherein said piperazine is connected at a single atom to another cycloalkyl or heterocycloalkyl to form a 10- to 15-membered spirocyclic heterocycle; and wherein said spirocyclic is optionally substituted with one or more R⁵³.

In another embodiment, X¹ is -NR⁵¹R⁵², wherein R⁵¹ and R⁵², together with the nitrogen atom to which they are attached, combine to form a heterocycloalkyl, wherein said heterocycloalkyl is an azetidine, wherein said azetidine is optionally substituted with one or more R⁵³. In another embodiment, said heterocycloalkyl is an azetidine, wherein said azetidine is fused to one or more additional aryl or heteroaryl rings to form a 10- to 15-membered heteroaryl, wherein said 10- to 15-membered heteroaryl is optionally substituted with one or more R⁵³. In another embodiment, said heterocycloalkyl is an azetidine, and wherein said azetidine is connected at a single atom to another cycloalkyl or heterocycloalkyl to form a 10-to 15-membered spirocyclic heterocycle; and wherein said spirocyclic is optionally substituted with one or more R⁵³.

In another embodiment, X¹ is -NR⁵¹R⁵², wherein R⁵¹ and R⁵², together with the nitrogen atom to which they are attached, combine to form a heterocycloalkyl, wherein said heterocycloalkyl is a piperidine.

In another preferred embodiment, X¹ is -NR⁵¹R⁵², wherein R⁵³ and R^{53A} are independently -C₁-C₄ alkyl, -C₁-C₃ alkylene-R^{57A}, 5- to 15-membered heterocycloalkyl, 5- to 15-membered heteroaryl, or phenyl, -OR⁵⁴, -C(O)R⁵⁴, -CO₂R⁵⁴, oxo, or -NR⁵⁵R⁵⁶, wherein each alkylene, heterocycloalkyl, heteroaryl and phenyl is optionally substituted with one or more R⁵⁷;
wherein R⁵⁴ is independently hydrogen, -C₁-C₄ alkyl, -C₅-C₆ cycloalkyl, or phenyl; wherein each alkyl, cycloalkyl or phenyl is optionally substituted with one or more R⁶¹; and
R⁵⁵ and R⁵⁶ are each independently hydrogen, -C₁-C₄ alkyl, -C₁-C₄ alkylene-R^{61A}, phenyl, -CO₂C₁-C₆ alkyl, -S(O)₂(phenyl), wherein phenyl or cycloalkyl is optionally substituted with one or more R⁶¹.

In another preferred embodiment, X¹ is -NR⁵¹R⁵², wherein R⁵³ and R^{53A} are independently -C₁-C₄ alkyl, -C₁-C₃ alkylene-R^{57A}, 5- to 6-membered heterocycloalkyl, 5- to 10-membered heteroaryl, or phenyl, -OR⁵⁴, -C(O)R⁵⁴, -CO₂R⁵⁴, oxo, or -NR⁵⁵R⁵⁶, wherein each alkylene, heterocycloalkyl, heteroaryl and phenyl is optionally substituted with one or more R⁵⁷;
wherein R⁵⁴ is independently hydrogen, -C₁-C₂ alkyl, --C₆ cycloalkyl, or phenyl; wherein each alkyl, cycloalkyl or phenyl is optionally substituted with one or more R⁶¹; and
R⁵⁵ and R⁵⁶ are each independently hydrogen, -C₁-C₂ alkyl, -C₁-C₃ alkylene-R^{61A}, phenyl, -CO₂C₁-C₂ alkyl, -S(O)₂(phenyl), wherein phenyl or cycloalkyl is optionally substituted with one or more R⁶¹.

In another preferred embodiment, X¹ is -NR⁵¹R⁵², wherein R⁵⁷ and R^{57A} are independently -C₁-C₆ alkyl, 3-10 membered heterocycloalkyl, phenyl, 5-10 membered heteroaryl, oxo, halogen, -OH, or -SO₂NH₂, wherein each alkyl, heterocycloalkyl, phenyl, and heteroaryl is optionally substituted with one or more R⁶¹.

In another preferred embodiment, X¹ is -NR⁵¹R⁵², wherein R⁵⁷ and R^{57A} are independently -C₁-C₂ alkyl, 5-6 membered heterocycloalkyl, phenyl, 5-10 membered heteroaryl, oxo, halogen, -OH, or -SO₂NH₂, wherein each alkyl, heterocycloalkyl, phenyl, and heteroaryl is optionally substituted with one or more R⁶¹.

In another preferred embodiment, X¹ is -NR⁵¹R⁵², wherein R⁶¹ and R^{61A} are independently -C₁-C₂ alkyl, phenyl, -C₁-C₂ alkoxy, -C₁-C₂ haloalkyl, or halogen.

In some preferred embodiments, X¹ is -NR⁵¹R⁵², wherein R⁵¹ and R⁵², together with the nitrogen atom to which they are attached, combine to form a heterocycloalkyl, wherein said heterocycle is a piperazine or piperidine, wherein each piperazine or piperidine is optionally substituted with one or more R⁵³; R⁵³ is -C₁-C₆ alkyl, -C₃-C₆ cycloalkyl, 3-10 membered heterocycloalkyl, -C₆-C₁₀ aryl, 5-10 membered heteroaryl, -OR⁵⁴, or -NR⁵⁵R⁵⁶, wherein each alkyl, cycloalkyl, heterocycloalkyl, aryl, or heteroaryl is optionally substituted with one or more R⁵⁷; R⁵⁴ is independently hydrogen, -C₆-C₁₀ aryl, or -C₅-C₆ cycloalkyl, wherein each aryl or cycloalkyl is optionally substituted with one or more R⁶¹; R⁵⁵ and R⁵⁶ are each independently hydrogen, -C₁-C₆ alkyl, -C₆-C₁₀ aryl, -S(O)₂C₆-C₁₀ aryl, or -C(O)O -C₁-C₆ alkyl; wherein each alkyl or aryl is optionally substituted with one or more R⁶¹; R⁵⁷ is independently -C₁-C₆ alkyl, 3-10 membered heterocycloalkyl, -C₆-C₁₀ aryl, 5-10 membered heteroaryl, oxo, halogen, -OH, or -SO₂NH₂, wherein each alkyl, heterocycloalkyl, aryl, and heteroaryl is optionally substituted with one or more R⁶¹; and R⁶¹ is independently -C₁-C₆ alkyl, -C₆-C₁₀ aryl, 5-10 membered heteroaryl, -C₁-C₆ alkoxy, -C₁-C₆ haloalkyl, or halogen.

In some preferred embodiments, X¹ is -NR⁵¹R⁵², wherein R⁵¹ and R⁵², together with the nitrogen atom to which they are attached, combine to form a heterocycloalkyl, wherein said heterocycle is a piperidine, wherein said piperidine is optionally substituted with one or more R⁵³; R⁵³ is -C₁-C₆ alkyl, -C₃-C₆ cycloalkyl, 3-10 membered heterocycloalkyl, -C₆-C₁₀ aryl, 5-10 membered heteroaryl, -OR⁵⁴, or -NR⁵⁵R⁵⁶, wherein each alkyl, cycloalkyl, heterocycloalkyl, aryl, or heteroaryl is optionally substituted with one or more R⁵⁷; R⁵⁴ is independently hydrogen or -C₆-C₁₀ aryl; R⁵⁵ and R⁵⁶ are each independently hydrogen, -C₁-C₆ alkyl, -C₆-C₁₀ aryl, - S(O)₂C₆-C₁₀ aryl, or -C(O)OC₁-C₆ alkyl; wherein each alkyl or aryl is optionally substituted with one or more R⁶¹; R⁵⁷ is independently -C₁-C₆ alkyl, 3-10 membered heterocycloalkyl, -C₆-C₁₀ aryl, 5-10 membered heteroaryl, oxo, halogen, -OH, or -SO₂NH₂, wherein each alkyl, heterocycloalkyl, aryl, and heteroaryl is optionally substituted with one or more R⁶¹; and R⁶¹ is independently -C₁-C₆ alkyl, -C₆-C₁₀ aryl, 5-10 membered heteroaryl, -C₁-C₆ alkoxy, -C₁-C₆ haloalkyl, or halogen.

In some preferred embodiments, X¹ is -NR⁵¹R⁵², wherein R⁵¹ and R⁵², together with the nitrogen atom to which they are attached, combine to form a heterocycloalkyl, wherein said heterocycle is a piperidine, wherein said piperidine is optionally substituted with one or more R⁵³; R⁵³ is -C₁-C₄ alkyl, -C₃-C₆ cycloalkyl, 3-10 membered heterocycloalkyl, phenyl, 5-10 membered heteroaryl, -OR⁵⁴, or -NR⁵⁵R⁵⁶, wherein each alkyl, cycloalkyl, heterocycloalkyl, phenyl, or heteroaryl is optionally substituted with one or more R⁵⁷; R⁵⁴ is independently hydrogen or phenyl; R⁵⁵ and R⁵⁶ are each independently hydrogen, -C₁-C₄ alkyl, phenyl, -S(O)₂ phenyl; or -C(O)OC₁-C₄ alkyl; R⁵⁷ is independently -C₁-C₄ alkyl, 3-10 membered heterocycloalkyl, phenyl, 5-10 membered heteroaryl, oxo, fluorine, chlorine, -OH, or -SO₂NH₂, wherein each alkyl, heterocycloalkyl, aryl, and heteroaryl is optionally substituted with one or more R⁶¹; and R⁶¹ is independently -C₁-C₄ alkyl, phenyl, 5-10 membered heteroaryl, -C₁-C₄ alkoxy, -C₁-C₄ haloalkyl, fluorine or chlorine.

In some preferred embodiments, X¹ is -NR⁵¹R⁵², wherein R⁵¹ and R⁵², together with the nitrogen atom to which they are attached, combine to form a heterocycloalkyl, wherein said heterocycle is a piperidine, wherein said piperidine is optionally substituted with one or more R⁵³; R⁵³ is -C₁-C₂ alkyl, -C₃-C₆ cycloalkyl, 3-10 membered heterocycloalkyl, phenyl, 5-10 membered heteroaryl, -OR⁵⁴, or -NR⁵⁵R⁵⁶, wherein each alkyl, cycloalkyl, heterocycloalkyl, phenyl, or heteroaryl is optionally substituted with one or more R⁵⁷; R⁵⁴ is independently hydrogen or phenyl; R⁵⁵ and R⁵⁶ are each independently hydrogen, -C₁-C₂ alkyl, phenyl, -S(O)₂ phenyl; or -C(O)OC₁-C₄ alkyl; R⁵⁷ is independently -C₁-C₂ alkyl, 3-10 membered heterocycloalkyl, phenyl, 5-10 membered heteroaryl, oxo, fluorine, chlorine, -OH, or -SO₂NH₂, wherein each alkyl, heterocycloalkyl, phenyl, and heteroaryl is optionally substituted with one or more R⁶¹; and R⁶¹ is independently -C₁-C₂ alkyl, phenyl, 5-10 membered heteroaryl, -C₁-C₂ alkoxy, -C₁-C₂ haloalkyl, fluorine or chlorine.

In some preferred embodiments, X¹ is -NR⁵¹R⁵², wherein R⁵¹ and R⁵², together with the nitrogen atom to which they are attached, combine to form a heterocycloalkyl, wherein said heterocycle is a piperidine, wherein said piperidine is optionally substituted with one or two R⁵³, preferably wherein said piperidine is substituted at the 4-position only; R⁵³ is -C₁-C₂ alkyl, -C₃-C₆ cycloalkyl, 3-10 membered heterocycloalkyl, phenyl, 5-10 membered heteroaryl, -OR⁵⁴, or -NR⁵⁵R⁵⁶, wherein each alkyl, cycloalkyl, heterocycloalkyl, phenyl, or heteroaryl is optionally substituted with one or more R⁵⁷; R⁵⁴ is independently hydrogen or phenyl; R⁵⁵ and R⁵⁶ are each independently hydrogen, -C₁-C₂ alkyl, phenyl, -S(O)₂ phenyl; or -C(O)OC₁-C₄ alkyl; R⁵⁷ is independently -C₁-C₂ alkyl, 3-10 membered heterocycloalkyl, phenyl, 5-10 membered heteroaryl, oxo, fluorine, chlorine, -OH, or -SO₂NH₂, wherein each alkyl, heterocycloalkyl, phenyl, and heteroaryl is optionally substituted with one or more R⁶¹; and R⁶¹ is independently -C₁-C₂ alkyl, phenyl, 5-10 membered heteroaryl, -C₁-C₂ alkoxy, -C₁-C₂ haloalkyl, fluorine or chlorine.

In some preferred embodiments, X¹ is -NR⁵¹R⁵², wherein R⁵¹ and R⁵², together with the nitrogen atom to which they are attached, combine to form a heterocycloalkyl, wherein said heterocycle is a piperidine, wherein said piperidine is substituted with exactly one R⁵³ at the 4-position; R⁵³ is -NR⁵⁵R⁵⁶, wherein R⁵⁵ and R⁵⁶ are each independently -C₁-C₂ alkyl, or phenyl.

In some preferred embodiments, X¹ is -NR⁵¹R⁵², wherein R⁵¹ and R⁵², together with the nitrogen atom to which they are attached, combine to form a heterocycloalkyl, wherein said heterocycle is a piperidine fused to an indole to form a 2,3,4,5-tetrahydro-1H-pyrido[4,3-b]indole, wherein said 2,3,4,5-tetrahydro-1H-pyrido[4,3-b]indole is optionally substituted with one or more R⁵³. In some embodiments, said heterocycloalkyl is a piperidine fused to an indole to form a 2,3,4,5-tetrahydro-1H-pyrido[4,3-b]indole.

In some preferred embodiments, X¹ is -NR⁵¹R⁵², wherein R⁵¹ and R⁵², together with the nitrogen atom to which they are attached, combine to form a heterocycloalkyl, wherein said heterocycle is a piperidine connected at a single atom to another cycloalkyl or heterocycloalkyl form a 10- to 15-membered spirocyclic heterocycle, wherein said spirocyclic heterocycle is optionally substituted with one or more R⁵³.

In some preferred embodiments, X¹ is -NR⁵¹R⁵², wherein R⁵¹ and R⁵², together with the nitrogen atom to which they are attached, combine to form a heterocycloalkyl, wherein said heterocycle is a piperidine connected at the 4-position to form a 10- to 15-membered spirocyclic heterocycle, wherein said spirocyclic heterocycle is optionally substituted with one or more R⁵³.

In some preferred embodiments, X¹ is -NR⁵¹R⁵², wherein R⁵¹ and R⁵², together with the nitrogen atom to which they are attached, combine to form a heterocycloalkyl, wherein said heterocycle is a piperidine connected at the 4-position to form a 10- to 15-membered spirocyclic heterocycle, wherein said spirocyclic heterocycle is optionally substituted with one or more R⁵³ and wherein R⁵³ is phenyl.

In some preferred embodiments, X¹ is -NR⁵¹R⁵², wherein R⁵¹ and R⁵², together with the nitrogen atom to which they are attached, combine to form a heterocycloalkyl, wherein said heterocycle is an azetidine, wherein said azetidine is optionally substituted with one or more R⁵³.

In some preferred embodiments, X¹ is -NR⁵¹R⁵², wherein R⁵¹ and R⁵², together with the nitrogen atom to which they are attached, combine to form a heterocycloalkyl, wherein said heterocycle is an azetidine, wherein said azetidine is optionally substituted with one or more R⁵³ and R⁵³ is 5- to 10-membered heteroaryl or -NR⁵⁵R⁵⁶; and R⁵⁵ and R⁵⁶ are each independently hydrogen or phenyl, wherein said phenyl is optionally substituted with halogen, preferably fluorine or chlorine, more preferably fluorine.

In some preferred embodiments, X¹ is -NR⁵¹R⁵², wherein R⁵¹ and R⁵², together with the nitrogen atom to which they are attached, combine to form a heterocycloalkyl, wherein said heterocycle is an azetidine, wherein said azetidine is optionally substituted at the 3-position with one or more R⁵³ and R⁵³ is 5- to 10-membered heteroaryl or -NR⁵⁵R⁵⁶; and R⁵⁵ and R⁵⁶ are each independently hydrogen or phenyl, wherein said phenyl is optionally substituted with halogen, preferably fluorine or chlorine, more preferably fluorine.

In some preferred embodiments, X¹ is -NR⁵¹R⁵², wherein R⁵¹ and R⁵², together with the nitrogen atom to which they are attached, combine to form a heterocycloalkyl, wherein said heterocycle is a 1,4-diazepane, wherein said 1,4-diazepane is optionally substituted with one or more R⁵³.

In some preferred embodiments, X¹ is -NR⁵¹R⁵², wherein R⁵¹ and R⁵², together with the nitrogen atom to which they are attached, combine to form a heterocycloalkyl, wherein said heterocycle is a [1,4-diazepane, wherein said 1,4-diazepane is optionally substituted with one or more R⁵³; and R⁵³ is phenyl, wherein said phenyl is optionally substituted with halogen, preferably fluorine or chlorine, more preferably fluorine.

In some preferred embodiments, X¹ is -NR⁵¹R⁵², wherein R⁵¹ is -H, and R⁵² is -C₅-C₆ heterocycle, -C₆-C₁₀ aryl, -C₁-C₆ alkylene-C₅-C₆ heterocycle or -C₁-C₄ alkylene-C₆-C₁₀ aryl; wherein each heterocycle or aryl is optionally substituted with one or more R⁵³.

In some preferred embodiments, X¹ is -NR⁵¹R⁵², R⁵¹ is -H, R⁵² is piperidine, piperazine, morpholine, pyrrolidine, -C₁-C₄ alkylene-piperidine, -C₁-C₄ alkylene-piperazine, -C₁-C₄ alkylene-morpholine, or -C₁-C₄ alkylene-pyrrolidine; wherein each piperidine, piperazine morpholine or pyrrolidine is optionally substituted with one or more -C₁-C₂ alkyl.

In some preferred embodiments, X¹ is -NR⁵¹R⁵², R⁵¹ is -H, R⁵² is piperidine, piperazine, morpholine, pyrrolidine, -C₁-C₂ alkylene-piperidine, -C₁-C₂ alkylene-piperazine, -C₁-C₂ alkylene-morpholine, or -C₁-C₂ alkylene-pyrrolidine; wherein each piperidine, piperazine morpholine or pyrrolidine is optionally substituted with one or more -C₁-C₂ alkyl.

In some preferred embodiments, X¹ is -NR⁵¹R⁵², R⁵¹ is -H, R⁵² is piperidine, piperazine, morpholine, pyrrolidine, -C₁-C₂ alkylene-piperidine, -C₁-C₂ alkylene-piperazine, -C₁-C₂ alkylene-morpholine, or -C₁-C₂ alkylene-pyrrolidine; wherein each piperidine, piperazine or pyrrolidine is optionally substituted at the 4-position with one or more -C₁-C₂ alkyl.

In some preferred embodiments, X¹ is -NR⁵¹R⁵², R⁵¹ is -H, R⁵² is piperidine or -C₁-C₂ alkylene-piperidine; wherein each piperidine is optionally substituted at the 4-position with one or more -C₁-C₂ alkyl.

In some preferred embodiments, X¹ is -NR⁵¹R⁵², R⁵¹ is -H, and R⁵² is phenyl or -C₁-C₄ alkylene-phenyl; wherein each phenyl is optionally substituted with one or more R⁵³.

In some preferred embodiments, X¹ is -NR⁵¹R⁵², R⁵¹ is -H, and R⁵² is phenyl or -C₁-C₄ alkylene-phenyl; wherein each phenyl is optionally substituted with one or more R⁵³; wherein R⁵³ is -C₁-C₂ alkyl optionally substituted by one or more -C₅-C₆ heterocycloalkyl; and wherein said heterocycloalkyl can be further substituted with one or more -C₁-C₂ alkyl.

In some preferred embodiments, X¹ is -NR⁵¹R⁵², R⁵¹ is -H, and R⁵² is phenyl or -C₁-C₃ alkylene-phenyl; wherein each phenyl is optionally substituted with one or more R⁵³; wherein R⁵³ is -C₁-C₂ alkyl optionally substituted by one or more piperidine or piperazine; and wherein said piperidine or piperazine can be further substituted with one or more -C₁-C₂ alkyl.

In some preferred embodiments, X¹ is -NR⁵¹R⁵², wherein R⁵¹ is -H, and R⁵² is -C₁-C₄ alkyl, wherein each alkyl is optionally substituted by one or more -CO₂C₁-C₄ alkyl, -OC₁-C₄ alkyl, -N(C₁-C₄ alkyl)₂, or -C₅-C₆ heterocycloalkyl, preferably wherein said heterocycloalkyl is optionally substituted with one or more -C₁-C₂ alkyl.

In some preferred embodiments, X¹ is -NR⁵¹R⁵², R⁵¹ is -H, and R⁵² is -C₁-C₄ alkyl, wherein each alkyl is optionally substituted by one or more -CO₂C₁-C₄ alkyl, -OC₁-C₄ alkyl, - N(C₁-C₄ alkyl)₂, piperidine, morpholine, or piperazine; preferably wherein said piperidine or piperazine is optionally substituted with one or more -C₁-C₂ alkyl at the 4-position.

In some embodiments, X¹ is -NR⁵¹R⁵², wherein R⁵¹ and R⁵², together with the nitrogen atom to which they are attached, combine to form a 3-15 membered heterocycloalkyl, wherein said heterocycloalkyl is optionally fused to one or more additional aryl or heteroaryl rings or said heterocycloalkyl is optionally connected at a single atom to another cycloalkyl or heterocycloalkyl to form a 10- to 15-membered spirocyclic heterocycle, or said heterocycloalkyl optionally comprises a C₂ bridging alkylene; and wherein said 3-15 membered heterocycloalkyl is optionally substituted with one or more, preferably one or two, R⁵³;
R⁵³ is independently selected from -C₁-C₆ alkyl, -C₃-C₆ cycloalkyl, -C₁-C₆ alkylene-C₃-C₆ cycloalkyl, -C₆-C₁₀ aryl, -C₁-C₆ alkylene-C₆-C₁₀ aryl, 3-10 membered heterocycloalkyl, -C₁-C₆ alkylene-3-10 membered heterocycloalkyl, 5-10 membered heteroaryl, -C₁-C₆ alkylene-5-10 membered heteroaryl, -NR⁵⁵R⁵⁶, -OR⁵⁴, or oxo;
wherein each alkylene is optionally substituted with one or more oxo or -OH;
wherein each alkyl, cycloalkyl, aryl, heterocycloalkyl, or heteroaryl is optionally substituted with one or more -C₁-C₆ alkyl, -C₁-C₆ haloalkyl, halogen, -NH₂, -NH(C₁-C₆ alkyl), -N(C₁-C₆ alkyl)₂, -OH, -OCH₃, -OCH₂CH₂OH, -SO₂NH₂, phenyl, or oxo;
R⁵⁵ and R⁵⁶ are each independently -H, -C₁-C₆ alkyl, -CO₂C₁-C₆ alkyl, -SO₂phenyl, - C₆-C₁₀ aryl, or 5- to 6-membered heteroaryl, wherein each -C₆-C₁₀ aryl or 5- to 6-membered heteroaryl is optionally substituted with one or more -C₁-C₆ alkyl, halogen -OC₁-C₆ alkyl, or - CN; and
R⁵⁴ is independently -H, -C₁-C₆ alkyl, -C₆-C₁₀ aryl or -C₅-C₆ cycloalkyl, wherein said cycloalkyl is optionally substituted by one or more -C₁-C₆ alkyl.

In some embodiments, X¹ is -NR⁵¹R⁵², wherein R⁵¹ and R⁵², together with the nitrogen atom to which they are attached, combine to form a 3-15 membered heterocycloalkyl, wherein said heterocycloalkyl is optionally fused to one or more additional aryl or heteroaryl rings or said heterocycloalkyl is optionally connected at a single atom to another cycloalkyl or heterocycloalkyl to form a 10- to 15-membered spirocyclic heterocycle, or said heterocycloalkyl optionally comprises a C₂ bridging alkylene; and wherein said 3-15 membered heterocycloalkyl is optionally substituted with one or more, preferably one or two, R⁵³;
R⁵³ is independently selected from -C₁-C₆ alkyl, -C₃-C₆ cycloalkyl, -C₁-C₆ alkylene-C₃-C₆ cycloalkyl, -C₆-C₁₀ aryl, -C₁-C₆ alkylene-C₆-C₁₀ aryl, -C(O)-C₆-C₁₀ aryl, 3-10 membered heterocycloalkyl, -C₁-C₆ alkylene-3-10 membered heterocycloalkyl, 5-10 membered heteroaryl, -C₁-C₆ alkylene-5-10 membered heteroaryl, -CH(OH)-5-10 membered heteroaryl, - NR⁵⁵R⁵⁶, -OR⁵⁴, or oxo;
wherein said -C₁-C₆ alkyl is optionally substituted with one or more -NH₂, -NH(C₁-C₆ alkyl), -N(C₁-C₆ alkyl)₂, -OH, -OCH₂CH₂OH, or phenyl;
wherein each aryl is optionally substituted with one or more -C₁-C₆ alkyl, -C₁-C₆ haloalkyl, phenyl, halogen, -OH, -OCH₃, or -SO₂NH₂;
wherein each heterocycloalkyl is optionally substituted with one or more -C₁-C₆ alkyl or oxo;
wherein each heteroaryl is optionally substituted with one or more oxo, -C₁-C₆ alkyl, - C₁-C₆ haloalkyl, or halogen;
R⁵⁵ and R⁵⁶ are each independently -H, -C₁-C₆ alkyl, -CO₂C₁-C₆ alkyl, -SO₂phenyl, - C₆-C₁₀ aryl, or 5- to 6-membered heteroaryl, wherein each -C₆-C₁₀ aryl or 5- to 6-membered heteroaryl is optionally substituted with one or more -C₁-C₆ alkyl, halogen -OC₁-C₆ alkyl, or - CN; and
R⁵⁴ is independently -H, -C₆-C₁₀ aryl or -C₅-C₆ cycloalkyl, wherein said cycloalkyl is optionally substituted by one or more -C₁-C₆ alkyl.

In some embodiments, X¹ is -NR⁵¹R⁵², wherein R⁵¹ and R⁵², together with the nitrogen atom to which they are attached, combine to form a 3-15 membered heterocycloalkyl, wherein said heterocycloalkyl is optionally fused to one or more additional aryl or heteroaryl rings or said heterocycloalkyl is optionally connected at a single atom to another cycloalkyl or heterocycloalkyl to form a 10- to 15-membered spirocyclic heterocycle, or said heterocycloalkyl optionally comprises a C₂ bridging alkylene; and wherein said 3-15 membered heterocycloalkyl is optionally substituted with one or more, preferably one or two, R⁵³;
R⁵³ is independently selected from -C₁-C₄ alkyl, -C₃-C₆ cycloalkyl, -C₁-C₄ alkylene-C₃-C₆ cycloalkyl, -C₆-C₁₀ aryl, -C₁-C₄ alkylene-C₆-C₁₀ aryl, -C(O)-C₆-C₁₀ aryl, 3-10 membered heterocycloalkyl, -C₁-C₄ alkylene-3-10 membered heterocycloalkyl, 5-10 membered heteroaryl, -C₁-C₄ alkylene-5-10 membered heteroaryl, -CH(OH)-5-10 membered heteroaryl, - NR⁵⁵R⁵⁶, -OR⁵⁴, or oxo;
wherein said -C₁-C₄ alkyl is optionally substituted with one or more -NH₂, -NH(C₁-C₄ alkyl), -N(C₁-C₄ alkyl)₂, -OH, -OCH₂CH₂OH, or phenyl;
wherein each aryl is optionally substituted with one or more -C₁-C₄ alkyl, -C₁-C₄ haloalkyl, phenyl, halogen, -OH, -OCH₃, or -SO₂NH₂;
wherein each heterocycloalkyl is optionally substituted with one or more -C₁-C₄ alkyl or oxo;
wherein each heteroaryl is optionally substituted with one or more oxo, -C₁-C₄ alkyl, - C₁-C₄ haloalkyl, or halogen;
R⁵⁵ and R⁵⁶ are each independently -H, -C₁-C₄ alkyl, -CO₂C₁-C₄ alkyl, -SO₂phenyl, - C₆-C₁₀ aryl, or 5- to 6-membered heteroaryl, wherein each -C₆-C₁₀ aryl or 5- to 6-membered heteroaryl is optionally substituted with one or more -C₁-C₄ alkyl, halogen -OC₁-C₄ alkyl, or - CN; and
R⁵⁴ is independently -H, -C₆-C₁₀ aryl or -C₅-C₆ cycloalkyl, wherein said cycloalkyl is optionally substituted by one or more -C₁-C₄ alkyl.

In some embodiments, X¹ is -NR⁵¹R⁵², wherein R⁵¹ and R⁵², together with the nitrogen atom to which they are attached, combine to form a 3-15 membered heterocycloalkyl, wherein said heterocycloalkyl is optionally fused to one or more additional aryl or heteroaryl rings or said heterocycloalkyl is optionally connected at a single atom to another cycloalkyl or heterocycloalkyl to form a 10- to 15-membered spirocyclic heterocycle, or said heterocycloalkyl optionally comprises a C₂ bridging alkylene; and wherein said 3-15 membered heterocycloalkyl is optionally substituted with one or more, preferably one or two, R⁵³;
R⁵³ is independently selected from -C₁-C₄ alkyl, -C₃-C₆ cycloalkyl, -C₁-C₄ alkylene-C₃-C₆ cycloalkyl, -C₆-C₁₀ aryl, -C₁-C₄ alkylene-C₆-C₁₀ aryl, -C(O)-C₆-C₁₀ aryl, 3-10 membered heterocycloalkyl, -C₁-C₄ alkylene-3-10 membered heterocycloalkyl, 5-10 membered heteroaryl, -C₁-C₄ alkylene-5-10 membered heteroaryl, -CH(OH)-5-10 membered heteroaryl, - NR⁵⁵R⁵⁶, -OR⁵⁴, or oxo;
wherein said -C₁-C₄ alkyl is optionally substituted with one or more -N(C₁-C₄ alkyl)₂, - OH, -OCH₂CH₂OH, or phenyl;
wherein each aryl is optionally substituted with one or more -C₁-C₄ alkyl, -C₁-C₄ haloalkyl, phenyl, halogen, -OCH₃, or -SO₂NH₂;
wherein each heterocycloalkyl is optionally substituted with one or more -C₁-C₄ alkyl or oxo;
wherein each heteroaryl is optionally substituted with one or more oxo, -C₁-C₄ alkyl, - C₁-C₄ haloalkyl, or halogen;
R⁵⁵ and R⁵⁶ are each independently -H, -C₁-C₄ alkyl, -CO₂C₁-C₄ alkyl, -SO₂phenyl, - C₆-C₁₀ aryl, or 5- to 6-membered heteroaryl, wherein each -C₆-C₁₀ aryl or 5- to 6-membered heteroaryl is optionally substituted with one or more -C₁-C₄ alkyl, halogen -OC₁-C₄ alkyl, or - CN; and
R⁵⁴ is independently -H, -C₆-C₁₀ aryl or -C₅-C₆ cycloalkyl, wherein said cycloalkyl is optionally substituted by one or more -C₁-C₄ alkyl.

In some embodiments, X¹ is -NR⁵¹R⁵², wherein R⁵¹ and R⁵², together with the nitrogen atom to which they are attached, combine to form a 3-15 membered heterocycloalkyl, wherein said heterocycloalkyl is optionally fused to one or more additional aryl or heteroaryl rings or said heterocycloalkyl is optionally connected at a single atom to another cycloalkyl or heterocycloalkyl to form a 10- to 15-membered spirocyclic heterocycle, or said heterocycloalkyl optionally comprises a C₂ bridging alkylene; and wherein said 3-15 membered heterocycloalkyl is optionally substituted with one or more, preferably one or two, R⁵³;
R⁵³ is independently selected from -C₁-C₄ alkyl, -C₃-C₆ cycloalkyl, -C₁-C₃ alkylene-C₃-C₆ cycloalkyl, -C₆-C₁₀ aryl, -C₁-C₃ alkylene-C₆-C₁₀ aryl, -C(O)-C₆-C₁₀ aryl, 3-10 membered heterocycloalkyl, -C₁-C₃ alkylene-3-10 membered heterocycloalkyl, 5-10 membered heteroaryl, -C₁-C₃ alkylene-5-10 membered heteroaryl, -CH(OH)-5-10 membered heteroaryl, - NR⁵⁵R⁵⁶, -OR⁵⁴, or oxo;
wherein said -C₁-C₄ alkyl is optionally substituted with one or more -N(C₁-C₂ alkyl)₂, - OH, -OCH₂CH₂OH, or phenyl;
wherein each aryl is optionally substituted with one or more -C₁-C₂ alkyl, -C₁-C₂ haloalkyl, phenyl, halogen, -OCH₃, or -SO₂NH₂;
wherein each heterocycloalkyl is optionally substituted with one or more -C₁-C₂ alkyl or oxo;
wherein each heteroaryl is optionally substituted with one or more oxo, -C₁-C₂ alkyl, - C₁-C₂ haloalkyl, or halogen;
R⁵⁵ and R⁵⁶ are each independently -H, -C₁-C₂ alkyl, -CO₂C₁-C₄ alkyl, -SO₂phenyl, - C₆-C₁₀ aryl, or 5- to 6-membered heteroaryl, wherein each -C₆-C₁₀ aryl or 5- to 6-membered heteroaryl is optionally substituted with one or more -C₁-C₂ alkyl, halogen -OC₁-C₂ alkyl, or - CN; and
R⁵⁴ is independently -H, -C₆-C₁₀ aryl or -C₅-C₆ cycloalkyl, wherein said cycloalkyl is optionally substituted by one or more -C₁-C₂ alkyl.

In some embodiments, X¹ is -NR⁵¹R⁵², wherein R⁵¹ and R⁵², together with the nitrogen atom to which they are attached, combine to form a membered heterocycloalkyl, wherein said heterocycloalkyl is selected from azetidine, pyrrolidine, imidazolidine, piperidine, piperazine, morpholine, or 1,4-diazepane, wherein said azetidine, pyrrolidine, imidazolidine, piperidine, piperazine, morpholine, or 1,4-diazepane is optionally fused to one or more additional aryl or heteroaryl rings or said heterocycloalkyl is optionally connected at a single atom to another cycloalkyl or heterocycloalkyl to form a 10- to 15-membered spirocyclic heterocycle, or said heterocycle is piperidine optionally comprising a C₂ bridging alkylene; and wherein said heterocycloalkyl is further optionally substituted with one or more, preferably one or two, R⁵³;
R⁵³ is independently selected from -C₁-C₄ alkyl, -C₃-C₆ cycloalkyl, -C₁-C₃ alkylene-C₃-C₆ cycloalkyl, -C₆-C₁₀ aryl, -C₁-C₃ alkylene-C₆-C₁₀ aryl, -C(O)-C₆-C₁₀ aryl, 3-10 membered heterocycloalkyl, -C₁-C₃ alkylene-3-10 membered heterocycloalkyl, 5-10 membered heteroaryl, -C₁-C₃ alkylene-5-10 membered heteroaryl, -CH(OH)-5-10 membered heteroaryl, - NR⁵⁵R⁵⁶, -OR⁵⁴, or oxo;
wherein said -C₁-C₄ alkyl is optionally substituted with one or more -N(C₁-C₂ alkyl)₂, - OH, -OCH₂CH₂OH, or phenyl;
wherein each aryl is optionally substituted with one or more -C₁-C₂ alkyl, -CF₃, phenyl, halogen, -OCH₃, or -SO₂NH₂;
wherein each heterocycloalkyl is optionally substituted with one or more -C₁-C₂ alkyl or oxo;
wherein each heteroaryl is optionally substituted with one or more oxo, -C₁-C₂ alkyl, - CF₃, or halogen;
R⁵⁵ and R⁵⁶ are each independently -H, -C₁-C₂ alkyl, -CO₂C₁-C₄ alkyl, -SO₂phenyl, - C₆-C₁₀ aryl, or 5- to 6-membered heteroaryl, wherein each -C₆-C₁₀ aryl or 5- to 6-membered heteroaryl is optionally substituted with one or more -C₁-C₂ alkyl, halogen -OC₁-C₂ alkyl, or - CN; and
R⁵⁴ is independently -H, -C₆-C₁₀ aryl or -C₅-C₆ cycloalkyl, wherein said cycloalkyl is optionally substituted by one or more -C₁-C₂ alkyl.

In some embodiments, X¹ is -NR⁵¹R⁵², wherein R⁵¹ and R⁵², together with the nitrogen atom to which they are attached, combine to form a membered heterocycloalkyl, wherein said heterocycloalkyl is selected from azetidine, pyrrolidine, imidazolidine, piperidine, piperazine, morpholine, or 1,4-diazepane, wherein said azetidine, pyrrolidine, imidazolidine, piperidine, piperazine, morpholine, or 1,4-diazepane is optionally fused to one or more additional aryl or heteroaryl rings or said heterocycloalkyl is optionally connected at a single atom to another cycloalkyl or heterocycloalkyl to form a 10- to 15-membered spirocyclic heterocycle, or said heterocycle is piperidine optionally comprising a C₂ bridging alkylene; and wherein said heterocycloalkyl is further optionally substituted with one or more, preferably one or two, R⁵³, and wherein when said heterocycloalkyl is azetidine, said azetidine is substituted at the 3-position and when said heterocycloalkyl is piperidine or piperazine, said piperidine or piperazine is substituted at the 4-position;
R⁵³ is independently selected from -C₁-C₄ alkyl, -C₃-C₆ cycloalkyl, -C₁-C₃ alkylene-C₃-C₆ cycloalkyl, -C₆-C₁₀ aryl, -C₁-C₃ alkylene-C₆-C₁₀ aryl, -C(O)-C₆-C₁₀ aryl, 3-10 membered heterocycloalkyl, -C₁-C₃ alkylene-3-10 membered heterocycloalkyl, 5-10 membered heteroaryl, -C₁-C₃ alkylene-5-10 membered heteroaryl, -CH(OH)-5-10 membered heteroaryl, - NR⁵⁵R⁵⁶, -OR⁵⁴, or oxo;
wherein said -C₁-C₄ alkyl is optionally substituted with one or more -N(C₁-C₂ alkyl)₂, - OH, -OCH₂CH₂OH, or phenyl;
wherein each aryl is optionally substituted with one or more -C₁-C₂ alkyl, -CF₃, phenyl, halogen, -OCH₃, or -SO₂NH₂;
wherein each heterocycloalkyl is optionally substituted with one or more -C₁-C₂ alkyl or oxo;
wherein each heteroaryl is optionally substituted with one or more oxo, -C₁-C₂ alkyl, - CF₃, or halogen;
R⁵⁵ and R⁵⁶ are each independently -H, -C₁-C₂ alkyl, -CO₂C₁-C₄ alkyl, -SO₂phenyl, - C₆-C₁₀ aryl, or 5- to 6-membered heteroaryl, wherein each -C₆-C₁₀ aryl or 5- to 6-membered heteroaryl is optionally substituted with one or more -C₁-C₂ alkyl, halogen -OC₁-C₂ alkyl, or - CN; and
R⁵⁴ is independently -H, -C₆-C₁₀ aryl or -C₅-C₆ cycloalkyl, wherein said cycloalkyl is optionally substituted by one or more -C₁-C₂ alkyl.

In some embodiments, X¹ is -NR⁵¹R⁵², wherein R⁵¹ and R⁵², together with the nitrogen atom to which they are attached, combine to form a membered heterocycloalkyl, wherein said heterocycloalkyl is selected from azetidine, pyrrolidine, imidazolidine, piperidine, piperazine, morpholine, or 1,4-diazepane, wherein said heterocycloalkyl is further optionally substituted with one or more, preferably one or two, R⁵³;
R⁵³ is independently selected from -C₁-C₄ alkyl, -C₃-C₆ cycloalkyl, -C₁-C₃ alkylene-C₃-C₆ cycloalkyl, -C₆-C₁₀ aryl, -C₁-C₃ alkylene-C₆-C₁₀ aryl, -C(O)-C₆-C₁₀ aryl, 3-10 membered heterocycloalkyl, -C₁-C₃ alkylene-3-10 membered heterocycloalkyl, 5-10 membered heteroaryl, -C₁-C₃ alkylene-5-10 membered heteroaryl, -CH(OH)-5-10 membered heteroaryl, - NR⁵⁵R⁵⁶, -OR⁵⁴, or oxo;
wherein said -C₁-C₄ alkyl is optionally substituted with one or more -N(C₁-C₂ alkyl)₂, - OH, -OCH₂CH₂OH, or phenyl;
wherein each aryl is optionally substituted with one or more -C₁-C₂ alkyl, -CF₃, phenyl, halogen, -OCH₃, or -SO₂NH₂;
wherein each heterocycloalkyl is optionally substituted with one or more -C₁-C₂ alkyl or oxo;
wherein each heteroaryl is optionally substituted with one or more oxo, -C₁-C₂ alkyl, - CF₃, or halogen;
R⁵⁵ and R⁵⁶ are each independently -H, -C₁-C₂ alkyl, -CO₂C₁-C₄ alkyl, -SO₂phenyl, - C₆-C₁₀ aryl, or 5- to 6-membered heteroaryl, wherein each -C₆-C₁₀ aryl or 5- to 6-membered heteroaryl is optionally substituted with one or more -C₁-C₂ alkyl, halogen -OC₁-C₂ alkyl, or - CN; and
R⁵⁴ is independently -H, -C₆-C₁₀ aryl or -C₅-C₆ cycloalkyl, wherein said cycloalkyl is optionally substituted by one or more -C₁-C₂ alkyl.

In one aspect, the present invention provides a pharmaceutical composition comprising at least one compound according to the present disclosure, or a pharmaceutically acceptable salt, tautomer, solvate or hydrate thereof, and a pharmaceutically acceptable excipient.

In one aspect, the present invention provides a compound according to the present disclosure or a pharmaceutically acceptable salt, tautomer, solvate or hydrate thereof, or a pharmaceutical composition comprising a compound of the present disclosure, for use in a method of preventing or treating a disease in a subject, preferably an infection, further preferably a bacterial infection, yet further preferably a bacterial infection caused by one or more bacterium belonging to a genus of non-tuberculous *Mycobacteria,* preferably *M. abscessus.*

In one aspect, the present invention provides a method of treating a bacterial infection in a subject in need thereof, comprising administering to the subject an effective amount of a compound of the present disclosure or a pharmaceutically acceptable salt, tautomer, solvate, or hydrate thereof. In one aspect, the present invention provides a method of treating a bacterial infection in a subject in need thereof, comprising administering to the subject an effective amount of a pharmaceutical composition comprising a compound of the present disclosure or a pharmaceutically acceptable salt, tautomer, solvate, or hydrate thereof.

In one aspect, the present invention provides the use of a compound of the present disclosure or a pharmaceutically acceptable salt, tautomer, solvate, or hydrate thereof in the manufacture of a medicament for treating a bacterial infection in a subject in need thereof. In one aspect, the present invention provides the use of a pharmaceutical composition comprising a compound of the present disclosure or a pharmaceutically acceptable salt, tautomer, solvate, or hydrate thereof in the manufacture of a medicament for treating a bacterial infection in a subject in need thereof.

In some embodiments, the bacterial infection is caused by one or more bacterium belonging to a genus of non-tuberculous *Mycobacteria,* preferably *M. abscessus.* In some embodiments, the bacterial infection is caused by *A. baumannii.* In some embodiments, the bacterial infection is caused by a strain of *A. baumannii* carrying a resistance mutation in the RpoB gene. In some preferred embodiments, the resistance mutation is H535Q; H535C; or H535L.

In one or more embodiments, X¹ of a compound of the present invention can form one of the structures selected from Table 1, below:

**Table 1. Exemplary Embodiments of X¹**

| Embodiment | Structure | Embodiment | Structure |
|---|---|---|---|
| 1 | 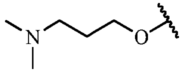 | 2 | 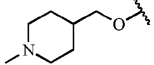 |
| 3 | 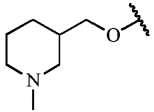 | 4 | 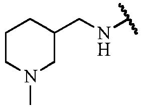 |
| 5 | 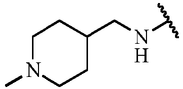 | 6 | 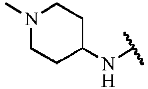 |
| 7 | 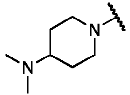 | 8 | 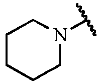 |
| 9 | 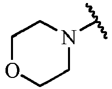 | 10 | 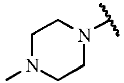 |
| 11 | 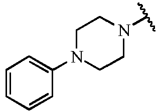 | 12 | 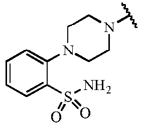 |
| 13 | 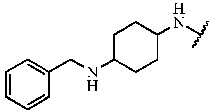 | 14 | 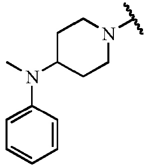 |
| 15 | 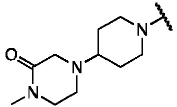 | 16 | 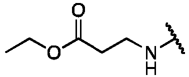 |
| 17 | 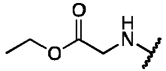 | 18 | 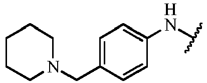 |
| 19 | 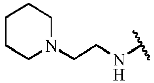 | 20 | 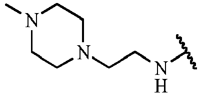 |
| 21 | 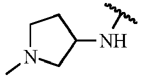 | 22 | 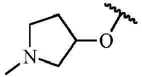 |
| 23 | 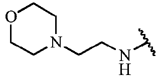 | 24 | 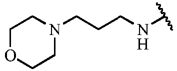 |
| 25 | 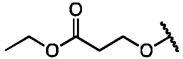 | 26 | 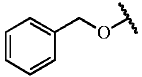 |
| 27 | 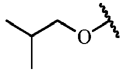 | 28 | 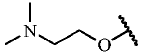 |
| 29 | 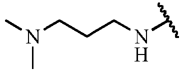 | 30 | 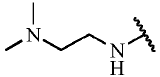 |
| 31 | 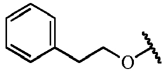 | 32 | 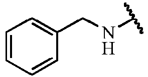 |
| 33 | 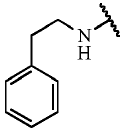 | 34 | 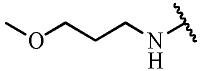 |
| 35 | 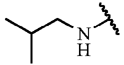 | 36 | 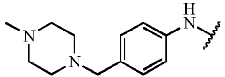 |
| 37 | 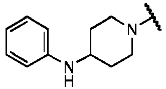 | 38 | 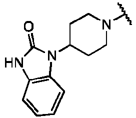 |
| 39 | 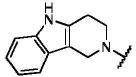 | 40 | 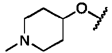 |
| 41 | 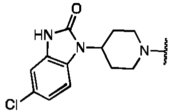 | 42 | 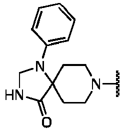 |
| 43 | 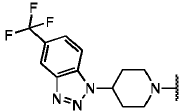 | 44 | 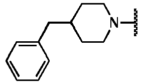 |
| 45 | 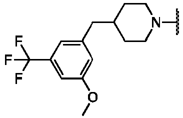 | 46 | 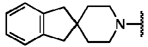 |
| 47 | 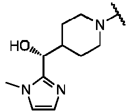 | 48 | 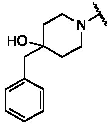 |
| 49 | 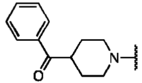 | 50 | 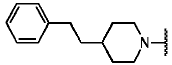 |
| 51 | 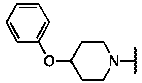 | 52 | 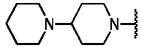 |
| 53 | 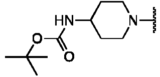 | 54 | 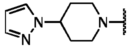 |
| 55 | 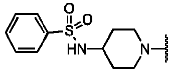 | 56 | 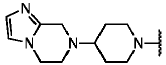 |
| 57 | 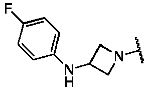 | 58 | 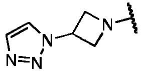 |
| 59 | 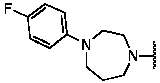 | 60 | 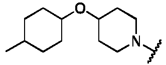 |
| 61 | 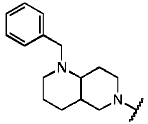 | 62 | 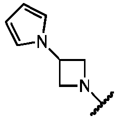 |
| 63 | 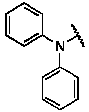 | 64 | 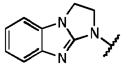 |
| 65 | 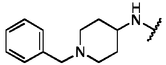 | 66 | 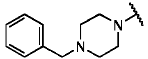 |
| 67 | 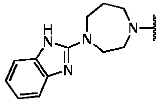 | 68 | 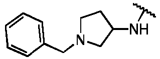 |
| 69 | 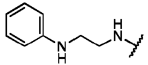 | 70 | 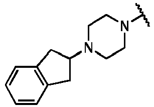 |
| 71 | 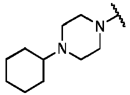 | 72 | 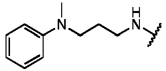 |
| 73 | 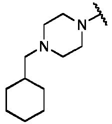 | 74 | 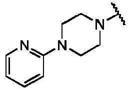 |
| 75 | 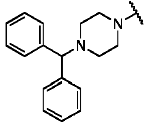 | 76 | 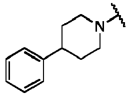 |
| 77 | 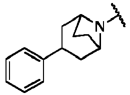 | 78 | 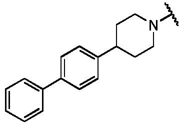 |
| 79 | 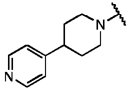 | 80 | 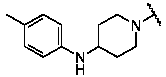 |
| 81 | 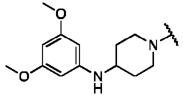 | 82 | 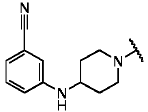 |
| 83 | 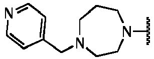 | 84 | 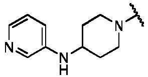 |
| 85 | 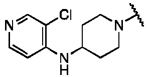 | 86 | 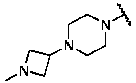 |
| 87 | 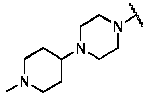 | 88 | 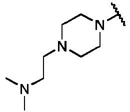 |
| 89 | 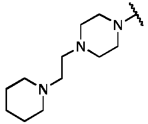 | 90 | 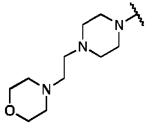 |
| 91 | 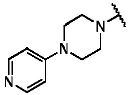 | 92 | 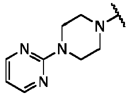 |
| 93 | 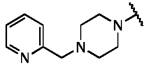 | 94 | 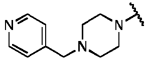 |
| 95 | 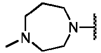 | 96 | 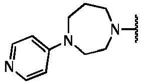 |
| 97 | 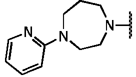 | 98 | 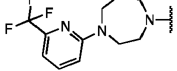 |
| 99 | 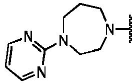 | 100 | 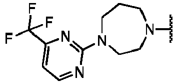 |
| 101 | 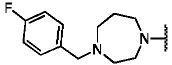 | 102 | 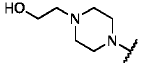 |
| 103 | 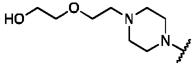 | 104 | 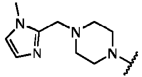 |
| 105 | 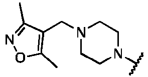 | 106 | 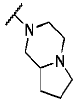 |
| 107 | 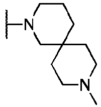 | 108 | 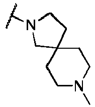 |
| 109 | 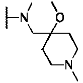 | 110 | 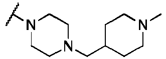 |

In one or more embodiments of any of the above aspects, the compound of Formula I is selected from Table 2, below:

**Table 2. Exemplary Compounds (Cpds) of the Invention**

| Cpd | Structure | Cpd | Structure |
|---|---|---|---|
| 1 | 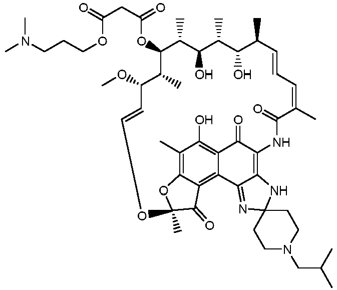 | 2 | 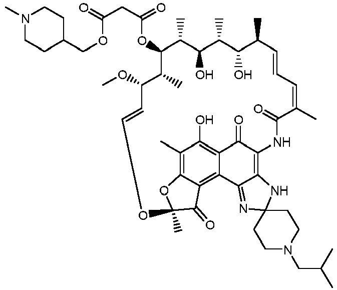 |
| 3 | 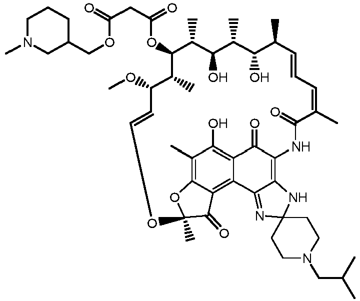 | 4 | 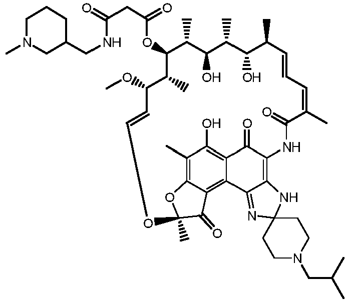 |
| 5 | 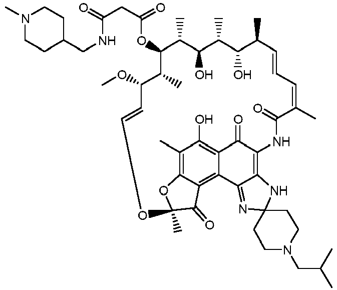 | 6 | 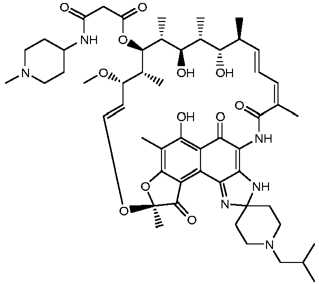 |
| 7 | 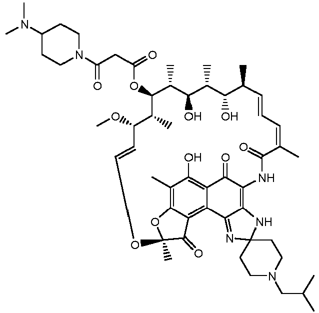 | 8 | 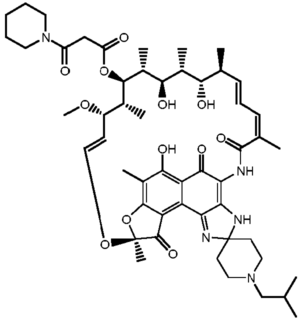 |
| 9 | 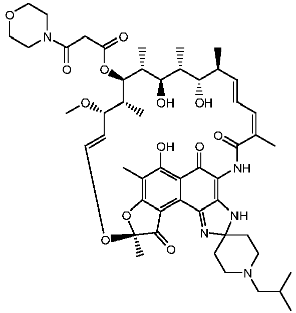 | 10 | 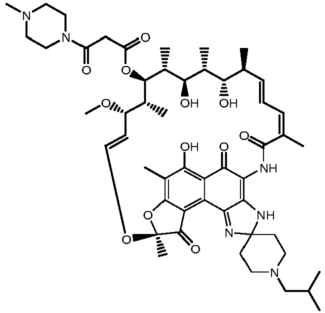 |
| 11 | 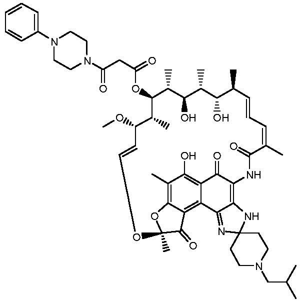 | 12 | 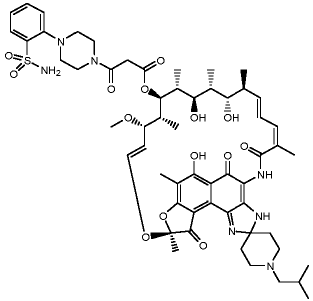 |
| 13 | 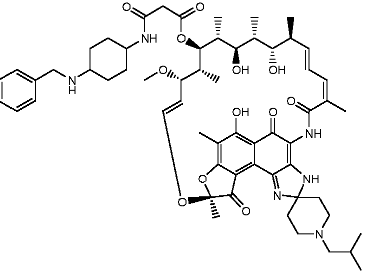 | 14 | 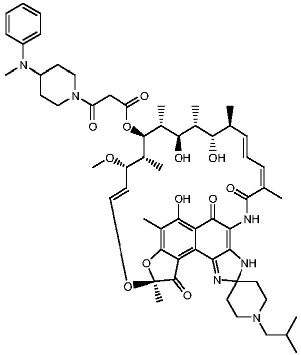 |
| 15 | 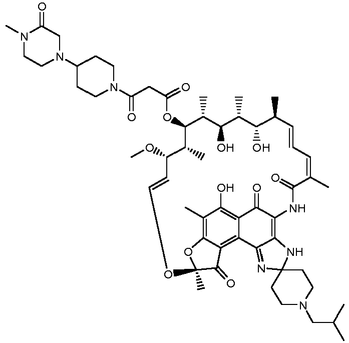 | 16 | 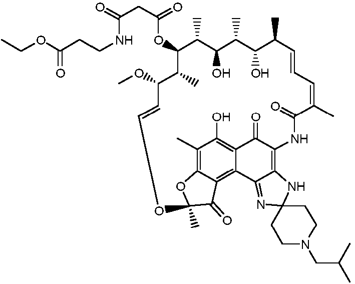 |
| 17 | 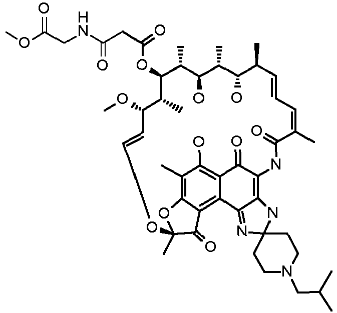 | 18 | 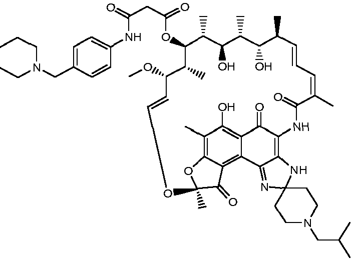 |
| 19 | 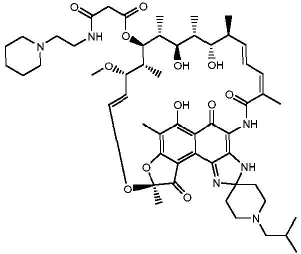 | 20 | 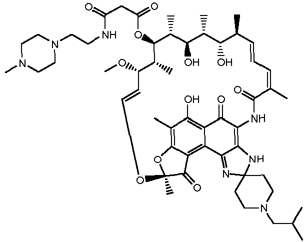 |
| 21 | 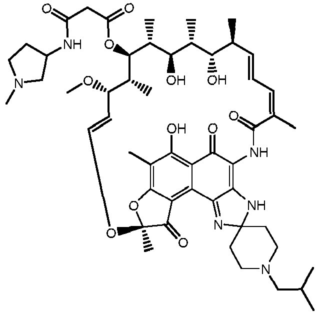 | 22 | 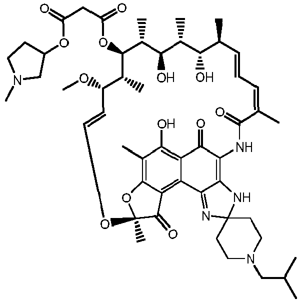 |
| 23 | 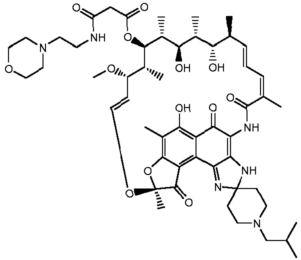 | 24 | 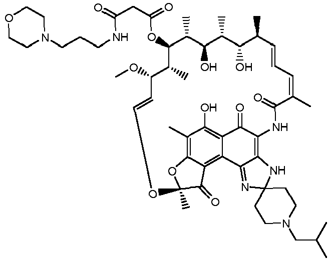 |
| 25 | 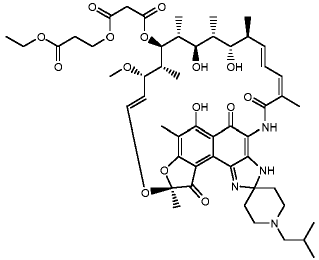 | 26 | 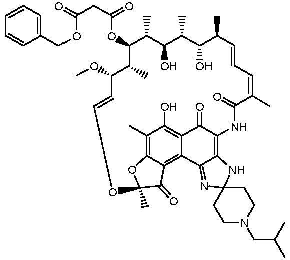 |
| 27 | 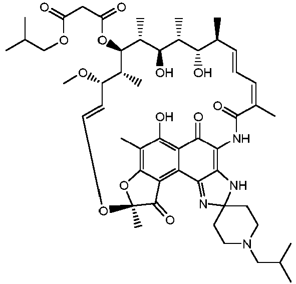 | 28 | 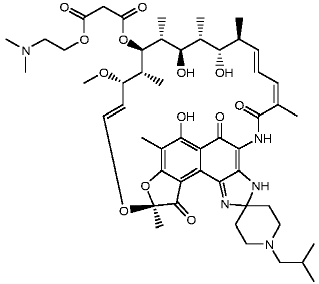 |
| 29 | 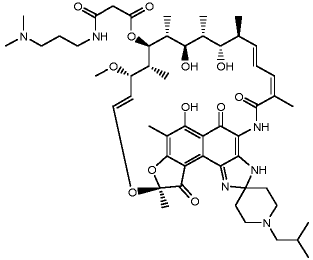 | 30 | 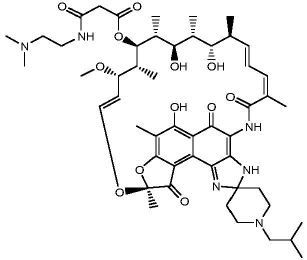 |
| 31 | 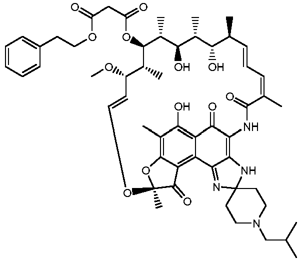 | 32 | 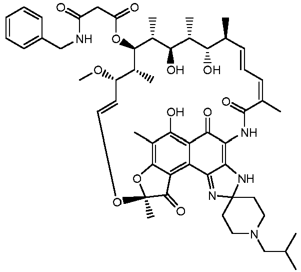 |
| 33 | 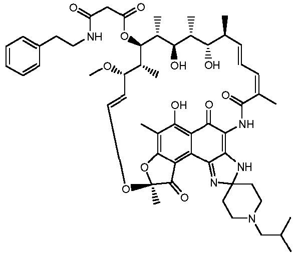 | 34 | 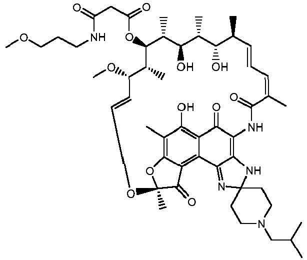 |
| 35 | 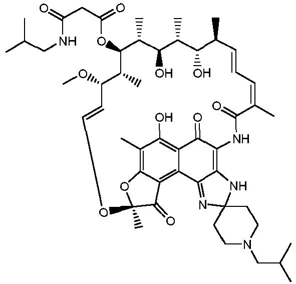 | 36 | 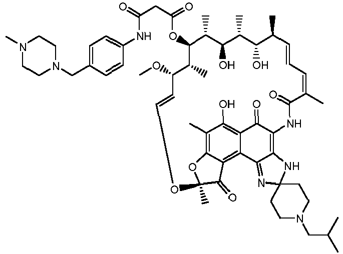 |
| 37 | 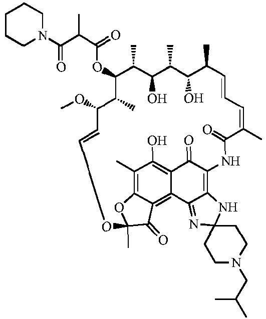 | 38 | 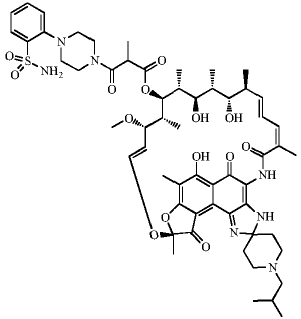 |
| 39 | 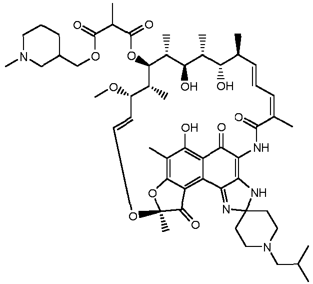 | 40 | 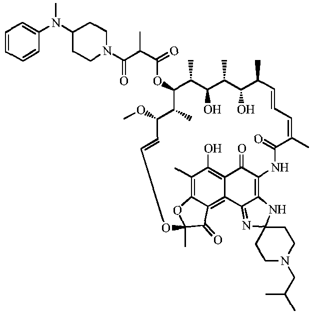 |
| 41 | 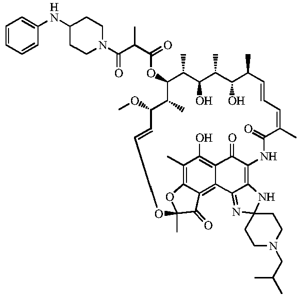 | 42 | 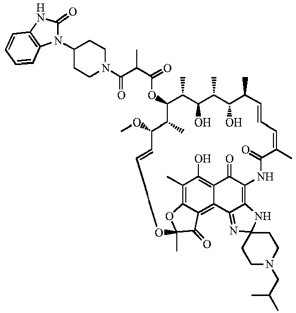 |
| 43 | 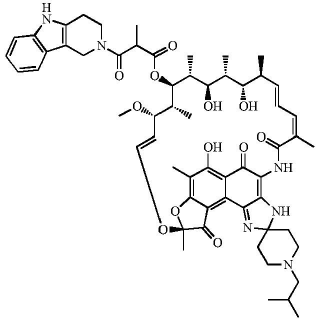 | 44 | 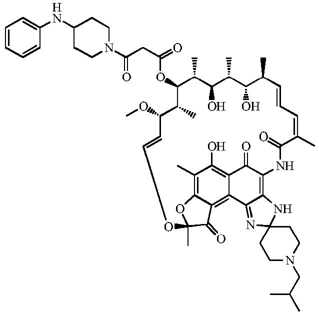 |
| 45 | 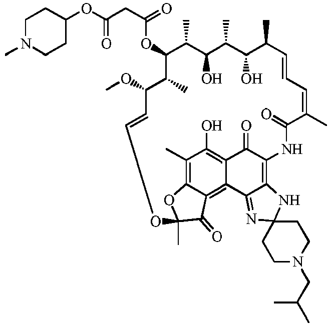 | 46 | 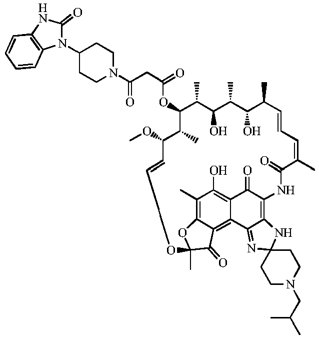 |
| 47 | 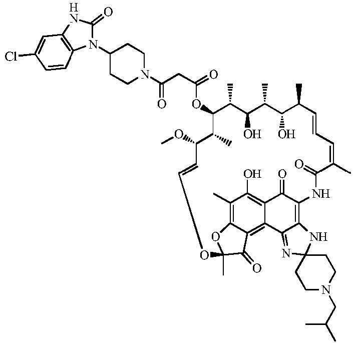 | 48 | 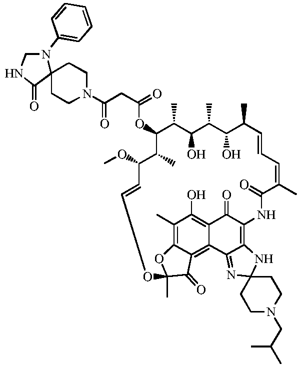 |
| 49 | 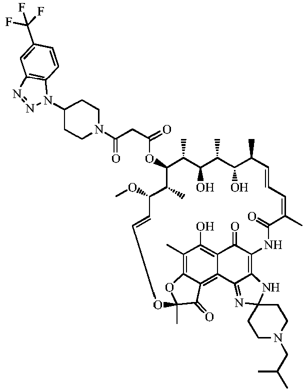 | 50 | 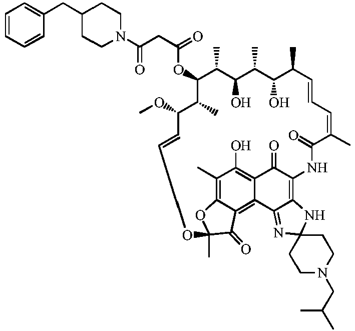 |
| 51 | 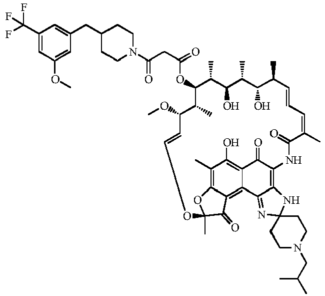 | 52 | 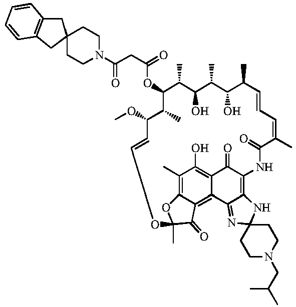 |
| 53 | 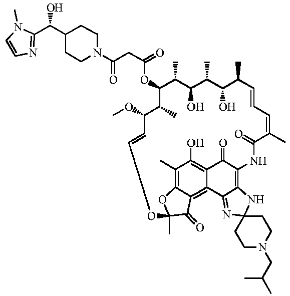 | 54 | 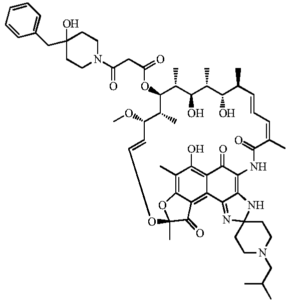 |
| 55 | 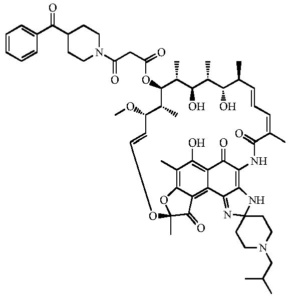 | 56 | 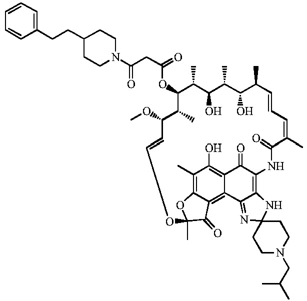 |
| 57 | 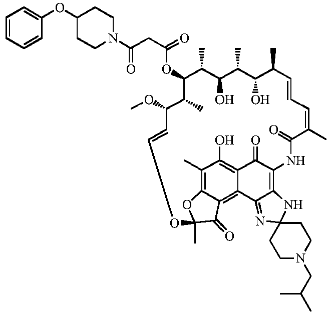 | 58 | 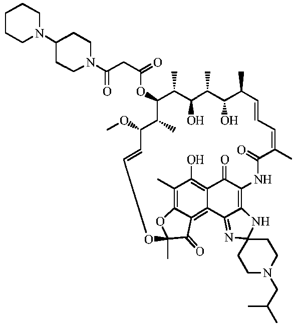 |
| 59 | 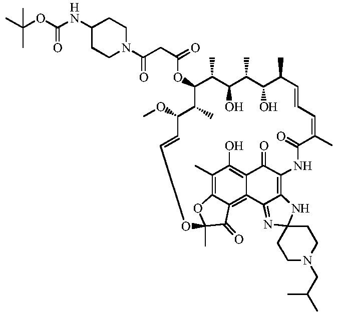 | 60 | 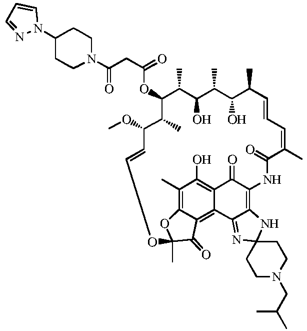 |
| 61 | 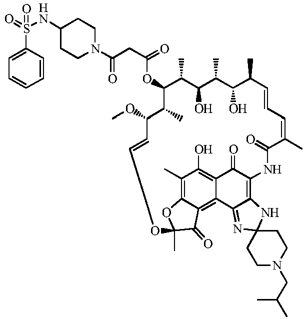 | 62 | 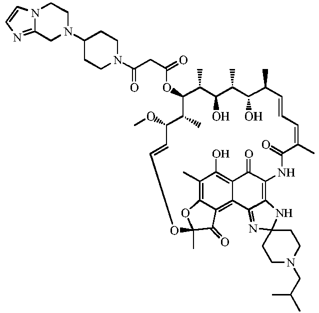 |
| 63 | 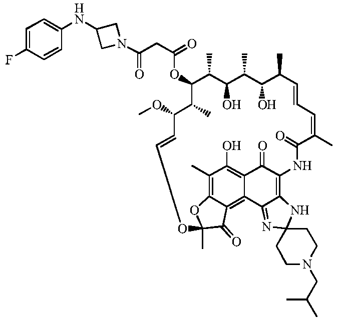 | 64 | 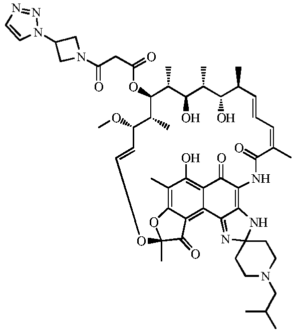 |
| 65 | 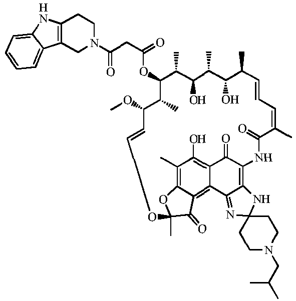 | 66 | 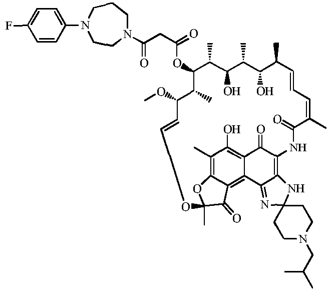 |
| 67 | 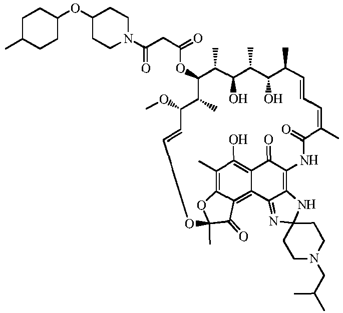 | 68 | 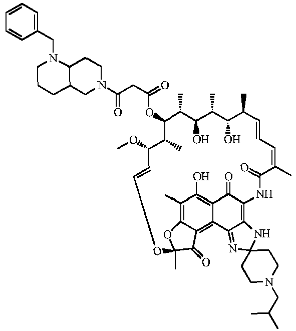 |
| 69 | 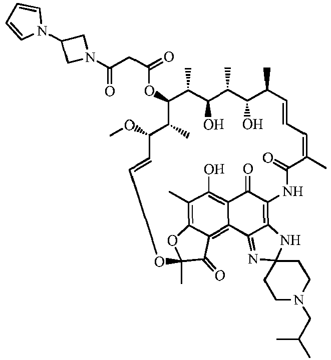 | 70 | 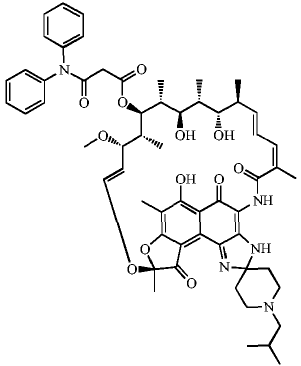 |
| 71 | 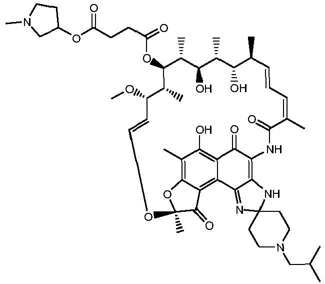 | 72 | 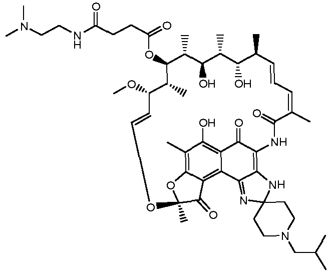 |
| 73 | 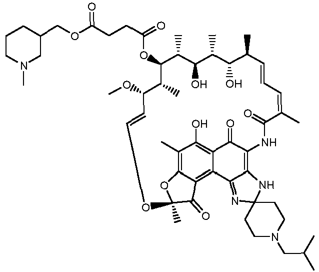 | 74 | 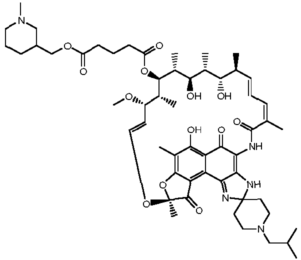 |
| 75 | 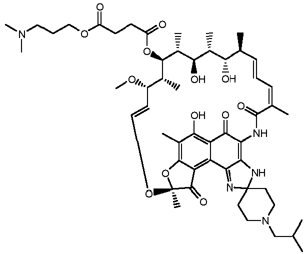 | 76 | 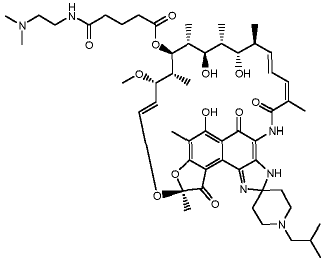 |
| 77 | 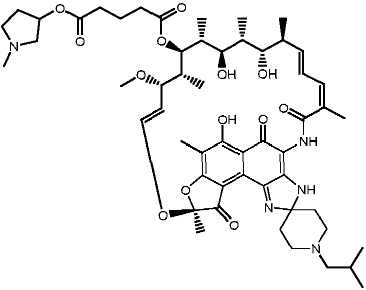 | 78 | 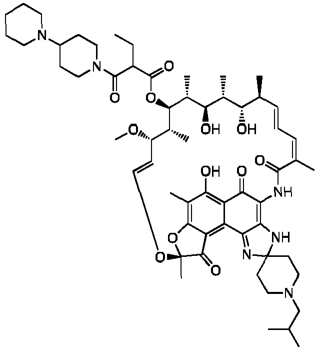 |
| 79 | 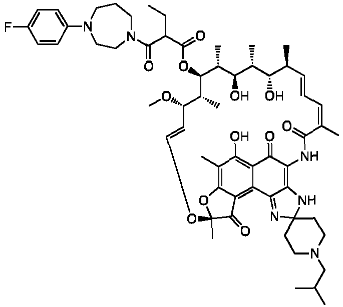 | 80 | 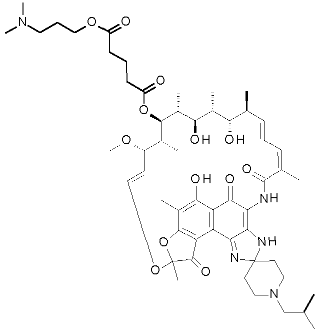 |
| 81 | 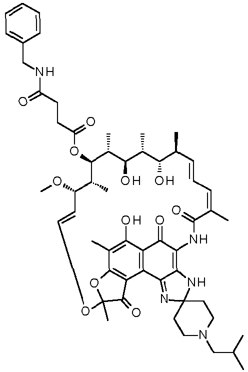 | 82 | 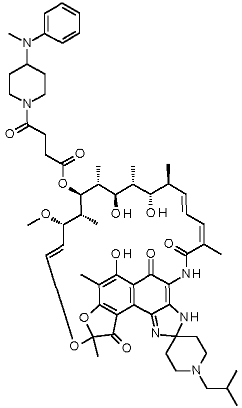 |
| 83 | 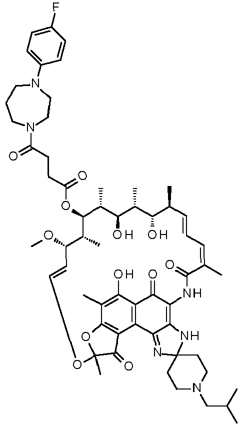 | 84 | 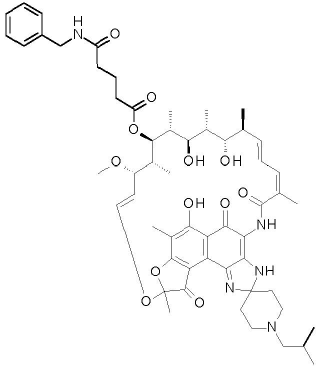 |
| 85 | 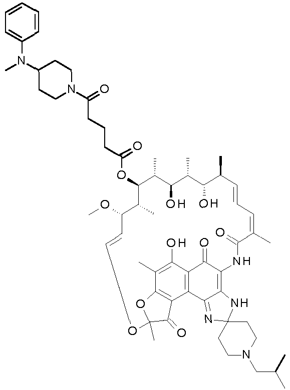 | 86 | 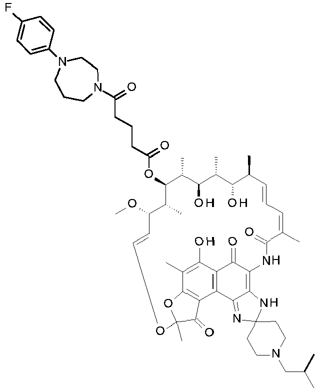 |
| 87 | 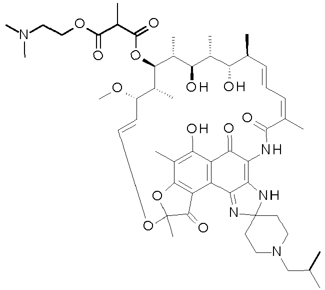 | 88 | 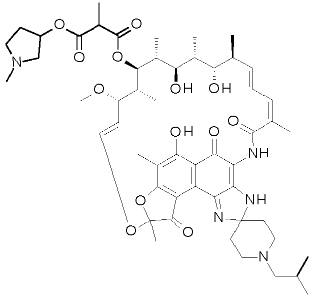 |
| 89 | 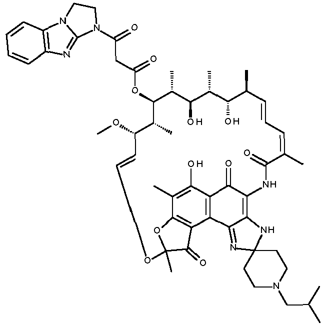 | 90 | 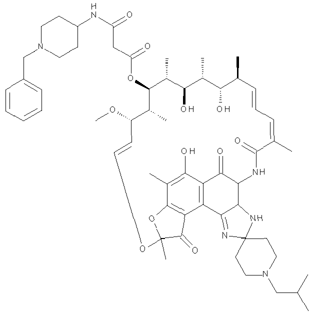 |
| 91 | 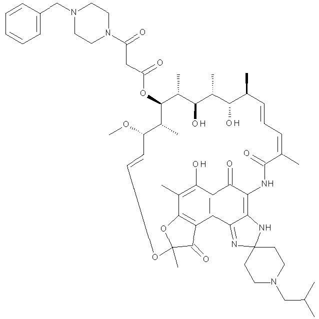 | 92 | 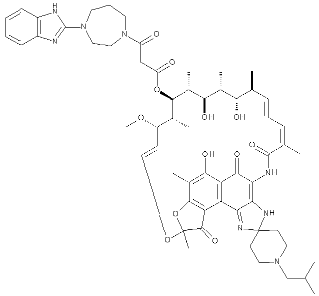 |
| 93 | 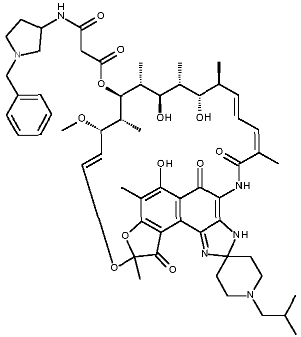 | 94 | 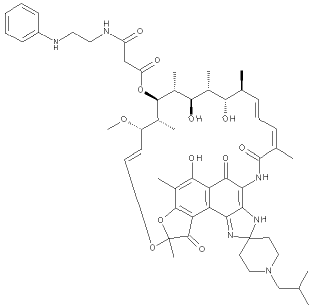 |
| 95 | 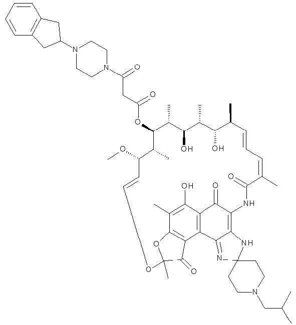 | 96 | 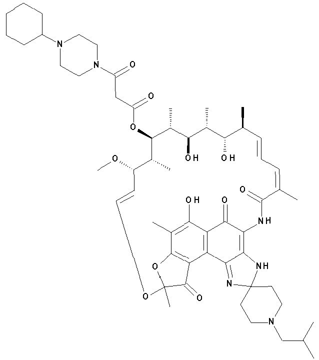 |
| 97 | 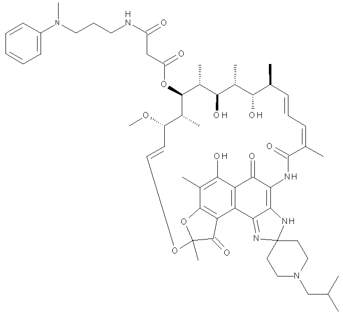 | 98 | 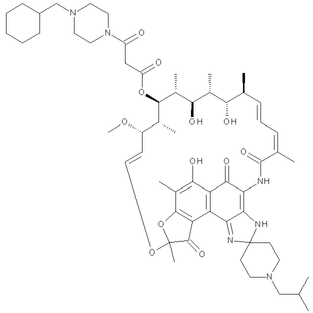 |
| 99 | 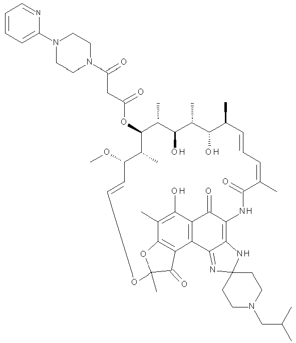 | 100 | 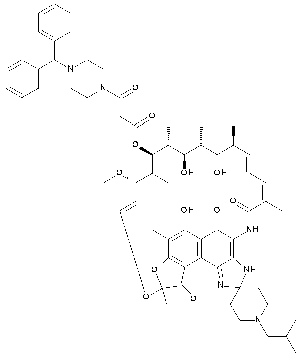 |
| 101 | 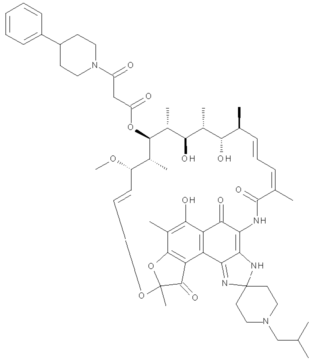 | 102 | 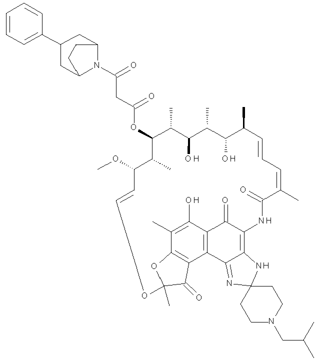 |
| 103 | 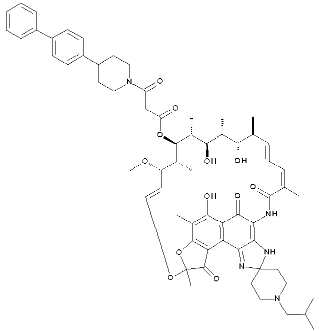 | 104 | 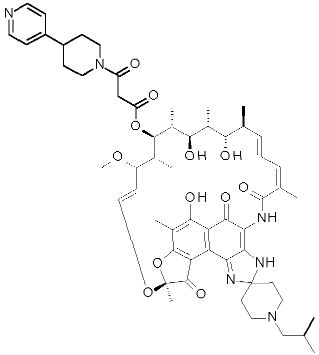 |
| 105 | 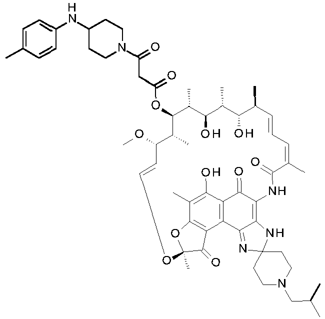 | 106 | 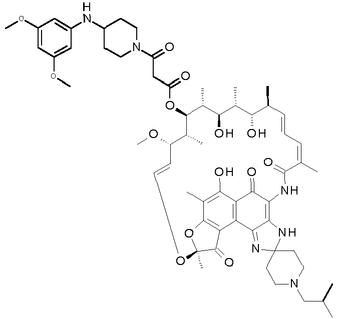 |
| 107 | 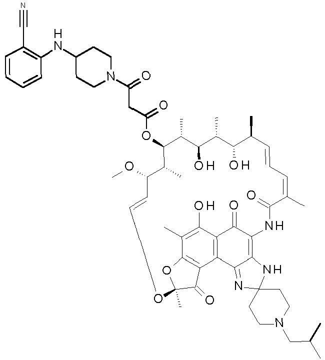 | 108 | 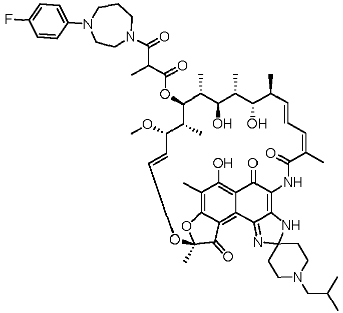 |
| 109 | 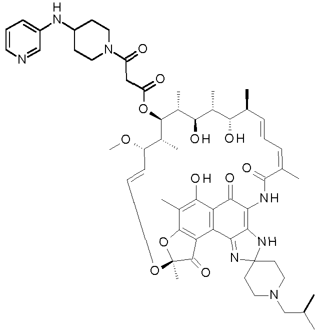 | 110 | 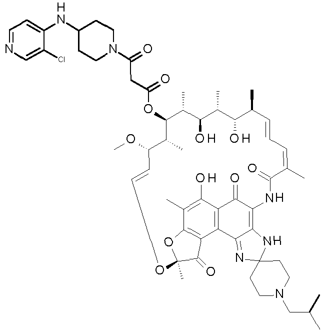 |
| 111 | 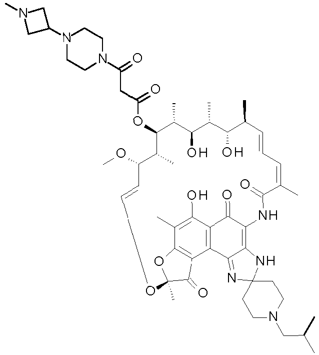 | 112 | 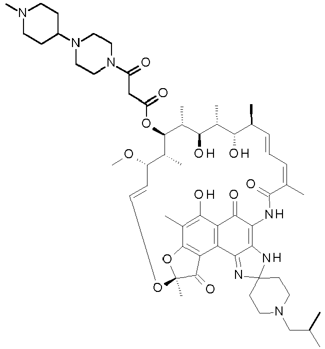 |
| 113 | 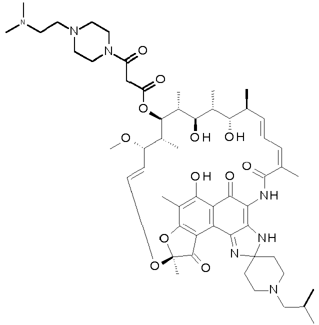 | 114 | 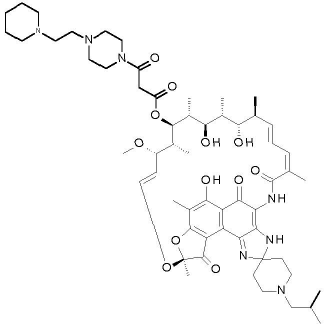 |
| 115 | 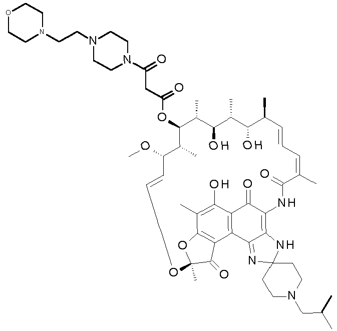 | 116 | 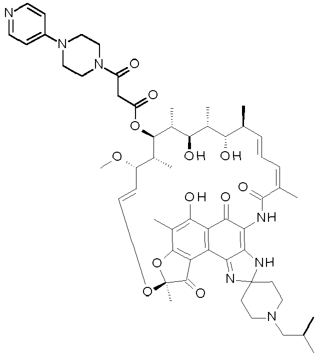 |
| 117 | 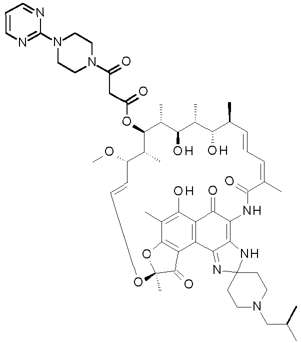 | 118 | 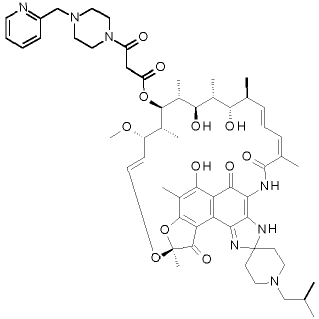 |
| 119 | 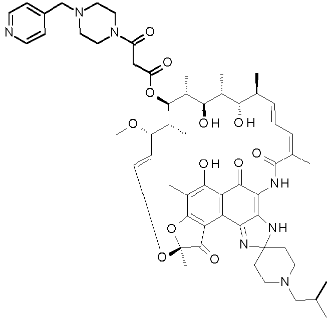 | 120 | 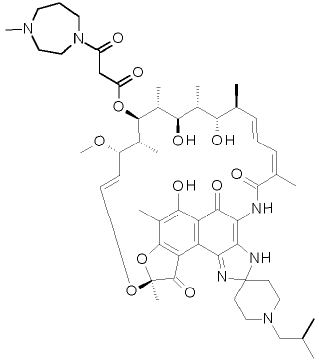 |
| 121 | 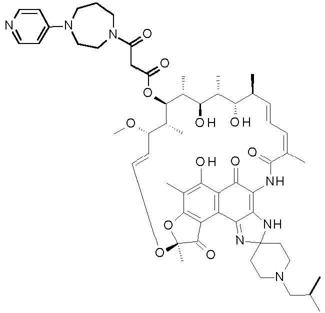 | 122 | 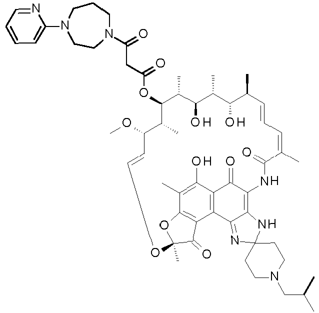 |
| 123 | 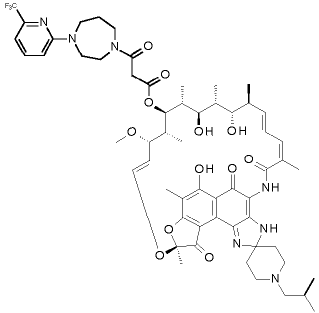 | 124 | 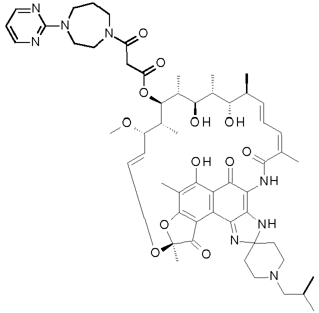 |
| 125 | 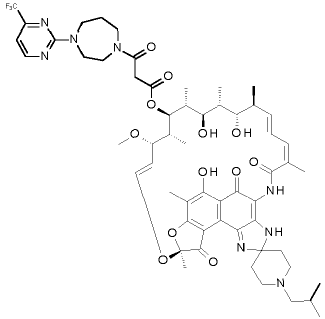 | 126 | 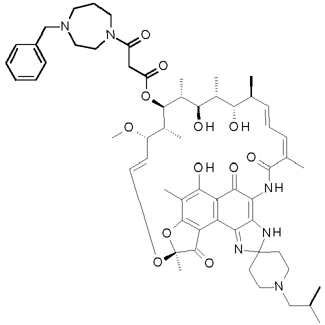 |
| 127 | 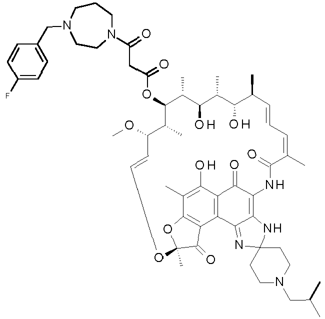 | 128 | 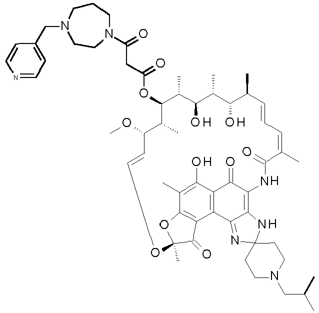 |
| 129 | 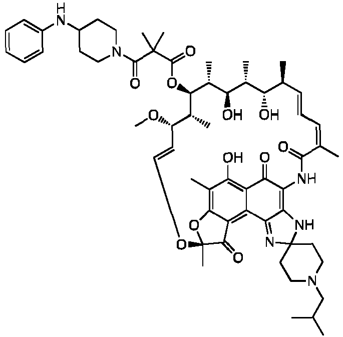 | 130 | 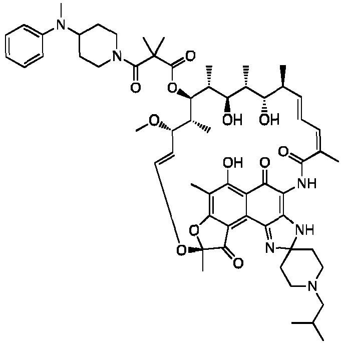 |
| 131 | 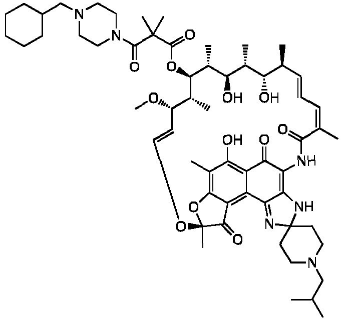 | 132 | 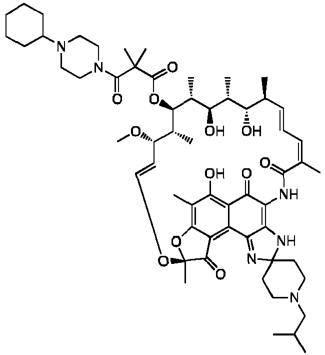 |
| 133 | 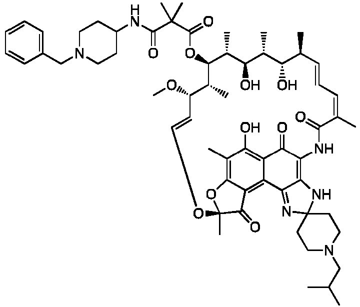 | 134 | 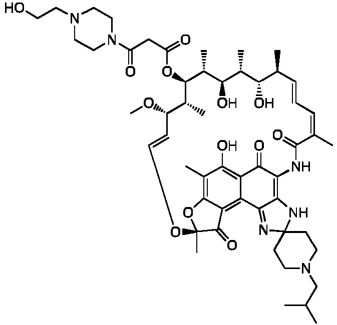 |
| 135 | 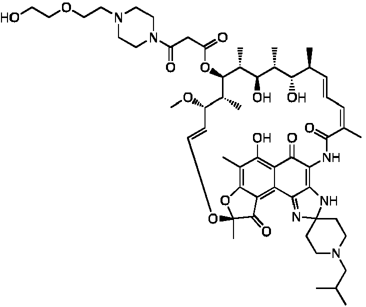 | 136 | 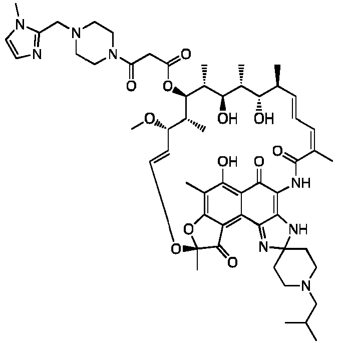 |
| 137 | 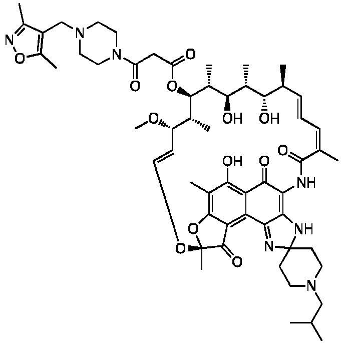 | 138 | 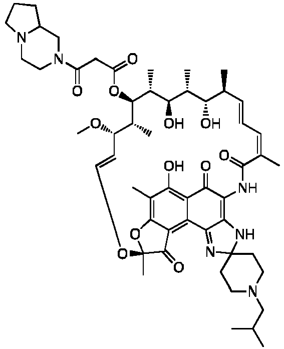 |
| 139 | 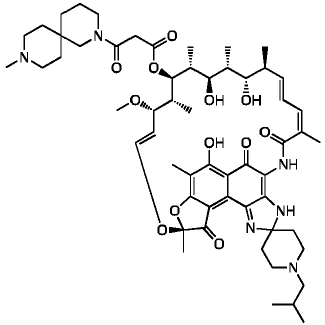 | 140 | 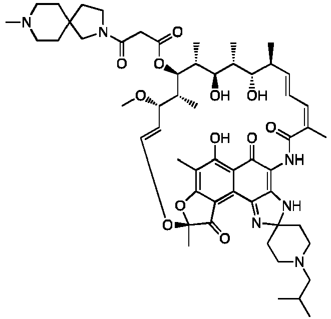 |
| 141 | 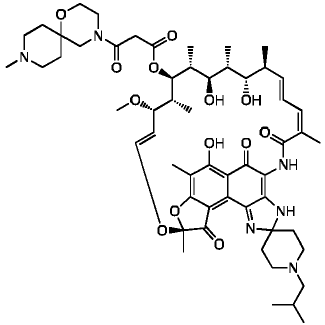 | 142 | 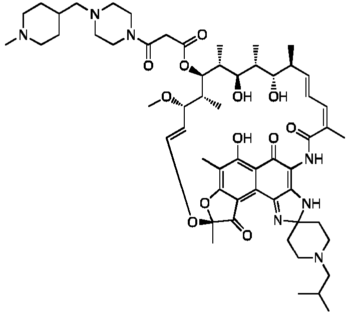 |

In one or more embodiments of any of the above aspects, the compound of Formula I is selected from Compound 44, Compound 49, Compound 52, Compound 67, Compound 85, Compound 90, Compound 91, Compound 98, Compound 105, Compound 106, Compound 109, Compound 110, Compound 118 and Compound 121.

In one or more embodiments of any of the above aspects, the compound of Formula I is selected from Compound 67, Compound 90, Compound 98 and Compound 106.

### Methods of Synthesizing the Compounds of the Invention

The compounds of the present disclosure may be made by a variety of methods, including standard chemistry. Suitable synthetic routes are depicted in the schemes given below.

The compounds of the present disclosure may be prepared by methods known in the art of organic synthesis as set forth in part by the following synthetic schemes and examples. In the schemes described below, it is well understood that protecting groups for sensitive or reactive groups are employed where necessary in accordance with general principles or chemistry. Protecting groups are manipulated according to standard methods of organic synthesis (T. W. Greene and P. G. M. Wuts, "Protective Groups in Organic Synthesis", Third edition, Wiley, New York 1999). These groups are removed at a convenient stage of the compound synthesis using methods that are readily apparent to those skilled in the art. The selection processes, as well as the reaction conditions and order of their execution, shall be consistent with the preparation of compounds of the present disclosure.

Those skilled in the art will recognize if a stereocenter exists in the compounds of Formula I. Accordingly, the present disclosure includes both possible stereoisomers (unless specified in the synthesis) and includes not only racemic compounds but the individual enantiomers and/or diastereomers as well. In preferred embodiments the present disclosure includes enantiomers and/or diastereomers within X¹ and Y¹ of Formula I. When a compound is desired as a single enantiomer or diastereomer, it may be obtained by stereospecific synthesis or by resolution of the final product or any convenient intermediate. Resolution of the final product, an intermediate, or a starting material may be affected by any suitable method known in the art. See, for example, "Stereochemistry of Organic Compounds" by E. L. Eliel, S. H. Wilen, and L. N. Mander (Wiley - lnterscience, 1994).

The compounds described herein may be made from commercially available starting materials or synthesized using known organic, inorganic, and/or enzymatic processes.

The compounds of the present disclosure can be prepared in a number of ways well known to those skilled in the art of organic synthesis. By way of example, compounds of the present disclosure can be synthesized using the methods described below, together with synthetic methods known in the art of synthetic organic chemistry, or variations thereon as appreciated by those skilled in the art. These methods include but are not limited to those methods described below. Compounds of the present disclosure can be synthesized by following the steps outlined in General Schemes 1, 2, and 3. Starting materials are either commercially available or made by known procedures in the reported literature or as illustrated.

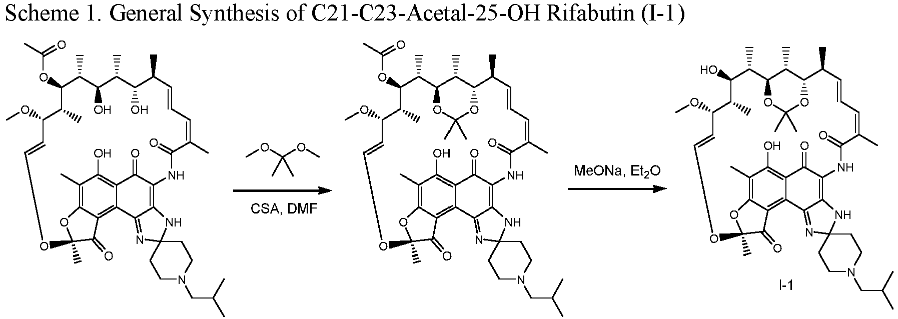

A general method of preparing C21-C23-Acetal-25-OH rifabutin is shown above in Scheme 1. Protection of the C21 and C23 hydroxy groups using dimethoxy propane and camphorsulphonic acid in DMF afforded an acetal-protected rifabutin. De-acetylation of the protected rifabutin using sodium methoxide in ether afforded the 25-OH rifabutin with the C21 and C23 hydroxy groups protected (I-1).

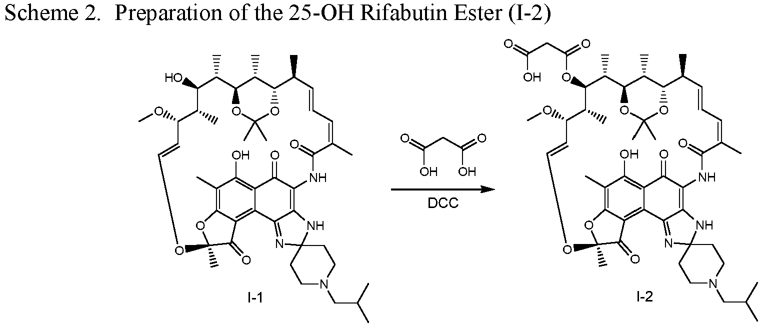

C21-C23-protected, 25-OH rifabutin (I-1) can be esterified using an appropriate carboxylic acid such as malonic acid in the presence of a coupling reagent such as DCC. Other di-carboxylic acids (e.g., glutaric acid, succinic acid, adipic acid, methyl- or ethylmalonic acid) can be used.

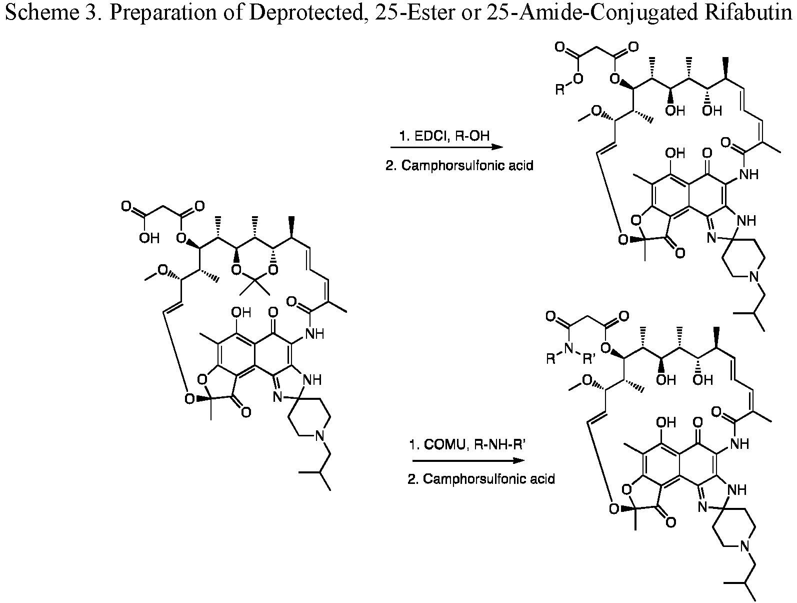

The C25-esterified, protected rifabutin can be coupled to an appropriate alcohol or amine using a coupling reagent such as EDCI or COMU (preferably wherein EDCI is used for coupling to an alcohol and COMU is used for coupling to an amine). Deprotection of the C21-C23 acetal in the conjugated rifabutin can be carried out by treating with an acid such as camphorsulphonic acid in an alcohol such as methanol.

### Antibacterial Efficacy of the Disclosed Compounds

The inventive compounds are C25-modified analogs of rifabutin that exhibit broad spectrum antibacterial activity characteristic of the rifamycin class. Additionally, the inventive compounds unexpectedly showed enhanced antibacterial activity against non-tuberculous *Mycobacteria* including *M. abscessus* compared to currently available antibiotics (e.g., rifabutin). The inventive compounds also retained antibacterial activity against *A. baumannii* and unexpectedly showed enhanced antibacterial activity against certain strains of *A. baumannii,* e.g., *A. baumannii* carrying one or more resistance mutations making it resistant to currently available antibiotics (e.g., rifabutin).

As shown below in Example 7, Table 5, the compounds of the disclosure are effective at inhibiting bacterial growth in strains of *S. aureus, A. baumannii,* and *M. abscessus* complex as well as other non-tuberculosis mycobacteria, i.e., *M. kansasii, M. xenopi,* and *M. avium.* Table 9 shows that the inventive compounds (e.g., Compound 3) are effective at inhibitiong bacterial growth in the strains *E. faecium, E. faecalis, S. epidermidis, S. pneumoniae, M. tuberculosis, S. pyogenes, H. influenzae, and M. smegmatis.*

Example 7, Tables 6 and 7 show the activity of the inventive compounds against particular isolates of *M. abscessus* compared to currently available antibiotics. Table 6 shows the activity of the inventive compounds in Middlebrook 7H9 broth supplemented with Middlebrook ADC growth supplement. Table 7 shows the activity of the inventive compounds in cation adjusted Mueller Hinton broth. Rifampicin exhibited MIC values above 32 mg/L and was not considered active against *M. abscessus* strains. Rifabutin and 3-(4-morpholinyl)rifamycin S, which are not modified on their C25 position, exhibited modest activity against the tested *M. abscessus* strains with MIC values ranging from 2 to 16 mg/L and 4 to 32 mg/L, respectively. In contrast, Compound 3 exhibited MIC values from 0.5 to 2 mg/L, corresponding to 8 to 16-fold increased activity over rifabutin in four of the five *M. abscessus* strains tested. Moreover, the C25 modified compound CGP 4832 showed lower activity (MIC from 2 to 8 mg/L) than Compound 3. Similarly, additional compounds of the invention (e.g., Compounds 85, 90, and 98) showed enhanced activity compared to rifabutin (e.g., 4 to 16-fold increase in activity) in the *M. abscessus* isolates tested as shown in Table 7. Accordingly, the present disclosure teaches compounds that display increased activity against *M. abscessus* beyond that of antibiotics known in the literature. In some embodiments, the inventive compounds can be used to treat *M. abscessus* infection. In some embodiments, the *M. abscessus* infection is resistant to current antibiotics.

As shown in Example 7, Table 8, compounds of the invention (e.g., Compound 3) retained strong antibacterial activity against *A. baumannii* wild type strain and overcame antibiotic resistance mechanisms such as ADP-ribosyl transferase (Arr)-mediated resistance and RpoB mutation-mediated resistance mechanisms. In contrast to the activity of Compound 3, CGP4832 did not exhibit strong activity against *A. baumannii* wild type and lost activity compared with rifabutin against RpoB mutant strains.

Accordingly, in some embodiments, the inventive compounds can be used to inhibit bacterial infection, preferably infection caused by a non-tuberculous *Mycobacteria,* more preferably M. *abscessus.* In some embodiments, the inventive compounds can be used to inhibit bacterial infection caused by *A. baumannii.* In some preferred embodiments, the inventive compounds can be used to treat a bacterial infection caused by a strain of bacteria that carries one or more resistance genes or resistance mechanisms against currently available antibiotics (e.g., rifampicin and/or rifabutin). In some preferred embodiments, the inventive compounds can be used to inhibit bacterial infection caused *by A. baumannii,* wherein the *A. baumannii* has a resistance mechanism, preferably a resistance mutation in the RpoB gene and/or expression of the ADP-ribosyltransferase (Arr) enzyme. In some preferred embodiments the resistance mutation in the RpoB gene is H535Q, H535C, or H535L.

### Methods of Using the Disclosed Compounds

An aspect of the present disclosure relates to a method of treating a bacterial infection in a subject in need thereof, the method comprising administering to the subject a compound of the present disclosure or a pharmaceutically acceptable salt thereof. An aspect of the present disclosure relates to a compound (or a pharmaceutically acceptable salt thereof) or a pharmaceutical composition as described herein for use as a medicament. An aspect of the present disclosure relates to a compound (or a pharmaceutically acceptable salt thereof) or a pharmaceutical composition as described herein for use in a method of treating a bacterial infection in a subject. An aspect of the present disclosure relates to the use of a compound (or a pharmaceutically acceptable salt thereof) or a pharmaceutical composition as described herein in the manufacture of a medicament for treating a bacterial infection. In some preferred embodiments, the bacterial infection is caused by a bacterial strain carries one or more resistance genes or resistance mechanisms against currently available antibiotics (e.g., rifampicin and/or rifabutin).

In some embodiments, the infection is caused by one or more bacterium belonging to the genera *Mycobacterium spp., Acinetobacter spp., Clostridium spp., Enterococcus spp., Hemophilus spp., Legionella spp., Neisseria spp., Staphylococcus spp., Streptococcus spp., Listeria monocytogenes, Moraxella catarrhalis, Bacillus spp., Bacteroides spp., Gardnerella vaginalis, Lactobacillus spp., Mobiluncus spp., Helicobacter pylori, Campylobacter jejuni, Chlamydia trachomatis* and/or *Toxoplasma gondii.*

In some embodiments, the infection is caused by one or more bacterium belonging to the species *M. abscessus, M. kansasii, M. xenopi, M. avium, A. baumannii, S. aureus, E. faecium, E. faecalis, S. epidermidis, S. pneumoniae, M. tuberculosis, S. pyogenes, H. influenzae,* and/or *M. smegmatis.*

In some preferred embodiments, the bacterial infection is caused by one or more bacterium belonging to the genus *Acinetobacter, Staphylococcus,* and/or *Mycobacteria.* In some preferred embodiments, the bacterial infection is caused by one or more bacterium belonging to the species *A. baumannii,* and/or *S. aureus,* and/or a genus of non-tuberculous *Mycobacteria,* preferably *M. abscessus.* In preferred embodiments, the bacterial infection is caused by one or more bacterium belonging to a genus of non-tuberculous *Mycobacteria,* preferably *M. abscessus.* In some embodiments, the infection is caused by one or more bacterium belonging to the genus *Acinetobacter* and/or *Staphylococcus,* preferably *A. baumannii* and/or S. *aureus.*

In some preferred embodiments, the infection is caused by one or more bacterium belonging to a genus of non-tuberculosis *Mycobacterium,* preferably *M. abscessus, M. avium, M. kansasii, M. xenopi* and/or *M. malmoense,* yet more *preferably M. abscessus.*

In some embodiments, the infection is caused by one or more bacterium belonging to the species *M. abscessus, A. baumannii,* and/or *S. aureus.* In some embodiments, the infection is caused by one or more bacterium belonging to the species *M. abscessus.* In some embodiments, the infection is caused by one or more bacterium belonging to the species A. *baumannii.*

In some embodiments, the infection is caused by one or more bacterium belonging to the strains *M. abscessus subsp. abscessus,* e.g., *M. abscessus subsp. abscessus* strain ID No. ATCC 19977, strain ID No. CCUG 71636, or strain ID No. CCUG 71382; *M. abscessus subsp. bolletii,* e.g., strain ID No. CCUG 50184; and/or *M. abscessus subsp. massiliense,* e.g., strain ID No. CCUG 48898.

In some preferred embodiments, the compounds of the present disclosure can be used to inhibit bacterial infection caused by *A. baumannii,* preferably wherein the *A. baumannii* has a resistance mechanism making it resistant to current antibiotics, preferably wherein the resistance mechanism is a mutation in the RpoB gene and/or expression of an ADP ribosyl transferase (Arr) enzyme. In some embodiments, the infection is caused by one or more bacterium belonging to the strains *A. baumannii* wild type; *A. baumannii* Arr-2; *A. baumannii* RpoB H535Q mutant; *A. baumannii* RpoB H535C mutant; and/or A. *baumannii* RpoB H535L mutant.

In some preferred embodiments, the inventive compounds are used for the treatment of a non-tuberculous *Mycobacteria* that causes a non-tuberculous *Mycobacteria* pulmonary infection. Accordingly, an aspect of the present disclosure relates to a method of treating a non-tuberculous *Mycobacteria* pulmonary infection in a subject in need thereof, the method comprising administering to the subject a compound of the present disclosure or a pharmaceutically acceptable salt thereof. An aspect of the present disclosure relates to a compound (or a pharmaceutically acceptable salt thereof) or a pharmaceutical composition as described herein for use in a method of treating a non-tuberculous *Mycobacteria* pulmonary infection in a subject. An aspect of the present disclosure relates to the use of a compound (or a pharmaceutically acceptable salt thereof) or a pharmaceutical composition as described herein in the manufacture of a medicament for treating a non-tuberculous *Mycobacteria* pulmonary infection in a subject. In some embodiments the non-tuberculosis *Mycobacterium* is *M. abscessus, M. avium, M. kansasii, M. xenopi* and/or *M. malmoense.*

Illustrative pharmaceutical compositions are tablets and gelatin capsules comprising a compound of the disclosure and a pharmaceutically acceptable carrier, such as a) a diluent, e.g., purified water, triglyceride oils, such as hydrogenated or partially hydrogenated vegetable oil, or mixtures thereof, com oil, olive oil, sunflower oil, safflower oil, fish oils, such as EPA or DHA, or their esters or triglycerides or mixtures thereof, omega-3 fatty acids or derivatives thereof, lactose, dextrose, sucrose, mannitol, sorbitol, cellulose, sodium, saccharin, glucose and/or glycine; b) a lubricant, e.g., silica, talcum, stearic acid, its magnesium or calcium salt, sodium oleate, sodium stearate, magnesium stearate, sodium benzoate, sodium acetate, sodium chloride and/or polyethylene glycol; for tablets also; c) a binder, e.g., magnesium aluminum silicate, starch paste, gelatin, tragacanth, methylcellulose, sodium carboxymethylcellulose, magnesium carbonate, natural sugars such as glucose or beta-lactose, corn sweeteners, natural and synthetic gums such as acacia, tragacanth or sodium alginate, waxes and/or polyvinylpyrrolidone, if desired; d) a disintegrant, e.g., starches, agar, methyl cellulose, bentonite, xanthan gum, algiic acid or its sodium salt, or effervescent mixtures; e) absorbent, colorant, flavorant and sweetener; f) an emulsifier or dispersing agent, such as Tween 80, Labrasol, HPMC, DOSS, caproyl 909, labrafac, labrafil, peceol, transcutol, capmul MCM, capmul PG-12, captex 355, gelucire, vitamin E TGPS or other acceptable emulsifier; and/or g) an agent that enhances absorption of the compound such as cyclodextrin, hydroxypropyl-cyclodextrin, PEG400, PEG200.

Liquid, particularly injectable, compositions can, for example, be prepared by dissolution, dispersion, etc. For example, the disclosed compound is dissolved in or mixed with a pharmaceutically acceptable solvent such as, for example, water, saline, aqueous dextrose, glycerol, ethanol, and the like, to thereby form an injectable isotonic solution or suspension. Proteins such as albumin, chylomicron particles, or serum proteins can be used to solubilize the disclosed compounds.

The disclosed compounds can be also formulated as a suppository that can be prepared from fatty emulsions or suspensions; using polyalkylene glycols such as propylene glycol, as the carrier.

The disclosed compounds can also be administered in the form of liposome delivery systems, such as small unilamellar vesicles, large unilamellar vesicles and multilamellar vesicles. Liposomes can be formed from a variety of phospholipids, containing cholesterol, stearylamine or phosphatidylcholines. In some embodiments, a film of lipid components is hydrated with an aqueous solution of drug to a form lipid layer encapsulating the drug, as described in U.S. Pat. No. 5,262,564.

Parental injectable administration is generally used for subcutaneous, intramuscular or intravenous injections and infusions. Injectables can be prepared in conventional forms, either as liquid solutions or suspensions or solid forms suitable for dissolving in liquid prior to injection.

Another aspect of the disclosure relates to a pharmaceutical composition comprising a compound of the present disclosure and a pharmaceutically acceptable carrier. The pharmaceutically acceptable carrier can further include an excipient, diluent, or surfactant.

Compositions can be prepared according to conventional mixing, granulating or coating methods, respectively, and the present pharmaceutical compositions can contain from about 0.1% to about 99%, from about 5% to about 90%, or from about 1% to about 20% of the disclosed compound by weight or volume.

The dosage regimen utilizing the disclosed compound is selected in accordance with a variety of factors including type, species, age, weight, sex and medical condition of the patient; the severity of the condition to be treated; the route of administration; the renal or hepatic function of the patient; and the particular disclosed compound employed. A physician or veterinarian of ordinary skill in the art can readily determine and prescribe the effective amount of the drug required to prevent, counter or arrest the progress of the condition.

Effective dosage amounts of the disclosed compounds, when used for the indicated effects, range from about 0.5 mg to about 5000 mg of the disclosed compound as needed to treat the condition. Compositions for in vivo or in vitro use can contain about 0.5, 5, 20, 50, 75, 100, 150, 250, 500, 750, 1000, 1250, 2500, 3500, or 5000 mg of the disclosed compound, or, in a range of from one amount to another amount in the list of doses. In one embodiment, the compositions are in the form of a tablet that can be scored.

### Equivalents

While the present technology has been described in conjunction with the specific embodiments set forth above, many altematives, modifications and other variations thereof will be apparent to those of ordinary skill in the art.

### EXAMPLES

The disclosure will now be illustrated by way of the following non-limiting examples. While particular embodiments of the disclosure are described below, a skilled person will appreciate that various changes and modifications can be made. References to preparations carried out in a similar manner to, or by the general method of, other preparations, may encompass variations in routine parameters such as time, temperature, workup conditions, minor changes in reagents amounts, and the like.

### Abbreviations

The following list provides definitions of certain abbreviations and symbols as used herein. It will be appreciated that the list is not exhaustive, but the meaning of those abbreviations and symbols not herein below defined will be readily apparent to those skilled in the art. In describing the disclosure, chemical elements are identified in accordance with the Periodic Table of the Elements.
- ACN: acetonitrile
- AcOH: acetic acid
- aq.: aqueous
- Ar: argon
- COMU: 1-cyano-2-ethoxy-2-oxoethylidenaminooxy)dimethylamino-morpholino-carbenium hexafluorophosphate
- CSA: camphorsulphonic acid
- DCC: dicyclohexylcarbodiimide
- DCM: dichloromethane
- DIEA: *N,N*-diisopropylethylamine
- DMF: dimethylformamide
- EDCI: 1-ethyl-3-(3-dimethylaminopropyl)carbodiimide
- eq.: equivalent
- EtOAc: ethyl acetate
- EtOH: ethanol
- Et₂O: diethylether
- h: hour(s)
- HPLC: high performance liquid chromatography
- i-PrOH: isopropanol
- LC: liquid chromatography
- M: molar
- MS: mass spectroscopy
- min: minutes
- NMR: nuclear magnetic resonance spectroscopy
- rt: room temperature
- T_{R}: retention time
- sat.: saturated

### Characterization of the Compounds

Unless specified otherwise, the purity and identity of Intermediate or example compounds were assessed by state-of-the-art LC/MS. Methods for reaction monitoring and compound purity determination were performed by liquid chromatography/mass spectrometry (LC/MS), as described below. Method A was used to characterize the intermediates I-1 to I-6. Method B was used to characterize the inventive compounds and confirm their purity. Percent purity was determined by reversed phase HPLC or UPLC using UV detection (254 nm). Compound structures were confirmed by MS, using electro spray ionization positive (ESI+) method and reported as [M+H]+, referring to the protonated molecular ion.

### Method A

UPLC system: UPLC I BIN SOL MGR with ACQUITY UPLC I-Class eK PDA Detector; Column: Acquity BEH C18 column (1.7µm particle size, dimensions 50mm x 2.1mm); Mobile phases: phase A (H₂O/ammonium formate, pH 3.75 or 9.2) and phase B (CH₃CN + 5% H₂O/ammonium formate, pH 3.75 or 9.2) were used according to the following methods:

| Method A | | | |
|---|---|---|---|
| Time (min) | Phase A | Phase B | Flow (mL/min) |
| 0.00 | 98.0 | 2.0 | 0.6 |
| 0.20 | 98.0 | 2.0 | 0.6 |
| 2.50 | 0.0 | 100.0 | 0.6 |
| 3.50 | 0.0 | 100.0 | 0.6 |
| 3.60 | 98.0 | 2.0 | 0.6 |
| 5.00 | 98.0 | 2.0 | 0.6 |

Mass Spectrometry Analysis: ACQUITY QDa (Performance) Xevo TQD. Ionization: electrospray (polarity: negative and positive).

### Method B

HPLC system: Waters 2695 LC with photodiode array detector Waters 996; Column: XBridge C18 column (3.5µm particle size, dimensions 50mm x 4.6mm); Mobile phases: phase A (H₂O/ammonium formate, pH 9.2) and phase B (CH₃CN + 5% H₂O/ammonium formate, pH 9.2) were used according to the following methods:

| Method B | | | |
|---|---|---|---|
| Time (min) | Phase A | Phase B | Flow (mL/min) |
| 0.00 | 100.0 | 0.0 | 2.00 |
| 1.00 | 100.0 | 0.0 | 2.00 |
| 25.00 | 0.0 | 100.0 | 2.00 |
| 27.00 | 0.0 | 100.0 | 2.00 |
| 27.10 | 100.0 | 0.0 | 2.00 |
| 30.00 | 100.0 | 0.0 | 2.00 |

Mass Spectrometry Analysis: Waters Alliance Micromass ZQ 2000. Ionization: electrospray (polarity: negative and positive).

NMR spectra were recorded on a Bruker DRX-300 spectrometer or Bruker 500 MHz spectrometer with a TXI probe. Chemical shifts were measured in parts per million (ppm). The assignments were made using one-dimensional (1D) ¹H and ¹³C spectra and two-dimensional (2D) HSQC, HMBC spectra.

All commercial reagents and solvents were used without further purification. Normal phase purifications were performed on Interchim PuriFlash 430 with prepacked columns (FlashPure, BUCHI^{®}, silica 40µm irregular). Reversed phase purifications were performed by HPLCprep (Buchi Pure C-830, Column: OmniSphere C18 Dynamax, using as mobile phases H₂O (0.1%HCOOH)/ACN(0.1%HCOOH).

### Example 1: Synthesis of C21-C23 Acetal 25-OH Rifabutin (Intermediate I-1)

The inventive compounds were prepared from the free 25-OH rifabutin (Intermediate I-1) prepared from commercially available rifabutin as shown below.

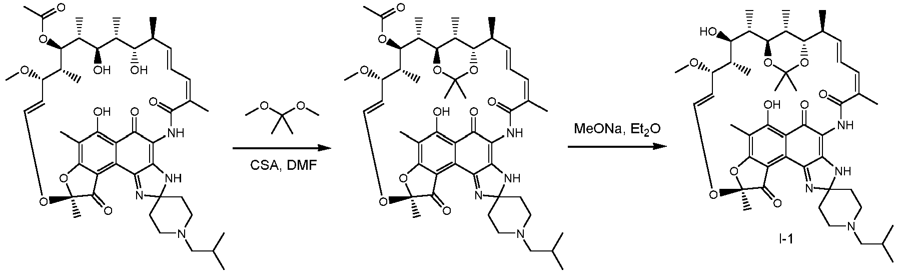

### Synthesis of Intermediate I-1:

Rifabutin (40.0 g, 47.2 mmol) was dissolved in dry DMF (80 mL) at rt under nitrogen atmosphere. 2,2-Dimethoxy-propane (58.1 mL, 472 mmol) and camphorsulphonic acid (12.6 g, 54.3 mmol) were sequentially added to the solution. The reaction mixture was stirred at rt for 26h under nitrogen atmosphere. The mixture was then cooled at 0°C and poured into a mixture of sat. aq. solution of NaHCO₃ (700 mL) and water (500 mL). The reaction flask was washed with acetone (100 mL). The resulting suspension was stirred on an ice bath for 10 min and filtered. The cake was rinsed with sat. aq. solution of NaHCO₃ (100 mL) and water (50 mL) and dried under vacuum at 40°C for 24h. The crude product was purified by flash chromatography (DCM to 50% of the mixture DCM/MeOH/NH₄OH 90/9/1.5 in DCM) to afford the desired product as a purple solid (37.68 g, 90% yield).

21,23-Dimethylacetonide-rifabutin (10.6 g, 11.9 mmol) was dissolved in dry diethyl ether (500 mL). The solution was cooled to -10°C with Ar bubbling. After 15 min, a solution of NaOMe (30 mL, 25w% in MeOH) was slowly added. Precipitation occurred and another portion of diethyl ether (100 mL) was added to homogenize. NaOMe solution was again added (27.4 mL). The solution was stirred at -5°C for 10 min before the ice bath was removed. The reaction mixture was stirred at rt for 6h. A saturated aqueous solution of NaHCO₃ (400 mL) was added and the layers were separated. The aqueous layer was extracted with diethyl ether (400 mL) and the combined organic layers were washed with brine (300 mL) and evaporated to afford the desired crude product as a black-purple powder. The product was purified by flash chromatography (DCM to 50% of the mixture DCM/MeOH/NH₄0H 90/9/1.5 in DCM). After evaporation, the product was redissolved in acetone and slowly added into water under vigorous stirring. The solid was collected by filtration and dried at 40°C to yield the de-acetylated analog of rifabutin Intermediate I-1 (97% purity).

### Example 2: Synthesis of 25-Malonate Rifabutin (Intermediate I-2)

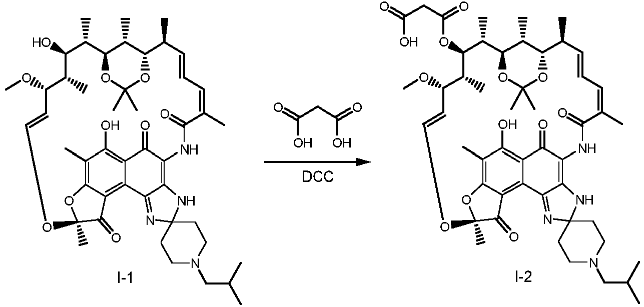

Intermediate I-1 (1 eq.) and malonic acid (3 eq.) were dissolved in THF and cooled to 0°C. DCC (3 eq.) was added and the reaction was stirred at 0°C for 1.5h. Dicyclohexylurea was removed by filtration and the filtrate was evaporated under vacuum. The crude product was purified by flash chromatography (CHCl₃/MeOH) to yield Intermediate I-2 as a black solid.

### Example 3: Synthesis of 25-Methyl-malonate Rifabutin (Intermediate I-3)

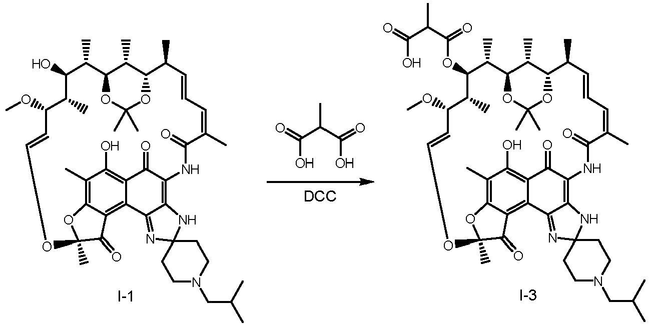

Intermediate I-1 (500mg, 0.59mmol, 1eq) and 2-methylpropanedioic acid (210mg, 1.78mmol, 3eq.) were dissolved in DCM (10mL). Then DCC (366mg, 1.78mmol, 3eq.) was added and reaction stirred at rt. After 1h, the mixture was filtered, and DCM was evaporated. The obtained oil was solubilized in EtOAc (30mL) and washed with an aqueous solution of HCl 1N (30mL) and brine (30mL). The organic layer was dried over MgSO₄ and concentrated under vacuum. The obtained solid was purified by flash chromatography (CHCl₃/MeOH, from 100/0 to 95/5) to yield Intermediate I-3 as a black solid, obtained as a mixture of diastereoisomers (m=395mg, yield=70%).

LC/MS A (ESI+): T_{R} = 2.15 min, m/z [M+H]⁺ = 945.52, UV purity: 99 % (254 nm).

¹H NMR (300 MHz, CDCl₃): (mixture of diastereoisomers) δ 0.35 (d, *J* = 7.0 Hz, 1.5H), 0.41 (d, *J=* 7.0 Hz, 1.5H), 0.62-0.73 (m, 3H), 0.73-0.88 (m, 9H), 0.99-1.10 (m, 6H), 1.16-1.25 (m, 3H), 1.25-1.36 (m, 3H), 1.40-1.55 (m, 3H), 1.64-1.91 (m, 3H), 1.74 (s, 1.5 H), 1.75 (s, 1.5H), 1.99 (s, 3H), 2.05-2.16 (m, 1H), 2.16-2.26 (m, 1H), 2.30 (s, 1.5H), 2.31 (s, 1.5H), 2.68 (s, 1.5H), 2.73 (s, 1.5H), 2.77-3.00 (m, 4H), 3.01-3.66 (m, 8H), 4.90-5.08 (m, 2H), 5.72-5.86 (m, 1H), 6.02 (dd, *J=* 15.4, 6.8 Hz, 1H), 6.14 (d, *J=* 10.5 Hz, 1H), 6.19-6.34 (m, 1H), 7.66 (s, 0.5H), 7.67 (s, 0.5H), 8.70 (brs, 1H), 14.61 (s, 0.5H), 14.62 (s, 0.5H).

¹³C NMR (75 MHz, CDCl₃): (mixture of diastereoisomers) δ 7.8, 8.2, 8.6, 9.8, 10.1, 12.8, 14.3, 14.4, 15.3, 17.7, 20.1, 20.2, 20.3, 20.7, 20.8, 23.8, 23.9, 24.5, 25.8, 33.0, 33.7, 34.3, 36.2, 39.9, 40.5, 41.5, 41.7, 44.7, 47.4, 47.6, 50.9, 51.1, 55.7, 55.9, 64.4, 64.7, 70.7, 70.8, 75.1, 75.5, 76.9, 77.3, 78.6, 78.8, 91.9, 100.0, 100.1, 104.9, 106.1, 108.5, 111.4, 111.5, 114.1, 114.2, 114.9, 115.1, 123.7, 124.9, 125.0, 130.4, 130.5, 133.3, 133.5, 140.6, 140.8, 140.9, 141.5, 141.6, 156.1, 156.2, 168.1, 168.7, 168.8, 171.0, 171.2, 172.3, 172.4, 174.55, 174.58, 177.1, 181.7, 181.8, 193.0, 193.1.

### Example 4: Synthesis of 25-Succinate Rifabutin (Intermediate I-4)

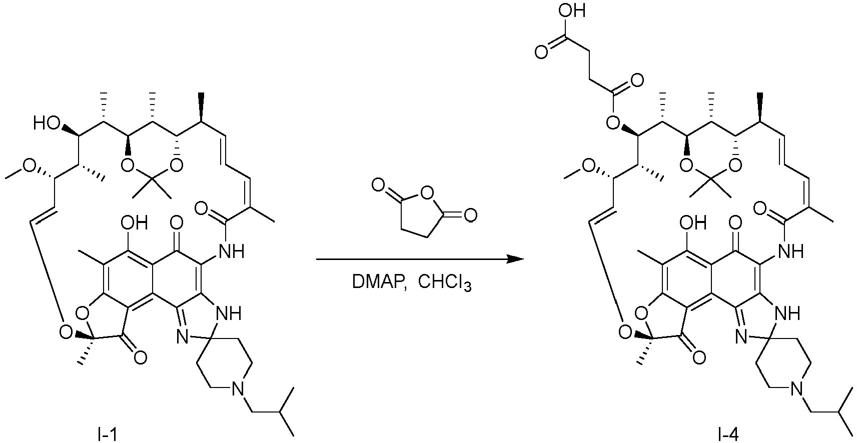

Intermediate I-1 (1 g, 1.18 mmol, 1 eq.), succinic anhydride (591.5 mg, 5.92 mmol, 5 eq.) and DMAP (722.2 mg, 5.92 mmol, 5 eq.) were dissolved in 10 mL of CHCl₃ and the reaction was stirred 60°C. After 70h, the mixture was diluted in 50 mL of DCM, washed with an aqueous solution of HCl 0.5N (2x30mL), with a saturated aqueous solution of NaHCO₃ (2x30mL) and with brine (30mL). The organic layer was dried with MgSO₄ and evaporated under vacuum. The obtained solid was purified by flash chromatography (DCM/MeOH from 100/0 to 90/10) to yield Intermediate I-4 as a black solid (791 mg, 71% yield).

LC/MS A (ESI+): T_{R} = 2.14 min, m/z [M+H]⁺ = 945.60, UV purity: 99 % (254 nm).

¹H NMR (300 MHz, CDCl₃): δ 0.29 (d, *J=* 7.0 Hz, 3H), 0.68 (d, *J=* 7.0 Hz, 3H), 0.79-0.85 (m, 9H), 1.16-1.26 (m, 9H), 1.31-1.72 (m, 5H), 1.75 (s, 3H), 2.00 (s, 3H), 2.18-2.29 (m, 2H), 2.31 (s, 3H), 2.53-2.59 (m, 4H), 2.80 (s, 3H), 2.96-3.08 (m, 3H), 3.30-3.72 (m, 8H), 4.92 (d, *J=* 8.1 Hz, 1H), 5.04 (dd, *J=* 12.1, 6.2 Hz, 1H), 5.85 (d, *J = 11.5* Hz, 1H), 5.99-6.35 (m, 3H), 7.62 (s, 1H), 8.72 (s, 1H), 14.62 (s, 1H).

¹³CNMR(75 MHz, CDCl₃): δ 7.79, 8.98, 9.84, 12.79, 17.68, 20.16, 20.24, 21.19, 21.23, 23.67, 24.47, 25.73, 29.09, 29.16, 32.49, 33.30, 34.26, 36.30, 40.70, 41.18, 51.72, 51.37, 56.26, 65.02, 70.98, 74.69, 76.93, 78.38, 91.42, 99.90, 104.79, 106.09, 108.44, 111.50, 114.25, 115.27, 123.84, 124.87, 130.51, 133.39, 140.53, 140.85, 141.68, 156.46, 168.13, 168.66, 171.46, 172.19, 176.15, 182.05, 193.17.

### Example 5: Synthesis of 25-Glutarate Rifabutin (I-5)

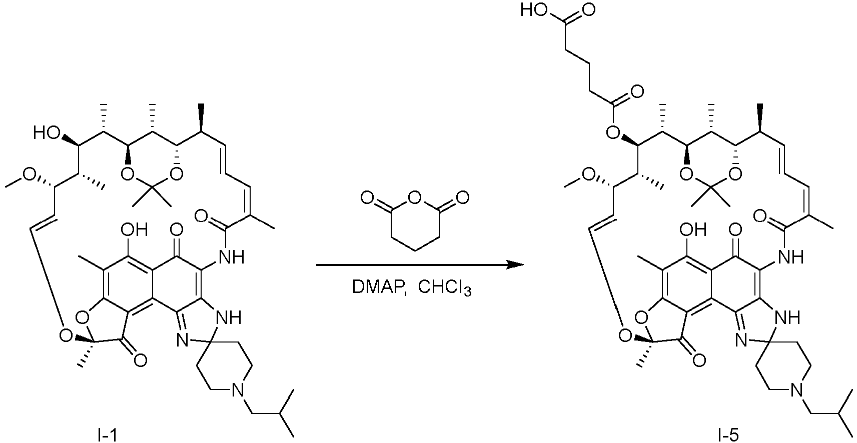

Intermediate I-1 (1 g, 1.18 mmol, 1 eq.), glutaric anhydride (675 mg, 5.92 mmol, 5 eq.) and DMAP (723 mg, 5.92 mmol, 5 eq.) were dissolved in 10 mL of CHCl₃. The reaction was stirred at 60°C. After 22h, glutaric anhydride (675 mg, 5.92 mmol, 5 eq.) and DMAP (723 mg, 5.92 mmol, 5 eq.) were added and the mixture was stirred at 60°C. After 24h, the mixture was diluted in 50 mL of DCM, washed with an aqueous solution of HCl 0.5N (2x30mL), with a saturated aqueous solution of NaHCO₃ (2x30mL) and with brine (30mL). The organic layer was dried with MgSO₄ and evaporated under vacuum. The obtained solid was purified by flash chromatography (DCM/MeOH from 100/0 to 90/10) to yield Intermediate I-5 as a black solid (554 mg, 49% yield).

LC/MS A (ESI+): T_{R} = 2.19min, m/z [M+H]⁺ = 959.65, UV purity: 99 % (254 nm).

¹H NMR (300 MHz, CDCl₃): δ 0.33 (d, *J=* 7.0 Hz, 3H), 0.68 (d, *J=* 7.0 Hz, 3H), 0.80-0.86 (m, 9H), 1.14 (dd, *J=* 2.1, 6.5 Hz, 6H), 1.22 (s, 3H), 1.36-1.41 (m, 1H), 1.51 (m, 2H), 1.67-1.80(m, 5H), 1.85-1.91 (m, 2H), 2.02 (s, 3H), 2.15-2.40 (m, 9H), 2.80 (s, 3H), 2.92 (d, *J* = 6.6 Hz, 2H), 3.02-3.08 (m, 1H), 3.10-3.71 (m, 8H), 4.91 (d, *J* = 7.6 Hz, 1H), 5.04 (dd, *J* = 12.1, 6.4 Hz, 1H), 5.85 (d, *J* = 12.1 Hz, 1H), 6.05 (dd, *J* = 15.5, 6.1 Hz, 1H), 6.16 (dd, *J* = 10.7, 1.1 Hz, 1H), 6.29 (dd, *J=* 15.6, 10.6 Hz, 1H), 7.75 (s, 1H), 8.69 (s, 1H), 14.65 (s, 1H).

¹³C NMR (75 MHz, CDCl₃): δ 7.92, 9.09, 10.08, 12.92, 17.86, 20.26, 20.34, 21.29, 21.33; 23.75, 25.87, 32.95, 33.53, 33.64, 33.77, 34.35, 36.52, 40.78, 41.43, 51.39, 51.64, 56.31, 65.19, 71.12, 74.54, 77.05, 78.66, 91.99, 100.05, 104.96, 106.19, 108.58, 111.62, 114.30, 115.26, 123.93, 125.06, 130.77, 133.33, 140.74, 140.94, 141.79, 156.38, 168.25, 168.78, 172.31, 172.39, 177.06, 182.04.

### Example 6: Synthesis of 25-Ethyl-malonate Rifabutin (I-6)

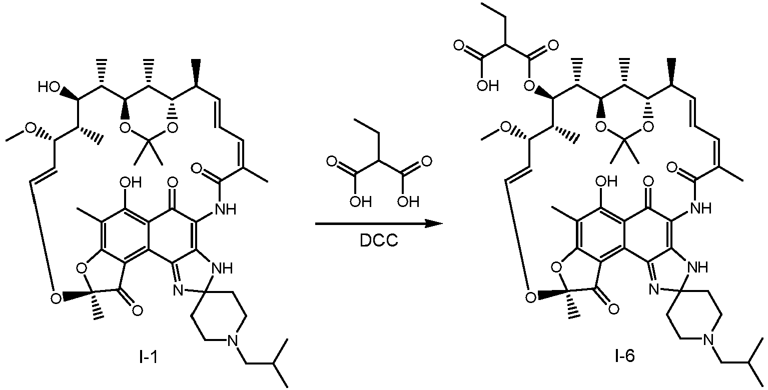

Intermediate I-1 (1.00g, 1.18mmol, 1 eq.) in 20 mL of dry DCM was added to ethylmalonic acid (469 mg, 3.55 mmol, 3.0 eq.) and DCC (732 mg, 3.55 mmol, 3.0 eq.). The mixture was stirred 2 h 30 min at 40 °C then was cooled down to 0 °C. The precipitated DCU was filtered off and rinsed with DCM. The filtrate was concentrated *in vacuo* to give a crude residue which was purified by flash chromatography (solid deposited in DCM, 80 g Buchi, DCM/MeOH gradient from 1/0 to 96/4, 40 min). Intermediate I-6 was obtained as a violet solid as a mixture of diastereoisomers (104 mg, 9 %).

MS A (ESI+): m/z [M+H]⁺ = 957.70, UV purity: 99 % (254 nm).

### Example 7: General procedure for the Synthesis of Ester Conjugates

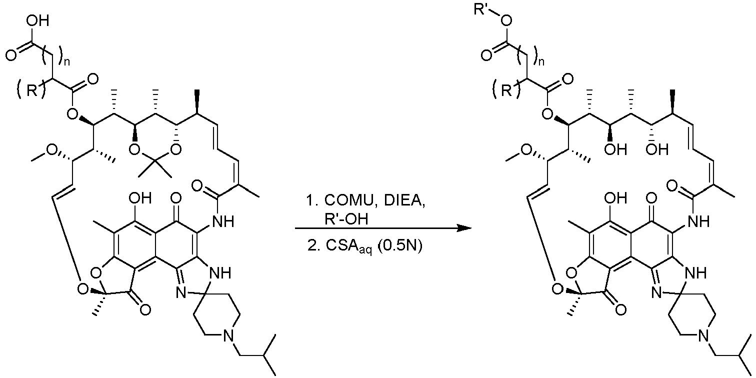

A solution of the desired Intermediate (I-2, I-3, I-4, I-5 or I-6) in THF (0.07M) was prepared. The corresponding alcohols (0.064mmol, 3eq) and EDCI (12.5mg, 0.64mmol, 3eq) were packed in Kimble reactor, and 300µL of the solution of the rifabutin Intermediate in THF (corresponding 1eq of the rifabutin-intermediate) was added. The reaction was stirred at rt. After 24h, 600µL of a solution of camphorsulphonic in water (0.5N) was added to the mixtures. The reaction tubes were stirred at 4°C for 16h. The mixtures were then deposited on Column PoraPak (Rxn RP 6CC) equilibrated with water. The elution was processed by 5mL of water, 5mL of a solution of water/ACN (90/10) and 10mL of ACN. The 10mL ACN fractions were collected, frozen and freeze-dried to give the desired products as purple powders.

### Example 8: General procedure for the Synthesis of Amide Conjugates

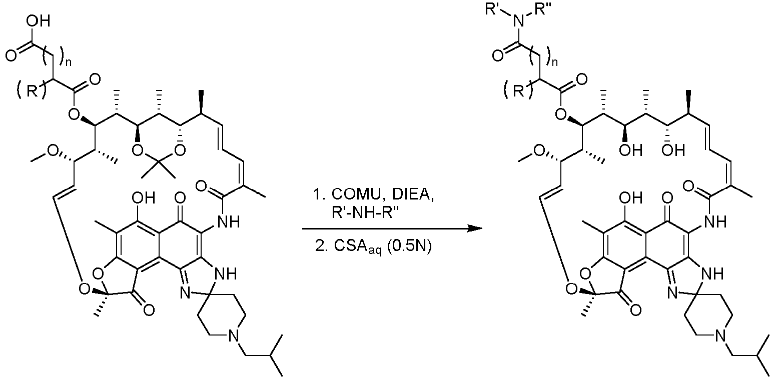

A solution of the desired Intermediate (I-2, I-3, I-4, I-5 or I-6) in THF (0.07M) was prepared. The corresponding amines (0.043mmol, 2eq) and COMU (18mg, 0.43mmol, 2eq) were packed in Kimble reactor, and 300µL of the solution of the rifabutin Intermediate in THF (corresponding to 1eq of the rifabutin Intermediate) followed by DIEA (15µL, 0.082mmol, 4eq) were added. The reaction was stirred at rt. After 3h, 600µL of a solution of camphorsulphonic in water (0.5N) was added to the mixtures. The reaction was stirred at 4°C for 16h. The mixture was then deposited on Column PoraPak (Rxn RP 6CC) equilibrated with water. The elution was processed by 5mL of water, 5mL of a sat. aq. solution of NaHCO₃, 5mL of a solution of a solution of water/ACN (90/10), 5mL of water and 10mL of ACN. The 10mL ACN fractions were collected, frozen and freeze-dried to give the desired products as purple powders.

Table 3 below summarizes exemplary compounds of the invention that were prepared according to the protocols of Examples 7 and 8 above. Data given in Table 3 were obtained following the analytic method B.

**Table 3: Compounds (Cpd) of the Disclosure from Intermediate I-2, I-3, I-4, I-5, or I-6.**

| Cpd | Structure | MW | Rt | [M+H]+ |
|---|---|---|---|---|
| 1 | 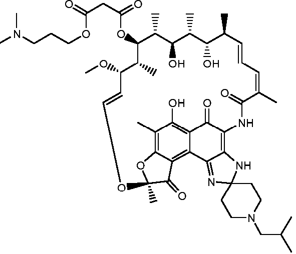 | 975.52 | 14.77 | 976.62 |
| 2 | 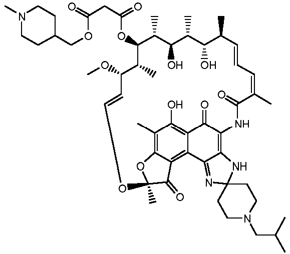 | 1001.53 | 14.03 | 976.78 |
| 3 | 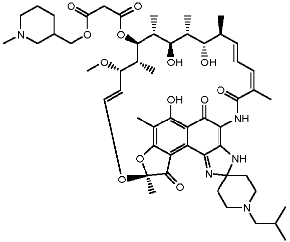 | 1001.53 | 15.73 | 1002.77 |
| 4 | 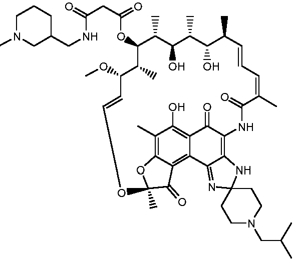 | 1000.55 | 14.00 | 1001.37 |
| 5 | 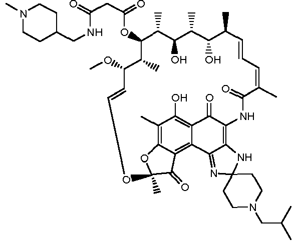 | 1000.55 | 13.27 | 1001.78 |
| 6 | 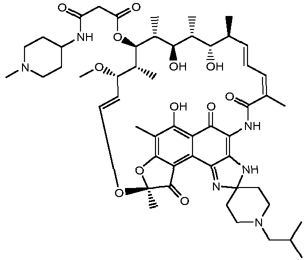 | 986.53 | 13.49 | 987.78 |
| 7 | 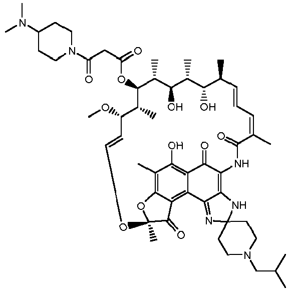 | 1000.55 | 13.94 | 1001.82 |
| 8 | 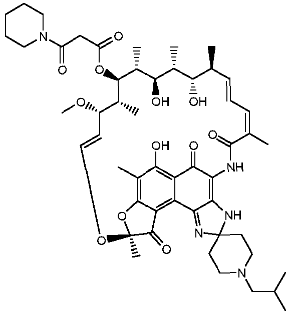 | 957.50 | 16.24 | 958.78 |
| 9 | 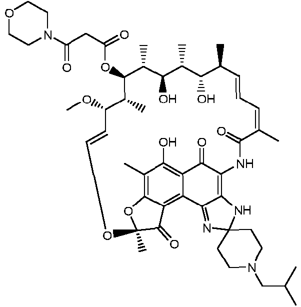 | 959.48 | 14.47 | 960.72 |
| 10 | 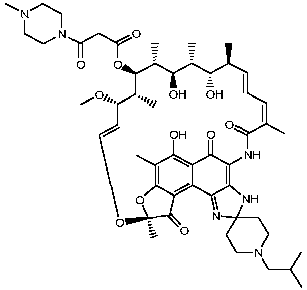 | 972.52 | 13.90 | 973.77 |
| 11 | 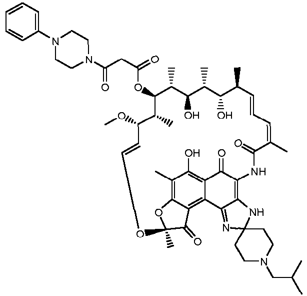 | 1034.54 | 17.68 | 1035.54 |
| 12 | 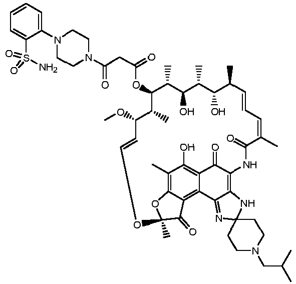 | 1113.50 | 15.50 | 1114.78 |
| 13 | 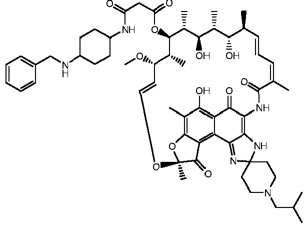 | | | |
| 14 | 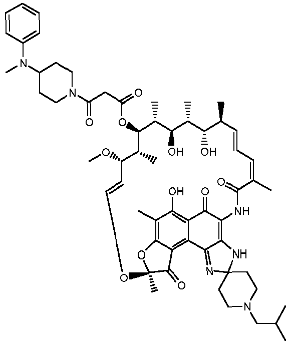 | 1062.56 | 18.58 | 1063.85 |
| 15 | 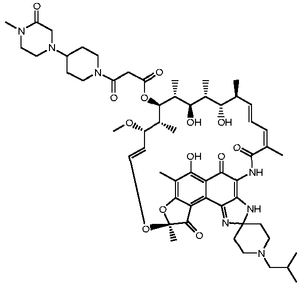 | 1069.57 | 13.15 | 1070.86 |
| 16 | 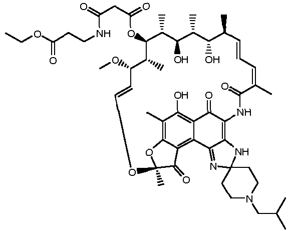 | 989.49 | 15.09 | 990.78 |
| 17 | 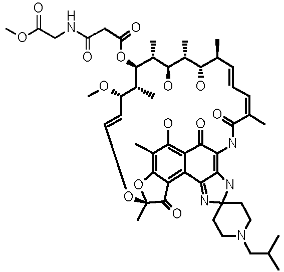 | 975.48 | 14.32 | 962.74 |
| 18 | 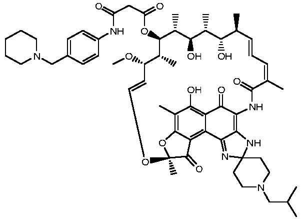 | 1062.56 | 16.58 | 1063.85 |
| 19 | 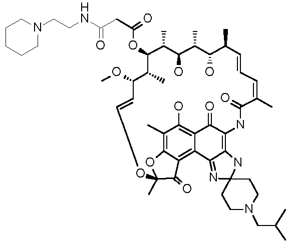 | | | |
| 20 | 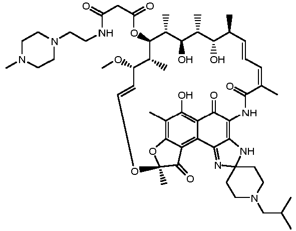 | 1015.56 | 12.92 | 1016.82 |
| 21 | 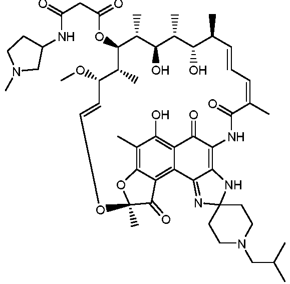 | 972.52 | 14.03 | 973.44 |
| 22 | 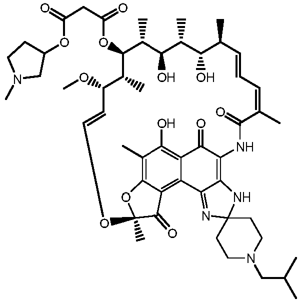 | 973.50 | 15.34 | 974.57 |
| 23 | 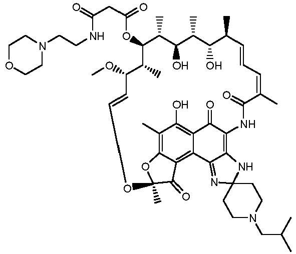 | 1002.53 | 13.47 | 1003.84 |
| 24 | 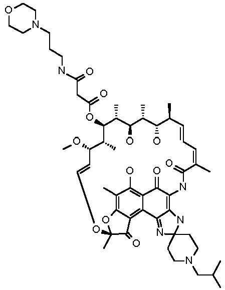 | | | |
| 25 | 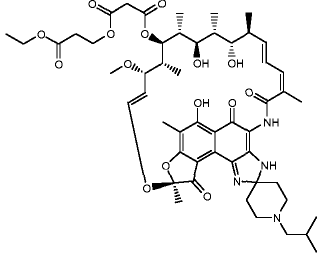 | | | |
| 26 | 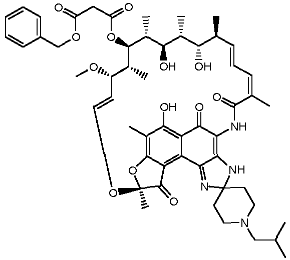 | 980.47 | 18.50 | 981.77 |
| 27 | 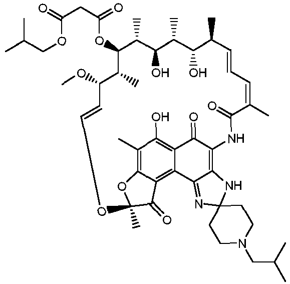 | 946.49 | 18.77 | 947.70 |
| 28 | 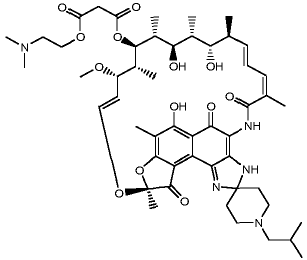 | 961.50 | 15.40 | 962.74 |
| 29 | 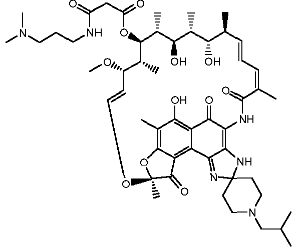 | 974.53 | 13.32 | 975.83 |
| 30 | 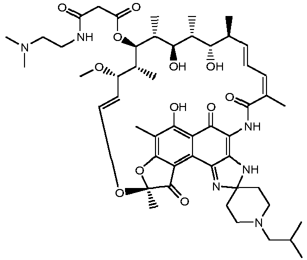 | 960.52 | 13.64 | 961.78 |
| 31 | 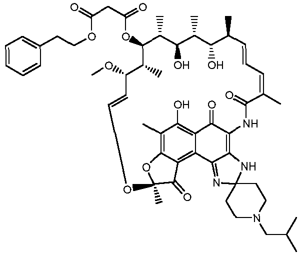 | 994.49 | 19.10 | 995.73 |
| 32 | 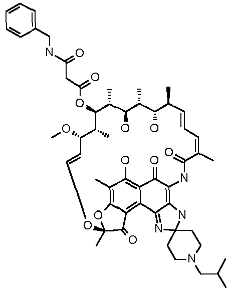 | | | |
| 33 | 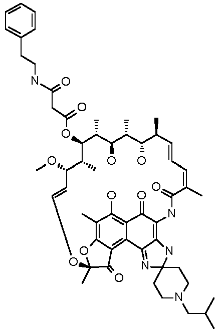 | | | |
| 34 | 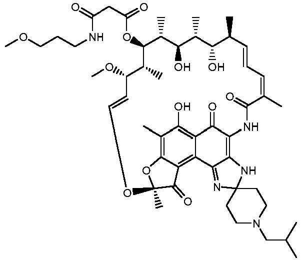 | 961.50 | 14.30 | 962.77 |
| 35 | 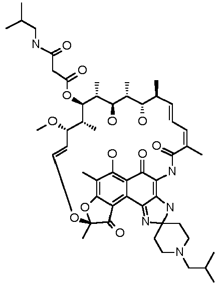 | | | |
| 36 | 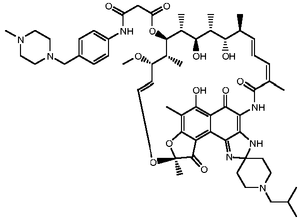 | 1077.57 | 14.32 | 1078.85 |
| 37 | 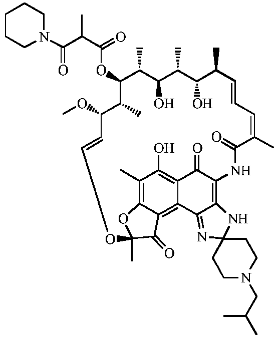 | 971.52 | 16.18 &16.53 | 972.77 |
| 38 | 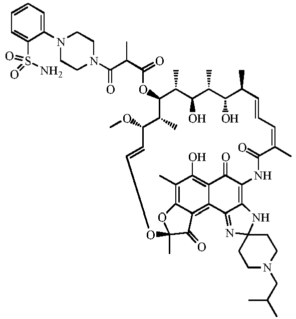 | 1127.52 | 15.72 | 1128.78 |
| 39 | 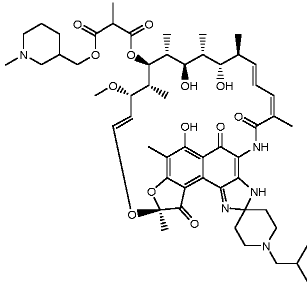 | 1015.55 | 16.20 | 1016.67 |
| 40 | 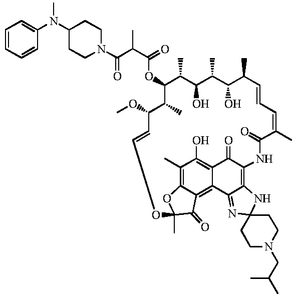 | 1076.58 | 18.89 | 1077.84 |
| 41 | 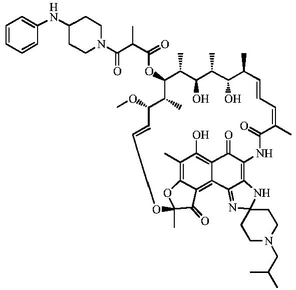 | 1062.56 | 17.90 | 1063.82 |
| 42 | 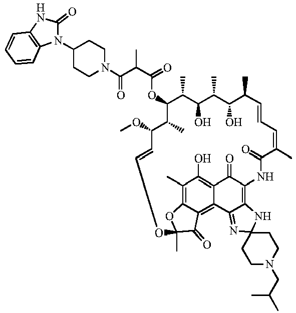 | 1103.55 | 15.27 | 1104.82 |
| 43 | 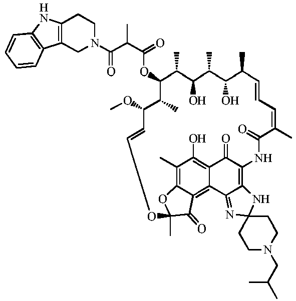 | 1058.53 | 16.70 &17.08 | 1059.80 |
| 44 | 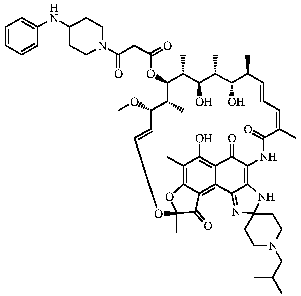 | 1048.55 | 17.45 | 1049.81 |
| 45 | 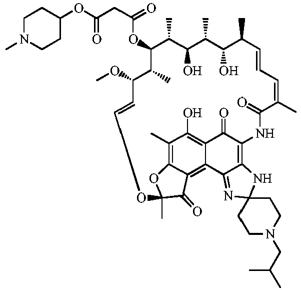 | 987.52 | 15.40 | 988.77 |
| 46 | 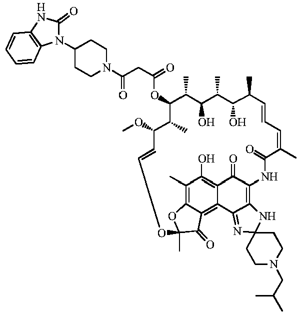 | 1089.54 | 15.09 | 1090.80 |
| 47 | 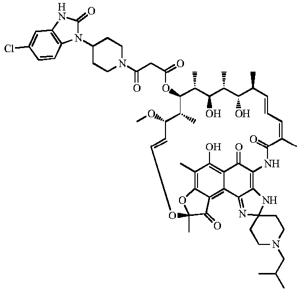 | 1123.50 | 16.10 | 1124.79 |
| 48 | 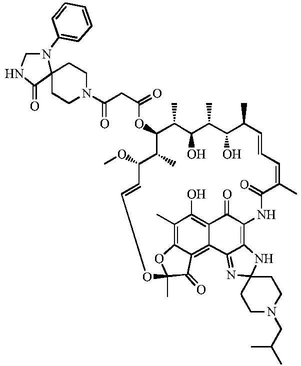 | 1103.55 | 15.67 | 1104.83 |
| 49 | 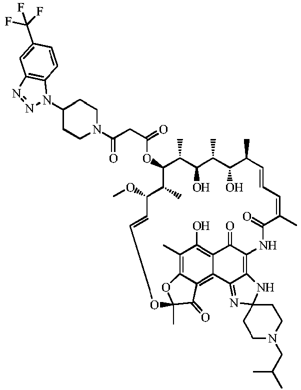 | 1142.53 | 17.83 | 1143.82 |
| 50 | 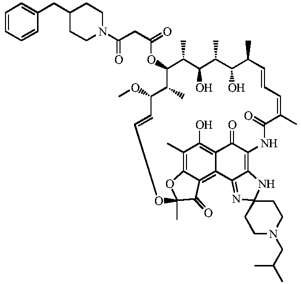 | 1047.55 | 19.00 | 1048.82 |
| 51 | 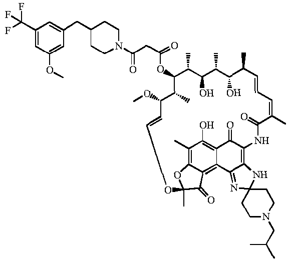 | 1145.55 | 20.02 | 1146.83 |
| 52 | 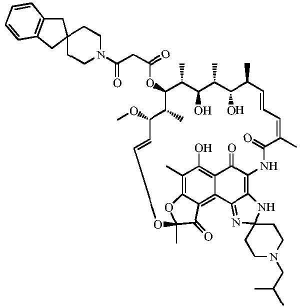 | 1059.55 | 19.20 | 1060.84 |
| 53 | 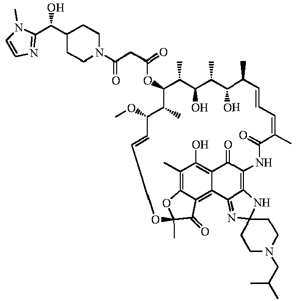 | 1067.55 | 13.47 | 1068.83 |
| 54 | 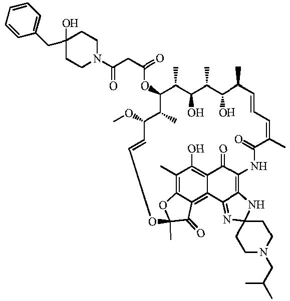 | 1063.55 | 16.08 | 1064.83 |
| 55 | 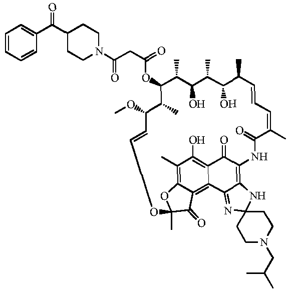 | 1061.53 | 17.07 | 1062.82 |
| 56 | 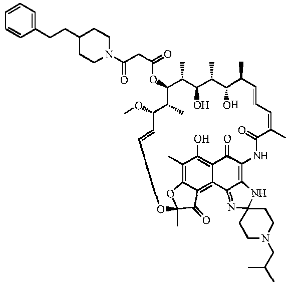 | 1061.57 | 19.70 | 1062.86 |
| 57 | 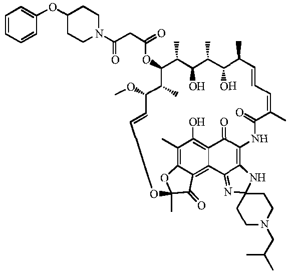 | 1049.53 | 17.97 | 1050.82 |
| 58 | 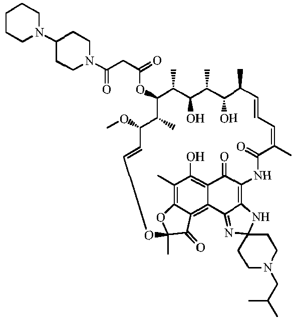 | 1040.58 | 15.14 | 1041.87 |
| 59 | 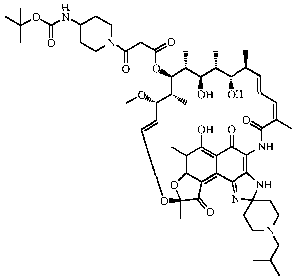 | 1072.57 | 16.57 | 1073.89 |
| 60 | 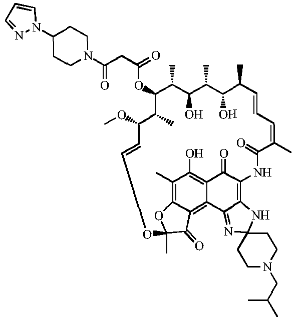 | 1023.53 | 15.10 | 1024.81 |
| 61 | 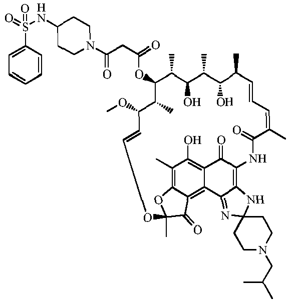 | 1112.51 | 15.85 | 1113.80 |
| 62 | 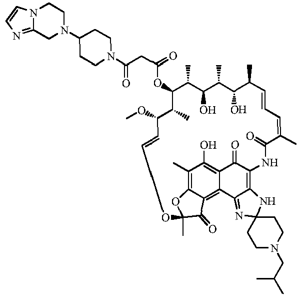 | 1078.57 | 13.52 | 1079.86 |
| 63 | 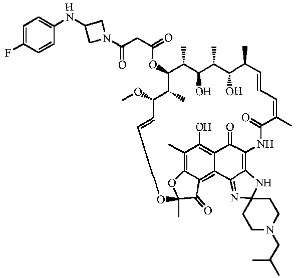 | 1038.51 | 16.51 | 1039.80 |
| 64 | 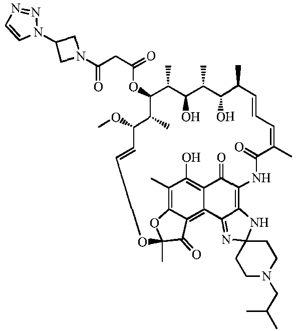 | 996.49 | 13.50 | 997.77 |
| 65 | 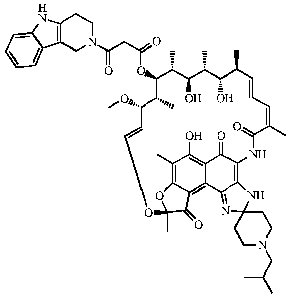 | 1044.52 | 16.52 | 1045.78 |
| 66 | 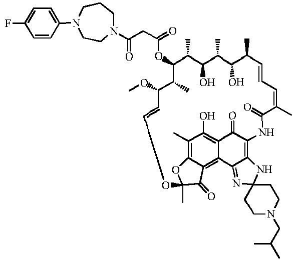 | 1066.54 | 17.60 | 1067.84 |
| 67 | 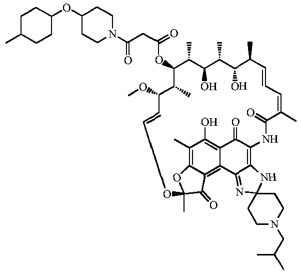 | 1069.60 | 20.85 | 1070.59 |
| 68 | 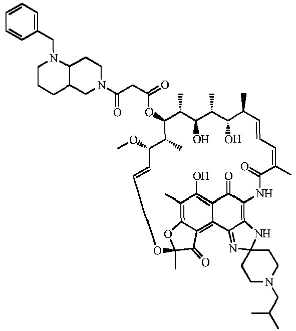 | 1102.60 | 19.14 | 1103.65 |
| 69 | 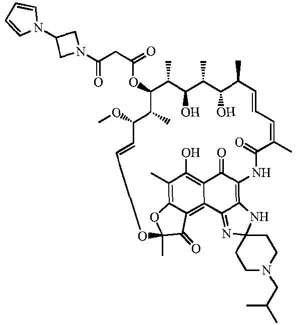 | | | |
| 70 | 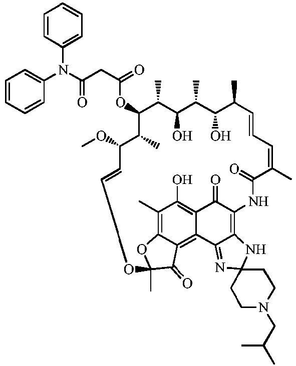 | | | |
| 71 | 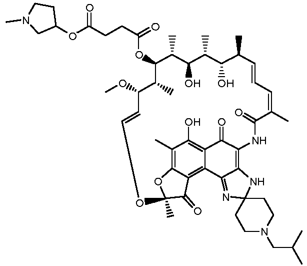 | 987.52 | 15.47 | 988.57 |
| 72 | 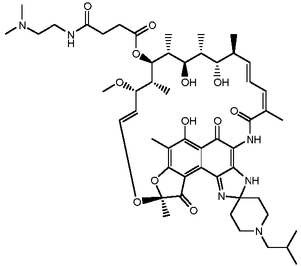 | 974.53 | 13.75 | 975.63 |
| 73 | 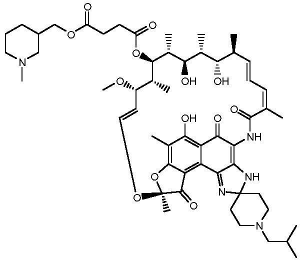 | 1015.55 | 15.87 | 1016.64 |
| 74 | 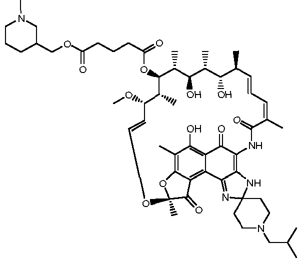 | 1029.56 | 16.22 | 1030.72 |
| 75 | 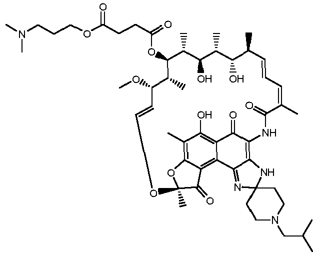 | 989.53 | 15.20 | 990.60 |
| 76 | 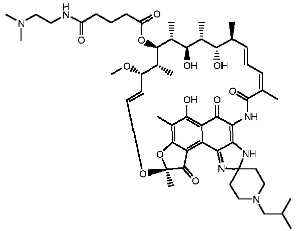 | 988.55 | 14.02 | 989.65 |
| 77 | 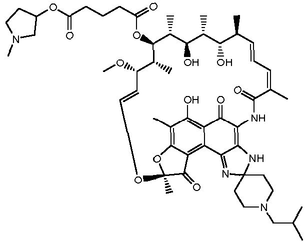 | 1001.53 | 15.85 | 1002.59 |
| 78 | 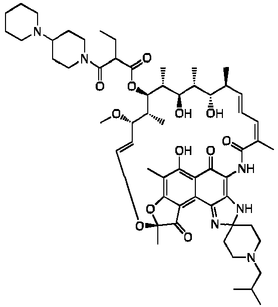 | 1068.61 | 16.15 | 1069.82 |
| 79 | 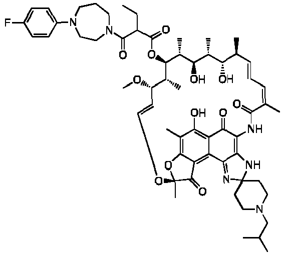 | 1094.57 | 18.64 | 1095.77 |
| 80 | 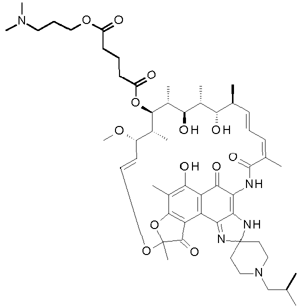 | 1003.55 | 15.60 | 1004.63 |
| 81 | 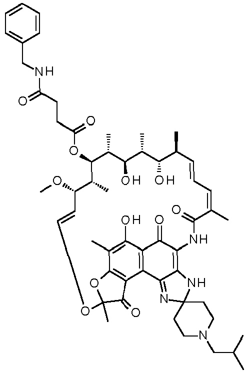 | 993.50 | 16.52 | 994.60 |
| 82 | 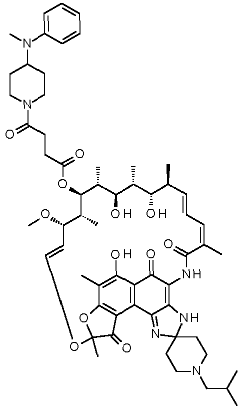 | 1076.58 | 18.85 | 1077.70 |
| 83 | 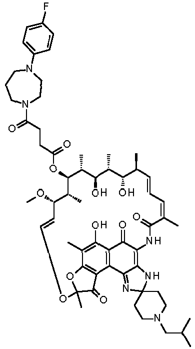 | 1080.55 | 18.20 | 1081.66 |
| 84 | 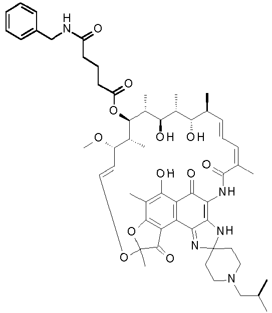 | 1007.52 | 16.49 | 1008.64 |
| 85 | 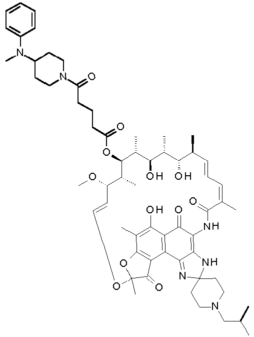 | 1090.59 | 18.87 | 1091.72 |
| 86 | 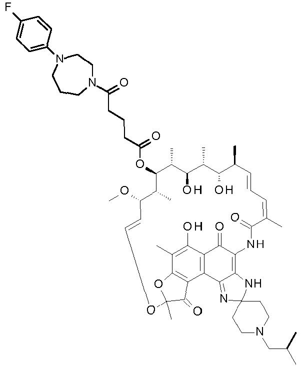 | 1094.57 | 18.17 | 1095.68 |
| 87 | 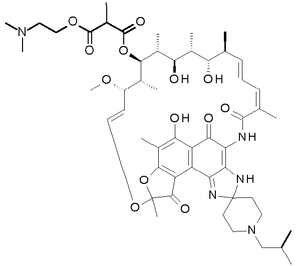 | 989.53 | 15.60 | 990.62 |
| 88 | 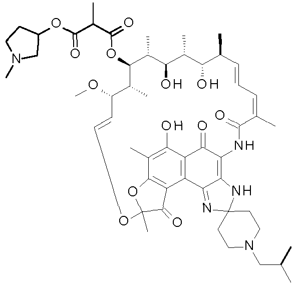 | 987.52 | 15.89 | 988.65 |
| 89 | 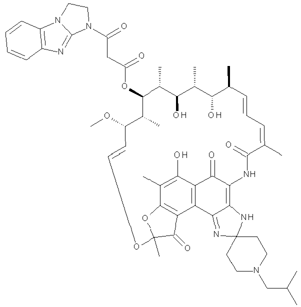 | 1031.50 | 16.80 | 1032.59 |
| 90 | 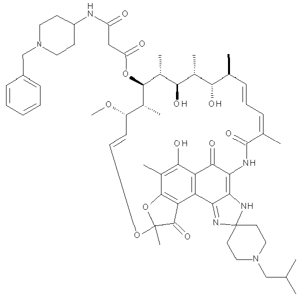 | 1062.57 | 16.80 | 1063.69 |
| 91 | 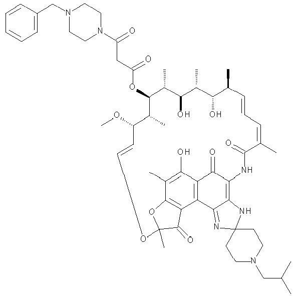 | 1048.55 | 17.47 | 1049.66 |
| 92 | 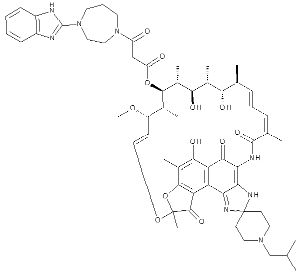 | 1088.56 | 14.77 | 1089.59 |
| 93 | 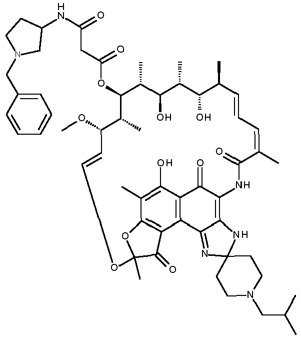 | 1048.55 | 16.72 | 1049.56 |
| 94 | 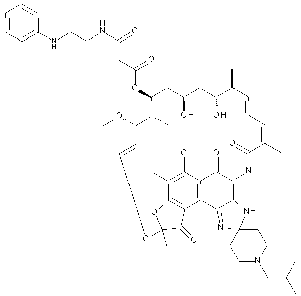 | 1008.52 | 16.52 | 1009.47 |
| 95 | 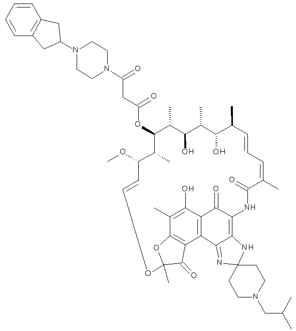 | 1074.57 | 17.53 | 1075.69 |
| 96 | 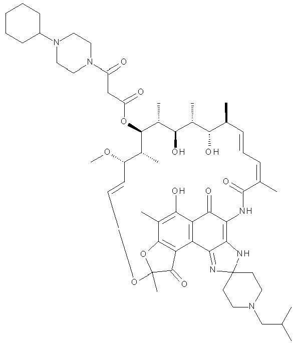 | 1040.58 | 17.65 | 1041.62 |
| 97 | 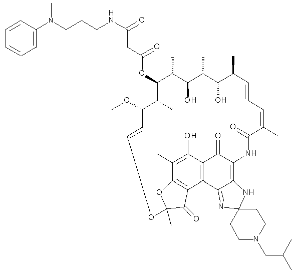 | 1036.55 | 17.57 | 1037.63 |
| 98 | 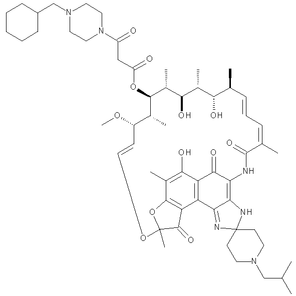 | 1054.60 | 20.02 | 1055.64 |
| 99 | 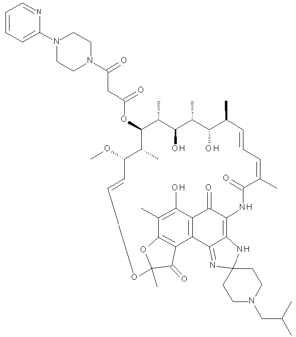 | 1035.53 | 16.14 | 1036.58 |
| 100 | 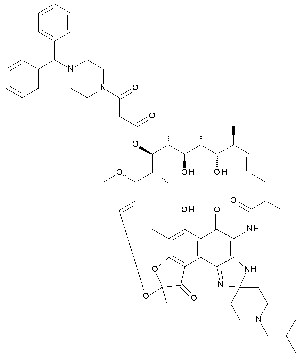 | 1124.58 | 20.03 | 1125.60 |
| 101 | 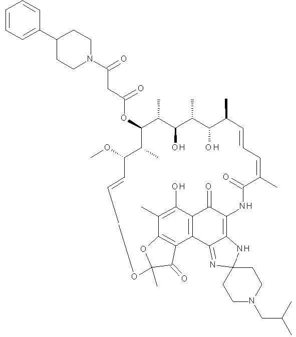 | 1033.54 | 18.49 | 1034.59 |
| 102 | 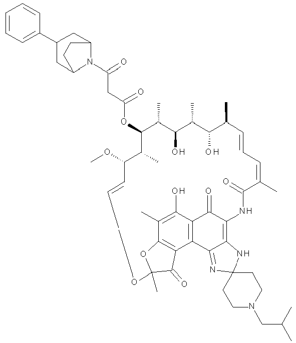 | 1059.56 | 18.78 | 1060.58 |
| 103 | 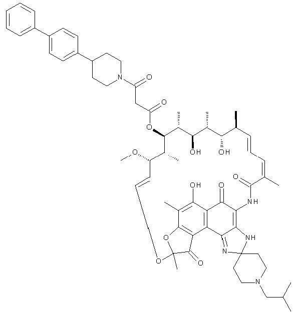 | 1109.57 | 20.49 | 1110.59 |
| 104 | 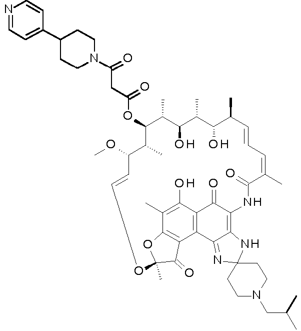 | 1034.54 | 14.92 | 1035.65 |
| 105 | 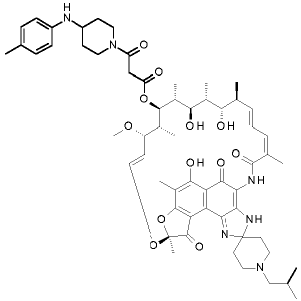 | 1062.57 | 18.15 | 1063.69 |
| 106 | 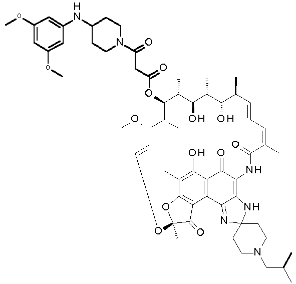 | 1108.57 | 16.97 | 1109.72 |
| 107 | 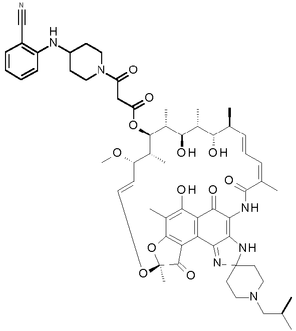 | 1073.55 | 17.33 | 1074.75 |
| 108 | 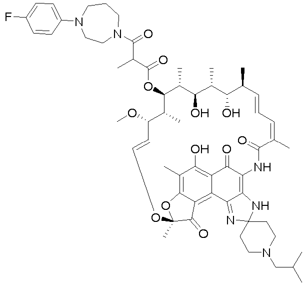 | 1080.55 | 18.07 | 1081..66 |
| 109 | 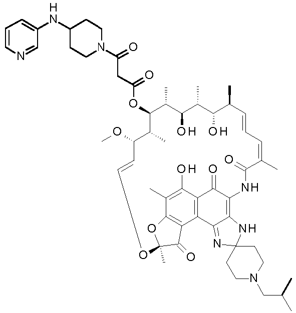 | 1049.55 | 14.60 | 1050.76 |
| 110 | 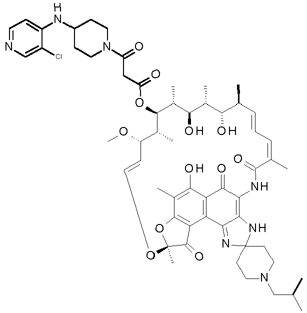 | 1083.51 | 15.30 | 1084.63 |
| 111 | 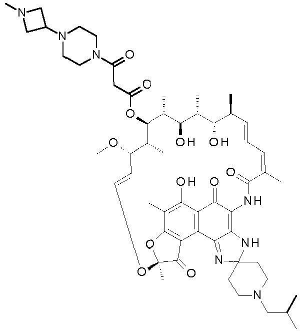 | 1027.26 | 13.22 | 1028.69 |
| 112 | 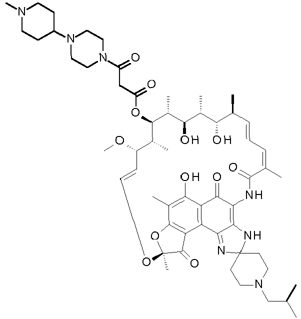 | 1055.59 | 13.67 | 1056.59 |
| 113 | 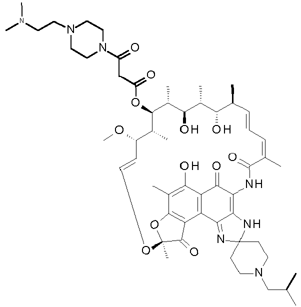 | 1029.58 | 13.60 | 1030.79 |
| 114 | 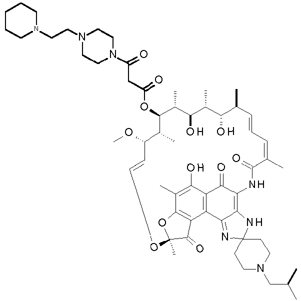 | 1069.61 | 14.60 | 1070.85 |
| 115 | 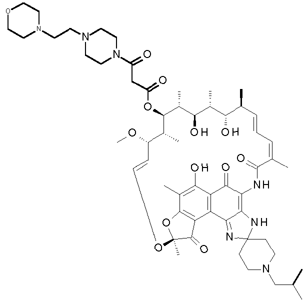 | 1071.59 | 13.55 | 1072.80 |
| 116 | 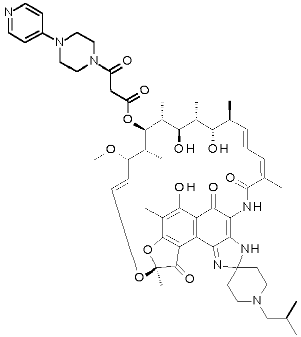 | 1035.53 | 14.09 | 1036.65 |
| 117 | 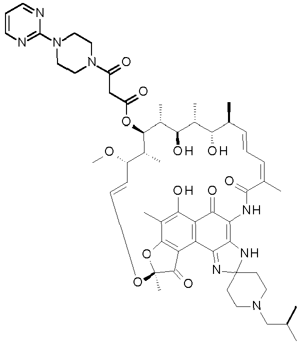 | 1036.53 | 15.47 | 1037.61 |
| 118 | 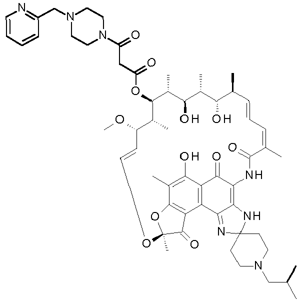 | 1049.54 | 14.62 | 1050.75 |
| 119 | 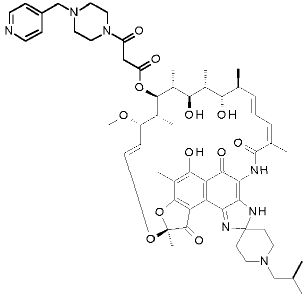 | 1049.54 | 14.34 | 1050.74 |
| 120 | 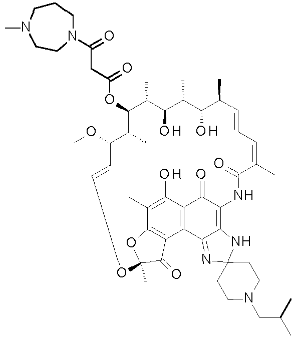 | 986.54 | 14.03 | 987.67 |
| 121 | 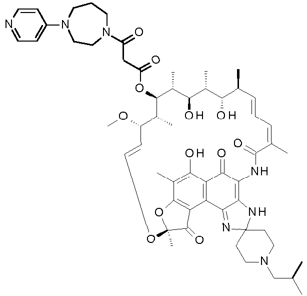 | 1049.55 | 14.02 | 1050.75 |
| 122 | 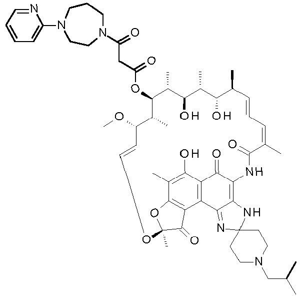 | 1049.55 | 15.97 | 1050.76 |
| 123 | 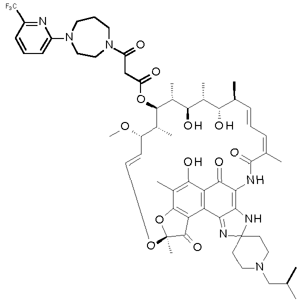 | 1117.53 | 18.58 | 1118.75 |
| 124 | 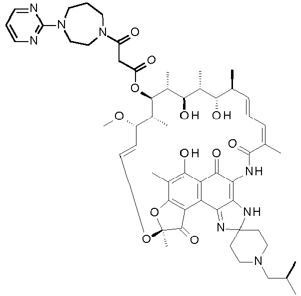 | 1050.54 | 15.53 | 1051.72 |
| 125 | 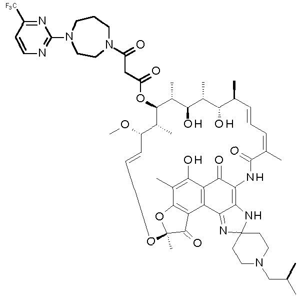 | 1118.53 | 18.25 | 1119.74 |
| 126 | 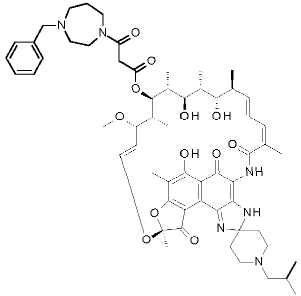 | 1062.57 | 17.65 | 1063.74 |
| 127 | 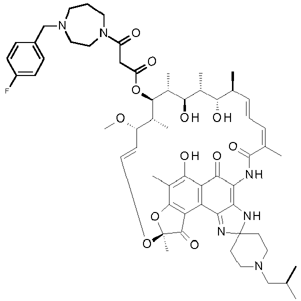 | 1080.56 | 17.80 | 1081.78 |
| 128 | 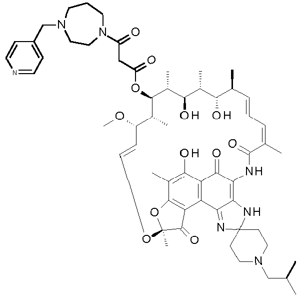 | 1063.56 | 14.50 | 1064.74 |

### Example 9: Synthesis of Compound 3

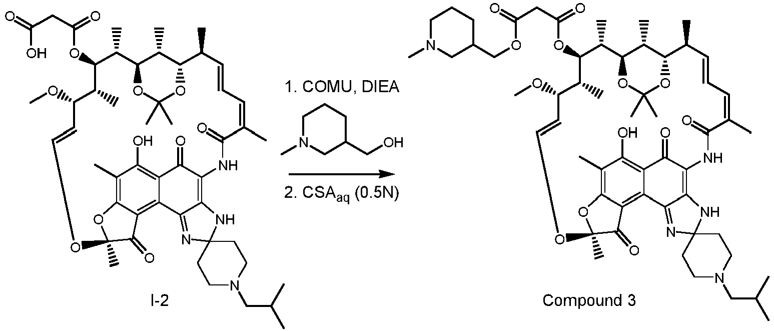

To a solution of I-2 (200mg, 0.21mmol, 1eq) in THF (5mL) was added (1-methyl-3-piperidyl)methanol (55µL, 0.43mmol, 2eq), and EDCI (113mg, 0.64mmol, 3eq). The reaction mixture was stirred at rt for 18h, filtered, and the solvent was evaporated. The obtained solid was re-dissolved in DCM (40mL) and washed with a sat. aq. solution of NaHCO₃ (3x40mL), brine (40mL) and dried over MgSO₄. The organic phase was then evaporated under vacuum. The obtained solid was purified by flash chromatography (DCM/MeOH from 100/0 to 90/10) to give a black solid (yield: 71.5%, Rt: 2.77min, purity (254nm): 99%, MS (ESI+) m/z = 1042.69 [M+H]⁺, 521.96 [M+2H]2+). The C21-C23-protected Compound 3 (160mg, 0.15mmol) was dissolved in 5mL of a solution of THF/0.5 N acid camphorsulfonic in water (1/1). The reaction was stirred at rt for 4h and 40mL of DCM was subsequently added. The mixture was washed with a sat. aq. solution of NaHCO₃ (3x40mL) and brine (40mL). The organic layer was dried over MgSO₄ and evaporated under vacuum. The obtained solid was purified by flash chromatography (DCM/MeOH: from 100/0 to 90/10) to give compound 3 as a black solid (yield: 52%, Rt: 2.43 min, purity (254nm)=99%, MS (ESI+) m/z = 1002.71 [M+H]⁺, 501.95 [M+2H]²⁺).

Compound 3 was further synthesized as follows: To a solution of I-2 (150mg, 0.16mmol, 1eq.) in THF (5mL) was added (1-methyl-3-piperidyl)methanol (0.32mmol, 2eq.), DIEA (110µL, 0.64mmol, 4eq) and COMU (137mg, 0.32mmol, 2eq). The reaction was stirred at rt. After 3h, the solvent was evaporated under vacuum. The obtained solid was dissolved in DCM (40mL) and washed with an aqueous solution of HCl 0.5N (3x40mL), a saturated aqueous solution of NaHCO₃ (3x40mL), brine (40mL), and dried over MgSO₄. The DCM was evaporated under vacuum. The obtained solid was then dissolved in 5mL of a solution of THF/camphorsulphonic acid in water 0.5 N (1/1). The reaction mixture was stirred at rt. After 24h, the mixture was diluted in 50mL of DCM and washed with a sat. aq. solution of NaHCO₃ (3x50mL), brine (50mL) and dried over MgSO₄. The solvent was evaporated under vacuum and the resulting solid was purified by preparative HPLC to afford Compound 3 as a purple solid (yield = 23 %). LC/MS A (ESI+): T_{R} = 1.73 min, m/z [M+H]⁺ = 1002.56, UV purity: 99 % (254 nm). HRMS (Maldi-TOF): m/z calculated for [M+H]⁺ = 1002.5434, found 1002.5461.

¹H NMR (300 MHz, CDCl₃): (mixture of diastereoisomers) δ -0.12 (d, *J* = 7.0 Hz, 3H), 0.58 (d, *J* = 6.7 Hz, 3H), 0.81 (d, *J* = 6.8 Hz, 3H), 0.92 (d, *J =* 6.5 Hz, 6H), 1.02 (d, *J =* 6.8 Hz, 3H), 1.33-1.45 (m, 1H), 1.60-2.22 (m, 14H), 1.71 (s, 3H), 2.02 (s, 3H), 2.24-2.45 (m, 3H), 2.28 (s, 3H), 2.41 (d, *J =* 12.1 Hz, 3H), 2.55-2.79 (m, 2H), 2.90-3.08 (m, 4H), 3.05 (s, 3H), 3.11 (d, *J* = 10.1 Hz, 1H), 3.20-3.34 (m, 3H), 3.37-3.60 (m, 2H), 3.66 (dd, *J* = 9.8 Hz, 2.8 Hz, 1H), 3.89-4.09 (m, 2H), 4.86 (dd, *J* = 9.83 Hz, 1H), 5.12-5.24 (m, 1H), 5.96 (dd, *J=* 15.5 Hz, 6.3 Hz, 1H), 6.10-6.41 (m, 3H), 8.22 (s, 0.5H) , 8.23 (m, 0.5H), 9.16 (s, 1H), 14.67 (s, 1H).

¹³C NMR (75 MHz, CDCl₃): (mixture of diastereoisomers) δ 7.87, 7.91, 8.98, 11.28, 11.38, 11.42, 17.53, 17.58, 20.45, 20.46, 21.07, 21.08, 22.22, 22.27, 23.78, 23.96, 25.96, 26.13, 33.17, 35.17, 35.38, 36.28, 37.73, 38.02, 38.61, 41.70, 41.79, 45.49, 45.72, 51.56, 51.62, 55.27, 55.42, 56.88, 56.93, 57.80, 58.00, 66.35, 67.38, 67.69, 72.58, 72.58, 72.65, 74.30, 74.38, 76.55, 76.69, 81.23, 81.43, 94.67, 94.71, 104.77, 107.60, 107.63, 109.20, 109.22, 111.90, 114.54, 114.60, 116.07, 116.22, 124.14, 124.17, 125.28, 131.62, 131.69, 133.23, 133.34, 141.04, 141.09, 142.12, 142.21, 144.81, 144.95, 155.39, 166.77, 166.78, 167.20, 167.33, 168.36, 168.38, 168.64, 168.95, 171.65, 181.14, 181.21, 192.68.

### Example 10: Synthesis of Compound 5

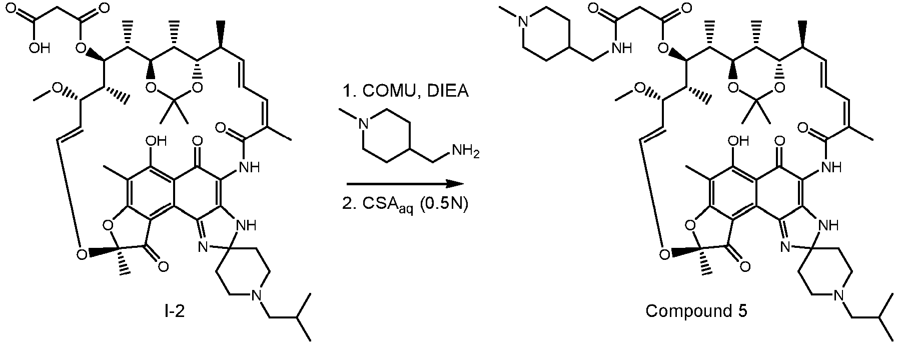

To a solution of I-2 (150mg, 0.16mmol, 1eq) in THF (5mL) was added (1-methyl-4-piperidyl)methanamine (27µL, 0.19mmol, 1.2eq), DIEA (110µL, 0.64mmol, 4eq) and COMU (137mg, 0.32mmol, 2eq). After 1h, the solvent was evaporated under vacuum. The obtained solid was dissolved in DCM (40mL) and washed with a sat. aq. solution of NaHCO₃ (3x40mL), brine (40mL), and dried over MgSO₄. The solvent was then evaporated under vacuum to give a black solid (quantitative yield) which was used without further purification (Rt: 2.65min, MS(ESI+) m/z = 1041.70 [M+H]⁺). The obtained C21-C23-protected Compound 5 (160mg, 0.15mmol) was dissolved in a solution of THF/0.5 N camphorsulphonic in water (1/1). The reaction mixture was stirred at rt for 3h and 40mL of DCM was added. The mixture was washed with a sat. aq. solution of NaHCO₃ (3x40mL), brine (40mL) and dried over MgSO₄. The solvent was evaporated under vacuum and the resulting solid was purified by flash chromatography (DCM/MeOH from 100/0 to 90/10) to give compound 5 as a black solid (yield: 51%, Rt: 2.28min, purity (254nm): 99%, MS(ESI+) m/z = 1001.7 [M+H]⁺, 501.4 [M+2H]²⁺).

Compound 5 was further synthesized as follows: To a solution of I-2 (150mg, 0.16mmol, 1eq) in THF (5mL) was added (1-methyl-4-piperidyl)methanamine (27µL, 0.19mmol, 1.2eq), DIEA (110µL, 0.64mmol, 4eq) and COMU (137mg, 0.32mmol, 2eq). After 1h, the solvent was evaporated under vacuum. The obtained solid was dissolved in DCM (40mL) and washed with a sat. aq. solution of NaHCO₃ (3x40mL), brine (40mL), and dried over MgSO₄. The solvent was then evaporated under vacuum to give a black solid (quantitative yield) which was used without further purification (Rt: 2.65min, MS(ESI+) m/z = 1041.70 [M+H]⁺).

The obtained C21-C23-protected Compound 5 (160mg, 0.15mmol) was dissolved in a solution of THF/0.5 N camphorsulphonic in water (1/1). The reaction mixture was stirred at rt for 3h and 40mL of DCM was added. The mixture was washed with a sat. aq. solution of NaHCO₃ (3x40mL), brine (40mL) and dried over MgSO₄. The solvent was evaporated under vacuum and the resulting solid was purified by flash chromatography (DCM/MeOH from 100/0 to 90/10) to give compound 5 as a black solid (yield: 51%, Rt: 2.28min, purity (254nm): 99%, MS(ESI+) m/z = 1001.7 [M+H]⁺, 501.4 [M+2H]²⁺).

### Example 11: Synthesis of Compound 14

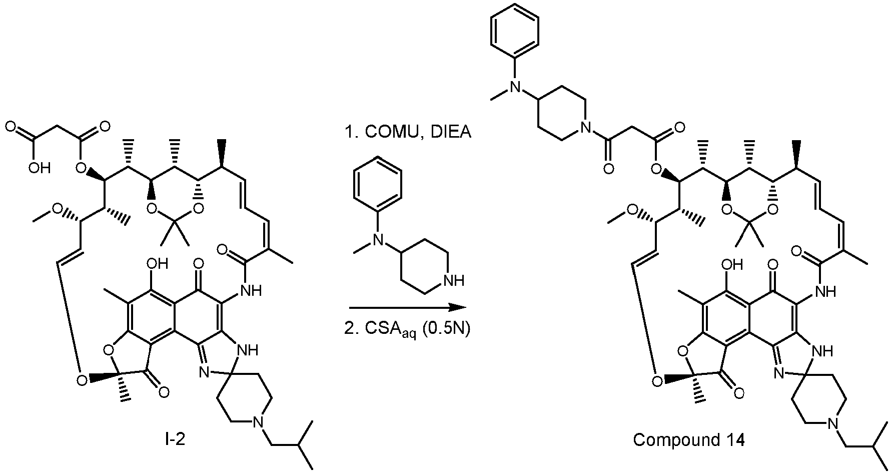

To a solution of I-2 (150mg, 0.16mmol, 1eq.) in THF (5mL) was added methyl-phenyl-piperidin-4-yl-amine (0.32mmol, 2eq.), DIEA (110µL, 0.64mmol, 4eq) and COMU (137mg, 0.32mmol, 2eq). The reaction was stirred at rt. After 3h, the solvent was evaporated under vacuum. The obtained solid was dissolved in DCM (40mL) and washed with an aqueous solution of HCl 0.5N (3x40mL), a saturated aqueous solution of NaHCO₃ (3x40mL), brine (40mL), and dried over MgSO₄. The DCM was evaporated under vacuum. The obtained solid was then dissolved in 5mL of a solution of THF/Camphorsulphonic acid in water 0.5 N (1/1). The reaction mixture was stirred at rt. After 24h, the mixture was diluted in 50mL of DCM and washed with a sat. aq. solution of NaHCO₃ (3x50mL), brine (50mL) and dried over MgSO₄. The solvent was evaporated under vacuum and the resulting solid was purified by preparative HPLC to afford Compound 14 as a purple solid (yield = 64 %). LC/MS A (ESI+): T_{R} = 2.33 min, m/z [M+H]⁺ = 1063.61, UV purity: 99 % (254 nm).

¹H NMR (300 MHz, CDCl₃): δ -0.06 (d, J = 7.1 Hz, 1.2H), -0.05 (d, J = 7.1 Hz, 1.8H), 0.83 (d, J = 6.8 Hz, 3H), 0.61 (d, J = 6.9 Hz, 3H), 0,96 (d, J = 6.6 Hz, 6H), 1.03 (d, J = 6.8 Hz, 1.2H), 1.04 (d, J = 6.9 Hz, 1.8H), 1.49-1.38 (m, 1H), 1.70-1.53 (m, 2H), 1.72 (s, 1.8H), 1.74 (s, 1.2H), 1.99-1.76 (m, 7H), 2.04 (s, 3H), 2.10-2.03 (m, 3H), 2.32 (s, 1.2H), 2.33 (s, 1.8H), 2.42-2.33 (m, 3H), 2.66-2.54 (m, 1H), 2.74 (s, 1.2H), 2.79-2.68 (m, 2H), ), 2.81 (s, 1.8H), 3.05-2.95 (m, 2H), 3.07 (s, 1.8H), 3.09 (s, 1.2H), 3.27-3.10 (m, 3H), 3.36-3.28 (m, 1H), 3.48-3.38 (m, 3H), 3.66 (d, J = 9.9 Hz, 1H), 3.88-3.74 (m, 2H), 4.73 (d, J = 13.0 Hz, 1H), 4.82 (t, J = 12.3 Hz, 1H), 5.18 (dd, J = 12.7 Hz, 7.7 Hz, 0.4H), 5.23 (dd, J = 12.6 Hz, 8.0 Hz,0.6H), 5.23 (dd, J = 12.6 Hz, 8.0 Hz,0.6H), 5.95 (t, J = 6.2 Hz, 0.4H), 5.95 (t, J = 6.2 Hz, 0.4H), 6.00 (t, J = 6.3 Hz, 0.6H), 6.17 (d, J = 12.5 Hz, 0.4H), 6.19 (d, J = 12.6 Hz), 6.25 (d, J = 10.5 Hz, 1H), 6.37 (dd, J = 15.2 Hz, 10.0 Hz, 1H), 6.75 (td, J = 7.3 Hz, 0.9 Hz, 1H), 6.86-6.78 (m, 2H), 7.24 (t, J = 7.8 Hz, 2H), 8.24 (s, 0.4H), 8.25 (s, 0.6H), 9.11 (brs, 0.4H), 9.20 (brs, 0.6H), 14.69 (s, 1H).

¹³C NMR (75 MHz, CDCl₃): δ 7.6, 8.9, 11.19, 11.22, 11.3, 11.5, 17.4, 17.5, 20.32, 20.34, 20.98, 20.99, 22.1, 22.2, 25.8, 28.7, 29.45, 29.48, 31.6, 31.7, 32.9, 33.0, 35.2, 36.1, 37.4, 37.7, 37.8, 38.4, 38.5, 41.4, 41.9, 42.0, 46.4, 51.5, 56.7, 56.8, 56.9, 57.0, 66.3, 72.4, 72.6, 74.1, 74.2, 76.6, 81.1, 81.8, 94.4, 104.7, 104.8, 107.5, 107.6, 109.0, 109.1, 111.8, 114.0, 114.1, 114.5, 114.6, 115.4, 115.6, 117.5, 117.7, 124.1, 125.0, 125.08, 125.1, 129.3, 131.40, 131.43, 133.1, 133.2, 140.9, , 141.9, 142.0, 144.7, 145.3, 149.8, 149.9, 155.3, 164.2, 164.3, 168.3, 168.37, 168.43, 168.5, 171.4, 171.5, 181.0, 181.2, 192.5, 192.6.

### Example 12: Synthesis of Compound 40

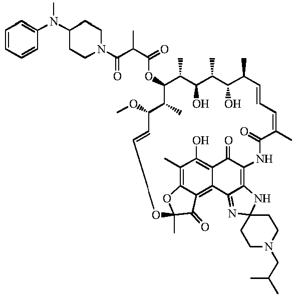

Following the above procedure for Compound 14, the title compound was obtained as a purple solid (yield = 33 %). LC/MS A (ESI+): T_{R} = 2.37 min, m/z [M+H]⁺ = 1077.65, UV purity: 92 % (254 nm). HRMS (Maldi-TOF): m/z calculated for [M+H]⁺ = 1077.5907, found 1077.5912.

¹H NMR (300 MHz, CDCl₃): (mixture of diastereoisomers and rotamers) δ -0.12-0.00 (m, 3H), 0.51-0.67 (m, 3H), 0.76-0.86 (m, 3H), 0.92-1.06 (m, 9H), 1.26-1.51 (m, 4H), 1.45-2.10 (m, 16H), 2.11-2.45 (m, 6H), 2.46-2.64 (m, 3H), 2.65-3.50 (m, 13H), 3.55-3.71 (m, 2H), 3.56-3.87 (m, 1H), 3.89-4.06 (m, 1H), 4.64-4.88 (m, 2H), 5.08-5.27 (m, 1H), 5.88-6.06 (m, 1H), 607-6.47 (m, 3H), 6.69-6.88 (m, 3H), 7.16-7.30 (m, 2H), 8.16-8.27 (m, 1H), 8.75-9.29 (m, 1H), 14.54-14.75 (m, 1H).

¹³C NMR (75 MHz, CDCl₃): (mixture of diastereoisomers and rotamers) δ 7.68, 8.81, 8.88, 8.97, 10.51, 11.02, 11.23, 11.26, 11.31, 11.67, 13.87, 14.02, 14.05, 14.16, 17.18, 17.27, 17.42, 20.21, 20.32, 20.82, 20.84, 21.74, 22.02, 22.08, 22.22, 25.34, 25.39, 28.65, 28.79, 28.84, 29.54, 29.60, 29.65, 31.59, 32.82, 32.94, 33.02, 34.53, 35.41, 37.15, 37.71, 37.81, 37.91, 38.08, 38.33, 38.39, 38.51, 42.12, 42.18, 42.25, 42.79, 43.07, 43.44, 43.54, 45.73, 46.05, 46.08, 46.17, 51.11, 56.46, 56.60, 56.70, 56.86, 56.91, 56.96, 57.02, 65.48, 65.54, 72.11, 72.52, 72.60, 72.74, 73.52, 73.64, 73.68, 74.12, 76.53, 76.75, 79.78, 80.98, 81.34, 81.99, 93.38, 93.53, 104.74, 104.90, 104.94, 107.23, 107.41, 107.46, 107.66, 108.78, 108.82, 108.99, 111.64, 111.78, 113.83, 114.09, 114.15, 114.37, 114.63, 114.66, 114.89, 115.02, 115.19, 115.40, 117.26, 117.65, 123.94, 124.19, 124.79, 124.87, 125.03, 129.24, 130.88, 130.99, 131.29, 133.21, 133.27, 140.79, 140.90, 140.96, 141.50, 141.68, 141.74, 141.91, 143.76, 144.47, 144.76, 145.47, 149.75, 149.91, 155.48, 155.60, 155.64, 167.71, 167.79, 167.85, 168.12, 168.26, 168.41, 168.47, 168.65, 170.97, 171.08, 171.36, 171.41, 171.48, 181.01, 181.38, 181.43, 192.48, 192.58, 192.62, 192.69.

### Example 13: Synthesis of Compound 50

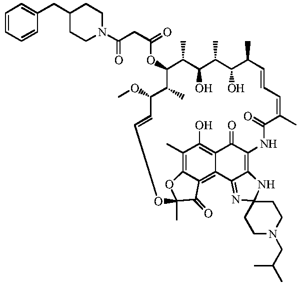

Following the above procedure for Compound 14, the title compound was obtained as a purple solid (yield = 15 %). LC/MS A (ESI+): T_{R} = 2.38 min, m/z [M+H]+ = 1048.67, UV purity: 99 % (254 nm). HRMS (Maldi-TOF): m/z calculated for [M+H]⁺ = 1048.5647, found 1048.5693.
¹H NMR (300 MHz, CDCl₃): (mixture of rotamers) δ -0.06 (dd, *J =* 6.2 Hz, 62 Hz, 3H), 0.59 (d, *J* = 6.7 Hz, 3H), 0.82 (d, *J =* 6.8 Hz, 3H), 0.91-1.03 (m, 9H), 1.11-1.25 (m, 2H), 1.36-1.51 (m, 1H), 1.64-2.07 (m, 10H), 1.73 (s, 3H), 2.03 (s, 3H), 2.26-2.40 (m, 6H), 2.51-2.58 (m, 2H), 2.61-3.10 (m, 6H), 3.06 (s, 3H), 3.13-3.24 (m, 1H), 3.30-3.39 (m, 3H), 3.45-3.74 (m, 4H), 4.46-4.60 (m, 1H), 4.81 (dd, *J* = 10.4 Hz, 10.4 Hz, 1H), 5.07-5.20 (m, 1H), 5.95 (dd, *J =* 6.6 Hz, 6.6 Hz, 0.5H), 6.00 (dd, *J* = 6.6 Hz, 6.6 Hz, 0.5H), 6.09-6.29 (m, 2H), 6.34 (dd, *J* = 15.4 Hz, 10.4Hz, 1H), 7.10-7.30 (m, 5H), 8.22 (s, 0.5H), 8.24 (s, 0.5H), 9.06 (s, 1H), 14.72 (s, 0.5H), 14.73 (s, 0.5H).
¹³C NMR (75 MHz, CDCl₃): (mixture of rotamers) δ 7.66, 7.71, 8.79, 10.83, 11.09, 11.17, 11.19, 17.31, 17.38, 20.28, 20.80, 21.90, 22.06, 25.69, 31.61, 32.26, 32.34, 32.91, 32.96, 35.16, 36.03, 37.47, 37.58, 37.64, 38.00, 38.17, 38.20, 38.28, 41.21, 42.24, 42.31, 42.84, 42.91, 46.66, 46.69, 51.41, 51.47, 56.70, 56.82, 66.17, 72.57, 72.66, 74.07, 74.15, 76.57, 80.50, 81.00, 94.26, 94.30, 104.69, 107.29, 107.43, 108.89, 111.74, 111.77, 114.32, 114.36, 115.19, 115.54, 124.12, 124.16, 125.00, 125.06, 126.10, 128.33, 129.09, 131.04, 131.08, 133.11, 139.75, 139.88, 140.87, 140.93, 141.86, 141.98, 144.20, 144.71, 155.29, 164.04, 164.09, 168.23, 168.26, 168.30, 168.38, 168.58, 168.63, 171.34, 171.42, 181.10, 181.15, 192.50, 192.56.

### Example 14: Synthesis of Compound 57

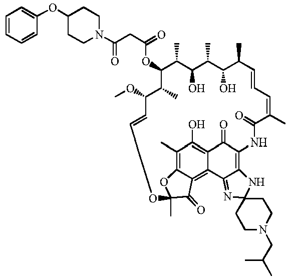

Following the above procedure for Compound 14, the title compound was obtained as a purple solid (yield = 42 %). LC/MS A (ESI+): T_{R} = 2.27 min, m/z [M+H]⁺ = 1050.56, UV purity: 99 % (254 nm). HRMS (Maldi-TOF): m/z calculated for [M+H]⁺ = 1050.5434, found 1050.5505.

¹H NMR (300 MHz, CDCl₃): (mixture of rotamers) δ -0.05 (dd, *J* = 1.6 Hz, *J* = 7.0 Hz, 3H), 0.60 (d, *J* = 6.4 Hz, 3H), 0.82 (d, *J* = 7.0 Hz, 3H), 0.95 (d, *J =* 6.5Hz, 6H), 1.0 (d, *J* = 6.6 Hz, 3H), 1.36-1.50 (m, 1H), 1.66-2.18 (m, 11H), 1.69 (s, 1.5H), 1.73 (s, 1.5H), 2.03 (s, 3H), 2.28-2.43 (m, 3H), 2.30 (s, 1.5H), 2.31 (s, 1.5H), 2.73-2.90 (m, 2H), 2.98-3.11 (m, 2H), 3.07 (s, 1.5H), 3.08 (s, 1.5H), 3.18 (t, *J* = 9 Hz, 1H), 3.27-3.41 (m, 4H), 3.48-3.88 (m, 6H), 4.50-4.62 (m, 1H), 4.80 (dd, *J =* 9.6, 5.7 Hz, 1H), 5.14 (quad d, *J* = 4.5 Hz, 12.5 Hz, 1H), 5.96 (ddd, *J =* 2.6 Hz, 6.6 Hz, 15.6 Hz, 1H), 6.17 (dd, *J* = 3.8 Hz, 12.5 Hz, 1H), 6.24 (d, J= 9.9 Hz, 1H), 6.36 (dd, *J* = 15.6 Hz, 10.4 Hz, 1H), 6.86-6.99 (m, 3H), 7.23-7.32 (m, 2H), 8.20 (s, 0.5H), 8.21 (s, 0.5H), 9.05 (s, 0.5H), 9.07 (s, 0.5H), 14.68 (s, 0.5H), 14.69 (s, 0.5H).

¹³C NMR (75 MHz, CDCl₃): (mixture of rotamers) δ 7.67, 8.81, 11.06, 11.17, 11.20, 17.29, 17.32, 20.26, 20.82, 20.83, 21.95, 22.10, 25.56, 29.68, 29.85, 29.99, 30.75, 30.81, 32.90, 32.94, 34.92, 35.79, 37.40, 37.50, 37.66, 38.31, 38.36, 38.52, 41.21, 41.24, 42.84, 43.06, 51.26, 51.30, 56.70, 56.72, 65.88, 70.79, 71.03, 72.50, 72.54, 74.11, 74.17, 76.56, 80.95, 81.21, 93.98, 104.73, 107.39, 107.46, 108.92, 111.70, 111.72, 114.44, 114.50, 115.04, 115.13, 116.05, 116.07, 121.23, 124.11, 124.94, 129.65, 131.11, 133.12, 140.88, 141.76, 141.81, 144.68, 144.88, 155.37, 156.94, 156.96, 164.19, 164.25, 168.10, 168.22, 168.29, 168.32, 168.49, 171.30, 171.36, 181.11, 181.15, 192.48, 192.56.

### Example 15: Synthesis of Compound 66

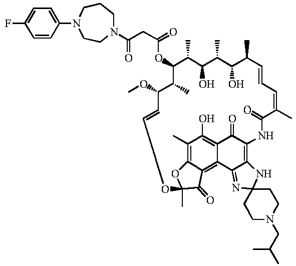

Following the above procedure for Compound 14, the title compound was obtained as a purple solid (yield = 38 %). LC/MS A (ESI+): T_{R} = 2.23 min, m/z [M+H]⁺ = 1067.61, UV purity: 97 % (254 nm). HRMS (Maldi-TOF): m/z calculated for [M+H]⁺ = 1067.5500, found 1067.5509.

¹H NMR (300 MHz, CDCl₃): (mixture of rotamers) δ -0.08 (d, *J =* 7.7 Hz, 3H), 0.56 (d, J = 5.0 Hz, 3H), 0.82 (d, J = 6.8 Hz, 3H), 0.97 (d, J = 6.5 Hz, 6H), 1.01 (d, J = 6.9 Hz, 3H), 1.35-1.47 (m, 1H), 1.66-2.06 (m, 7H), 1.73 (s, 3H), 2.03 (s, 3H), 2.12-2.32 (m, 2H), 2.29 (s, 3H), 2.32-2.42 (m, 1H), 2.46 (d, J = 6.7 Hz, 2H), 2.81-3.81 (m, 19H), 3.06 (s, 1.5H), 3.07 (s, 1.5H), 4.77-4.84 (m, 1H), 5.12-5.20 (m, 1H), 5.96 (dd, J = 6.5, 15.4 Hz, 1H), 6.16 (dd, J = 4.5, 12.6 Hz, 1H), 6.24 (d, J =10.4 Hz, 1H), 6.36 (ddd, J = 4.3, 10.7, 15.2 Hz, 1H), 6.57-6.65 (m, 2H), 6.87-6.95 (m, 2H), 8.25 (s, 1H), 8.98 (s, 1H), 14.65 (s, 0.5H), 14.67 (s, 0.5H).

¹³C NMR (75 MHz, CDCl₃): (mixture of rotamers) δ 7.73,8.86,10.99, 11.09, 11.21, 17.40, 20.37, 20.91, 22.03, 22.06, 24.37, 25.51, 26.27, 32.99, 34.73, 35.61, 37.37, 37.65, 37.72, 38.37, 38.46, 40.68, 41.27, 45.24, 46.03, 47.61, 47.87, 47.94, 49.07, 50.10, 51.01, 51.23, 51.26, 56.81, 56.84, 65.72, 72.56, 72.64, 74.10, 74.17, 76.57, 80.84, 81.06, 93.78, 104.84, 104.89, 107.44, 107.47, 108.94, 109.00, 111.77, 111.82, 112.73, 112.82, 112.91, 114.59, 114.62, 115.41, 115.93, 116.01, 116.22, 116.30, 124.13, 124.22, 125.02, 131.11, 131.26, 133.20, 133.26, 140.94, 140.96, 141.93, 143.54, 143.57, 143.80, 143.82, 144.47, 144.64, 153.73, 153.77, 155.55, 155.58, 156.85, 156.89, 165.59, 165.89, 168.27, 168.32, 168.40, 171.46, 171.48, 181.31, 181.37, 192.70, 192.72.

### Example 16: Synthesis of Compound 82

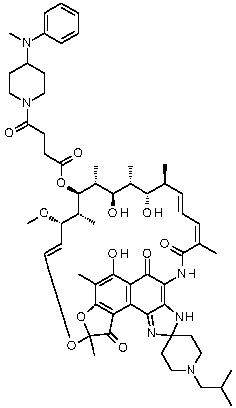

Following the above procedure for Compound 14, the title compound was obtained as a purple solid (yield = 42 %). LC/MS A (ESI+): T_{R} = 2.29 min, m/z [M+H]⁺ = 1077.54, UV purity : 99 % (254 nm). HRMS (Maldi-TOF): m/z calculated for [M+H]⁺ = 1077.5907, found 1077.5912.

¹H NMR (300 MHz, CDCl₃): (mixture of rotamers) δ -0.04 (d, *J* = 7.0 Hz, 1.5H), -0.03 (d, *J* = 7.0 Hz, 1.5H), 0.62 (d, *J =* 6.8 Hz, 3H), 0.82 (d, *J =* 6.9 Hz, 3H), 0.92 (d, *J =* 6.5 Hz, 6H), 1.02 (d, *J* = 6.9 Hz, 3H), 1.38-1.47 (m, 1H), 1.60-1.84 (m, 7H), 1.73 (s, 3H), 1.90-2.22 (m, 4H), 2.03 (s, 3H), 2.26-2.76 (m, 10H), 2.28 (s, 3H), 2.73 (d, *J* = 13.8 Hz, 3H), 2.86-3.00 (m, 2H), 3.01-3.17 (m, 1H), 3.05 (s, 3H), 3.17-3.30 (m, 1H), 3.35 (dd, *J* = 2.1 Hz, 7.1 Hz, 1H), 3.57-3.84 (m, 4H), 3.95 (d, *J =* 13.3 Hz, 1H), 4.27 (d, *J=* 13.3 Hz, 1H), 4.76 (dd, *J =* 8.7 Hz, 9.1 Hz, 1H), 5.16 (dd, *J* = 12.6 Hz, 7.2 Hz, 1H), 5.97 (dd, *J* = 15.5 Hz, 6.8 Hz, 1H), 6.15, (d, *J* = 12.5 Hz, 1H), 6.23 (d, *J* = 10.1 Hz, 0.8 Hz, 1H), 6.36 (dd, *J* = 15.6 Hz, 10.2 Hz, 1H), 6.73 (t, *J =* 7.2 Hz, 1H), 6.80 (d, *J* = 8.2 Hz, 2H), 7.17-7.26 (m, 2H), 8.23 (s, 1H), 8.97 (s, 1H), 14.79 (s, 1H).

¹³C NMR (75 MHz, CDCl₃): (mixture of rotamers) δ 7.87, 7.99, 9.11, 11.09, 11.16, 11.34, 17.61, 20.62, 21.10, 22.24, 26.08, 28.12, 28.25, 29.05, 29.58, 29.67, 31.76, 31.80, 33.20, 33.24, 35.65, 36.52, 37.74, 37.77, 37.85, 38.51, 42.02, 42.18, 45.28, 45.42, 51.62, 51.72, 57.05, 57.21, 66.54, 73.18, 73.41, 73.46, 76.83, 80.76, 80.91, 94.86, 104.95, 107.54, 107.56, 109.12, 112.11, 114.15, 114.26, 114.40, 115.48, 115.63, 117.63, 117.78, 124.43, 125.39, 129.49, 131.24, 133.22, 141.32, 142.35, 144.26, 144.40, 150.03, 150.08, 155.47, 168.53, 168.58, 169.78, 169.90, 171.55, 171.60, 174.28, 174.43, 181.48, 192.76.

### Example 17: Synthesis of Compound 85

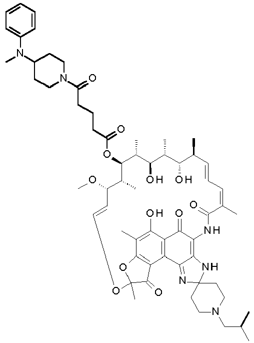

Following the above procedure for Compound 14, the title compound was obtained as a purple solid (yield = 35 %). LC/MS A (ESI+): T_{R} = 2.29 min, m/z [M+H]⁺ = 1091.57, UV purity : 99 % (254 nm). HRMS (Maldi-TOF): m/z calculated for [M+H]⁺ = 1091.6063, found 1091.6069.

¹H NMR (300 MHz, CDCl₃): (mixture of rotamers) δ -0.05 (d, *J* = 7.0 Hz, 3H), 0.62 (d, *J* = 6.8 Hz, 3H), 0.86 (d, *J =* 6.8 Hz, 3H), 0.96 (d, *J=* 6.5 Hz, 6H), 1.05 (d, *J* = 7.0 Hz, 3H), 1.37-1.51 (m, 1H), 1.62-2.08 (m, 13H), 1.74 (s, 1.5H), 1.75 (s, 1.5H), 2.07 (s, 3H), 2.29-2.46 (m, 7H), 2.34 (s, 3H), 2.56-2.71 (m, 3H), 2.77 (s, 3H), 2.94-3.17 (m, 4H), 3.08 (s, 1.5H), 3.08 (s, 1.5H), 3.32 (dd, *J* = 7.4 Hz, 2.2 Hz, 1H), 3.45 (s, 1H), 3.64-3.74 (m, 2H), 3.75-3.88 (m, 1H), 3.94-4.08 (m, 1H), 4.68-4.84 (m, 2H), 5.17 (dd, *J* = 7.6 Hz, 4.7 Hz, 0.5H), 5.21 (dd, *J* = 7.6 Hz, 4.7 Hz, 0.5H), 6.02 (dd, *J =* 15.3 Hz, 6.0 Hz, 1H), 6.19 (d, *J =* 12.3 Hz, 1H), 6.23-6.45 (m, 2H), 6.76 (t, *J =* 7.1 Hz, 1H), 6.84 (d, *J* = 8.25 Hz, 2H), 7.22-7.31 (m, 2H), 8.22 (s, 1H), 9.17 (s, 1H), 14.75 (s, 1H).

¹³C NMR (75 MHz, CDCl₃): (mixture of rotamers) δ 7.86, 9.04, 11.26, 11.35, 11.50, 17.62, 20.47, 20.64, 21.08, 22.17, 26.07, 29.07, 29.83, 31.78, 32.51, 32.63, 33.24, 33.69, 35.58, 36.48, 37.70, 37.75, 37.91, 38.50, 38.55, 41.86, 45.61, 51.61, 51.70, 56.98, 57.24, 66.52, 72.84, 73.09, 73.16, 77.10, 81.16, 81.36, 94.94, 104.76, 107.56, 109.19, 111.92, 114.19, 114.50, 116.00, 117.66, 124.21, 125.33, 131.58, 131.62, 133.20, 141.14, 142.24, 144.59, 144.71, 150.07, 155.32, 168.35, 168.64, 170.76, 170.81, 171.65, 174.31, 174.37, 181.18, 192.63.

### Example 18: Synthesis of Compound 90

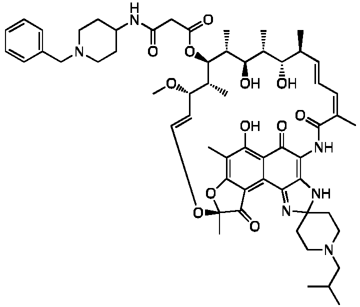

Following the above procedure for Compound 14, the title compound was obtained as a purple solid (yield = 47 %). LC/MS A (ESI+): T_{R} = 1.76 min, m/z [M+H]⁺ = 1063.51, UV purity : 99 % (254 nm).

¹H NMR (300 MHz, CDCl₃): (mixture of rotamers) δ -0.10 (d, *J =* 6.9 Hz, 3H), 0.59 (d, *J* = 6.6 Hz, 3H), 0.81 (d, *J* = 6.7 Hz, 3H), 0.93 (d, *J =* 6.4 Hz, 6H), 1.03 (d, *J =* 6.8 Hz, 3H), 1.24-1.32 (m, 1H), 1.44-1.59 (m, 2H), 1.71-2.20 (m, 11H), 1.72 (s, 3H), 2.04 (s, 3H), 2.27-2.45 (m, 3H), 2.34 (s, 3H), 2.60-2.83 (m, 4H), 2.89-3.13 (m, 4H), 3.02 (s, 3H), 3.16-3.27 (m, 3H), 3.37 (s, 1H), 3.50 (s, 2H), 3.65 (d, *J =* 9.7 Hz, 2H), 3.72-3.90 (m, 1H), 4.81 (d, *J* = 10.4 Hz, 1H), 5.32 (dd, *J =* 12.6 Hz, 9.2 Hz, 1H), 5.86-6.00 (m, 1H), 6.18-6.35 (m, 3H), 7.24-7.33 (m, 5H), 8.24 (s, 1H), 9.47 (s, 1H), 14.55 (s, 1H).

¹³C NMR (75 MHz, CDCl₃): (mixture of rotamers) δ 7.65, 8.79, 10.92, 12.02, 17.18, 20.11, 20.82, 20.84, 22.32, 25.81, 31.73, 31.84, 32.73, 35.19, 36.12, 37.04, 38.04, 38.93, 41.70, 46.30, 51.43, 51.98, 56.14, 63.10, 66.26, 71.85, 73.50, 76.32, 83.00, 94.74, 104.27, 107.66, 109.29, 111.43, 114.79, 115.63, 123.47, 124.82, 127.08, 128.23, 129.21, 132.28, 132.73, 138.15, 140.57, 141.75, 146.09, 154.89, 164.67, 167.91, 168.33, 169.29, 171.36, 180.56, 192.33.

### Example 19: Synthesis of Compound 91

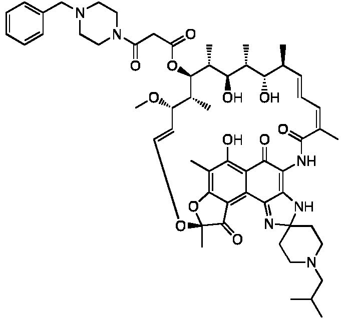

Following the above procedure for Compound 14, the title compound was obtained as a purple solid (yield = 35 %). LC/MS A (ESI+): T_{R} = 2.01 min, m/z [M+H]⁺ = 1049.57, UV purity : 99 % (254 nm).

¹H NMR (300 MHz, CDCl₃): (mixture of rotamers) δ -0.06 (d, *J* = 7.0 Hz, 3H), 0.59 (d, *J* = 6.8 Hz, 3H), 0.81 (d, *J =* 6.9 Hz, 3H), 0.93 (d, *J =* 6.7 Hz, 6H), 1.01 (d, *J =* 6.9 Hz, 3H), 1.35-1.52 (m, 1H), 1.68-2.14 (m, 7H), 1.73 (s, 3H), 2.03 (s, 3H), 2.27-2.48 (m, 7H), 2.31 (s, 3H), 2.60-2.75 (m, 2H), 2.91-3.03 (m, 2H), 3.06 (s, 3H), 3.18 (d, *J =* 9.9 Hz, 1H), 3.27-3.47 (m, 6H), 3.48-3.59 (m, 4H), 3.59-3.70 (m, 2H), 4.81 (d, *J* = 10.6 Hz, 1H), 5.14 (dd, *J* = 12.5 Hz, 7.6 Hz, 1H), 5.98 (dd, *J=* 15.6 Hz, 6.6 Hz, 1H), 6.16 (d, *J =* 12.5 Hz, 1H), 6.24 (d, *J =* 10.3 Hz, 1H), 6.37 (dd, *J =* 15.6 Hz, 10.4 Hz, 1H), 7.24-7.33 (m, 5H), 8.25 (s, 1H), 9.09 (s, 1H), 14.73 (s, 1H).

¹³C NMR (75 MHz, CDCl₃): (mixture of rotamers) δ 8.01, 9.10, 11.35, 11.47, 17.63, 20.57, 21.14, 22.30, 26.06, 33.22, 35.55, 36.41, 37.80, 37.91, 38.55, 41.42, 42.20, 46.60, 51.65, 51.71, 52.80, 53.17, 57.03, 63.11, 66.49, 72.85, 74.46, 76.84, 81.20, 94.77, 104.93, 107.66, 109.20, 112.06, 114.64, 115.60, 124.41, 125.35, 127.63, 128.66, 129.44, 131.45, 133.35, 137.76, 141.14, 142.27, 144.89, 155.51, 164.51, 168.55, 168.65, 168.68, 171.67, 181.37, 192.81.

### Example 20: Synthesis of Compound 95

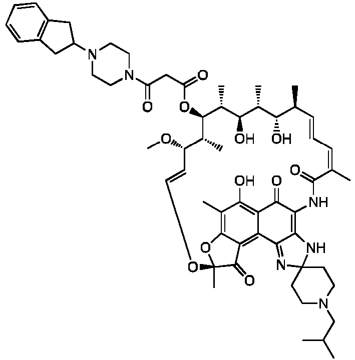

Following the above procedure for Compound 14, the title compound was obtained as a purple solid (yield = 45 %). LC/MS A (ESI+): T_{R} = 2.02 min, m/z [M+H]⁺ = 1075.60, UV purity : 99 % (254 nm).

¹H NMR (300 MHz, CDCl₃): (mixture of rotamers) δ -0.10 (d, *J =* 6.8 Hz, 3H), 0.60 (d, *J =* 6.5 Hz, 3H), 0.81 (d, *J* = 6.6 Hz, 3H), 0.98 (d, *J =* 6.5 Hz, 3H), 1.18 (d, *J =* 6.4 Hz, 6H), 1.28-1.48 (m, 2H), 1.60-1.80 (m, 2H), 1.65 (s, 3H), 1.81-1.95 (m, 1H), 1.99 (s, 3H), 2.17-2.39 (m, 2H), 2.29 (s, 3H), 2.65-2.91 (m, 4H), 2.97 (d, *J =* 7.0 Hz, 2H), 3.04-3.14 (m, 1H), 3.07 (s, 3H), 3.15-3.43 (m, 10H), 3.45-3.75 (m, 10H), 3.94-4.04 (m, 1H), 4.78 (d, *J =* 10.7 Hz, 1H), 5.20 (dd, *J =* 12.4 Hz, 8.8 Hz, 1H), 5.88 (dd, *J =* 15.1 Hz, 6.7 Hz, 1H), 6.12-6.36 (m, 3H), 7.08-7.23 (m, 4H), 8.25 (s, 1H), 8.98 (s, 1H), 14.47 (s, 1H).

¹³C NMR (75 MHz, CDCl₃): (mixture of rotamers) δ 8.09, 9.18, 11.37, 11.66, 17.38, 20.55, 21.57, 22.46, 24.83, 32.79, 33.04, 33.71, 36.06, 36.19, 36.72, 38.02, 39.01, 40.66, 41.41, 45.05, 50.71, 51.42, 51.77, 51.88, 56.87, 65.35, 67.12, 72.53, 74.09, 76.74, 82.09, 91.36, 105.51, 108.02, 109.27, 111.85, 115.02, 115.44, 124.47, 124.68, 124.72, 124.77, 127.33, 131.84, 133.13, 140.23, 140.54, 141.70, 145.63, 156.69, 164.54, 168.17, 168.35, 171.45, 182.05, 193.05.

### Example 21: Synthesis of Compound 98

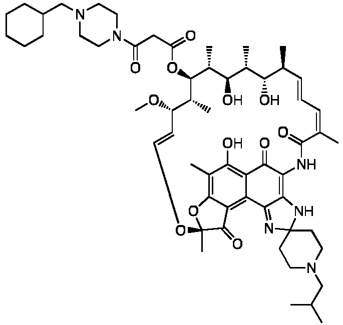

Following the above procedure for Compound 14, the title compound was obtained as a purple solid (yield = 56 %). LC/MS A (ESI+): T_{R} = 2.01 min, m/z [M+H]⁺ = 1055.58, UV purity : 99 % (254 nm).

¹H NMR (300 MHz, CDCl₃): (mixture of rotamers) δ -0.05 (d, *J =* 7.0 Hz, 3H), 0.59 (d, *J* = 6.9 Hz, 3H), 0.78-0.95 (m, 2H), 0.81 (d, *J =* 6.9 Hz, 3H), 0.92 (d, *J =* 6.5 Hz, 6H), 1.02 (d, *J* = 7.0 Hz, 3H), 1.14-1.21 (m, 2H), 1.37-1.53 (m, 2H), 1.63-1.87 (m, 9H), 1.73 (s, 3H), 1.89-2.00 (m, 2H), 2.01-2.16 (m, 4H), 2.03 (s, 3H), 2.23-2.41 (m, 7H), 2.30 (s, 3H), 2.53-2.71 (m, 2H), 2.87-3.01 (m, 2H), 3.07 (s, 3H), 3.14-3.22 (m, 1H), 3.29-3.41 (m, 5H), 3.42-3.57 (m, 3H), 3.58-3.70 (m, 2H), 4.79 (dd, *J =* 10.7 Hz, 0.9 Hz, 1H), 5.13 (dd, *J =* 12.6 Hz, 7.6 Hz, 1H), 5.98 (dd, *J* = 15.6 Hz, 6.6 Hz, 1H), 7.17 (d, *J =* 12.5 Hz, 1H), 6.24 (dd, *J =* 10.3 Hz, 1.0 Hz, 1H), 6.38 (dd, *J =* 15.6 Hz, 10.4 Hz, 1H), 8.21 (s, 1H), 9.11 (s, 1H), 14.73 (s, 1H).

¹³C NMR (75 MHz, CDCl₃): (mixture of rotamers) δ 8.02, 9.11, 11.40, 11.50, 17.64, 20.56, 21.13, 21.15, 22.33, 26.15, 26.38, 27.07, 32.09, 33.25, 35.25, 35.71, 36.56, 37.82, 37.92, 38.54, 41.46, 42.26, 46.64, 51.70, 51.77, 53.36, 53.91, 57.04, 65.70, 66.60, 72.87, 74.51, 76.90, 81.25, 94.92, 104.91, 107.68, 109.20, 112.08, 114.62, 115.57, 124.43, 125.37, 131.44, 133.38, 141.20, 142.23, 145.00, 155.47, 164.42, 168.57, 168.67, 168.83, 171.66, 181.30, 192.76.

### Example 22: Synthesis of Compound 99

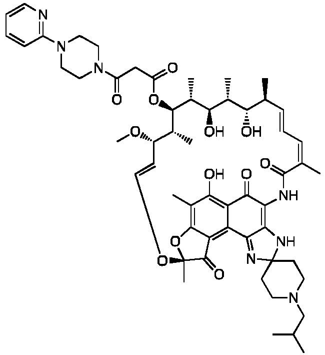

Following the above procedure for Compound 14, the title compound was obtained as a purple solid (yield = 23 %). LC/MS A (ESI+): T_{R} = 2.00 min, m/z [M+H]⁺ = 1036.54, UV purity : 99 % (254 nm).

¹H NMR (300 MHz, CDCl₃): (mixture of rotamers) δ -0.05 (d, *J =* 7.1 Hz, 3H), 0.60 (d, *J* = 6.8 Hz, 3H), 0.81 (d, *J =* 7.0 Hz, 3H), 0.92 (d, *J =* 6.5 Hz, 6H), 1.02 (d, *J =* 7.0 Hz, 3H), 1.36-1.48 (m, 1H), 1.72-2.11 (m, 7H), 1.72 (s, 3H), 2.03 (s, 3H), 2.23-2.31 (m, 2H), 2.28 (s, 3H), 2.31-2.42 (m, 1H), 2.50-2.75 (m, 2H), 2.86-3.02 (m, 2H), 3.09 (s, 3H), 3.13-3.23 (m, 1H), 3.34 (dd, *J* = 7.8 Hz, 2.5 Hz, 1H), 3.40-3.70 (m, 12H), 3.73-3.84 (m, 1H), 4.81 (dd, *J=* 10.6 Hz, 1.0 Hz, 1H), 5.17 (dd, *J* = 12.6 Hz, 7.9 Hz, 1H), 5.96 (dd, *J =* 15.5 Hz, 6.6 Hz, 1H), 6.15-6.28 (m, 2H), 6.36 (dd, *J* = 15.6 Hz, 10.4 Hz, 1H), 6.63-6.70 (m, 2H), 7.49 (dd, *J =* 7.2 Hz, 1.9 Hz, 0.5H), 7.52 (dd, *J* = 7.2 Hz, 1.9 Hz, 0.5H), 8.19 (dd, *J* = 5.1 Hz, 1.8 Hz, 1H), 8.24 (s, 1H), 9.16 (s, 1H), 14.71 (s, 1H).

¹³C NMR (75 MHz, CDCl₃): (mixture of rotamers) δ 7.97, 9.12, 11.47, 11.58, 17.61, 20.55, 21.13, 21.15, 22.39, 26.12, 33.21, 35.65, 36.51, 37.70, 37.99, 38.65, 41.54, 41.87, 45.34, 45.43, 46.23, 51.70, 51.76, 56.99, 66.58, 72.73, 74.48, 76.85, 81.63, 94.92, 104.85, 107.60, 107.74, 109.26, 112.02, 114.34, 114.72, 115.51, 124.34, 125.31, 131.61, 133.31, 138.03, 141.09, 142.21, 145.30, 148.32, 155.43, 159.29, 164.85, 168.52, 168.55, 168.64, 171.62, 181.24, 192.74.

### Example 23: Antibacterial Activity of Inventive Compounds

MIC values were determined by broth microdilution method according to the CLSI guideline. Unless otherwise mentioned, MIC against *A. baumannii* was performed in RPMI medium supplemented with 10% FCS. MIC against *S. pneumoniae* and *S. pyogenes* was performed in cation adjusted Mueller Hinton broth supplemented with 5% laked horse blood. MIC against *H. influenzae* was performed in Hemophilus test medium broth. MIC against *M*. *abscessus, M. tuberculosis* and *M*. *smegmatis* was performed in Middlebrook 7H9 broth supplemented with Middlebrook ADC growth supplement (10%). All other MIC were performed in standard cation adjusted Mueller Hinton broth, which was supplemented with Middlebrook ADC growth supplement (5%) for the slow growing mycobacteria (*M. xenopi*, *M*. *kansasii* and *M*. *avium*).

The following procedure applies to all species tested except *M*. *tuberculosis* and *M*. *smegmatis.* Cells were resuspended from a plate culture (overnight to 7 days culture depending on the species) in 0.9% (w/v) saline solution and bacterial inoculum was prepared in the respective testing medium with 5x10⁵ CFU/mL. For *M*. *xenopi*, the inoculum was prepared from a fresh liquid culture (7H9+ADC) instead of a plate. The appropriate volumes of a 10 mg/mL compound solution were directly dispensed in the 96-well assay plate using a digital dispenser to create two-fold dilution series from 32 to 0.00006 µg/mL final concentration (range adapted depending on the species). 100 µL of bacterial suspension was finally added to the compounds. Plates were covered and incubated without shaking at 35 °C for 20 hours to 7 days or more depending on the species. Every experiment contained an antibiotic as quality control. MIC was determined visually as the lowest concentration of a compound that prevents visible growth of the bacteria.

A similar procedure was used for *M*. *tuberculosis* and *M*. *smegmatis*, except that the inoculum was prepared from an exponentially growing culture and set to an OD₆₀₀ of 0.02 and 0.002 for *M*. *tuberculosis* and *M*. *smegmatis,* respectively. The MIC was determined at 90% growth inhibition using a GFP reporter after 5 days incubation for *M*. *tuberculosis* and using the BacTiter-Glo kit (Promega) after 20 hours incubation for *M*. *smegmatis.*

The *in vitro* activity of compounds described herein was determined against *S. aureus* (UAMS-1625 strain), *A*. *baumannii* (HUMC1 strain), *M*. *abscessus* (ATCC 19977), *M*. *kansasii* (ATCC 12478), *M*. *avium* (ATCC 25291), and *M. xenopi* (ATCC 19250). The MIC values, given in µg/mL, are given in Table 4, below.

**Table 4: In vitro activity of described compounds against S. aureus, A. baumannii, M. abscessus, M. kansasii, M. xenopi and M. avium.**

| Compound Number | *S. aureus* | *A. baumannii* | *M*. *abscessus* | *M*. *kansasii* | *M*. *xenopi* | *M. avium* |
|---|---|---|---|---|---|---|
| Rifabutin | 0.016 | ≤0.002 | 8 | 0.001 | 0.03 | 0.125 |
| 3 | 0.06 | ≤0.002 | 1 | 0.125 | 1 | 1 |
| 5 | 0.125 | ≤0.002 | 1 | | | |
| 6 | 0.03 | ≤0.002 | 1 | | | |
| 7 | 0.016 | ≤0.002 | 1 | | | |
| 8 | 0.016 | 0.004 | 0.5 | | | |
| 10 | 0.016 | ≤0.002 | 1 | | | |
| 11 | 0.25 | 0.016 | 0.125 | 0.008 | 0.06 | 0.125 |
| 12 | 0.125 | 0.03 | 0.5 | | | |
| 14 | 0.5 | 0.06 | 0.125 | 0.002 | 0.03 | 0.06 |
| 15 | 0.016 | 0.004 | 1 | | | |
| 16 | 0.03 | 0.004 | 2 | | | |
| 17 | 0.03 | ≤0.002 | 2 | | | |
| 18 | 0.06 | ≤0.002 | 1 | | | |
| 20 | 0.03 | ≤0.002 | 1 | | | |
| 23 | 0.03 | ≤0.002 | 1 | | | |
| 26 | 0.5 | 0.03 | 4 | | | |
| 27 | 0.125 | 0.016 | 4 | | | |
| 29 | 0.03 | ≤0.002 | 1 | | | |
| 30 | 0.03 | ≤0.002 | 1 | | | |
| 31 | 1 | 0.125 | 4 | | | |
| 34 | 0.016 | ≤0.002 | 1 | | | |
| 36 | 0.016 | ≤0.002 | 1 | | | |
| 37 | 0.03 | ≤0.002 | 0.5 | | | |
| 38 | 0.25 | 0.008 | 0.5 | | | |
| 40 | 0.5 | 0.03 | 0.125 | 0.001 | 0.03 | 0.06 |
| 41 | 0.5 | 0.016 | 0.5 | | | |
| 42 | 0.5 | 0.03 | 0.5 | | | |
| 43 | 0.5 | 0.06 | 0.5 | | | |
| 44 | 0.25 | 0.008 | 0.25 | 0.004 | 0.03 | 0.06 |
| 45 | 0.06 | 0.004 | 0.5 | | | |
| 46 | 0.25 | 0.03 | 0.5 | | | |
| 47 | 1 | 0.125 | 0.5 | | | |
| 48 | 0.25 | 0.03 | 0.5 | | | |
| 49 | 1 | 0.25 | 0.25 | 0.008 | 0.03 | 0.06 |
| 50 | 2 | 0.06 | 0.125 | 0.004 | 0.03 | 0.06 |
| 51 | 2 | 0.06 | 1 | | | |
| 52 | 0.5 | 0.016 | 0.125 | 0.002 | 0.03 | 0.03 |
| 53 | 0.125 | ≤0.002 | 1 | | | |
| 54 | 0.125 | 0.008 | 0.25 | 0.008 | 0.125 | 0.06 |
| 55 | 0.5 | 0.016 | 0.25 | 0.016 | 0.06 | 1 |
| 56 | 2 | 0.03 | 0.25 | 0.03 | 0.06 | 0.5 |
| 57 | 0.5 | 0.03 | 0.125 | 0.008 | 0.03 | 0.125 |
| 58 | 0.03 | ≤0.002 | 1 | | | |
| 59 | 0.125 | 0.008 | 0.5 | | | |
| 60 | 0.03 | ≤0.002 | 0.5 | | | |
| 61 | 0.125 | 0.008 | 0.5 | | | |
| 62 | 0.06 | ≤0.002 | 1 | | | |
| 63 | 0.25 | 0.016 | 0.5 | | | |
| 64 | 0.06 | ≤0.002 | 1 | | | |
| 65 | 0.125 | 0.016 | 0.5 | | | |
| 66 | 0.125 | 0.016 | 0.125 | 0.016 | 0.06 | 0.125 |
| 67 | 0.25 | 0.008 | 0.125 | 0.008 | 0.125 | 0.25 |
| 68 | 0.5 | 0.03 | 0.25 | 0.001 | 0.125 | 0.03 |
| 72 | 0.06 | ≤0.002 | 1 | | | |
| 73 | 0.06 | ≤0.002 | 1 | | | |
| 74 | 0.06 | ≤0.002 | 1 | | | |
| 77 | 0.06 | ≤0.002 | 0.5 | | | |
| 79 | 0.5 | 0.03 | 0.125 | 0.004 | 0.03 | 0.125 |
| 82 | 0.5 | 0.016 | 0.25 | 0.016 | 0.03 | 0.25 |
| 83 | 0.5 | 0.016 | 0.25 | 0.008 | 0.03 | 0.25 |
| 85 | 0.5 | 0.03 | 0.125 | 0.008 | 0.03 | 0.25 |
| 86 | 0.5 | 0.008 | 0.125 | 0.008 | 0.03 | 0.125 |
| 89 | 0.125 | 0.016 | 0.5 | | | |
| 90 | 0.125 | 0.016 | 0.25 | 0.008 | 0.25 | 0.125 |
| 91 | 0.25 | 0.016 | 0.125 | 0.001 | 0.03 | 0.125 |
| 93 | 0.125 | 0.008 | 0.25 | 0.008 | 0.25 | 0.5 |
| 95 | 0.5 | 0.03 | 0.125 | 0.002 | 0.03 | 0.125 |
| 96 | 0.125 | 0.008 | 0.25 | 0.008 | 0.125 | 0.03 |
| 97 | 0.25 | 0.008 | 0.25 | 0.004 | 0.06 | 0.125 |
| 98 | 0.5 | 0.03 | 0.125 | 0.002 | 0.016 | 0.03 |
| 99 | 0.06 | 0.008 | 0.25 | 0.004 | 0.125 | 0.125 |
| 101 | 0.5 | 0.06 | 0.125 | 0.002 | 0.03 | 0.03 |
| 102 | 0.5 | 0.03 | 0.25 | 0.008 | 0.06 | 0.03 |
| 104 | 0.06 | 0.004 | 0.25 | 0.004 | 0.125 | 0.25 |
| 105 | 0.25 | 0.06 | 0.25 | 0.004 | 0.03 | 0.125 |
| 106 | 0.25 | 0.008 | 0.25 | 0.03 | 0.125 | 0.5 |
| 107 | 0.25 | 0.5 | 0.125 | 0.016 | 0.06 | 0.125 |
| 108 | 0.25 | 0.06 | 0.25 | 0.008 | 0.06 | 0.25 |
| 109 | 0.06 | 0.004 | 0.25 | 0.03 | 0.25 | 1 |
| 110 | 0.125 | 0.06 | 0.125 | 0.008 | 0.125 | 0.25 |
| 111 | 0.125 | 0.06 | 0.5 | | | |
| 112 | 0.125 | 0.004 | 1 | | | |
| 113 | 0.06 | 0.06 | 0.5 | | | |
| 114 | 0.125 | ≤0.002 | 0.5 | | | |
| 115 | 0.06 | ≤0.002 | 0.5 | | | |
| 116 | 0.06 | ≤0.002 | 0.25 | 0.06 | 1 | 2 |
| 117 | 0.03 | ≤0.002 | 0.125 | 0.004 | 0.125 | 0.25 |
| 118 | 0.06 | ≤0.002 | 0.25 | 0.03 | 0.25 | 2 |
| 119 | 0.016 | ≤0.002 | 0.25 | 0.008 | 0.25 | 1 |
| 120 | 0.06 | 0.06 | 0.25 | 0.25 | 1 | 2 |
| 121 | 0.125 | ≤0.002 | 0.25 | 0.125 | | 2 |
| 122 | 0.06 | ≤0.002 | 0.125 | 0.002 | 0.03 | 0.125 |
| 123 | 0.5 | 0.25 | 0.25 | 0.008 | 0.125 | 0.06 |
| 124 | 0.03 | ≤0.002 | 0.125 | 0.008 | 0.125 | 0.125 |
| 125 | 0.5 | 0.25 | 0.125 | 0.008 | 0.06 | 0.03 |
| 126 | 0.125 | 0.004 | 0.125 | 0.004 | 0.03 | 0.03 |
| 127 | 0.25 | 0.06 | 0.125 | 0.008 | 0.03 | 0.125 |
| 128 | 0.06 | ≤0.002 | 0.25 | 0.03 | 0.25 | 0.5 |

### In vitro Activity Against M. abscessus Complex

Compound 3, rifabutin, rifampicin, 3-(4-morpholinyl)rifamycin S, and CGP4832 were tested for MIC activity against the following five isolates of the *Mycobacterium abscessus* complex: (1) *M. abscessus subsp. abscessus*; strain ID No. ATCC 19977; (2) *M. abscessus subsp. abscessus*; strain ID No. CCUG 71636; (3) *M. abscessus subsp. abscessus*; strain ID No. CCUG 71382; (4) *M. abscessus subsp. bolletii*; strain ID No. CCUG 50184; and (5) *M*. *abscessus subsp. massiliense*; strain ID No. CCUG 48898. The structures of rifabutin, rifampicin, 3-(4-morpholinyl)rifamycin S, and CGP4832 and results are given below.

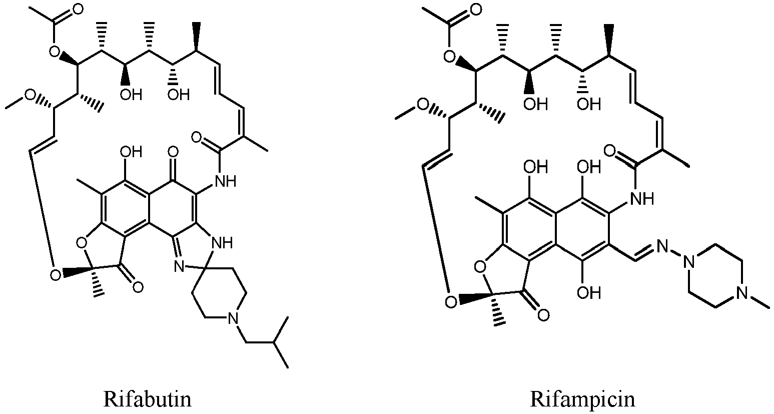

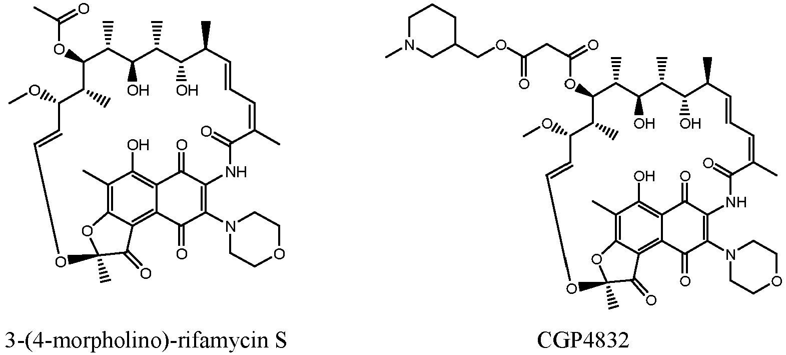

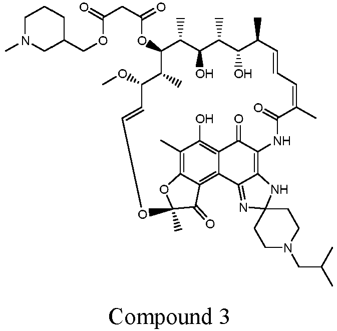

**Table 5: In vitro activity against M. abscessus isolates in Middlebrook 7H9 broth supplemented with Middlebrook ADC growth supplement**

| | *M*. *abscessus* MIC (mg/L) in 7H9 + ADC | | | | |
|---|---|---|---|---|---|
| Subspecies | *abscessus* | *abscessus* | *abscessus* | *bolletii* | *massiliense* |
| Strain ID | ATCC 19977 | CCUG 71636 | CCUG 71382 | CCUG 50184 | CCUG 48898 |
| Rifampicin | >32 | >32 | >32 | >32 | 32 |
| Rifabutin | 8 | 4 | 16 | 8 | 2 |
| Compound 3 | 1 | 0.5 | 1 | 0.5 | 2 |
| 3-(4-morpholinyl)rifamycin S | 4 | 4 | 8 | 4 | 32 |
| CGP 4832 | 4 | 2 | 8 | 8 | 4 |

In addition, Compounds 85, 90 and 98 were tested against the same five isolates of the *Mycobacterium abscessus* complex used above in Table 5, in CAMHB medium. Data are shown in Table 6 below.

**Table 6: In vitro activity against M. abscessus isolates in CAMHB**

| | *M*. *abscessus* MIC (mg/L) in CAMHB | | | | |
|---|---|---|---|---|---|
| Subspecies | *abscessus* | *abscessus* | *abscessus* | *bolletii* | *massiliense* |
| Strain ID | ATCC 19977 | CCUG 71636 | CCUG 71382 | CCUG 50184 | CCUG 48898 |
| Rifabutin | 16 | 2 | >16 | 16 | 8 |
| 90 | 1 | 0.125 | 1 | 0.5 | 1 |
| 98 | 0.25 | 0.125 | 0.5 | 0.25 | 0.5 |
| 85 | 1 | 0.5 | 1 | 1 | 1 |

### In vitro Activity Against A. baumannii

Compound 3, rifabutin, rifampicin, 3-(4-morpholinyl)rifamycin S, and CGP4832 were tested for MIC activity against the following isolates of *A. baumannii*: wild type (WT); Arr-2, RpoB H535Q mutant; RpoB H535C mutant; and RpoB H535L mutant. The results are given in Table 7, below.

**Table 7: In vitro activity against A. baumannii isolates.**

| | *A. baumannii* MIC (mg/L) in RPMI + FCS | | | | |
|---|---|---|---|---|---|
| Strain ID | WT | Arr-2 | RpoB H535Q | RpoB H535C | RpoB H535L |
| Rifampicin | 32 | > 32 | > 32 | > 32 | > 32 |
| Rifabutin | ≤ 0.002 | 0.125 | 0.004 | 0.03 | > 32 |
| Compound 3 | ≤ 0.002 | ≤ 0.002 | ≤ 0.002 | 0.008 | 0.25 |
| 3-(4-morpholinyl) rifamycin S | 16 | ND | ND | ND | ND |
| CGP 4832 | 8 | 4 | > 32 | > 32 | > 32 |

| | | | | | |
|---|---|---|---|---|---|
| ND: not determined | | | | | |

### In vitro Activity Against Additional Bacterial Species

In addition, Compound 3, rifabutin, rifampicin, 3-(4-morpholinyl)rifamycin S, and CGP4832 were tested for MIC activity against strains from the following species: *Enterococcus faecium* (strain NCTC 12204), *Enterococcus faecalis* (strain ATCC 51575), *Staphylococcus epidermidis* (strain ATCC 12228), *Streptococcus pneumoniae* (strain ATCC 49619), *Streptococcus pyogenes* (strain IHMA 1117021), *Hemophilus influenzae* (strain ATCC 49247), *Mycobacterium tuberculosis* (H37Rv, strain ATCC 27294) and *M. smegmatis* (strain ATCC 700084). The results are given in Table 8, below.

**Table 8: In vitro activity against other species**

| MIC (mg/mL) | *E. faecium* | *E. faecalis* | *S. epidermidis* | *S. pneumoniae* |
|---|---|---|---|---|
| Rifampicin | 0.5 | 1 | ≤ 0.002 | 0.016 |
| Rifabutin | 0.125 | 4 | 0.004 | ≤ 0.002 |
| Compound 3 | 1 | 2 | ≤ 0.002 | ≤ 0.002 |
| 3-(4-morpholinyl) rifamycin S | 0.25 | 1 | ≤ 0.002 | ≤ 0.002 |
| CGP 4832 | 2 | 2 | ≤ 0.002 | ≤ 0.002 |
| | | | | |

| MIC (mg/mL) | *M*. *tuberculosis* | *S. pyogenes* | *H. influenzae* | *M. smegmatis* |
|---|---|---|---|---|
| Rifampicin | 0.0041 | 0.03 | 0.25 | 1.12 |
| Rifabutin | 0.0035 | 0.004 | 0.5 | 0.24 |
| Compound 3 | 0.0049 | ≤ 0.002 | 1 | 0.03 |
| 3-(4-morpholinyl) rifamycin S | 0.0037 | 0.03 | 0.125 | 0.30 |
| CGP 4832 | 0.0036 | 0.008 | 0.5 | 0.03 |

## Claims

1. A compound of the Formula I or a pharmaceutically acceptable salt, tautomer, solvate, hydrate or enantiomer thereof: 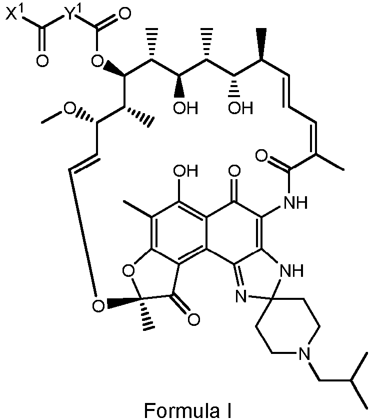 wherein:
Y¹ is (CR¹R²)ₙ, wherein n is an integer from 1 to 6 and R¹ and R² are independently, at each occurrence, selected from the group consisting of hydrogen, -C₁-C₆ alkyl, and -C₁-C₆ alkoxy, wherein each alkyl is optionally substituted with one or more halogen or -C₃-C₆ cycloalkyl, and wherein each cycloalkyl is further optionally substituted with one or more halogen;
X¹ is -OR¹¹ or -NR⁵¹R⁵²;
R¹¹ is independently -C₁-C₆ alkyl, -C₁-C₆ alkylene-R^{12A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, or 5-10 membered heteroaryl, wherein each alkyl, alkylene, alkenyl, alkynyl, cycloalkyl, cycloalkenyl, heterocycloalkyl, aryl or heteroaryl is optionally substituted with one or more R¹²;
R¹² and R^{12A} are each independently -C₁-C₆ alkyl, -C₁-C₆ alkylene-R^{16A}, -C₂-C₆ alkenyl, -C₂-C₆ alkynyl, -C₃-C₆ cycloalkyl, -C₅-C₈ cycloalkenyl, 3-10 membered heterocycloalkyl, - C₆-C₁₀ aryl, 5-10 membered heteroaryl, -C₁-C₆ alkoxy, -C₁-C₆ haloalkyl, halogen, cyano, oxo, -OR¹³, -CO₂R¹³, or -NR¹⁴R¹⁵, wherein each alkyl, alkylene, alkenyl, alkynyl, cycloalkyl, cycloalkenyl, heterocycloalkyl, aryl, heteroaryl and alkoxy is optionally substituted with one or more R¹⁶;
R¹³, R¹⁴ and R¹⁵ are each independently hydrogen, -C₁-C₆ alkyl, -C₁-C₆ alkylene-R^{20A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, -S(O)₂(C₁-C₆ alkyl), or -S(O)₂NR¹⁸R¹⁹, wherein each alkyl, alkylene, alkenyl, alkynyl, cycloalkyl, cycloalkenyl, heterocycloalkyl, aryl, heteroaryl and alkoxy is optionally substituted with one or more R²⁰;
R¹⁶ and R^{16A} are each independently -C₁-C₆ alkyl, -C₁-C₆ alkylene-R^{20A}, -C₂-C₆ alkenyl, -C₂-C₆ alkynyl, -C₃-C₆ cycloalkyl, -C₅-C₈ cycloalkenyl, 3-10 membered heterocycloalkyl, - C₆-C₁₀ aryl, 5-10 membered heteroaryl, -C₁-C₆ alkoxy, -C₁-C₆ haloalkyl, halogen, cyano, oxo, -OR¹⁷, -S(O)₂(C₁-C₆ alkyl), or -S(O)₂NR¹⁸R¹⁹, wherein each alkyl, alkylene, alkenyl, alkynyl, cycloalkyl, cycloalkenyl, heterocycloalkyl, aryl, heteroaryl and alkoxy is optionally substituted with one or more R²⁰;
R¹⁷, R¹⁸ and R¹⁹ are each independently hydrogen, halogen, -OH, -C₁-C₆ alkyl, -C₁-C₆ alkylene-R^{23A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, -S(O)₂(C₁-C₆ alkyl), or S(O)₂NR²¹R²², wherein each alkyl, alkylene, alkenyl, alkynyl, cycloalkyl, cycloalkenyl, heterocycloalkyl, aryl, heteroaryl and alkoxy is optionally substituted with one or more R²³;
R²⁰ and R^{20A} are each independently halogen, -OH, -C₁-C₆ alkyl, -C₁-C₆ alkylene-R^{23A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, halogen, cyano, oxo, -S(O)₂(C₁-C₆ alkyl), or S(O)₂NR²¹R²², wherein each alkyl, alkylene, alkenyl, alkynyl, cycloalkyl, cycloalkenyl, heterocycloalkyl, aryl, heteroaryl and alkoxy is optionally substituted with one or more R²³;
R²¹ and R²² are each independently hydrogen or -C₁-C₆ alkyl; and
R²³ and R^{23A} are each independently -C₁-C₆ alkyl, -C₁-C₆ alkoxy, -C₁-C₆ haloalkyl, halogen, or cyano;
R⁵¹ and R⁵² are each independently hydrogen, -C₁-C₆ alkyl, -C₁-C₆ alkylene-R^{53A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, or 5-10 membered heteroaryl, wherein each alkyl, alkylene, alkenyl, alkynyl, cycloalkyl, cycloalkenyl, heterocycloalkyl, aryl or heteroaryl is optionally substituted with one or more R⁵³; or R⁵¹ and R⁵², together with the nitrogen atom to which they are attached, combine to form a 3-15 membered heterocycloalkyl or 5-15 membered heteroaryl, wherein the heterocycloalkyl or heteroaryl are optionally substituted with one or more R⁵³;
R⁵³ and R^{53A} are each independently -C₁-C₆ alkyl, -C₁-C₆ alkylene-R^{57A}, -C₂-C₆ alkenyl, -C₂-C₆ alkynyl, -C₃-C₆ cycloalkyl, -C₅-C₈ cycloalkenyl, 3-10 membered heterocycloalkyl, - C₆-C₁₀ aryl, 5-10 membered heteroaryl, -C₁-C₆ alkoxy, -C₁-C₆ haloalkyl, halogen, cyano, oxo, -OR⁵⁴, -C(O)R⁵⁴, -CO₂R⁵⁴, or -NR⁵⁵R⁵⁶, wherein each alkyl, alkylene, alkenyl, alkynyl, cycloalkyl, cycloalkenyl, heterocycloalkyl, aryl, heteroaryl and alkoxy is optionally substituted with one or more R⁵⁷;
R⁵⁴, R⁵⁵ and R⁵⁶ are each independently hydrogen, -C₁-C₆ alkyl, -C₁-C₆ alkylene-R^{61A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, -CO₂R⁵⁸, -S(O)₂(C₁-C₆ alkyl), -S(O)₂(C₆-C₁₀ aryl), or -S(O)₂NR⁵⁹R⁶⁰, wherein each alkyl, alkylene, alkenyl, alkynyl, cycloalkyl, cycloalkenyl, heterocycloalkyl, aryl, heteroaryl and alkoxy is optionally substituted with one or more R⁶¹;
R⁵⁷ and R^{57A} are each independently -C₁-C₆ alkyl, -C₁-C₆ alkylene-R^{61A}, -C₂-C₆ alkenyl, -C₂-C₆ alkynyl, -C₃-C₆ cycloalkyl, -C₅-C₈ cycloalkenyl, 3-10 membered heterocycloalkyl, - C₆-C₁₀ aryl, 5-10 membered heteroaryl, -C₁-C₆ alkoxy, -C₁-C₆ haloalkyl, halogen, cyano, oxo, -OR⁵⁸, NR⁵⁹R⁶⁰, -S(O)₂(C₁-C₆ alkyl), or -S(O)₂NR⁵⁹R⁶⁰, wherein each alkyl, alkylene, alkenyl, alkynyl, cycloalkyl, cycloalkenyl, heterocycloalkyl, aryl, heteroaryl and alkoxy is optionally substituted with one or more R⁶¹;
R⁵⁸, R⁵⁹ and R⁶⁰ are each independently hydrogen, -C₁-C₆ alkyl, -C₁-C₆ alkylene-R⁶⁴, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, -S(O)₂(C₁-C₆ alkyl), or S(O)₂NR⁶²R⁶³, wherein each alkyl, alkylene, alkenyl, alkynyl, cycloalkyl, cycloalkenyl, heterocycloalkyl, aryl, heteroaryl and alkoxy is optionally substituted with one or more R⁶⁴;
R⁶¹ and R^{61A} are each independently halogen, -OH, -C₁-C₆ alkyl, -C₁-C₆ alkylene-R^{64A}, -C₂-C₆ alkenyl, -C₂-C₆ alkynyl, -C₃-C₆ cycloalkyl, -C₅-C₈ cycloalkenyl, 3-10 membered heterocycloalkyl, -C₆-C₁₀ aryl, 5-10 membered heteroaryl, -C₁-C₆ alkoxy, -C₁-C₆ haloalkyl, halogen, cyano, oxo, -S(O)₂(C₁-C₆ alkyl), or S(O)₂NR⁶²R⁶³, wherein each alkyl, alkylene, alkenyl, alkynyl, cycloalkyl, cycloalkenyl, heterocycloalkyl, aryl, heteroaryl and alkoxy is optionally substituted with one or more R⁶⁴; and
R⁶² and R⁶³ are each independently hydrogen and -C₁-C₆ alkyl; and
R⁶⁴ and R^{64A} are each independently -C₁-C₆ alkyl, -C₁-C₆ alkoxy, -C₁-C₆ haloalkyl, phenyl, halogen, or cyano.

2. The compound of claim 1, wherein the compound is of the Formula I-A: 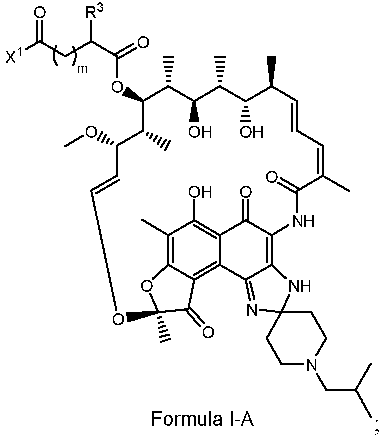 wherein
m is an integer between 0 and 4; and
R³ is selected from -H, methyl and ethyl, and wherein preferably the compound is of the Formula I-B, Formula I-C, Formula I-D, Formula I-E, Formula I-F, or Formula I-G:

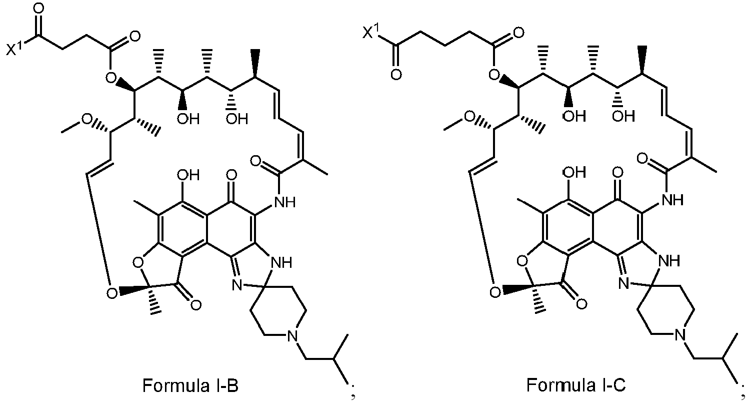

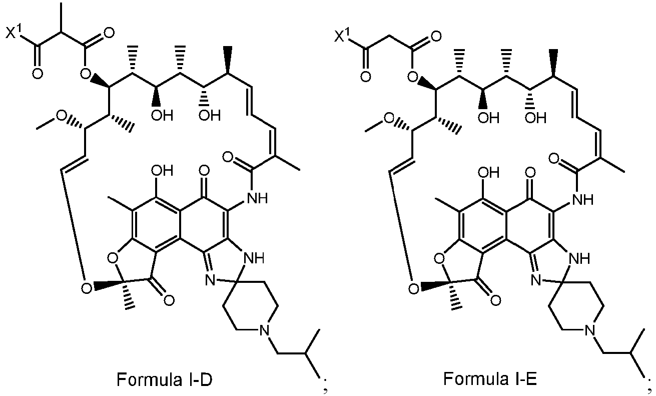

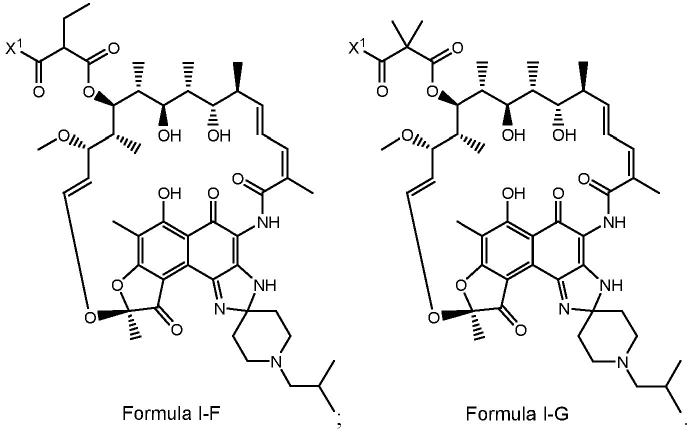

3. The compound according to claim 1 or claim 2, wherein X¹ is -OR¹¹.

4. The compound according to claim 1 or claim 2, wherein X¹ is -NR⁵¹NR⁵².

5. The compound according to claim 4, wherein R⁵¹ and R⁵² are each independently hydrogen, -C₁-C₆ alkyl, -C₁-C₆ alkylene-R^{53A}, -C₅-C₆ cycloalkyl, 5-6 membered heterocycloalkyl or phenyl, wherein preferably at least one of R⁵¹ and R⁵² is hydrogen, wherein each alkyl, alkylene, cycloalkyl, heterocycloalkyl or phenyl is optionally substituted with one or more, preferably one or two, R⁵³; or R⁵¹ and R⁵², together with the nitrogen atom to which they are attached, combine to form a 3-15 membered heterocycloalkyl, wherein said heterocycloalkyl is optionally substituted with one or more, preferably one or two, R⁵³.

6. The compound according to claim 4, wherein R⁵¹ and R⁵², together with the nitrogen atom to which they are attached, combine to form a 3-15 membered heterocycloalkyl, wherein said heterocycloalkyl is optionally substituted with one or more, preferably one or two, R⁵³; or wherein said heterocycle is fused to one or more additional aryl or heteroaryl rings to form a 10- to 15-membered heteroaryl, wherein said 10- to 15-membered heteroaryl is optionally substituted with one or more, preferably one or two, R⁵³; or wherein said heterocycle is connected at a single atom to another cycloalkyl or heterocycloalkyl to form a 10- to 15-membered spirocyclic heterocycle; and wherein said spirocyclic is optionally substituted with one or more, preferably one or two, R⁵³.

7. The compound according to claim 6, wherein said 3-15 membered heterocycloalkyl is a piperidine, morpholine, 1,4-diazepane, piperazine, or an azetidine.

8. The compound according to claim 6, wherein said heterocycloalkyl is a piperidine.

9. The compound according to any one of the claims 4 to 8, wherein:
R⁵³ and R^{53A} are independently -C₁-C₄ alkyl, -C₁-C₃ alkylene-R^{57A}, 5- to 15-membered heterocycloalkyl, 5- to 15-membered heteroaryl, or phenyl, -OR⁵⁴, -C(O)R⁵⁴, -CO₂R⁵⁴, oxo, or -NR⁵⁵R⁵⁶, wherein each alkylene, heterocycloalkyl, heteroaryl and phenyl is optionally substituted with one or more R⁵⁷;
wherein R⁵⁴ is independently hydrogen, -C₁-C₄ alkyl, -C₅-C₆ cycloalkyl, or phenyl; wherein each alkyl, cycloalkyl or phenyl is optionally substituted with one or more R⁶¹;
R⁵⁵ and R⁵⁶ are each independently hydrogen, -C₁-C₄ alkyl, -C₁-C₄ alkylene-R^{61A}, phenyl, -CO₂C₁-C₆ alkyl, -S(O)₂(phenyl), wherein phenyl or cycloalkyl is optionally substituted with one or more R⁶¹.

10. The compound according to any one of the claims 4 to 9, wherein R⁵⁷ and R^{57A} are independently -C₁-C₆ alkyl, 3-10 membered heterocycloalkyl, phenyl, 5-10 membered heteroaryl, oxo, halogen, -OH, or -SO₂NH₂, wherein each alkyl, heterocycloalkyl, phenyl, and heteroaryl is optionally substituted with one or more R⁶¹.

11. The compound according to any one of the claims 4 to 10, wherein R⁶¹ and R^{61A} are independently -C₁-C₂ alkyl, phenyl, -C₁-C₂ alkoxy, -C₁-C₂ haloalkyl, or halogen.

12. The compound according to claim 1, wherein X¹ is selected from the group consisting of:

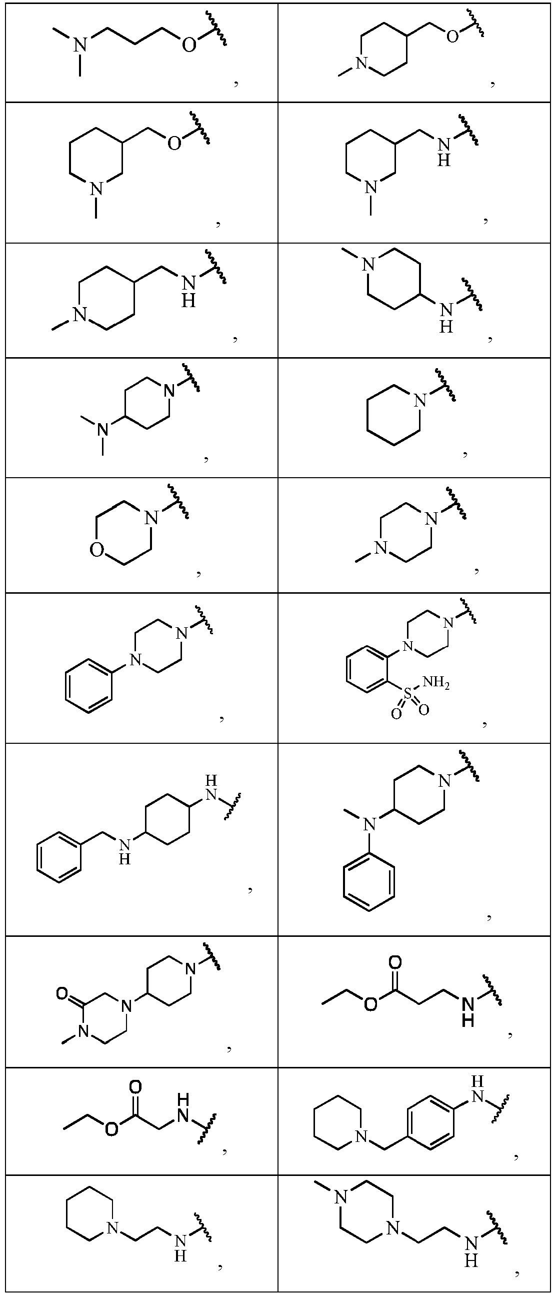

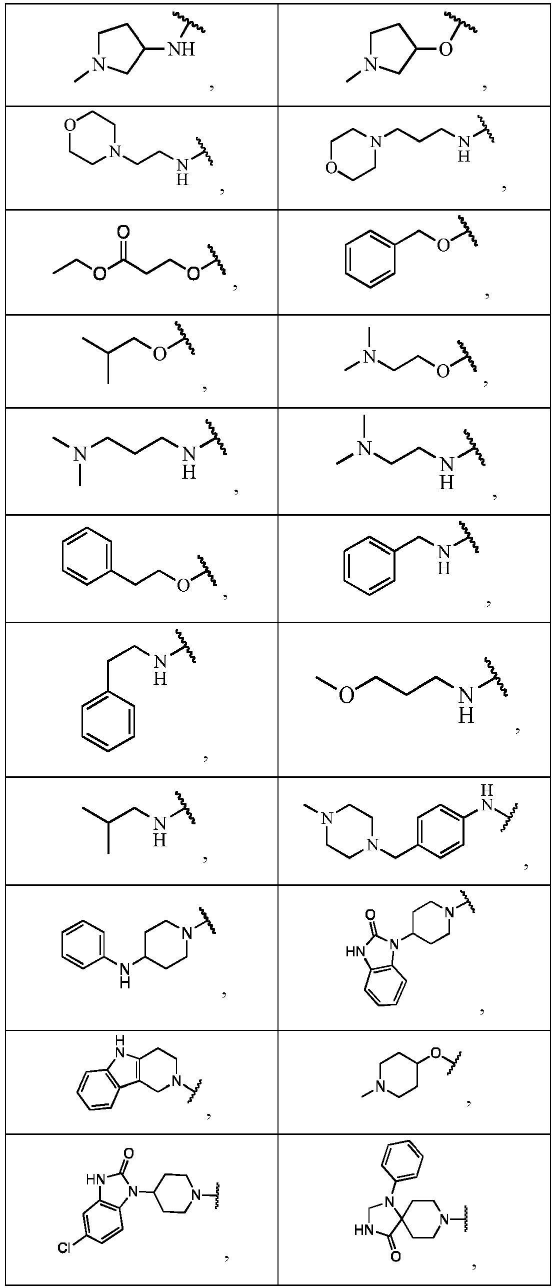

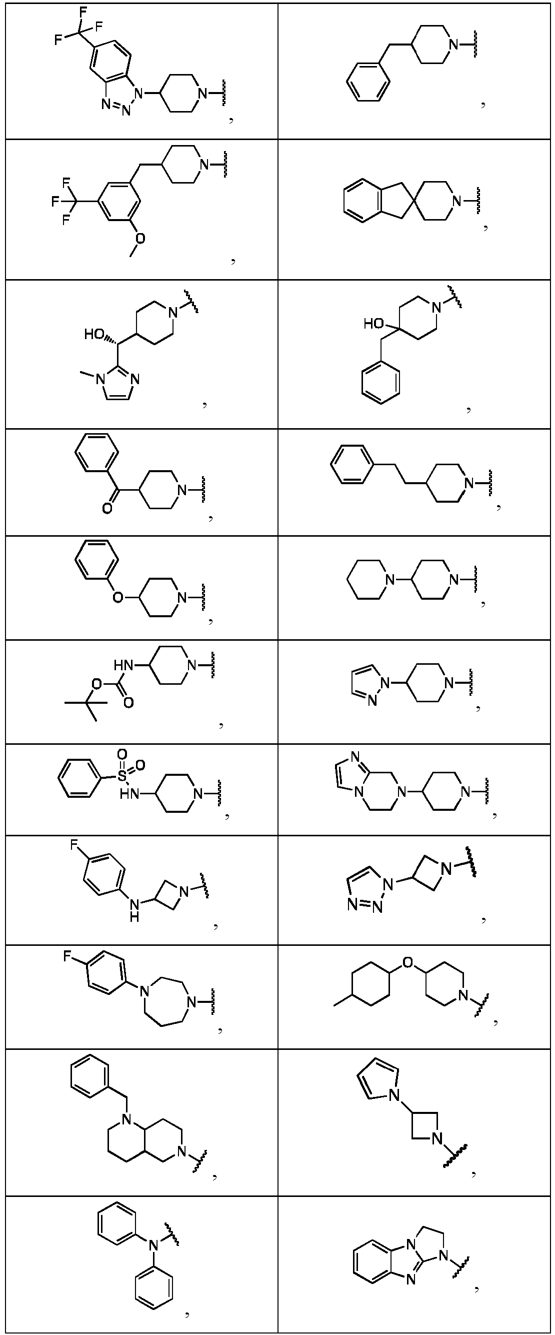

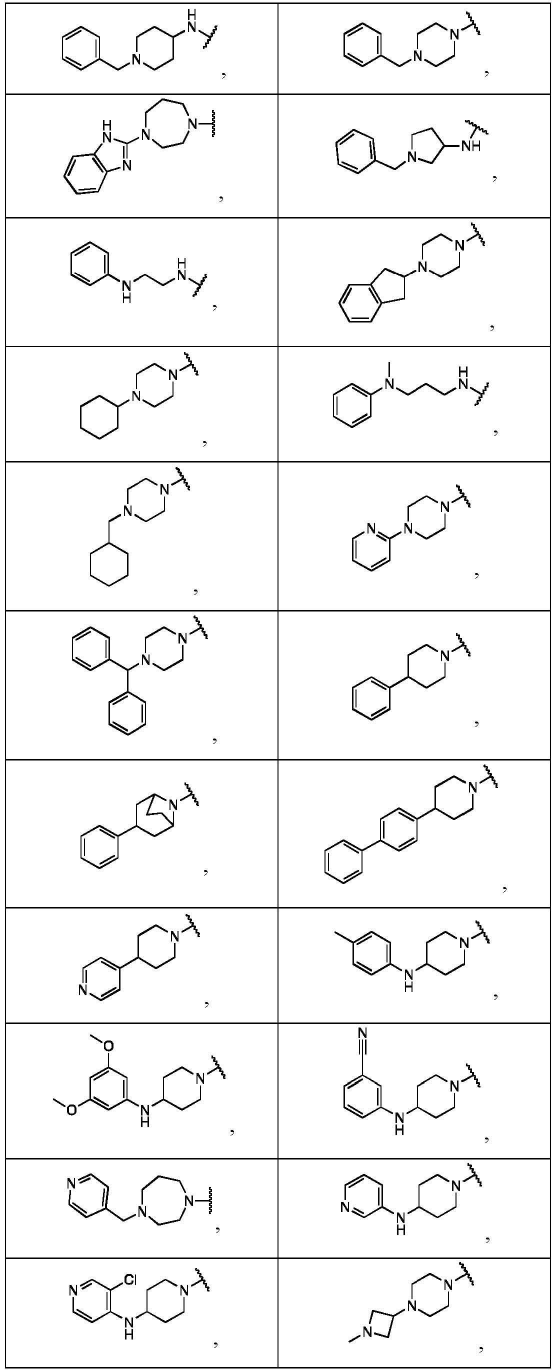

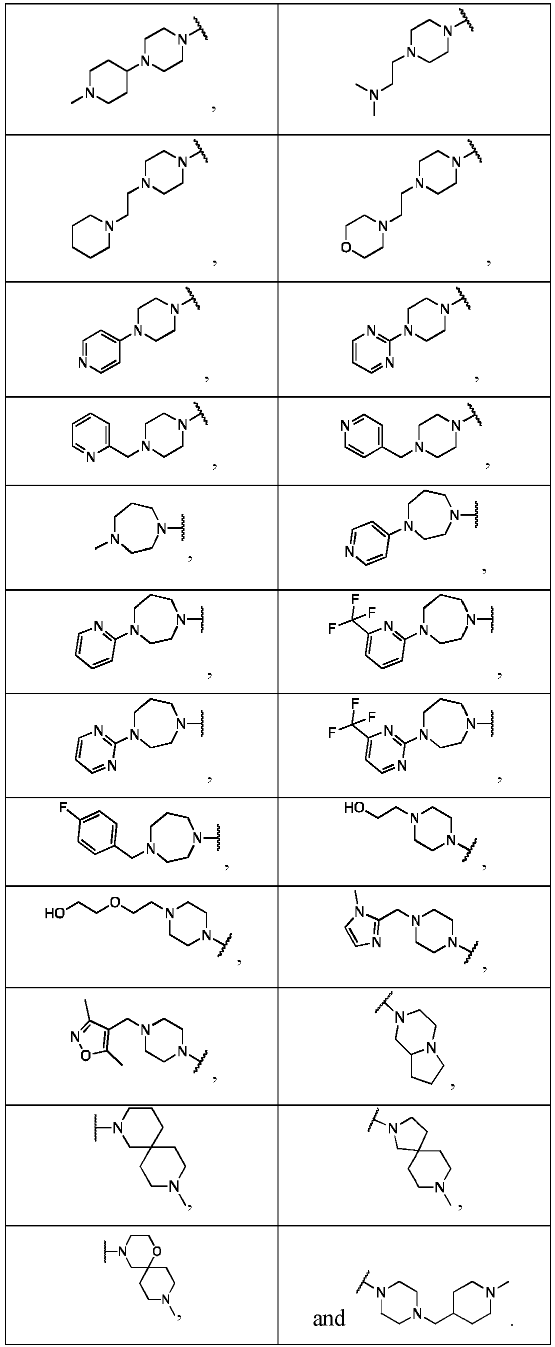

13. The compound according to claim 1, wherein the compound is selected from the group consisting of:

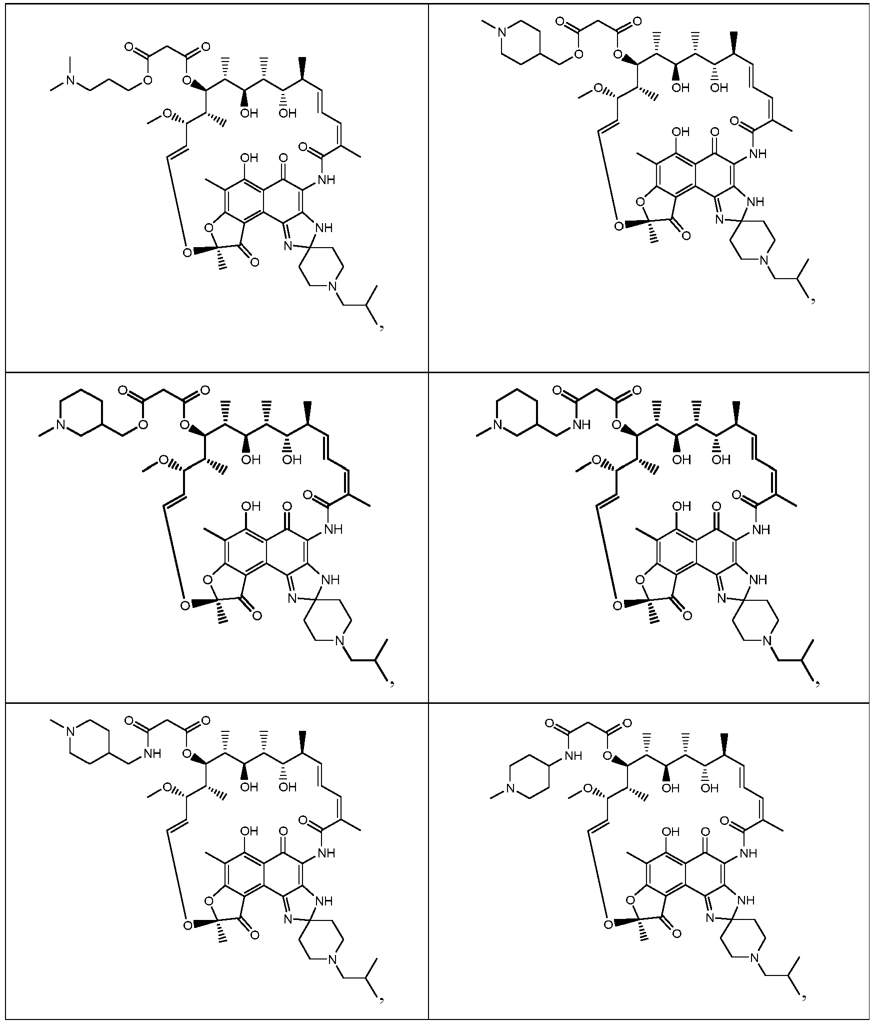

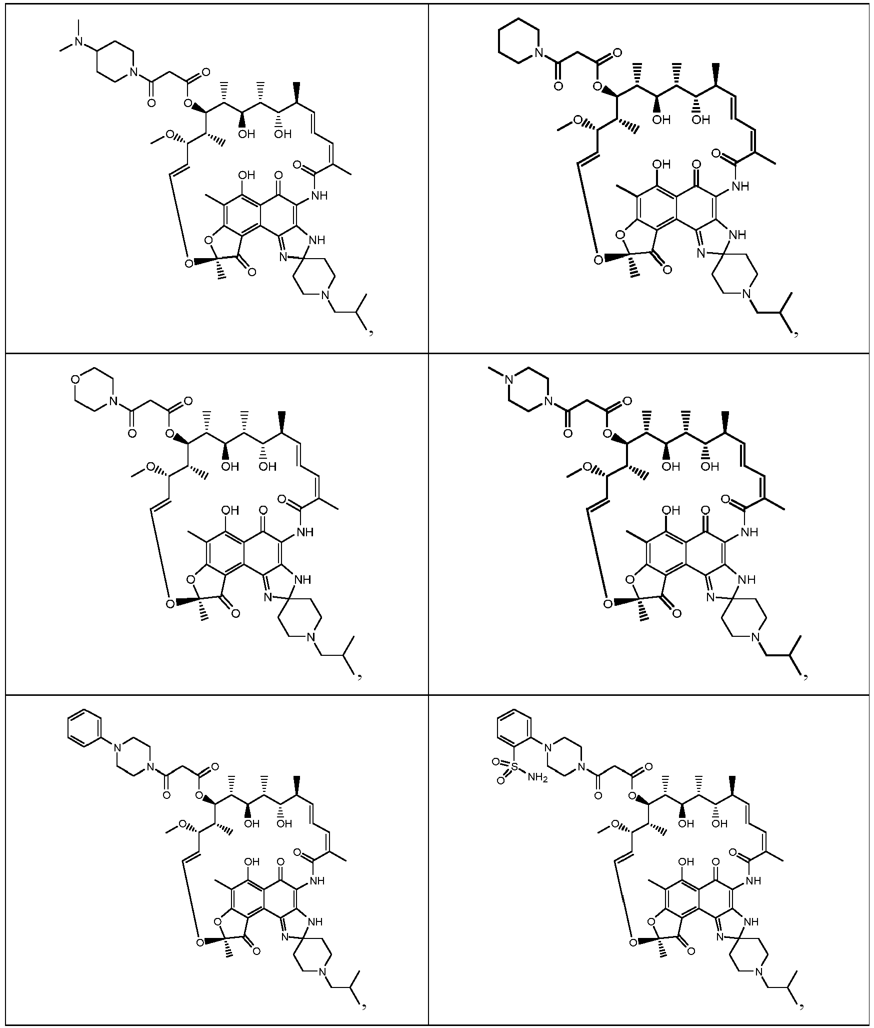

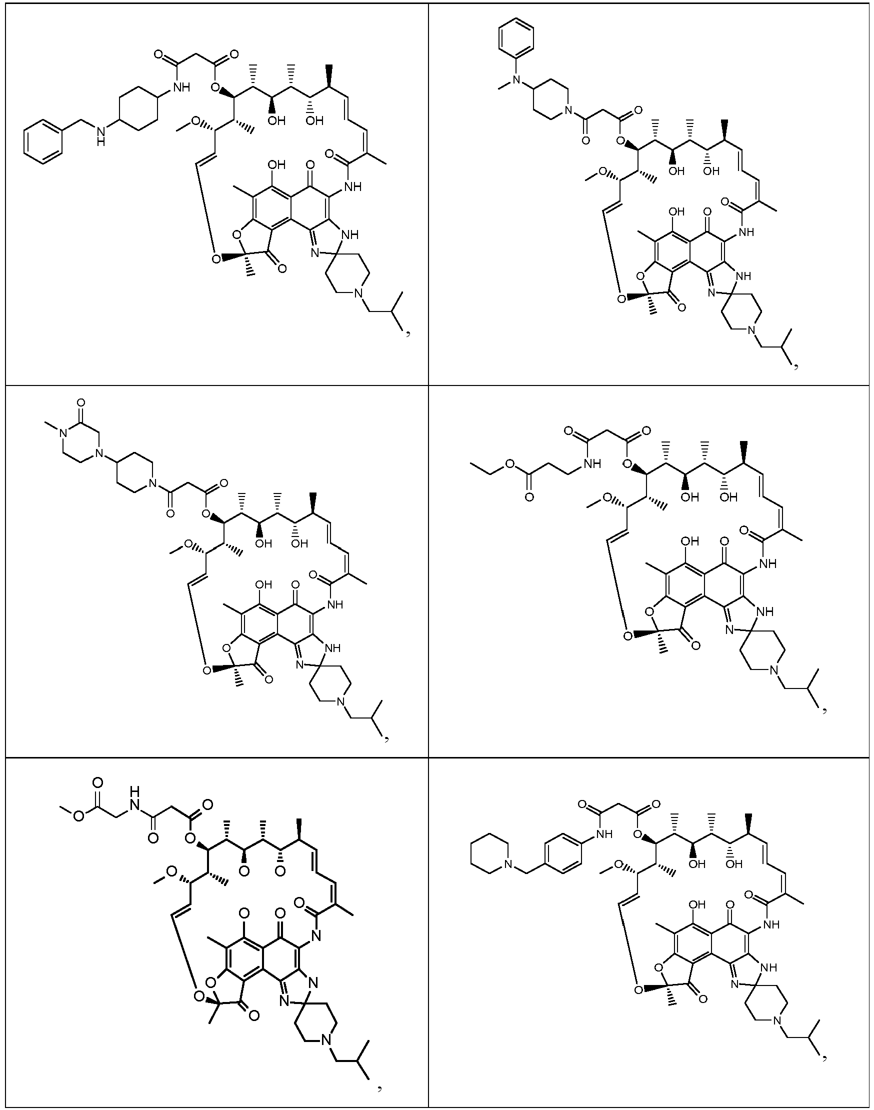

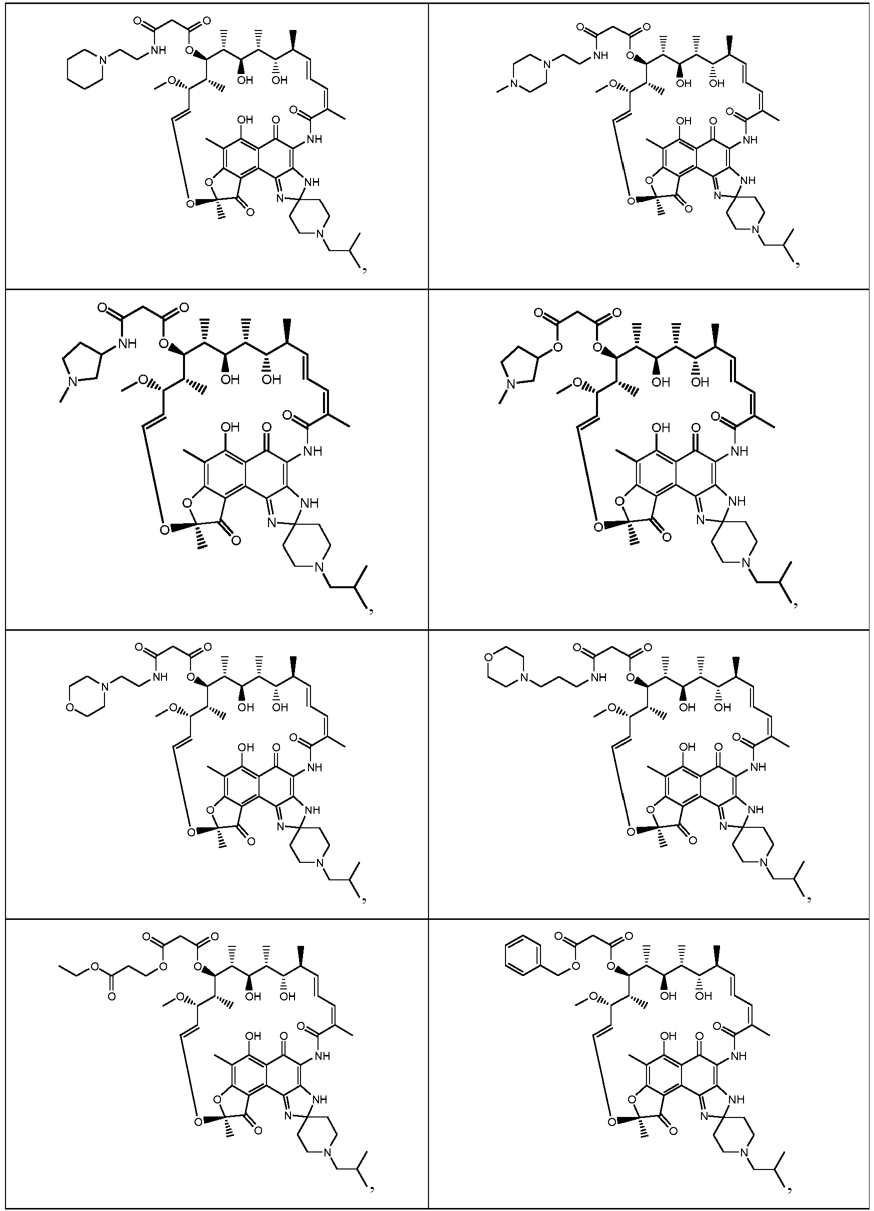

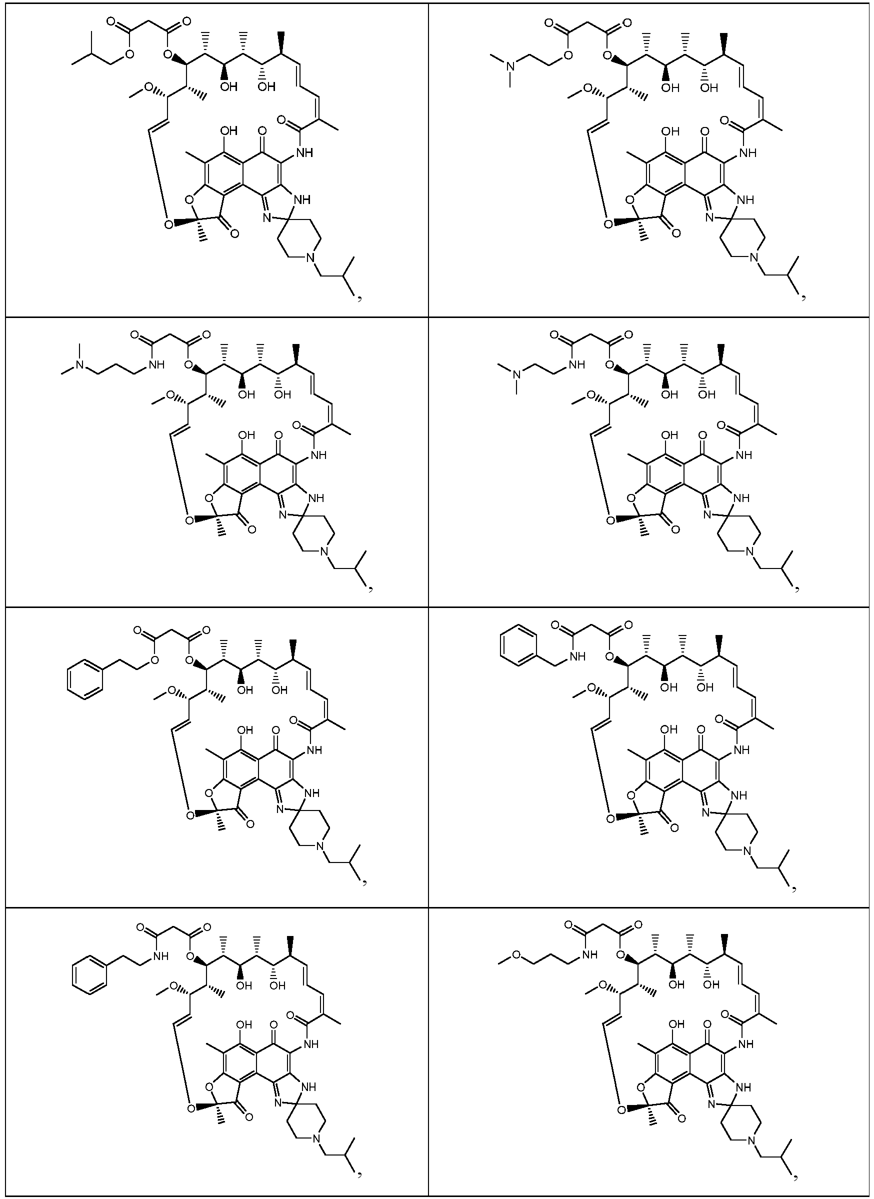

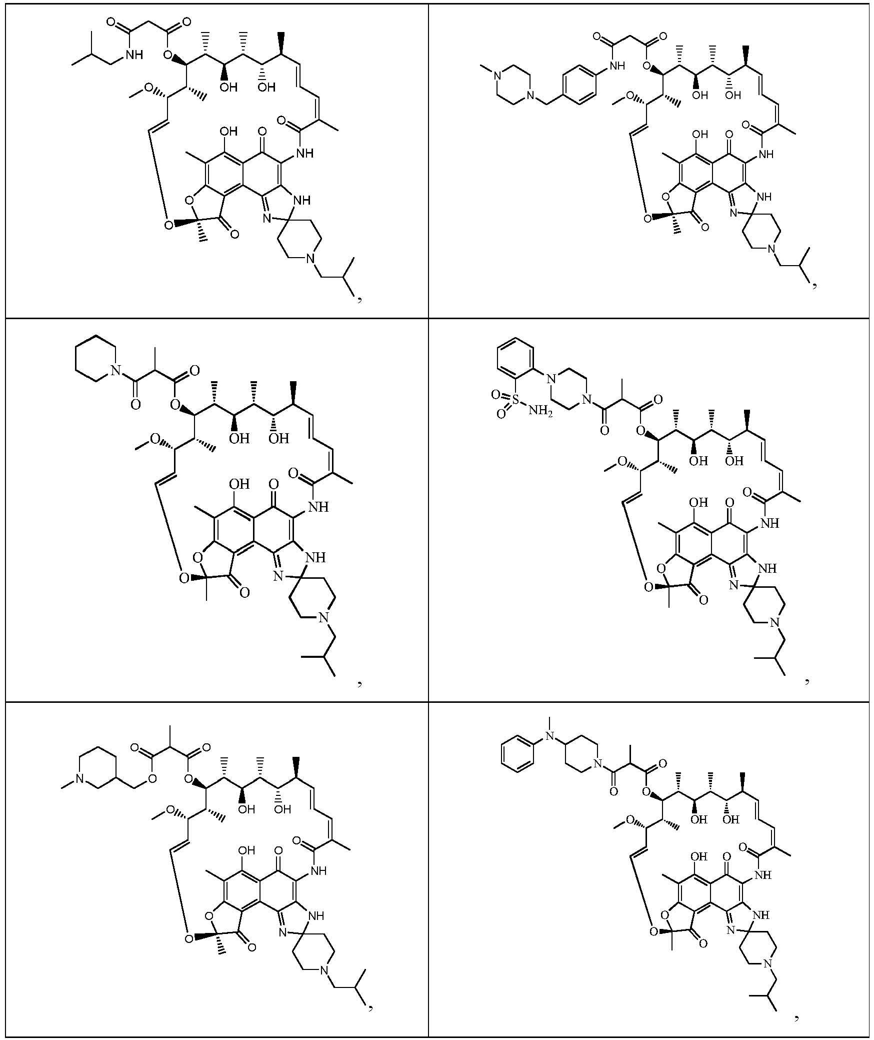

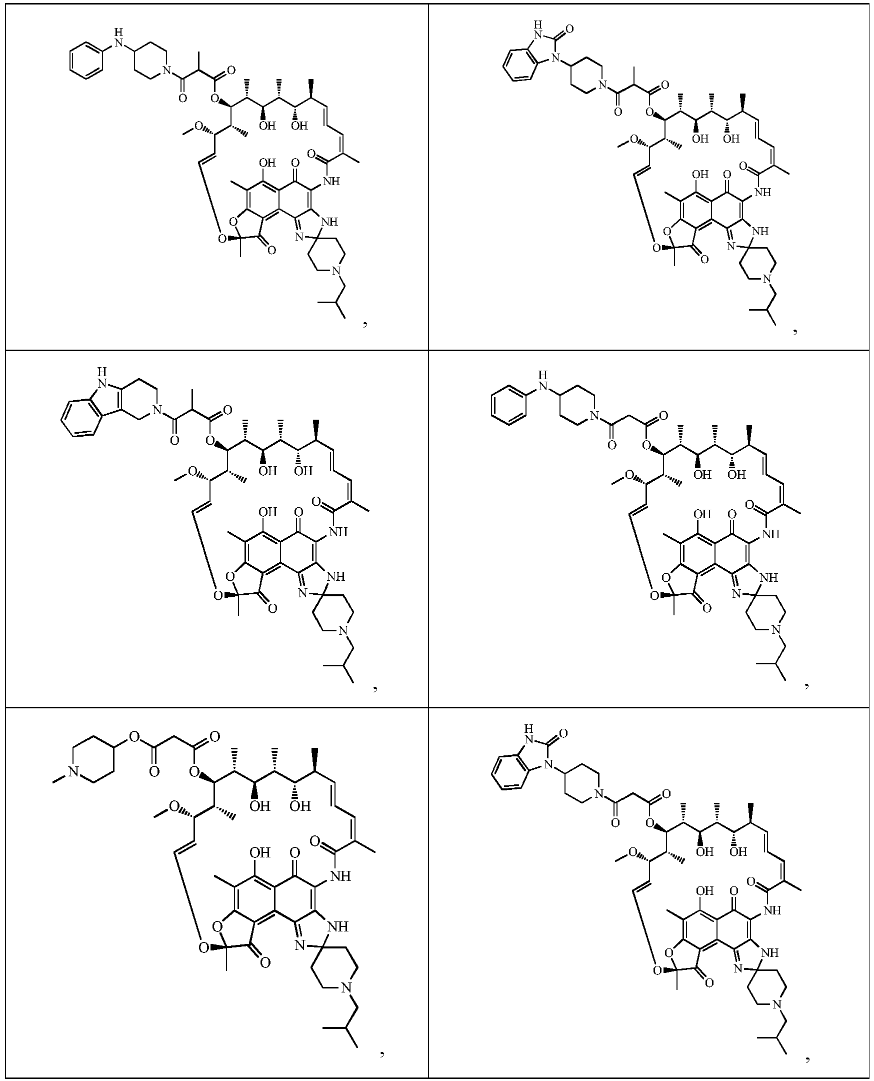

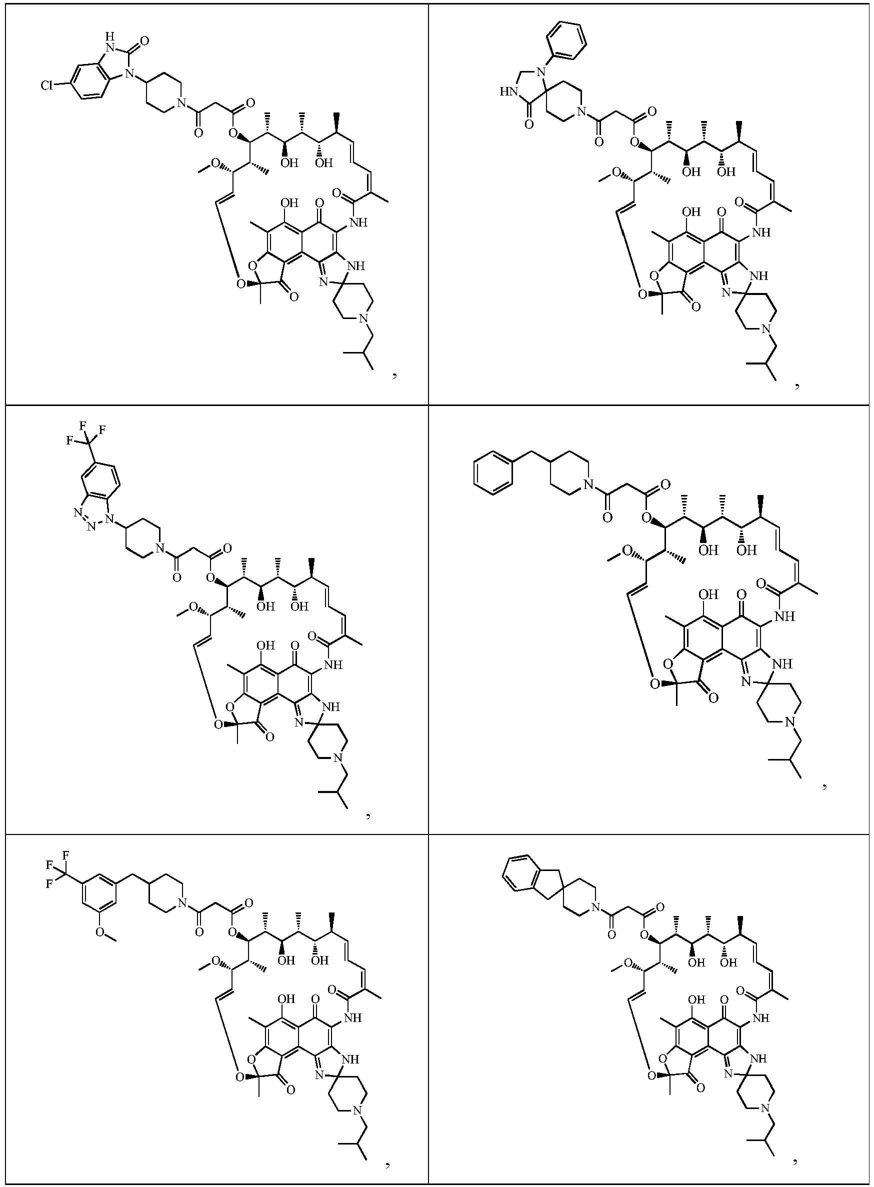

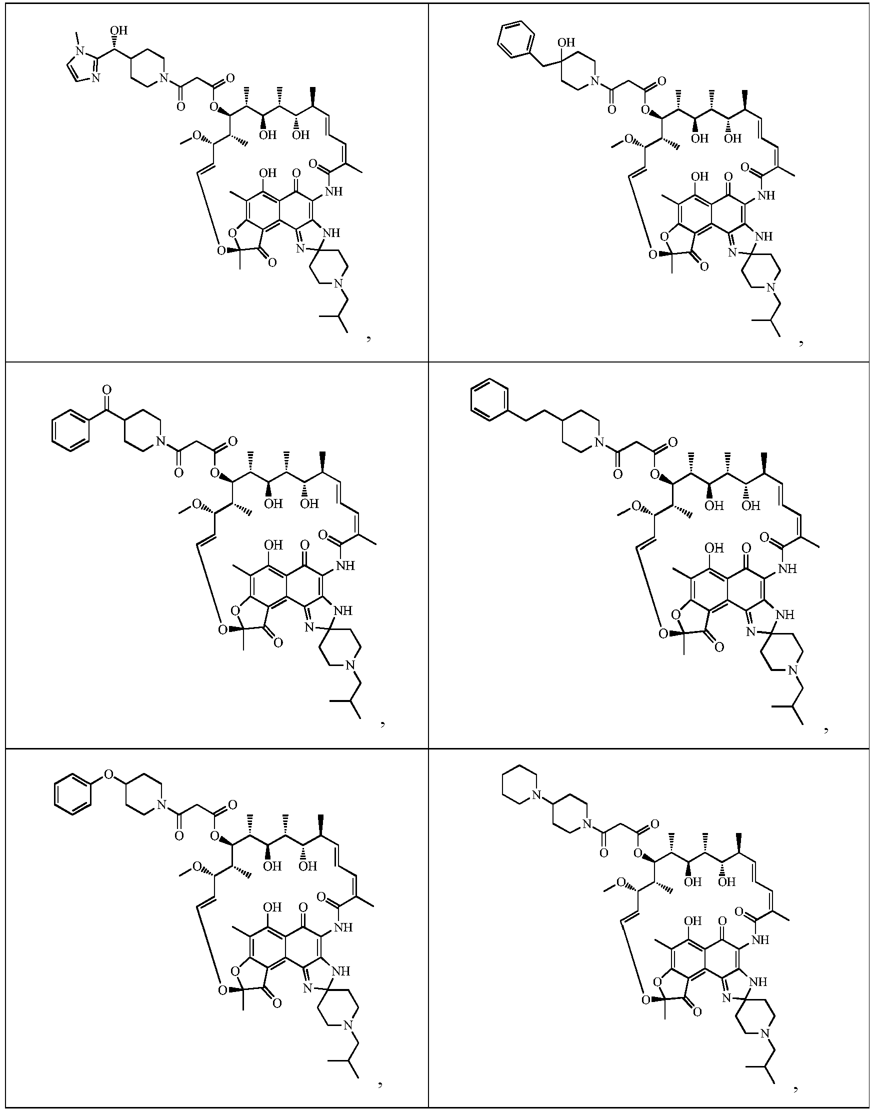

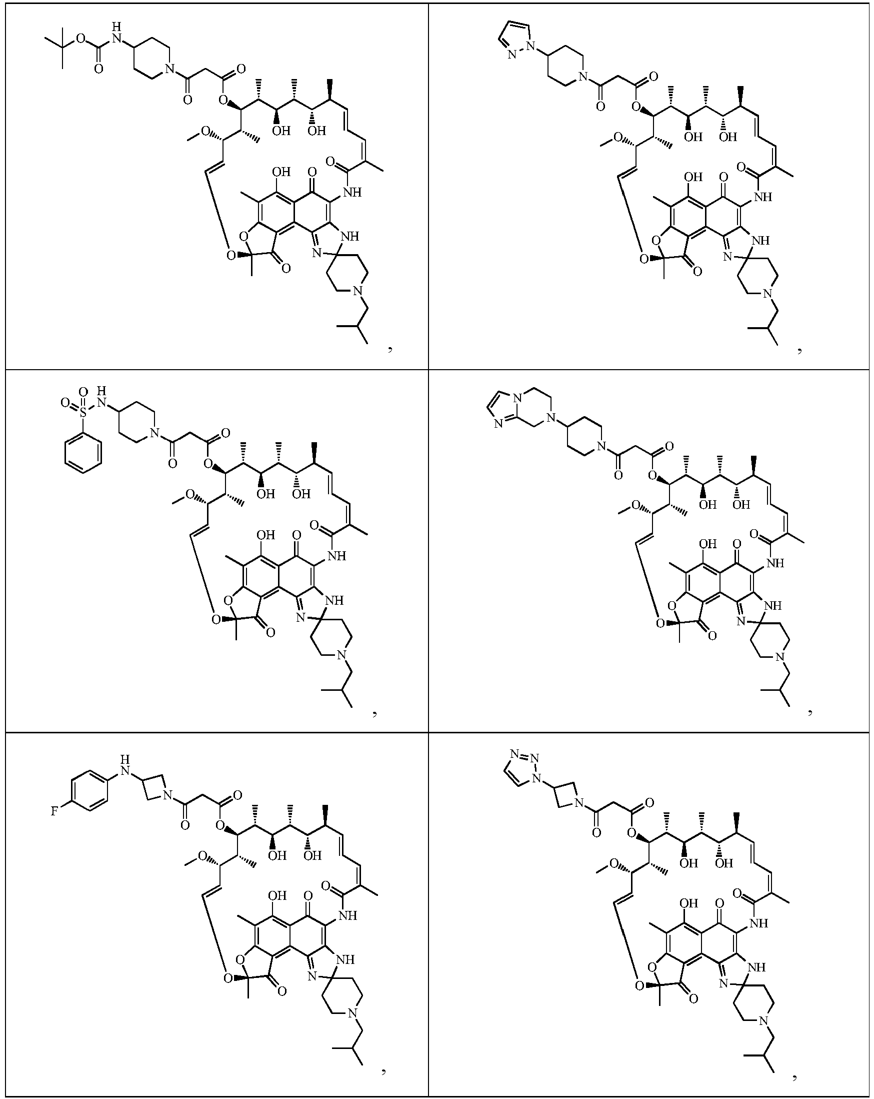

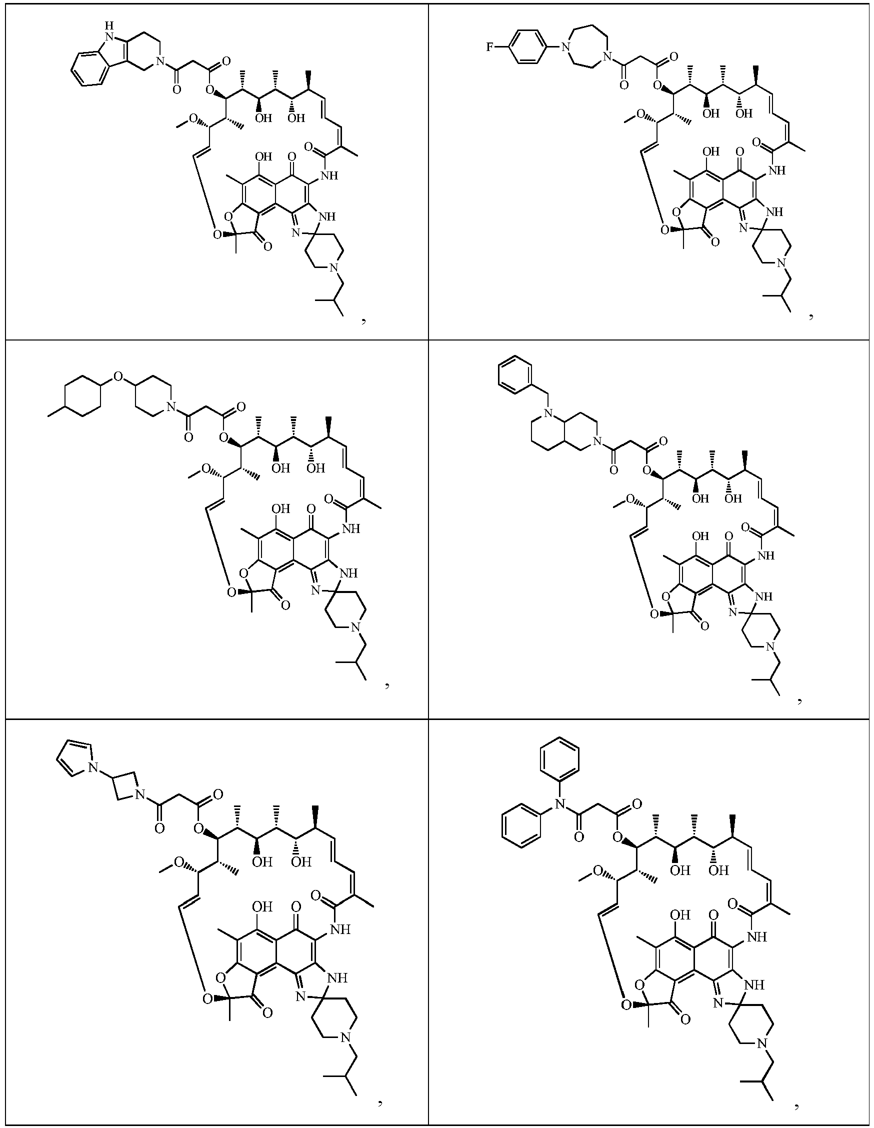

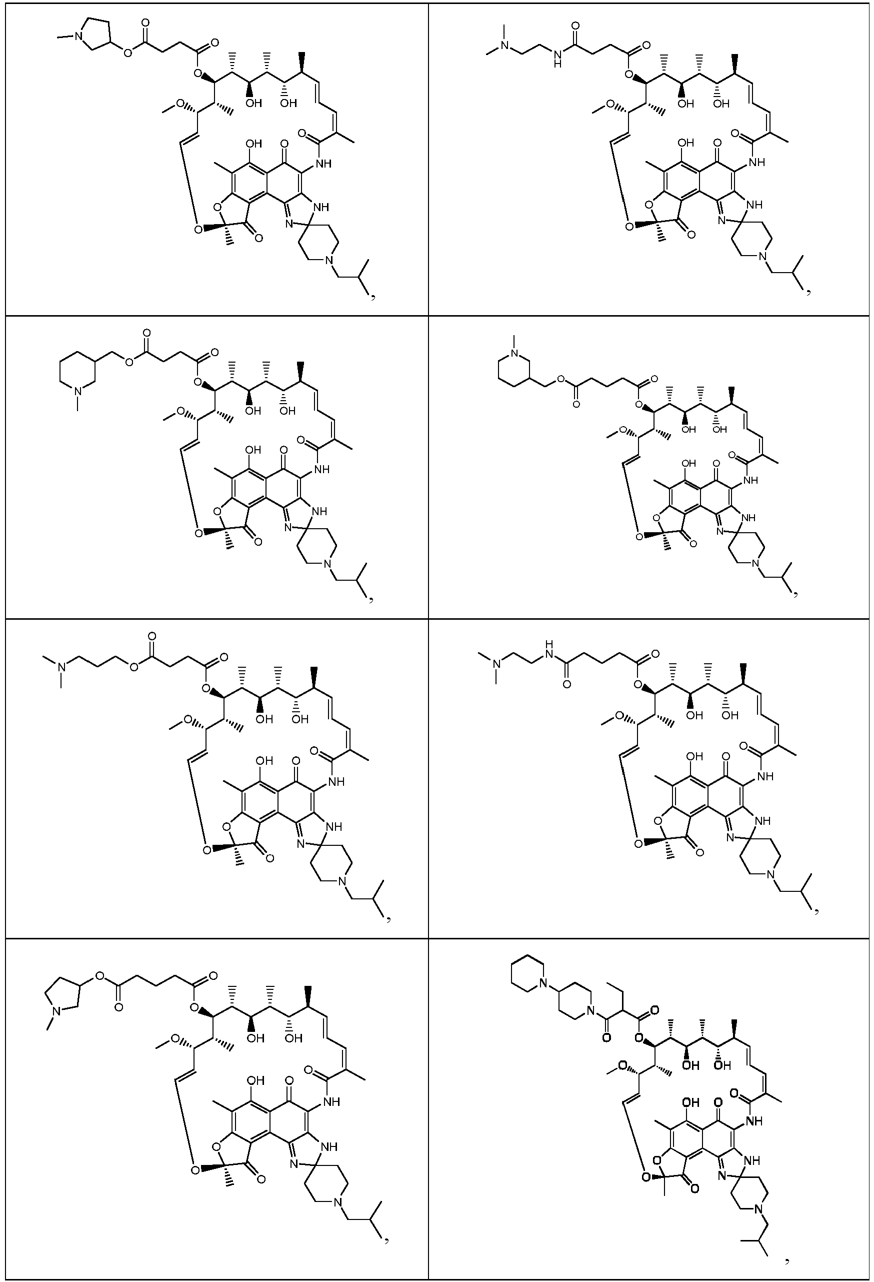

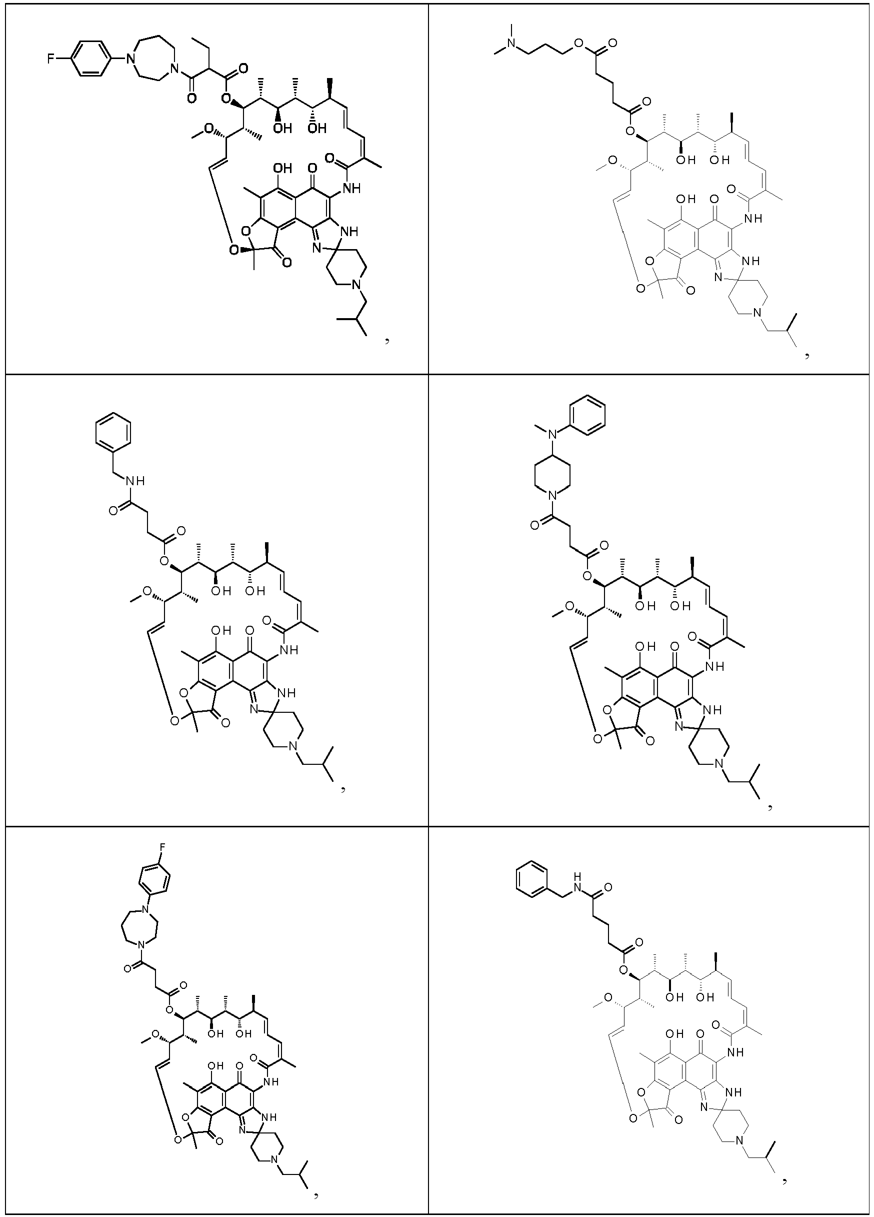

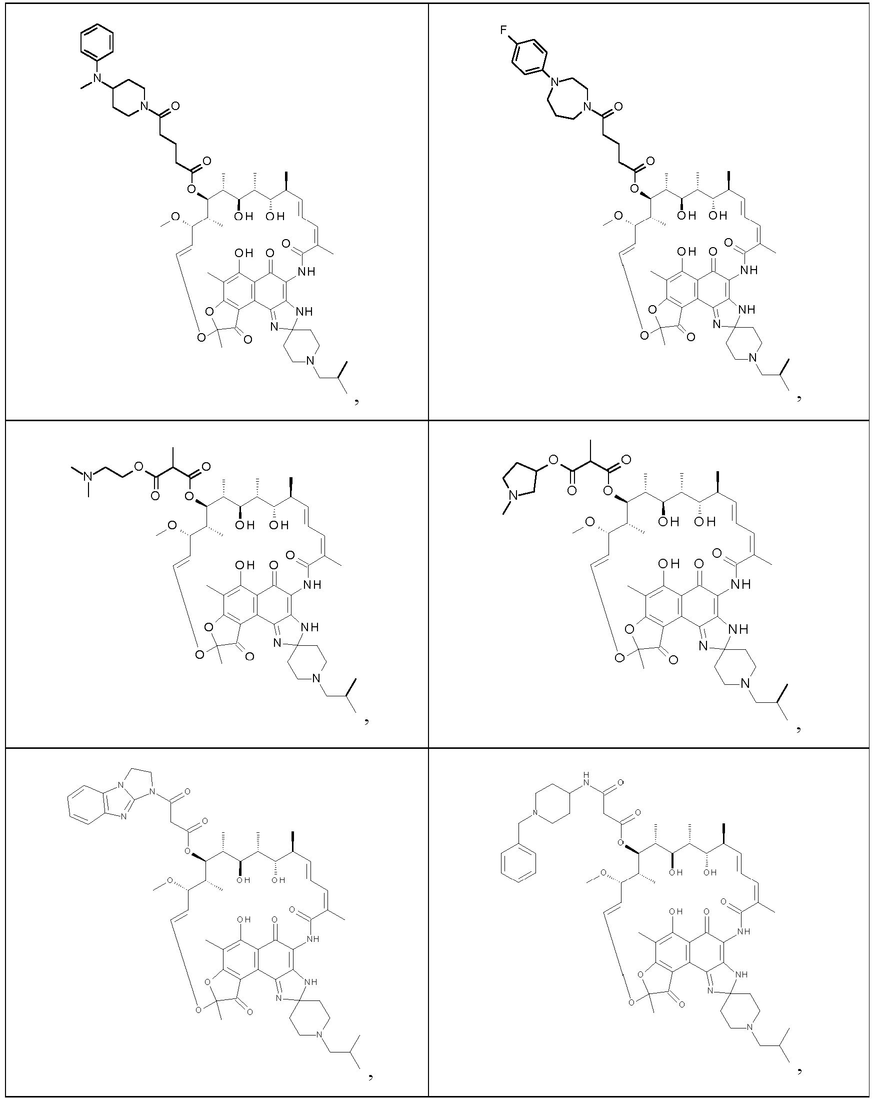

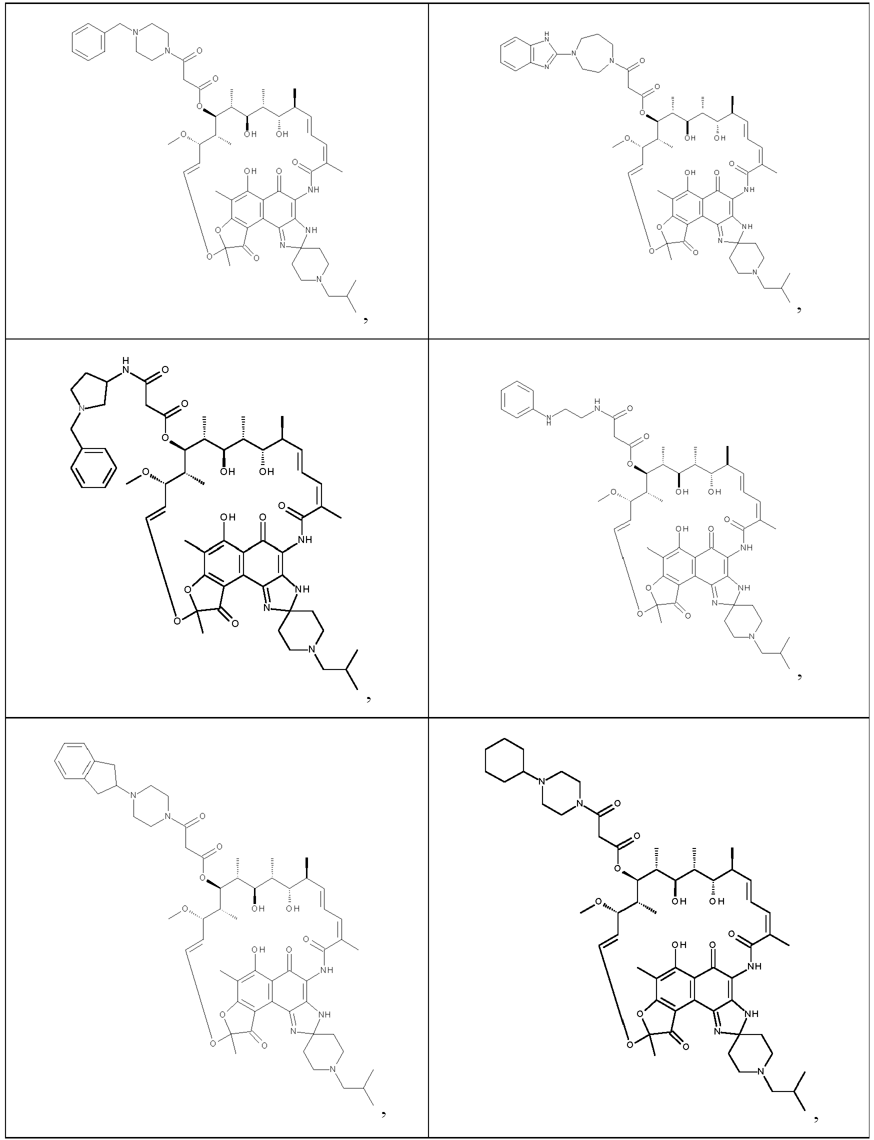

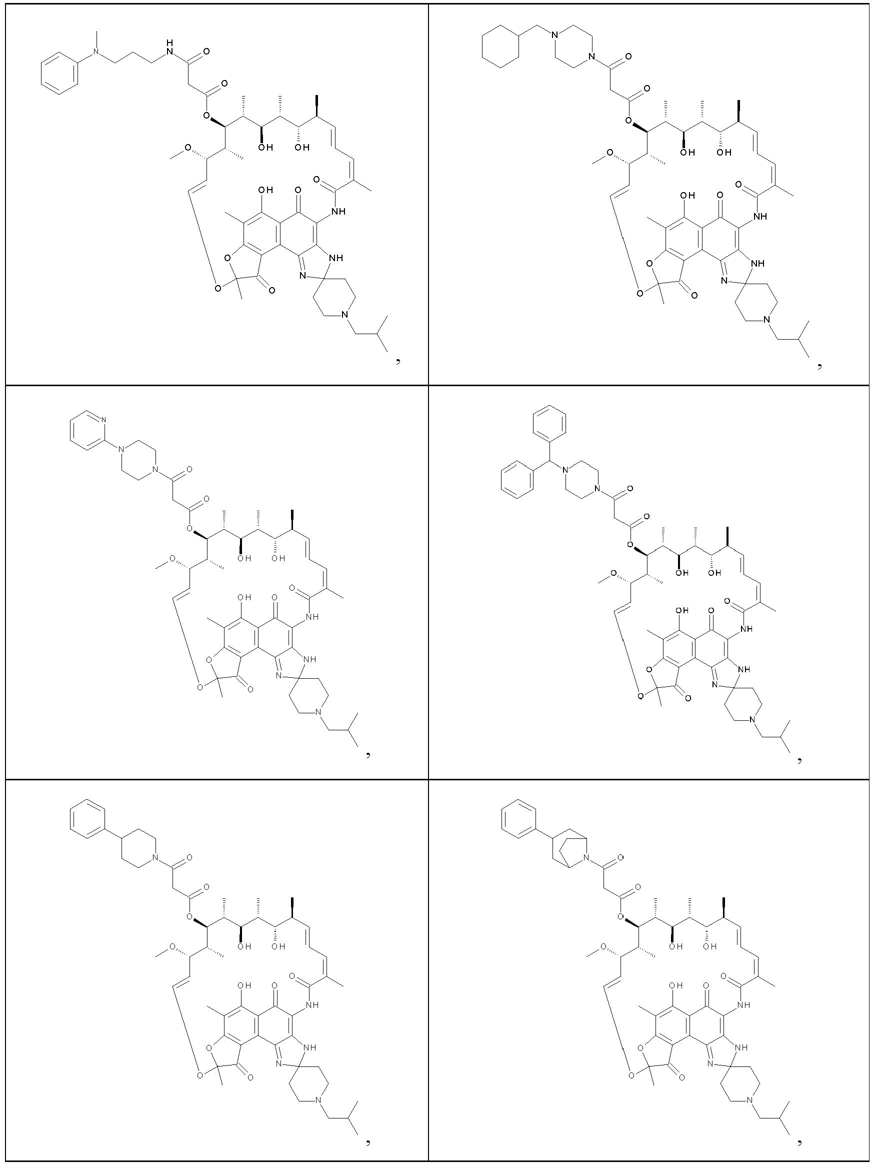

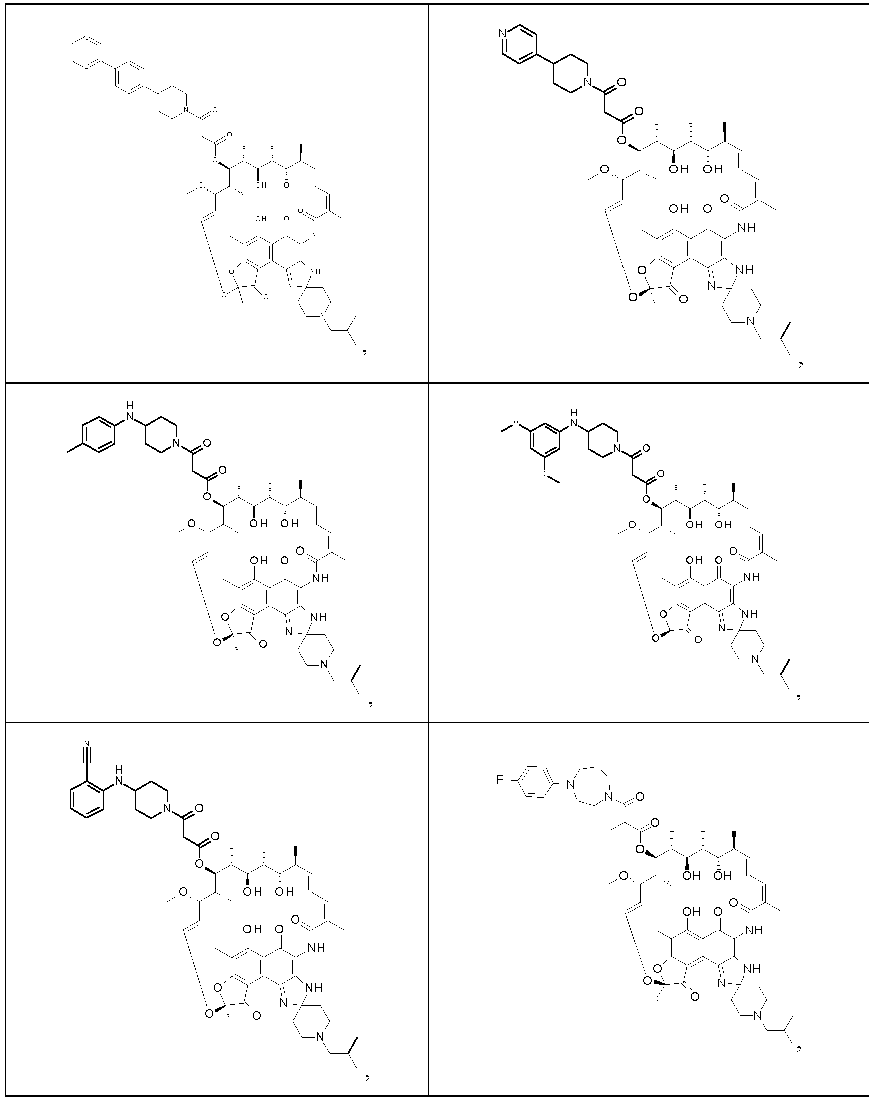

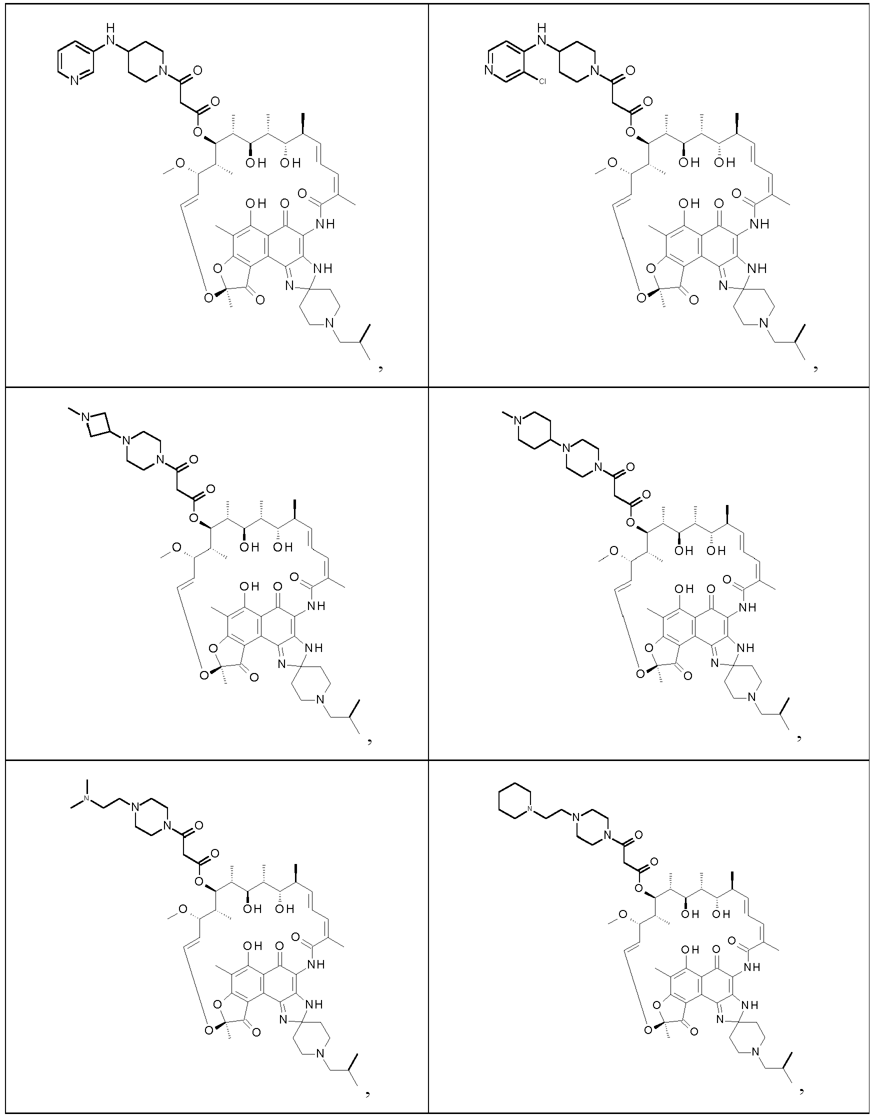

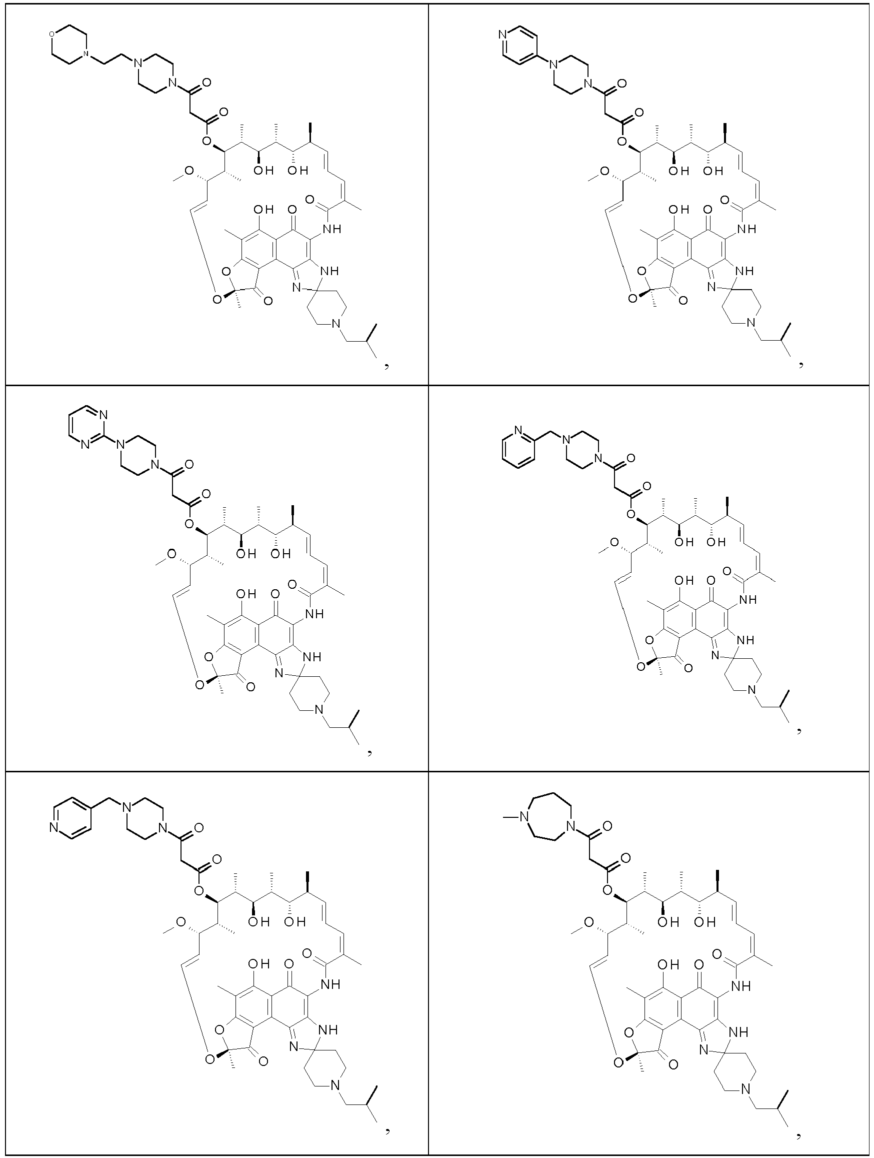

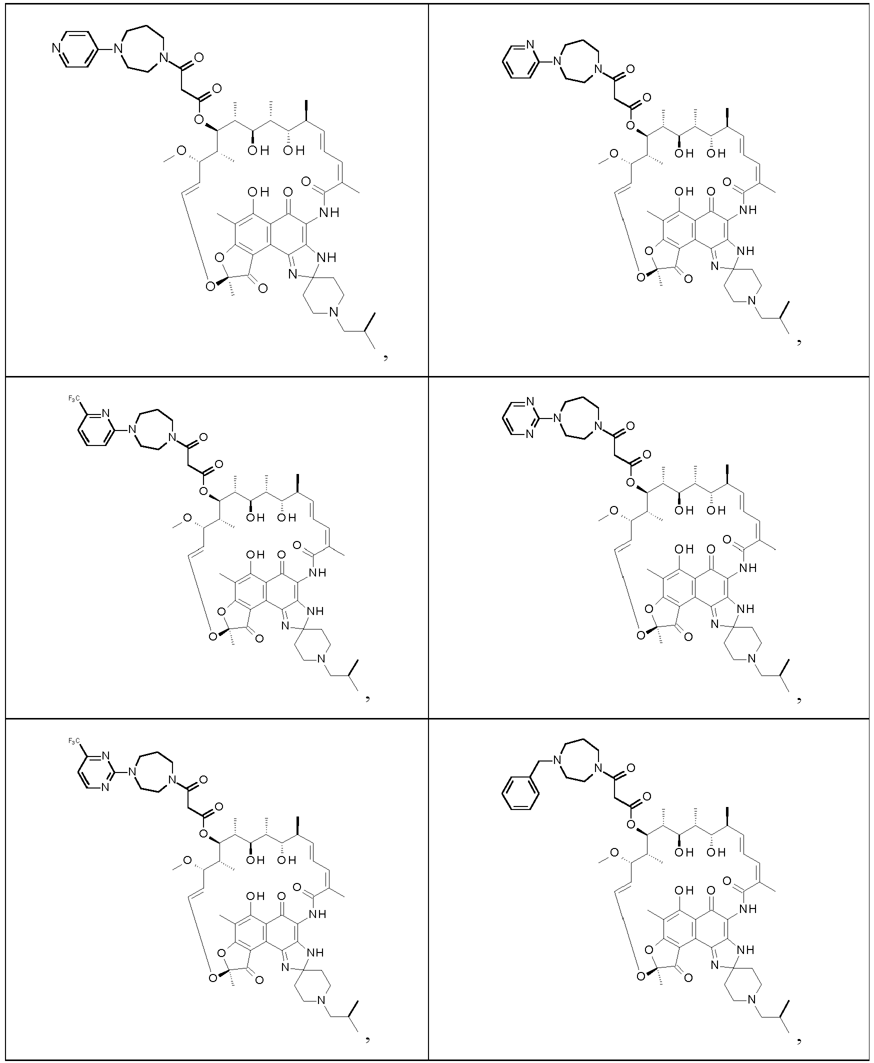

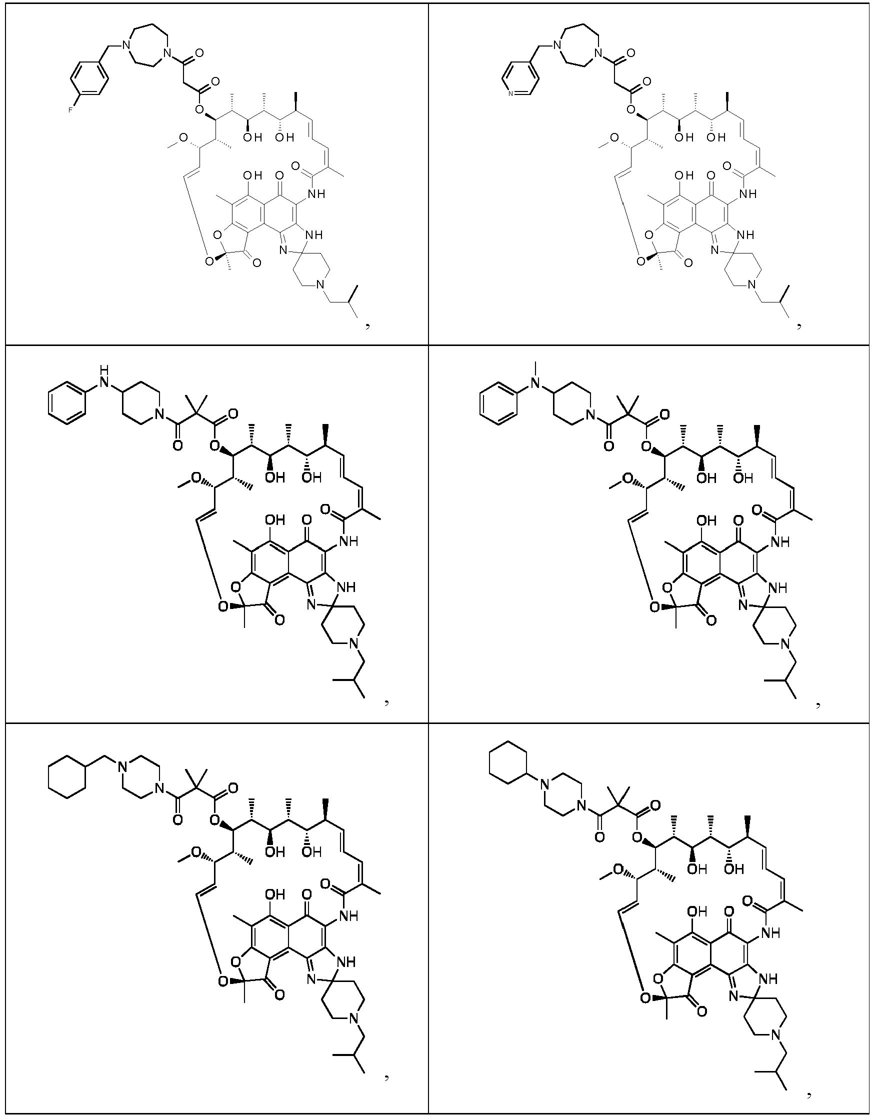

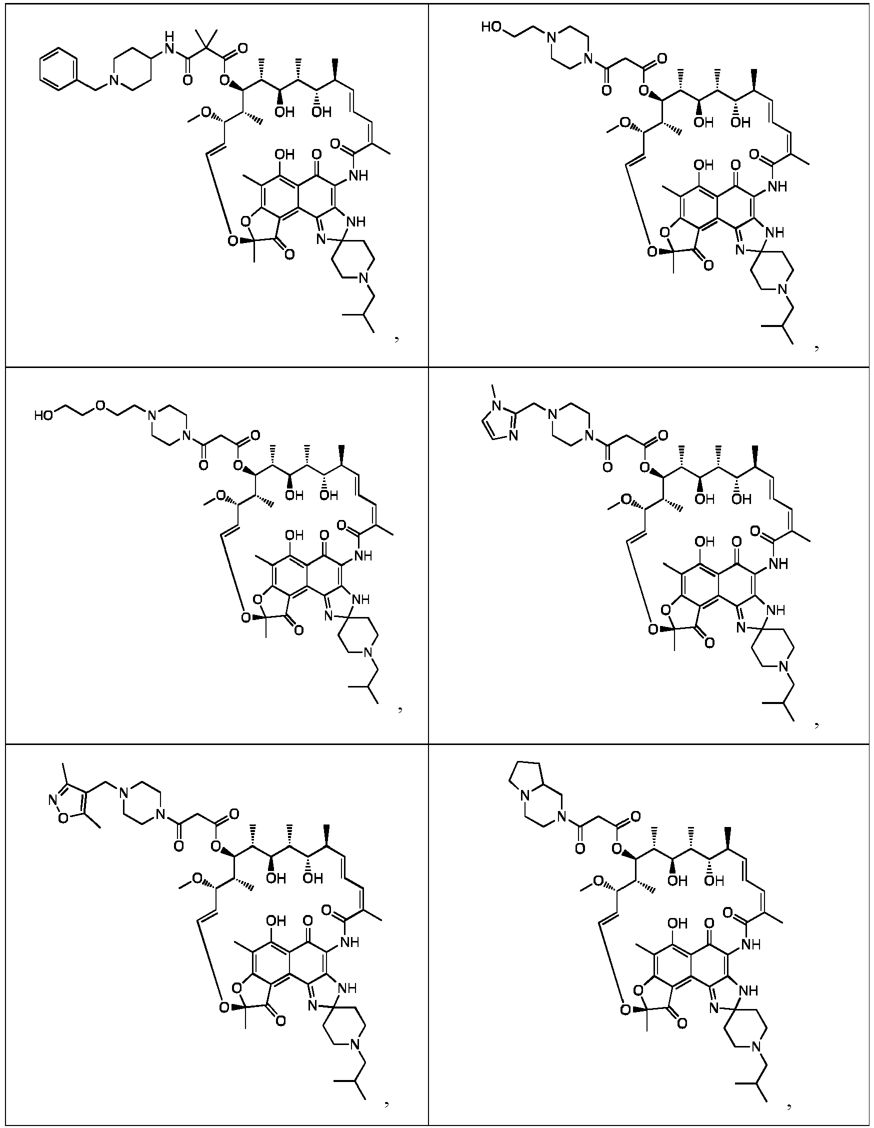

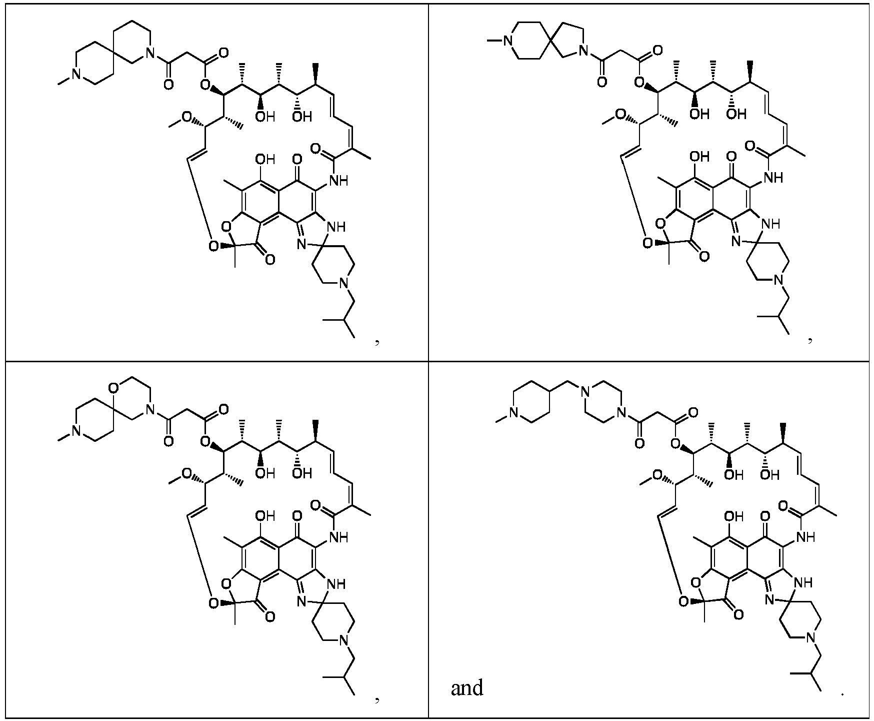

14. A pharmaceutical composition comprising at least one compound according to any of the preceding claims, or a pharmaceutically acceptable salt, tautomer, solvate or hydrate thereof, and a pharmaceutically acceptable excipient.

15. A compound according to any of the claims 1 to 13 or a pharmaceutically acceptable salt, tautomer, solvate or hydrate thereof, or a pharmaceutical composition according to claim 14, for use as a medicament, preferably for use in a method of treating a bacterial infection.

## Patentansprüche

1. Verbindung der Formel I oder ein pharmazeutisch verträgliches Salz, Tautomer, Solvat, Hydrat, oder Enantiomer davon: 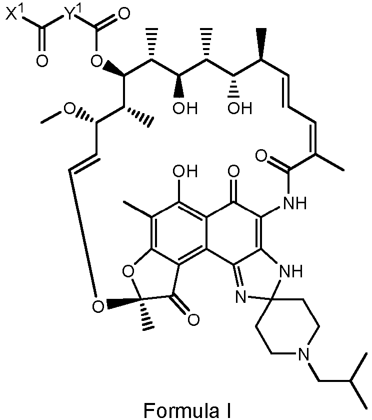 wobei:
Y¹ (CR¹R²)ₙ ist, wobei n eine ganze Zahl von 1 bis 6 ist und R¹ und R² unabhängig, bei jedem Vorkommen, aus der Gruppe ausgewählt sind, bestehend aus Wasserstoff, -C₁-C₆-Alkyl und -C₁-C₆-Alkoxy, wobei jedes Alkyl optional mit einem oder mehreren Halogenen oder -C₃-C₆-Cycloalkylen substituiert ist, und wobei jedes Cycloalkyl ferner optional mit einem oder mehreren Halogenen substituiert ist;
X¹ -OR¹¹ oder -NR⁵¹R⁵² ist;
R¹¹ unabhängig -C₁-C₆-Alkyl, -C₁-C₆ Alkylen-R^{12A}, -C₂-C₆-Alkenyl, -C₂-C₆-Alkinyl, -C₃-C₆-Cycloalkyl, -C₅-C₈-Cycloalkenyl, 3-10-gliedriges Heterocycloalkyl, -C₆-C₁₀-Aryl oder 5-10-gliedriges Heteroaryl ist, wobei jedes Alkyl, Alkylen, Alkenyl, Alkinyl, Cycloalkyl, Cycloalkenyl, Heterocycloalkyl, Aryl oder Heteroaryl optional mit einem oder mehreren R¹² substituiert ist;
R¹² und R^{12A} jeweils unabhängig -C₁-C₆-Alkyl, -C₁-C₆-Alkylen-R^{16A}, -C₂-C₆-Alkenyl, -C₂-C₆-Alkinyl, -C₃-C₆-Cycloalkyl, -C₅-C₈-Cycloalkenyl, 3-10-gliedriges Heterocycloalkyl, -C₆-C₁₀-Aryl, 5-10-gliedriges Heteroaryl, -C₁-C₆-Alkoxy, -C₁-C₆ Halogenalkyl, Halogen, Cyano, Oxo, -OR¹³, -CO₂R¹³ oder -NR¹⁴R¹⁵ sind, wobei jedes Alkyl, Alkylen, Alkenyl, Alkinyl, Cycloalkyl, Cycloalkenyl, Heterocycloalkyl, Aryl, Heteroaryl und Alkoxy optional mit einem oder mehreren R¹⁶ substituiert ist;
R¹³, R¹⁴ und R¹⁵ jeweils unabhängig Wasserstoff, -C₁-C₆-Alkyl, -C₁-C₆-Alkylen-R^{20A}, -C₂-C₆-Alkenyl, -C₂-C₆-Alkinyl, -C₃-C₆-Cycloalkyl, -C₅-C₈-Cycloalkenyl, 3-10-gliedriges Heterocycloalkyl, -C₆-C₁₀-Aryl, 5-10-gliedriges Heteroaryl, -C₁-C₆-Alkoxy, -C₁-C₆-Halogenalkyl, -S(O)₂(C₁-C₆-Alkyl) oder -S(O)₂NR¹⁸R¹⁹ sind, wobei jedes Alkyl, Alkylen, Alkenyl, Alkinyl, Cycloalkyl, Cycloalkenyl, Heterocycloalkyl, Aryl, Heteroaryl und Alkoxy optional mit einem oder mehreren R²⁰ substituiert ist;
R¹⁶ und R^{16A} jeweils unabhängig -C₁-C₆-Alkyl, -C₁-C₆-Alkylen-R^{20A}, -C₂-C₆-Alkenyl, -C₂-C₆-Alkinyl, -C₃-C₆-Cycloalkyl, -C₅-C₈-Cycloalkenyl, 3-10-gliedriges Heterocycloalkyl, -C₆-C₁₀-Aryl, 5-10-gliedriges Heteroaryl, -C₁-C₆-Alkoxy, -C₁-C₆-Halogenalkyl, Halogen, Cyano, Oxo, -OR¹⁷, -S(O)₂(C₁-C₆-Alkyl) oder -S(O)₂NR¹⁸R¹⁹ sind, wobei jedes Alkyl, Alkylen, Alkenyl, Alkinyl, Cycloalkyl, Cycloalkenyl, Heterocycloalkyl, Aryl, Heteroaryl und Alkoxy optional mit einem oder mehreren R²⁰ substituiert ist;
R¹⁷, R¹⁸ und R¹⁹ jeweils unabhängig Wasserstoff, Halogen, -OH, -C₁-C₆-Alkyl, -C₁-C₆-Alkylen-R^{23A}, -C₂-C₆-Alkenyl, -C₂-C₆-Alkinyl, -C₃-C₆-Cycloalkyl, -C₅-C₈-Cycloalkenyl, 3-10-gliedriges Heterocycloalkyl, -C₆-C₁₀-Aryl, 5-10-gliedriges Heteroaryl, -C₁-C₆-Alkoxy, -C₁-C₆-Halogenalkyl, -S(O)₂(C₁-C₆-Alkyl) oder S(O)₂NR²¹R²² sind, wobei jedes Alkyl, Alkylen, Alkenyl, Alkinyl, Cycloalkyl, Cycloalkenyl, Heterocycloalkyl, Aryl, Heteroaryl und Alkoxy optional mit einem oder mehreren R²³ substituiert ist;
R²⁰ und R^{20A} jeweils unabhängig Halogen, -OH, -C₁-C₆-Alkyl, -C₁-C₆-Alkylen-R^{23A}, -C₂-C₆-Alkenyl, -C₂-C₆-Alkinyl, -C₃-C₆-Cycloalkyl, -C₅-C₈-Cycloalkenyl, 3-10-gliedriges Heterocycloalkyl, -C₆-C₁₀-Aryl, 5-10-gliedriges Heteroaryl, -C₁-C₆-Alkoxy, -C₁-C₆-Halogenalkyl, Halogen, Cyano, Oxo, -S(O)₂(C₁-C₆-Alkyl) oder S(O)₂NR²¹R²² sind, wobei jedes Alkyl, Alkylen, Alkenyl, Alkinyl, Cycloalkyl, Cycloalkenyl, Heterocycloalkyl, Aryl, Heteroaryl und Alkoxy optional mit einem oder mehreren R²³ substituiert ist;
R²¹ und R²² jeweils unabhängig Wasserstoff oder -C₁-C₆-Alkyl sind; und
R²³ und R^{23A} jeweils unabhängig -C₁-C₆-Alkyl, -C₁-C₆-Alkoxy, -C₁-C₆-Halogenalkyl, Halogen oder Cyano sind;
R⁵¹ und R⁵² jeweils unabhängig Wasserstoff, -C₁-C₆-Alkyl, -C₁-C₆-Alkylen-R^{53A}, -C₂-C₆-Alkenyl, -C₂-C₆-Alkinyl, -C₃-C₆-Cycloalkyl, -C₅-C₈-Cycloalkenyl, 3-10-gliedriges Heterocycloalkyl, -C₆-C₁₀-Aryl oder 5-10-gliedriges Heteroaryl sind, wobei jedes Alkyl, Alkylen, Alkenyl, Alkinyl, Cycloalkyl, Cycloalkenyl, Heterocycloalkyl, Aryl oder Heteroaryl optional mit einem oder mehreren R⁵³ substituiert ist; oder R⁵¹ und R⁵², zusammen mit dem Stickstoffatom, an das sie gebunden sind, sich kombinieren, um ein 3-15-gliedriges Heterocycloalkyl oder 5-15-gliedriges Heteroaryl auszubilden, wobei das Heterocycloalkyl oder Heteroaryl optional mit einem oder mehreren R⁵³ substituiert ist;
R⁵³ und R^{53A} jeweils unabhängig -C₁-C₆-Alkyl, -C₁-C₆-Alkylen-R^{57A}, -C₂-C₆-Alkenyl, -C₂-C₆-Alkinyl, -C₃-C₆-Cycloalkyl, -C₅-C₈-Cycloalkenyl, 3-10-gliedriges Heterocycloalkyl, -C₆-C₁₀-Aryl, 5-10-gliedriges Heteroaryl, -C₁-C₆-Alkoxy, -C₁-C₆-Halogenalkyl, Halogen, Cyano, Oxo, -OR⁵⁴, -C(O)R⁵⁴, -CO₂R⁵⁴ oder -NR⁵⁵R⁵⁶ sind, wobei jedes Alkyl, Alkylen, Alkenyl, Alkinyl, Cycloalkyl, Cycloalkenyl, Heterocycloalkyl, Aryl, Heteroaryl und Alkoxy optional mit einem oder mehreren R⁵⁷ substituiert ist;
R⁵⁴, R⁵⁵ und R⁵⁶ jeweils unabhängig Wasserstoff, -C₁-C₆-Alkyl, -C₁-C₆-Alkylen-R^{61A}, -C₂-C₆-Alkenyl, -C₂-C₆-Alkinyl, -C₃-C₆-Cycloalkyl, -C₅-C₈-Cycloalkenyl, 3-10-gliedriges Heterocycloalkyl, -C₆-C₁₀-Aryl, 5-10-gliedriges Heteroaryl, -C₁-C₆-Alkoxy, -C₁-C₆-Halogenalkyl, -CO₂R⁵⁸, -S(O)₂(C₁-C₆-Alkyl), -S(O)₂(C₆-C₁₀-Aryl) oder -S(O)₂NR⁵⁹R⁶⁰ sind, wobei jedes Alkyl, Alkylen, Alkenyl, Alkinyl, Cycloalkyl, Cycloalkenyl, Heterocycloalkyl, Aryl, Heteroaryl und Alkoxy optional mit einem oder mehreren R⁵¹ substituiert ist;
R⁵⁷ und R^{57A} jeweils unabhängig -C₁-C₆-Alkyl, -C₁-C₆-Alkylen-R^{61A}, -C₂-C₆-Alkenyl, -C₂-C₆-Alkinyl, -C₃-C₆-Cycloalkyl, -C₅-C₈-Cycloalkenyl, 3-10-gliedriges Heterocycloalkyl, -C₆-C₁₀-Aryl, 5-10-gliedriges Heteroaryl, -C₁-C₆-Alkoxy, -C₁-C₆-Halogenalkyl, Halogen, Cyano, Oxo, -OR⁵⁸, NR⁵⁹R⁶⁰, -S(O)₂(C₁-C₆-Alkyl) oder -S(O)₂NR⁵⁹R⁶⁰ sind, wobei jedes Alkyl, Alkylen, Alkenyl, Alkinyl, Cycloalkyl, Cycloalkenyl, Heterocycloalkyl, Aryl, Heteroaryl und Alkoxy optional mit einem oder mehreren R⁶¹ substituiert ist;
R⁵⁸, R⁵⁹ und R⁶⁰ jeweils unabhängig Wasserstoff, -C₁-C₆-Alkyl, -C₁-C₆-Alkylen-R⁶⁴, -C₂-C₆-Alkenyl, -C₂-C₆-Alkinyl, -C₃-C₆-Cycloalkyl, -C₅-C₈-Cycloalkenyl, 3-10-gliedriges Heterocycloalkyl, -C₆-C₁₀-Aryl, 5-10-gliedriges Heteroaryl, -C₁-C₆-Alkoxy, -C₁-C₆-Halogenalkyl, -S(O)₂(C₁-C₆-Alkyl) oder S(O)₂NR⁶²R⁶³ sind, wobei jedes Alkyl, Alkylen, Alkenyl, Alkinyl, Cycloalkyl, Cycloalkenyl, Heterocycloalkyl, Aryl, Heteroaryl und Alkoxy optional mit einem oder mehreren R⁶⁴ substituiert ist;
R⁶¹ und R^{61A} jeweils unabhängig Halogen, -OH, -C₁-C₆-Alkyl, -C₁-C₆-Alkylen-R^{64A}, -C₂-C₆-Alkenyl, -C₂-C₆-Alkinyl, -C₃-C₆-Cycloalkyl, -C₅-C₈-Cycloalkenyl, 3-10-gliedriges Heterocycloalkyl, -C₆-C₁₀-Aryl, 5-10-gliedriges Heteroaryl, -C₁-C₆-Alkoxy, -C₁-C₆-Halogenalkyl, Halogen, Cyano, Oxo, -S(O)₂(C₁-C₆-Alkyl) oder S(O)₂NR⁶²R⁶³ sind, wobei jedes Alkyl, Alkylen, Alkenyl, Alkinyl, Cycloalkyl, Cycloalkenyl, Heterocycloalkyl, Aryl, Heteroaryl und Alkoxy optional mit einem oder mehreren R⁶⁴ substituiert ist; und
R⁶² und R⁶³ jeweils unabhängig Wasserstoff und -C₁-C₆-Alkyl sind; und
R⁶⁴ und R^{64A} jeweils unabhängig -C₁-C₆-Alkyl, -C₁-C₆-Alkoxy, -C₁-C₆-Halogenalkyl, Phenyl, Halogen oder Cyano sind.

2. Verbindung nach Anspruch 1, wobei die Verbindung von der Formel I-A ist: 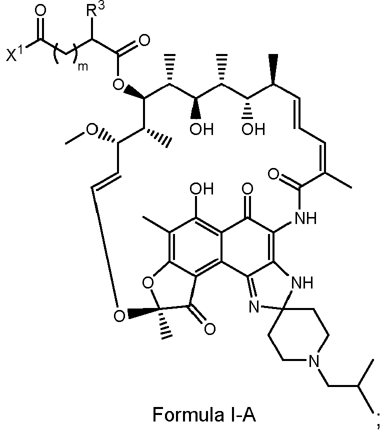 wobei
m eine ganze Zahl zwischen 0 und 4 ist; und
R³ aus -H, Methyl und Ethyl ausgewählt ist, und wobei vorzugsweise die Verbindung zu der Formel I-B, der Formel I-C, der Formel I-D, der Formel I-E, der Formel I-F oder der Formel I-G gehört:

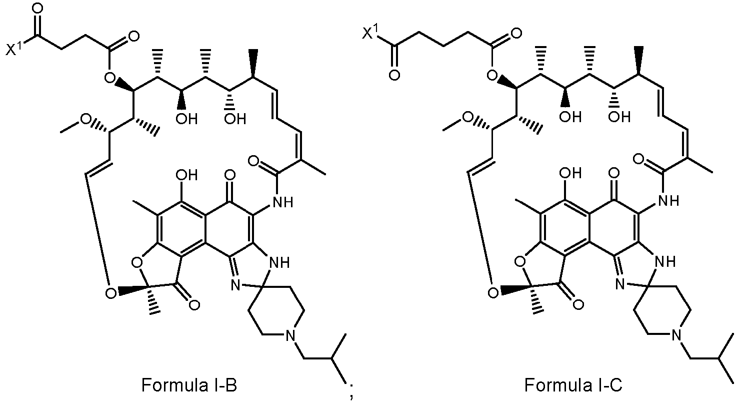

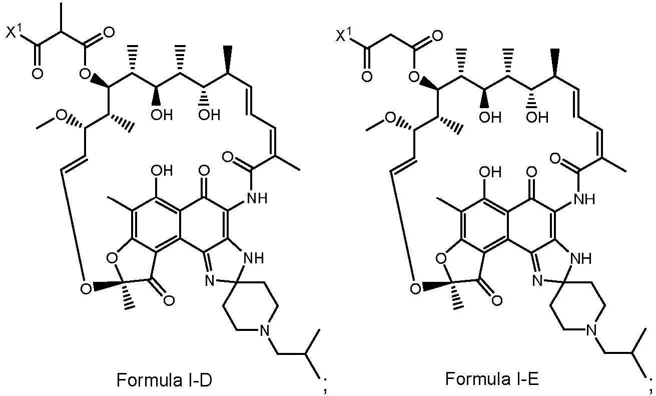

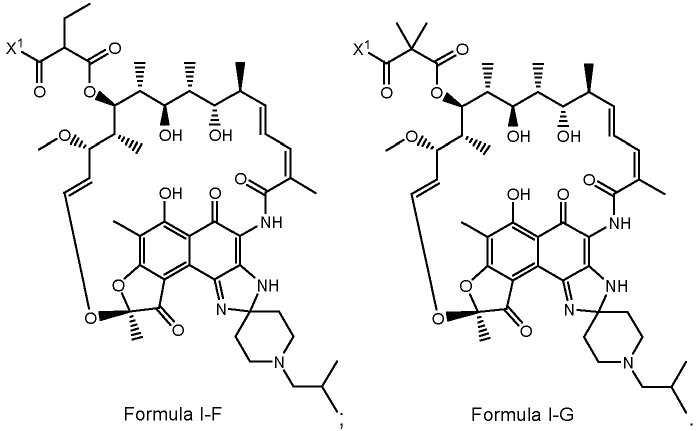

3. Verbindung nach Anspruch 1 oder 2, wobei X¹ -OR¹¹ ist.

4. Verbindung nach Anspruch 1 oder 2, wobei X¹ -NR⁵¹NR⁵² ist.

5. Verbindung nach Anspruch 4, wobei R⁵¹ und R⁵² jeweils unabhängig Wasserstoff, -C₁-C₆-Alkyl, -C₁-C₆-Alkylen-R^{53A}, -C₅-C₆-Cycloalkyl, 5-6-gliedriges Heterocycloalkyl oder Phenyl sind, wobei vorzugsweise mindestens eines von R⁵¹ und R⁵² Wasserstoff ist, wobei jedes Alkyl, Alkylen, Cycloalkyl, Heterocycloalkyl oder Phenyl optional mit einem oder mehreren, vorzugsweise einem oder zwei, R⁵³ substituiert ist; oder R⁵¹ und R⁵², zusammen mit dem Stickstoffatom, an das sie gebunden sind, sich kombinieren, um ein 3-15-gliedriges Heterocycloalkyl auszubilden, wobei das Heterocycloalkyl optional mit einem oder mehreren, vorzugsweise einem oder zwei, R⁵³ substituiert ist.

6. Verbindung nach Anspruch 4, wobei R⁵¹ und R⁵², zusammen mit dem Stickstoffatom, an das sie gebunden sind, sich kombinieren, um ein 3-15-gliedriges Heterocycloalkyl auszubilden, wobei das Heterocycloalkyl optional mit einem oder mehreren, vorzugsweise einem oder zwei, R⁵³ substituiert ist; oder wobei der Heterocyclus mit einem oder mehreren zusätzlichen Aryl- oder Heteroarylringen kondensiert ist, um ein 10- bis 15-gliedriges Heteroaryl auszubilden, wobei das 10- bis 15-gliedrige Heteroaryl optional mit einem oder mehreren, vorzugsweise einem oder zwei, R⁵³ substituiert ist; oder wobei der Heterocyclus an einem einzelnen Atom mit einem anderen Cycloalkyl oder Heterocycloalkyl verbunden ist, um einen 10- bis 15-gliedrigen spirocyclischen Heterocyclus auszubilden; und wobei der Spirocyclus optional mit einem oder mehreren, vorzugsweise einem oder zwei, R⁵³ substituiert ist.

7. Verbindung nach Anspruch 6, wobei das 3-15-gliedrige Heterocycloalkyl ein Piperidin, Morpholin, 1,4-Diazepan, Piperazin oder ein Azetidin ist.

8. Verbindung nach Anspruch 6, wobei das Heterocycloalkyl ein Piperidin ist.

9. Verbindung nach einem der Ansprüche 4 bis 8, wobei:
R⁵³ und R^{53A} unabhängig -C₁-C₄-Alkyl, -C₁-C₃-Alkylen-R^{57A}, 5- bis 15-gliedriges Heterocycloalkyl, 5- bis 15-gliedriges Heteroaryl oder Phenyl, -OR⁵⁴, -C(O)R⁵⁴, -CO₂R⁵⁴, Oxo oder -NR⁵⁵R⁵⁶ sind, wobei jedes Alkylen, Heterocycloalkyl, Heteroaryl und Phenyl optional mit einem oder mehreren R⁵⁷ substituiert ist;
wobei R⁵⁴ unabhängig Wasserstoff, -C₁-C₄-Alkyl, -C₅-C₆-Cycloalkyl oder Phenyl ist; wobei jedes Alkyl, Cycloalkyl oder Phenyl optional mit einem oder mehreren R⁶¹ substituiert ist;
R⁵⁵ und R⁵⁶ jeweils unabhängig Wasserstoff, -C₁-C₄-Alkyl, -C₁-C₄-Alkylen-R^{61A}, Phenyl, -CO₂C₁-C₆-Alkyl, -S(O)₂(Phenyl) sind, wobei Phenyl oder Cycloalkyl optional mit einem oder mehreren R⁶¹ substituiert ist.

10. Verbindung nach einem der Ansprüche 4 bis 9, wobei R⁵⁷ und R^{57A} unabhängig -C₁-C₆-Alkyl, 3-10-gliedriges Heterocycloalkyl, Phenyl, 5-10-gliedriges Heteroaryl, Oxo, Halogen, -OH oder -SO₂NH₂ sind, wobei jedes Alkyl, Heterocycloalkyl, Phenyl und Heteroaryl optional mit einem oder mehreren R⁶¹ substituiert ist.

11. Verbindung nach einem der Ansprüche 4 bis 10, wobei R⁶¹ und R^{61A} unabhängig -C₁-C₂-Alkyl, Phenyl, -C₁-C₂-Alkoxy, -C₁-C₂-Halogenalkyl oder Halogen sind.

12. Verbindung nach Anspruch 1, wobei X¹ aus der Gruppe ausgewählt ist, bestehend aus:

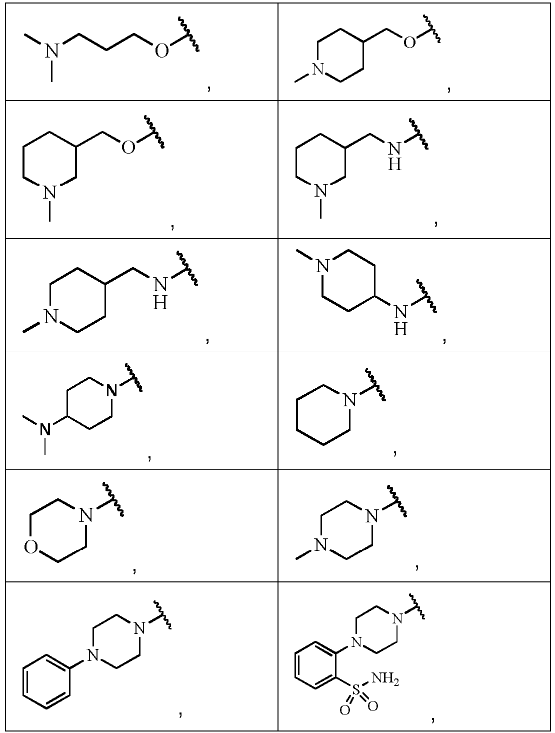

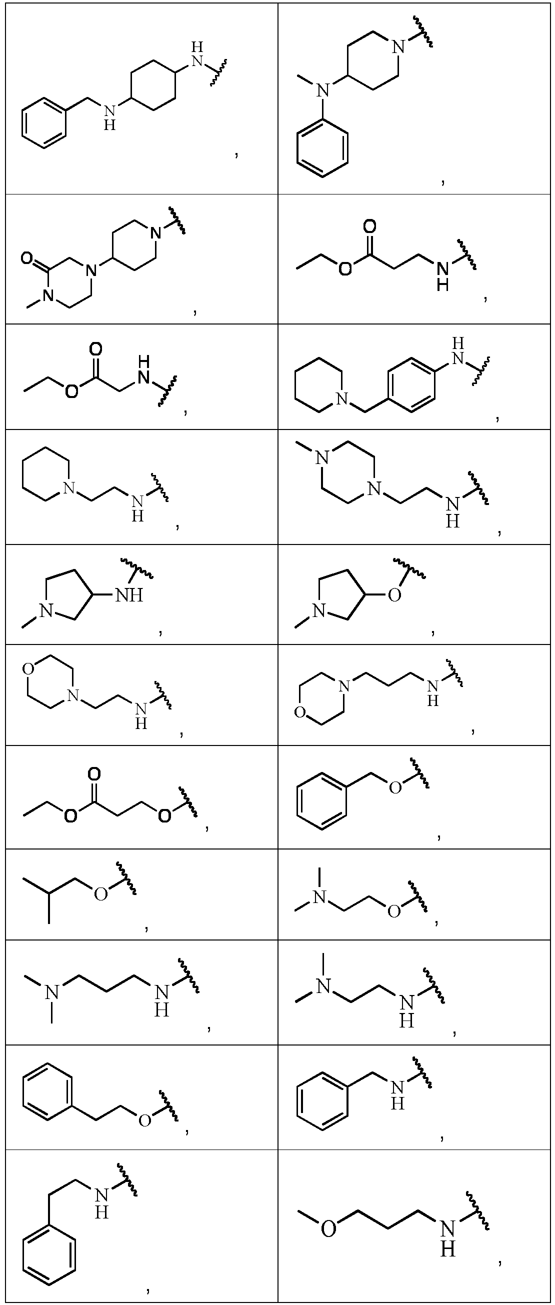

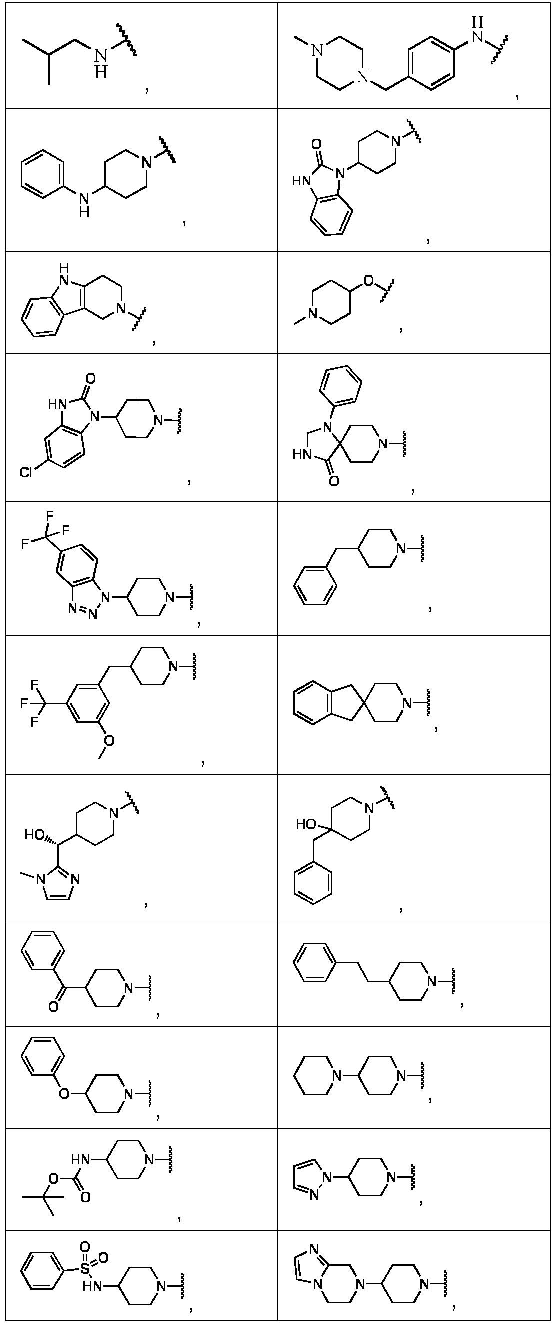

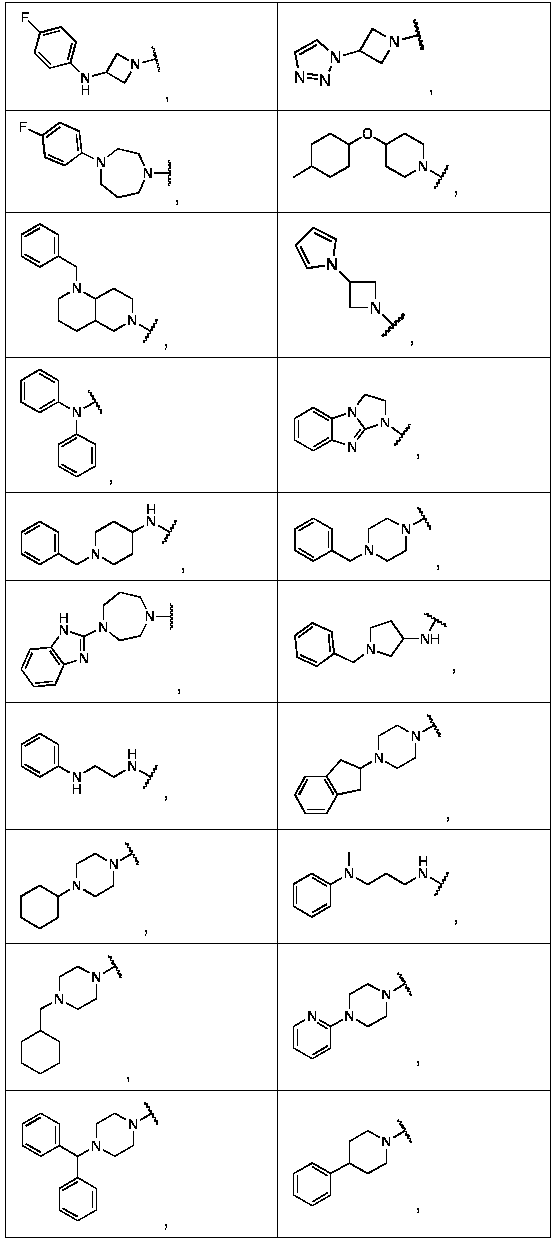

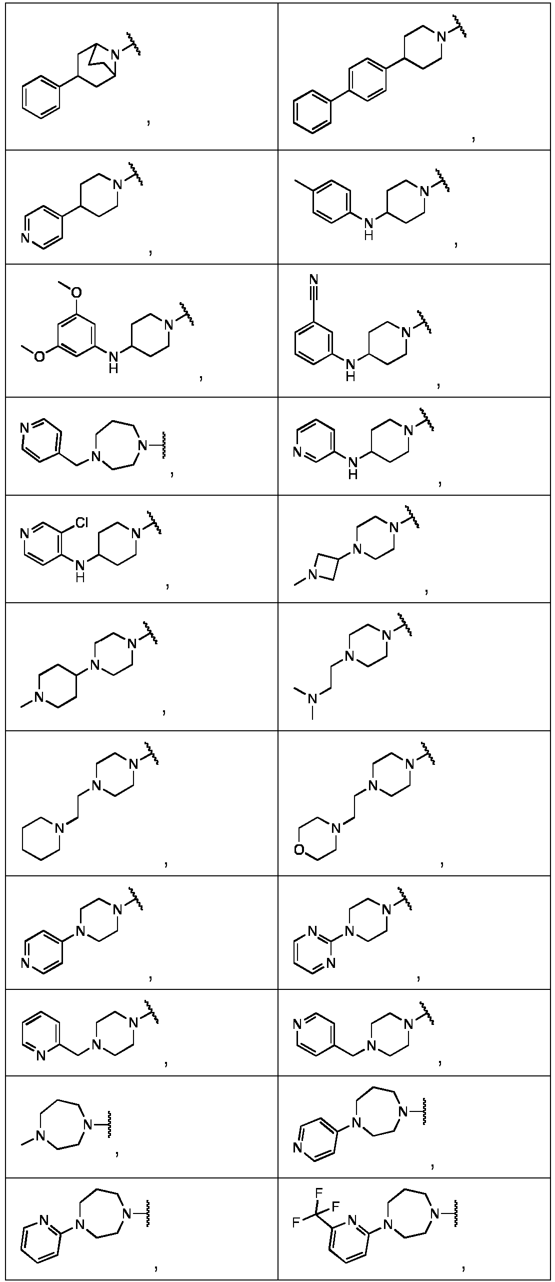

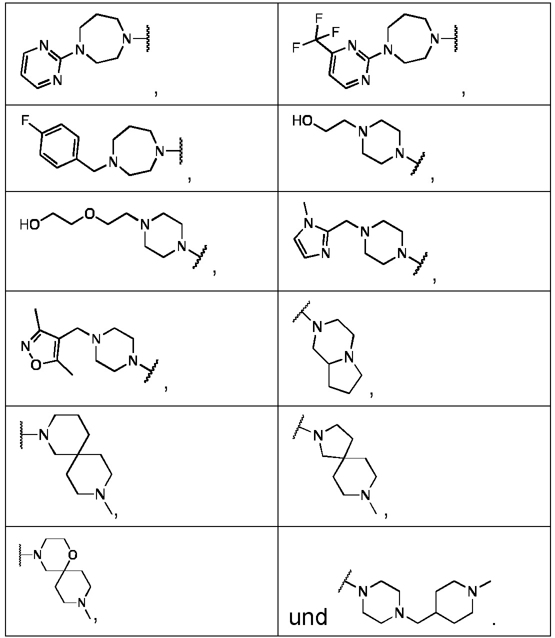

13. Verbindung nach Anspruch 1, wobei die Verbindung aus der Gruppe ausgewählt ist, bestehend aus:

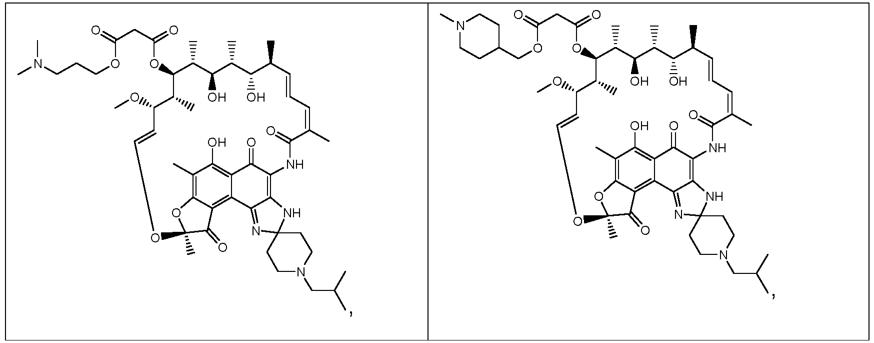

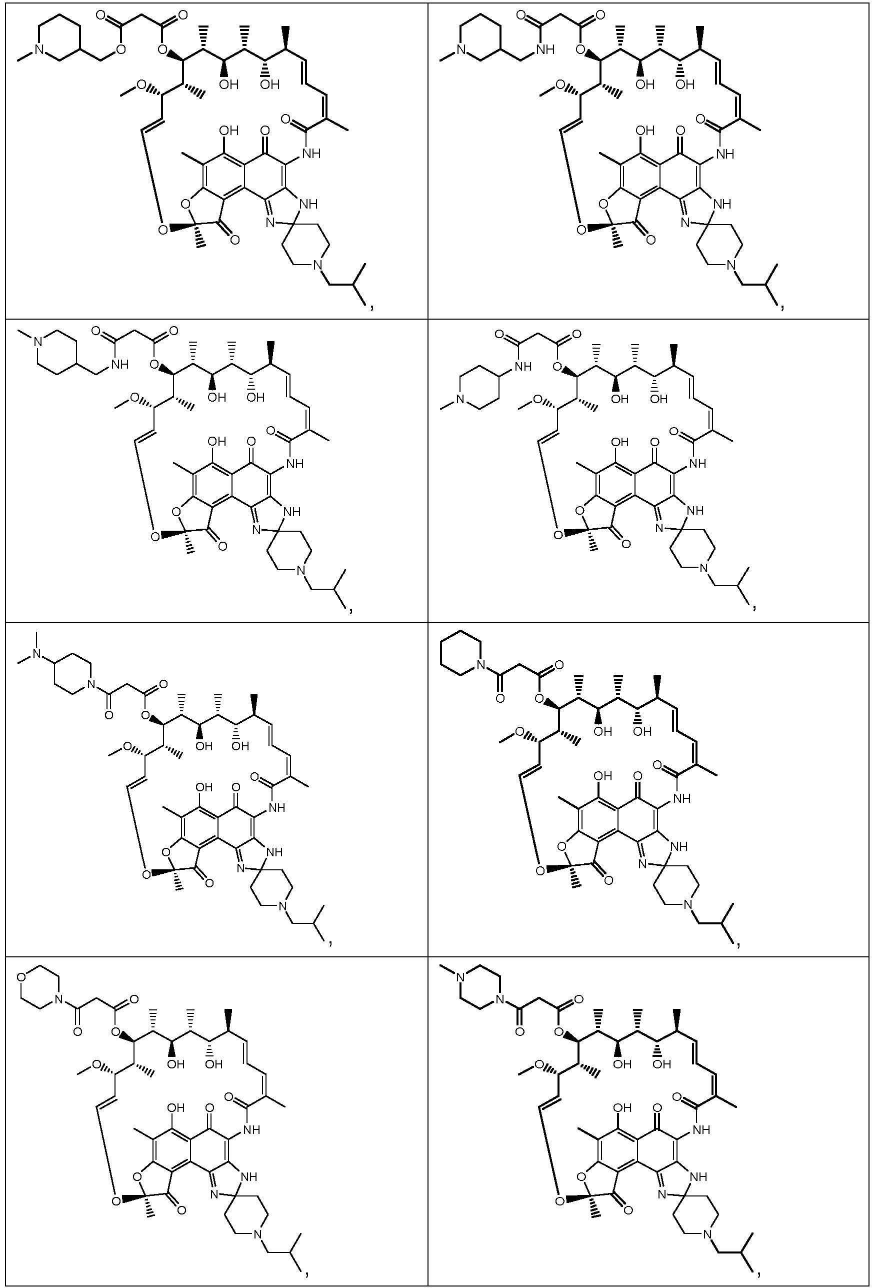

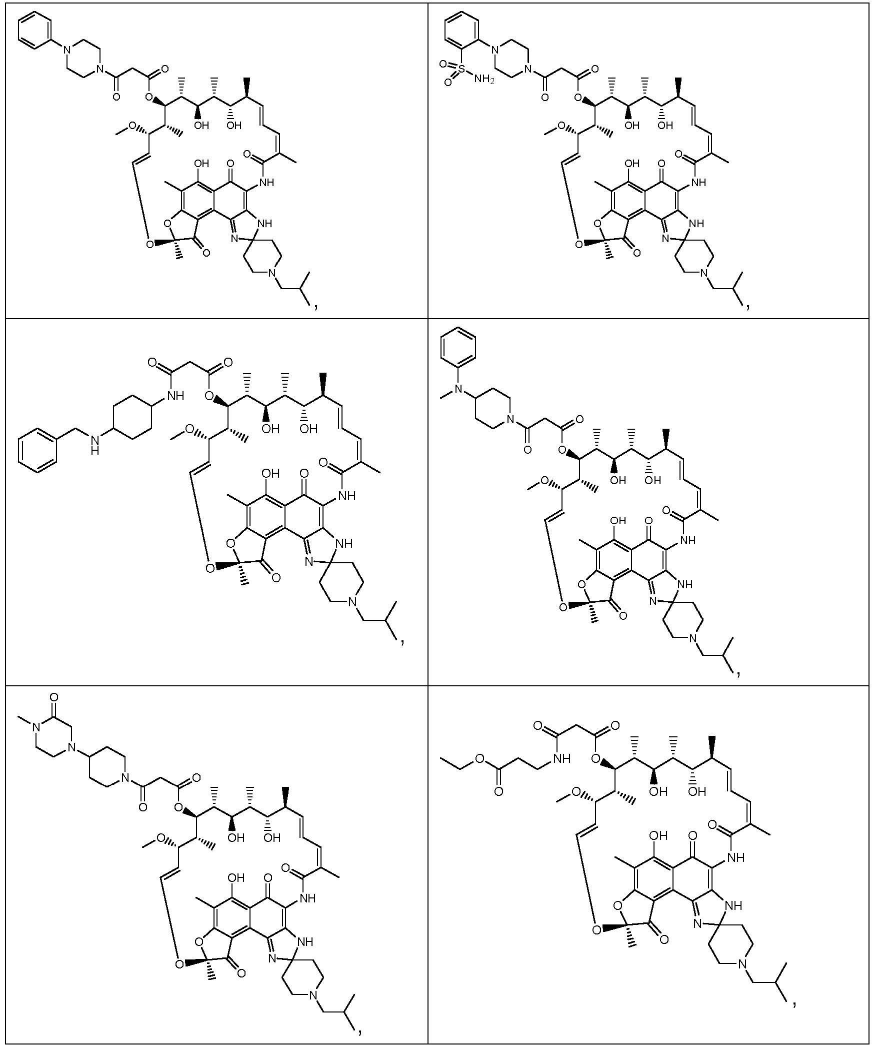

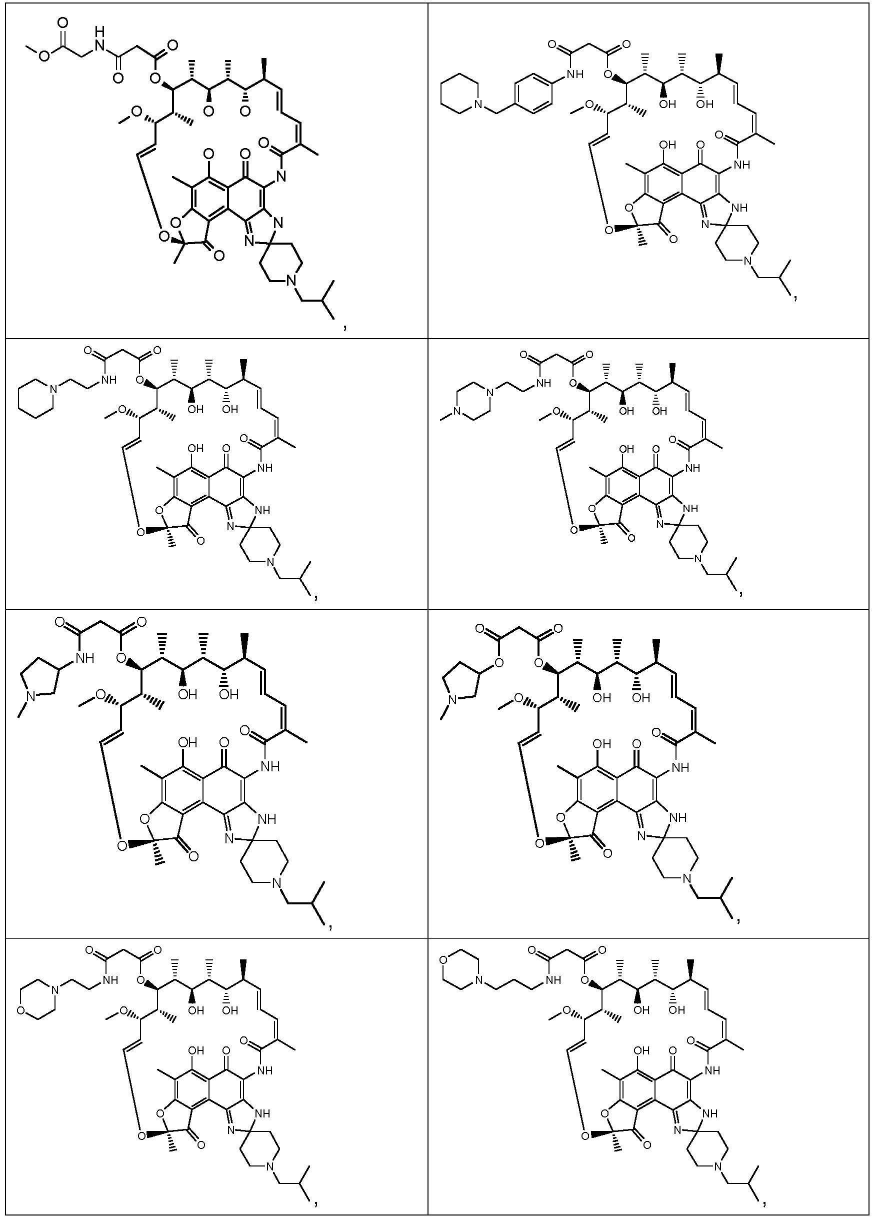

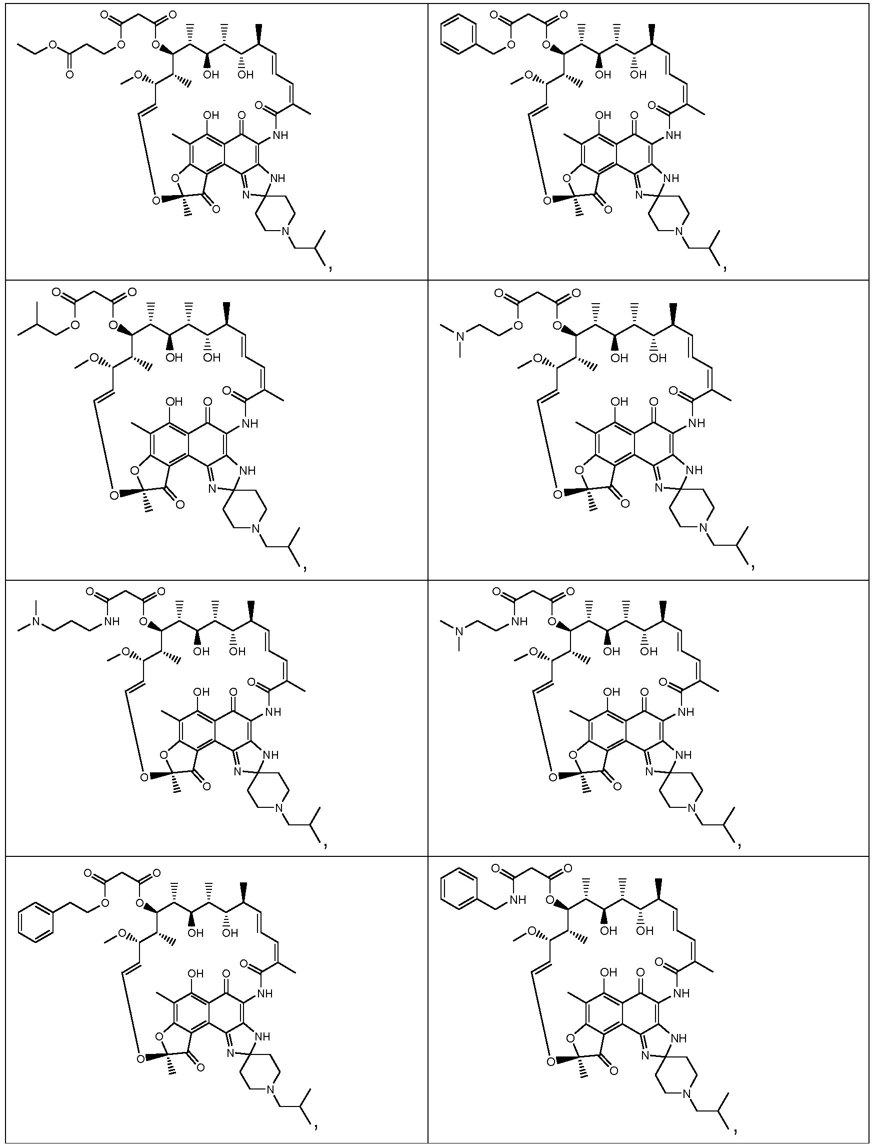

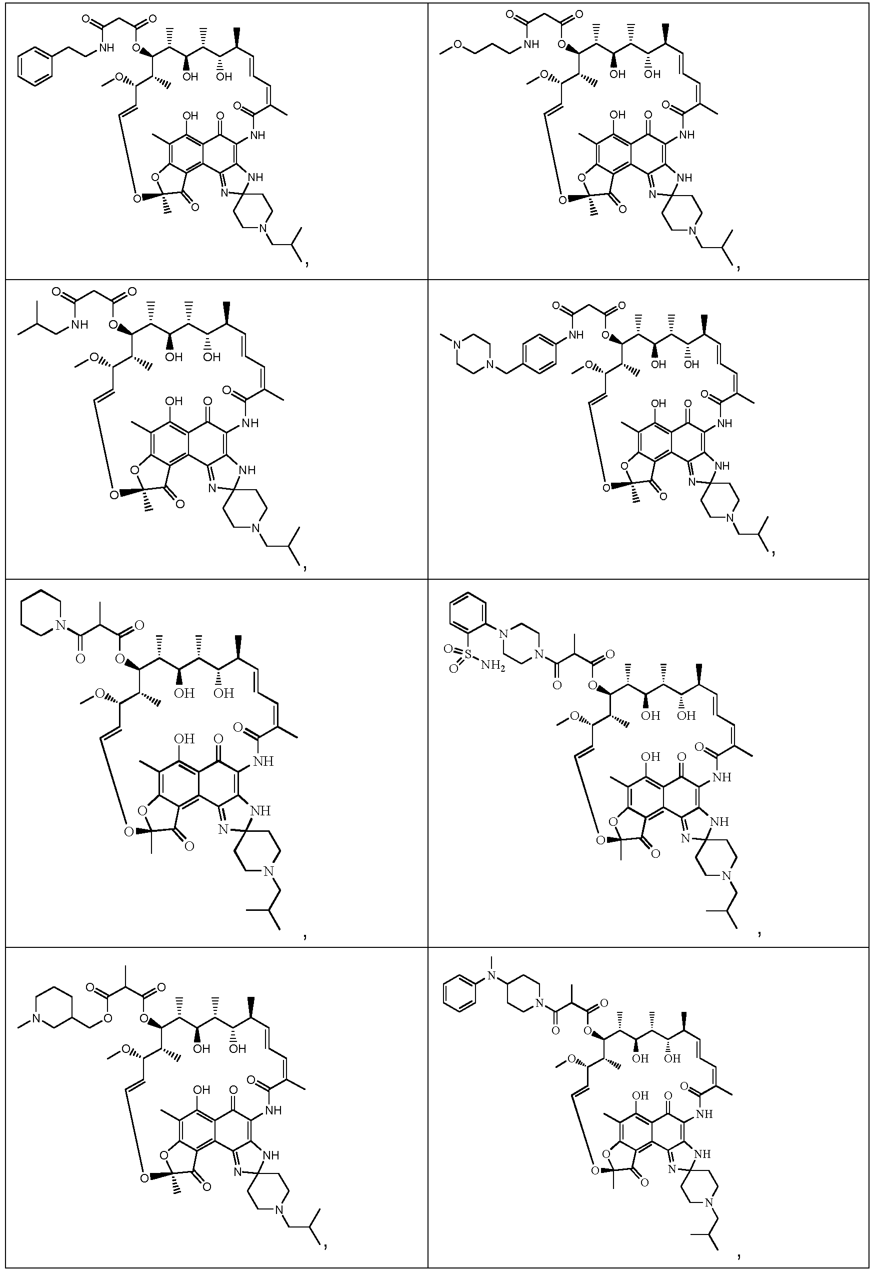

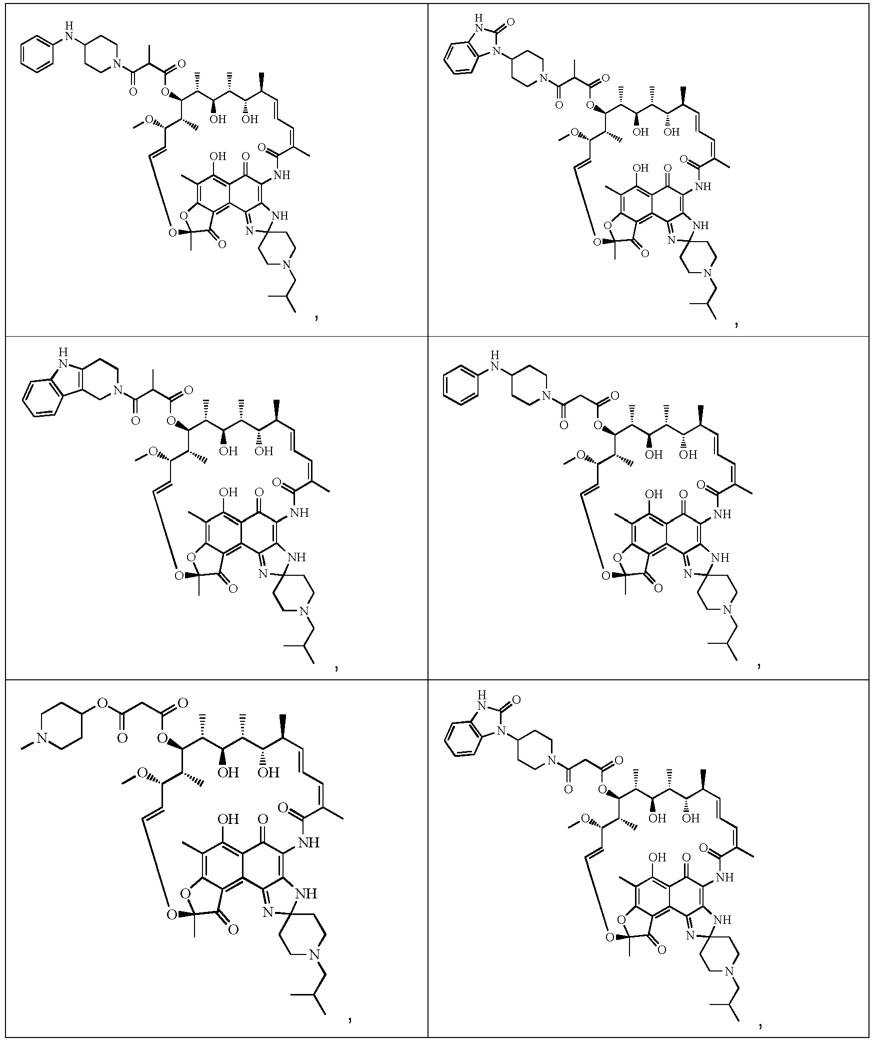

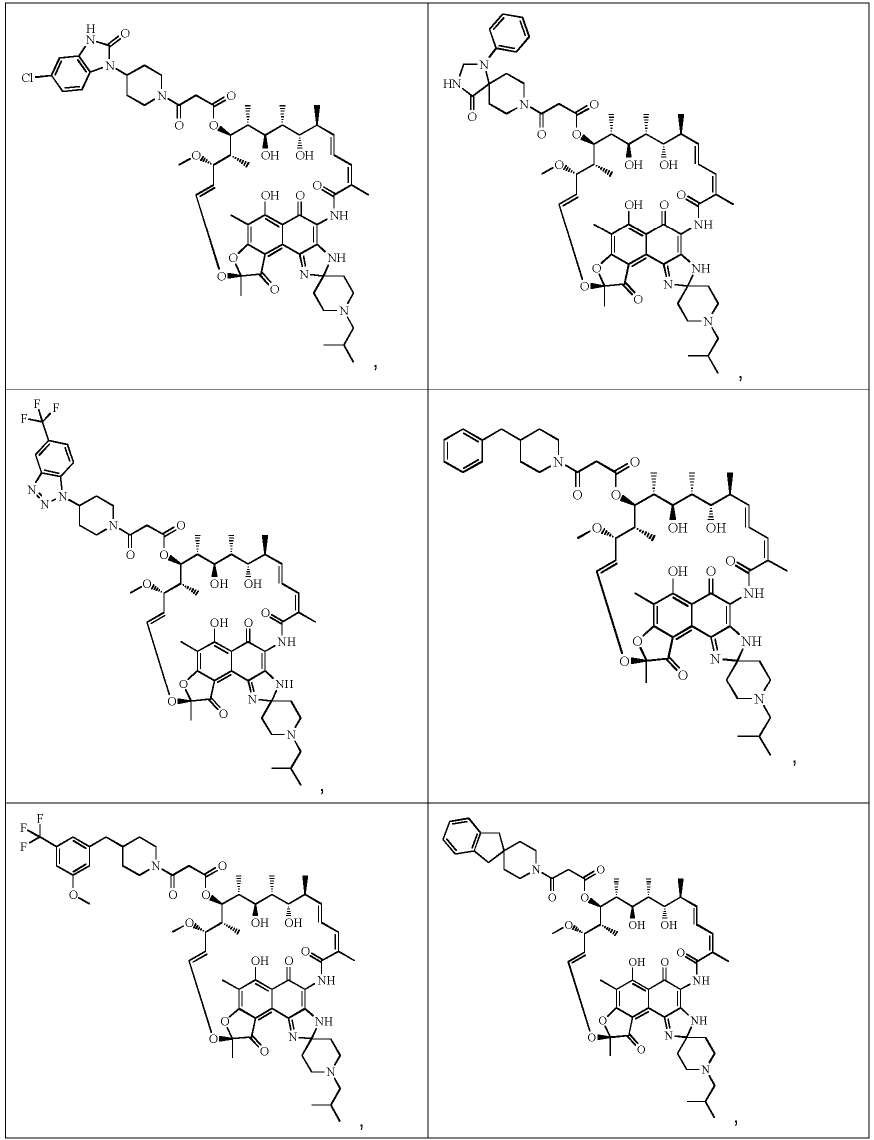

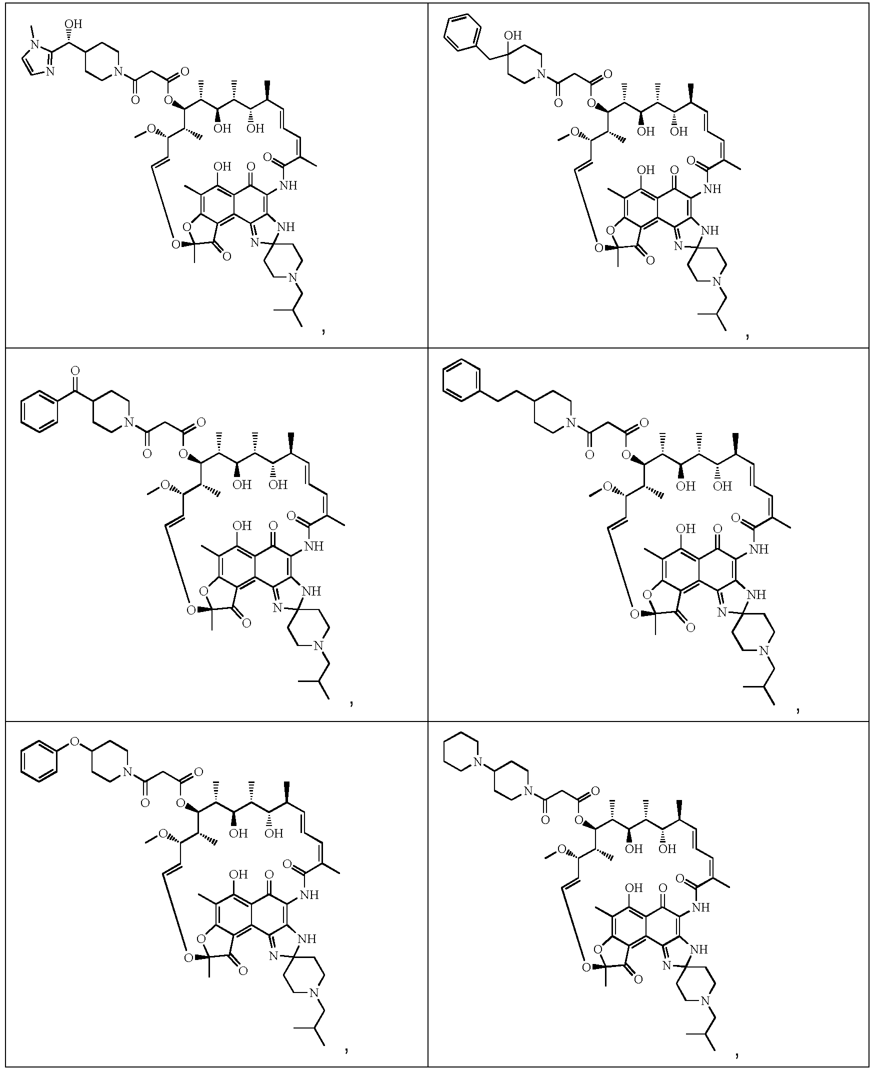

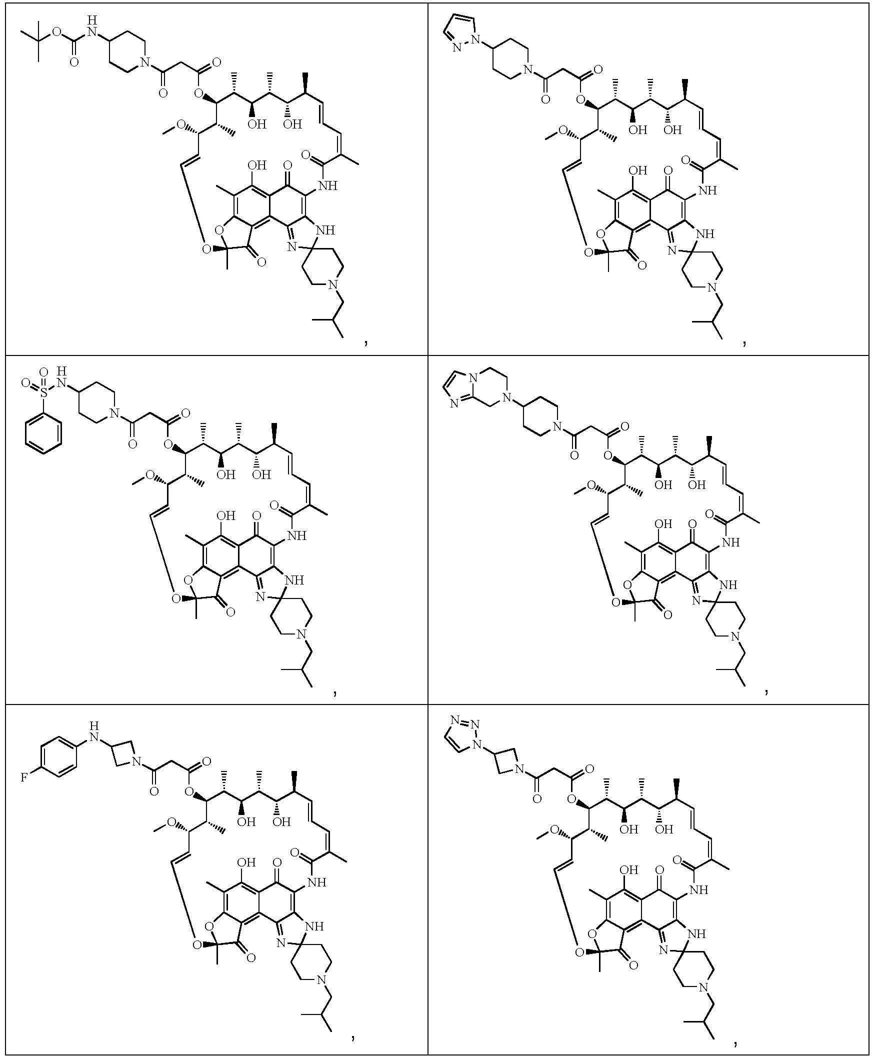

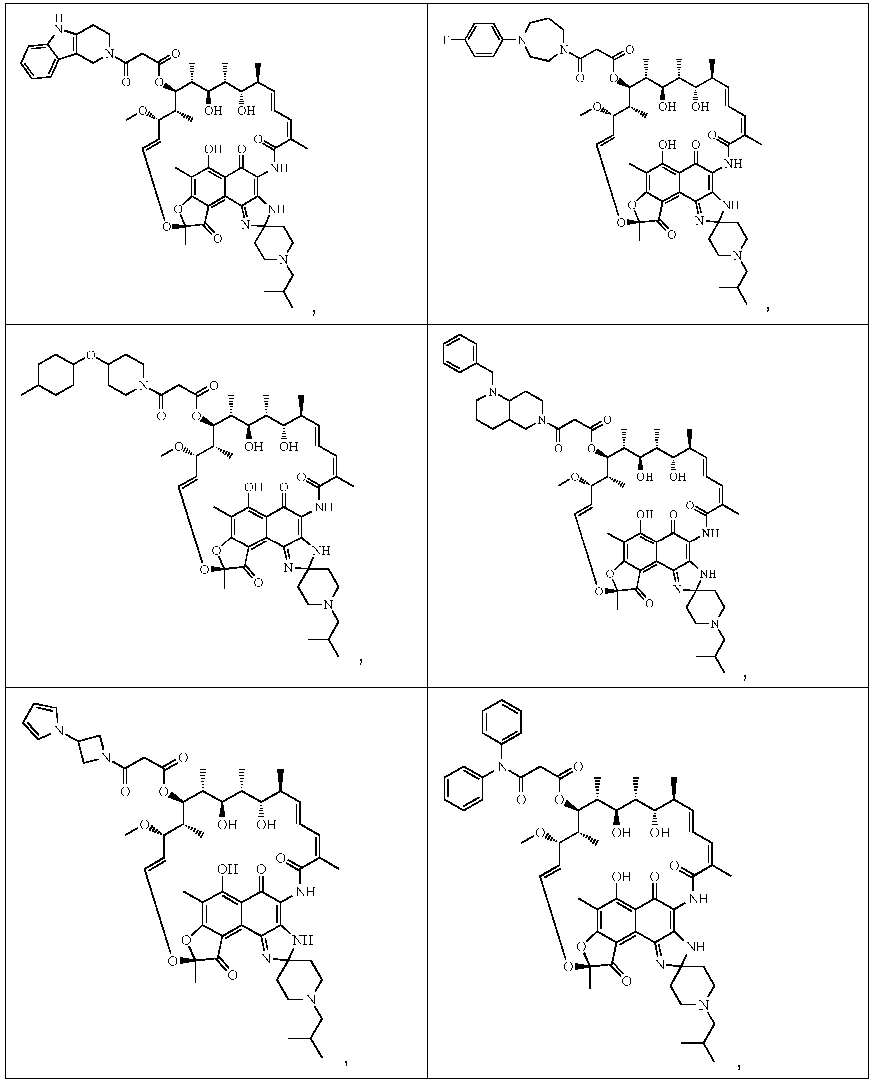

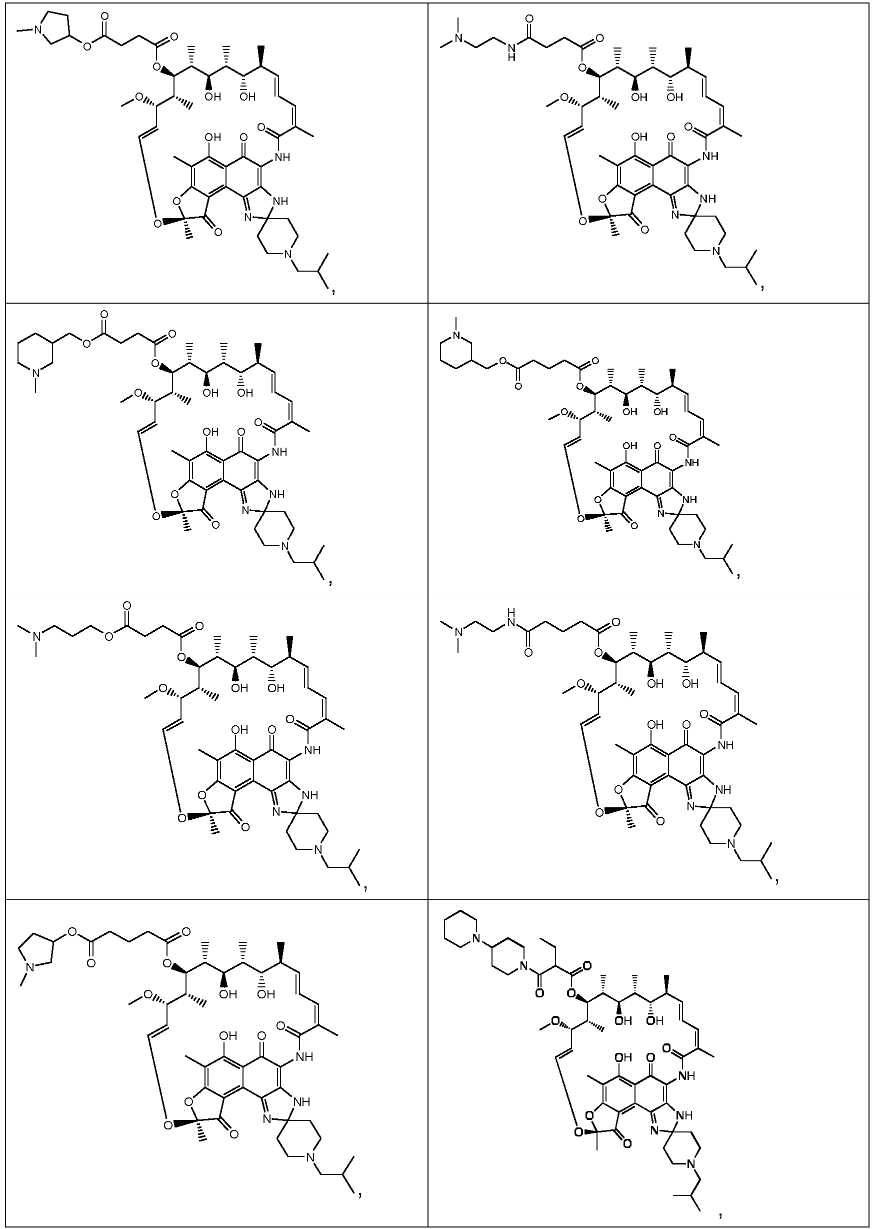

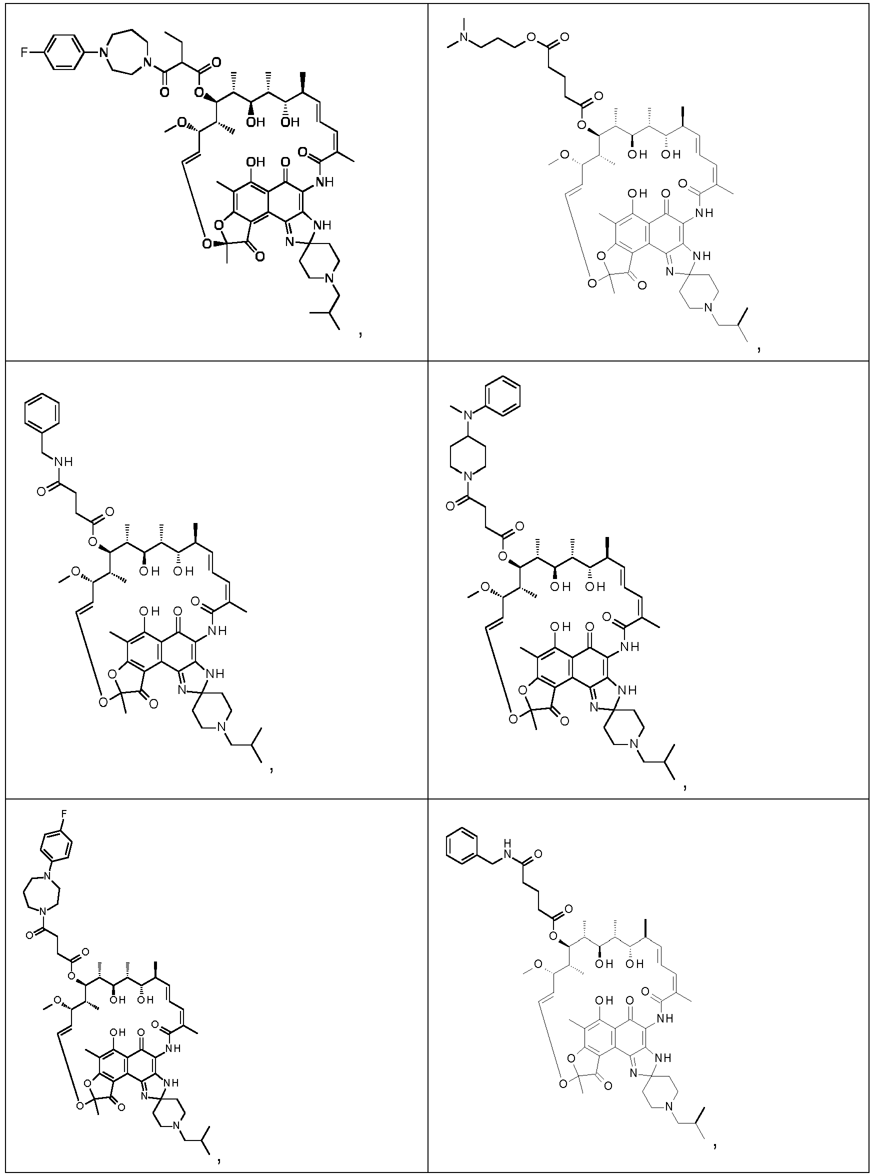

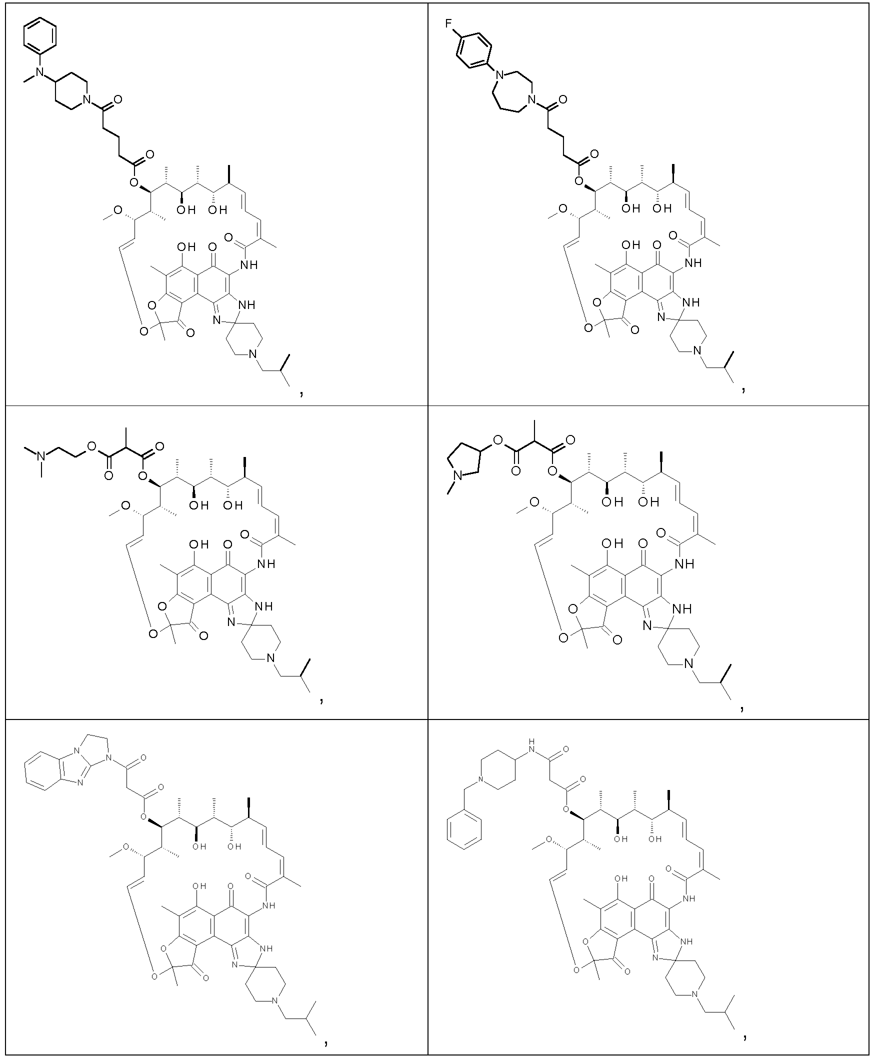

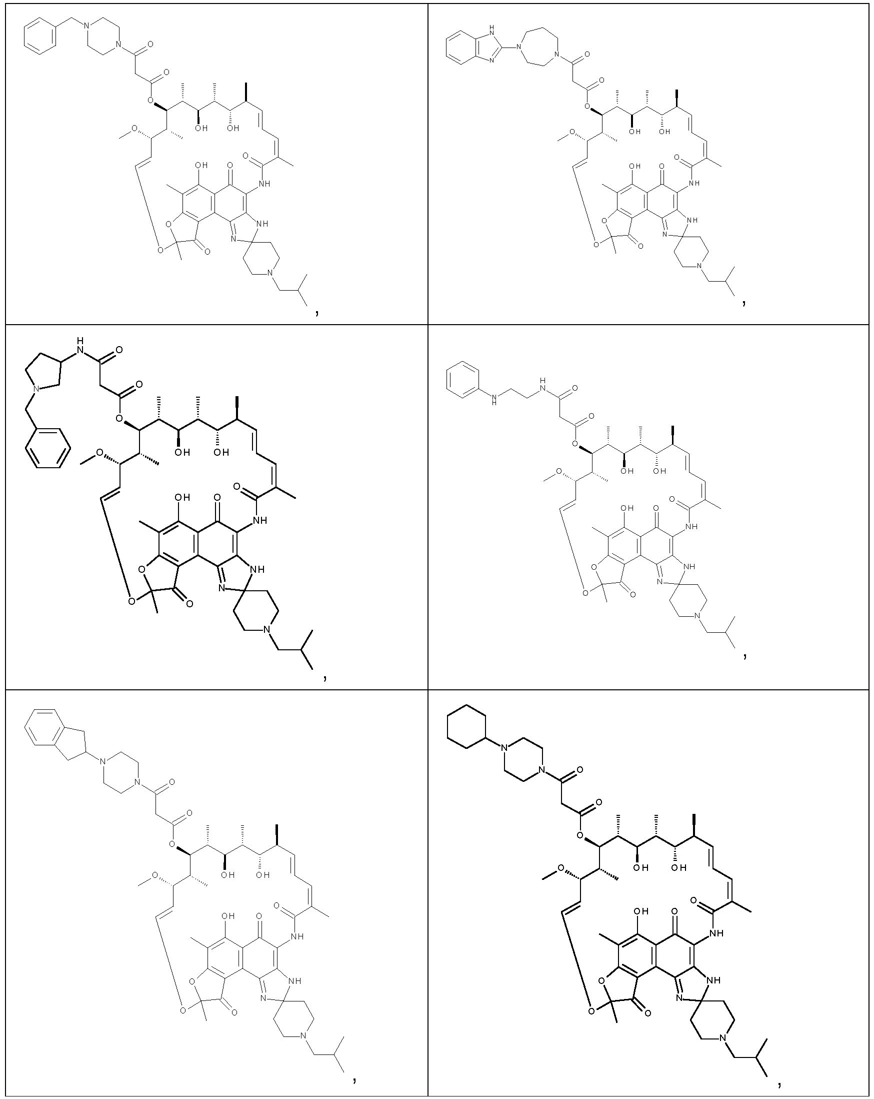

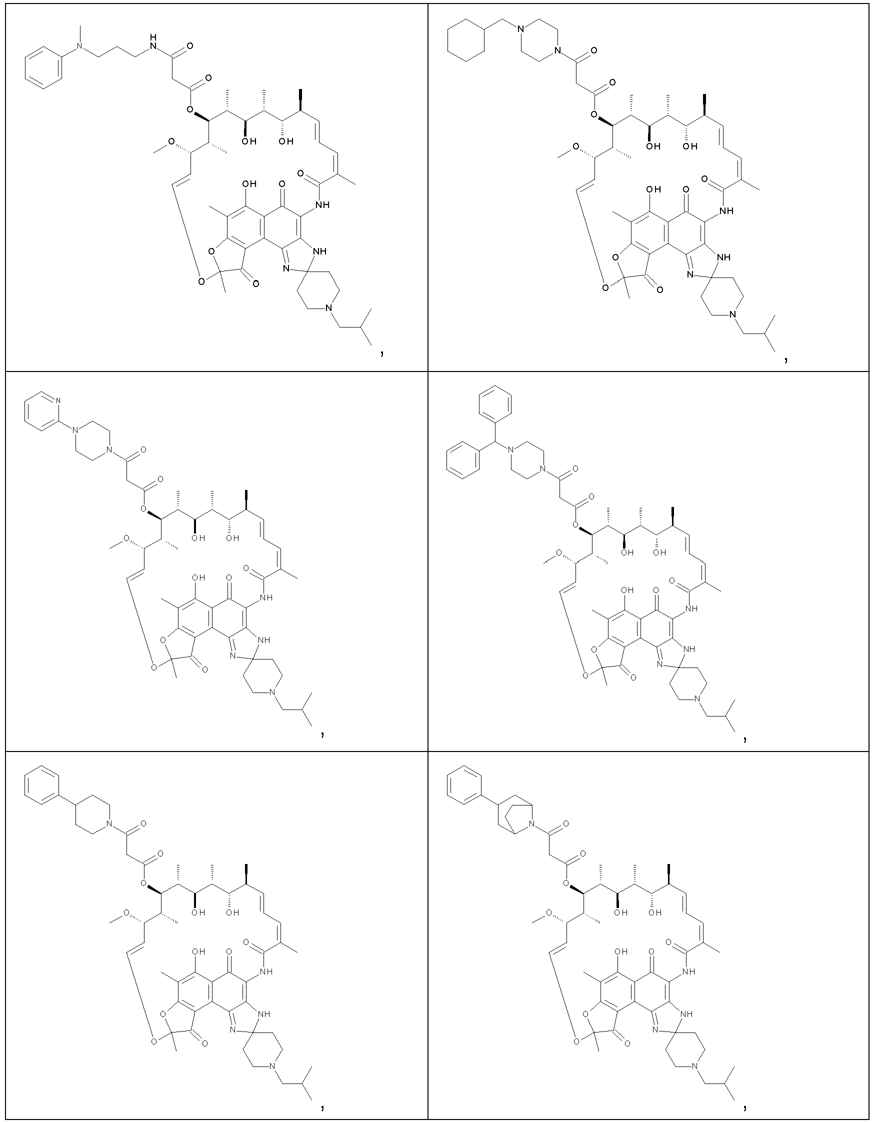

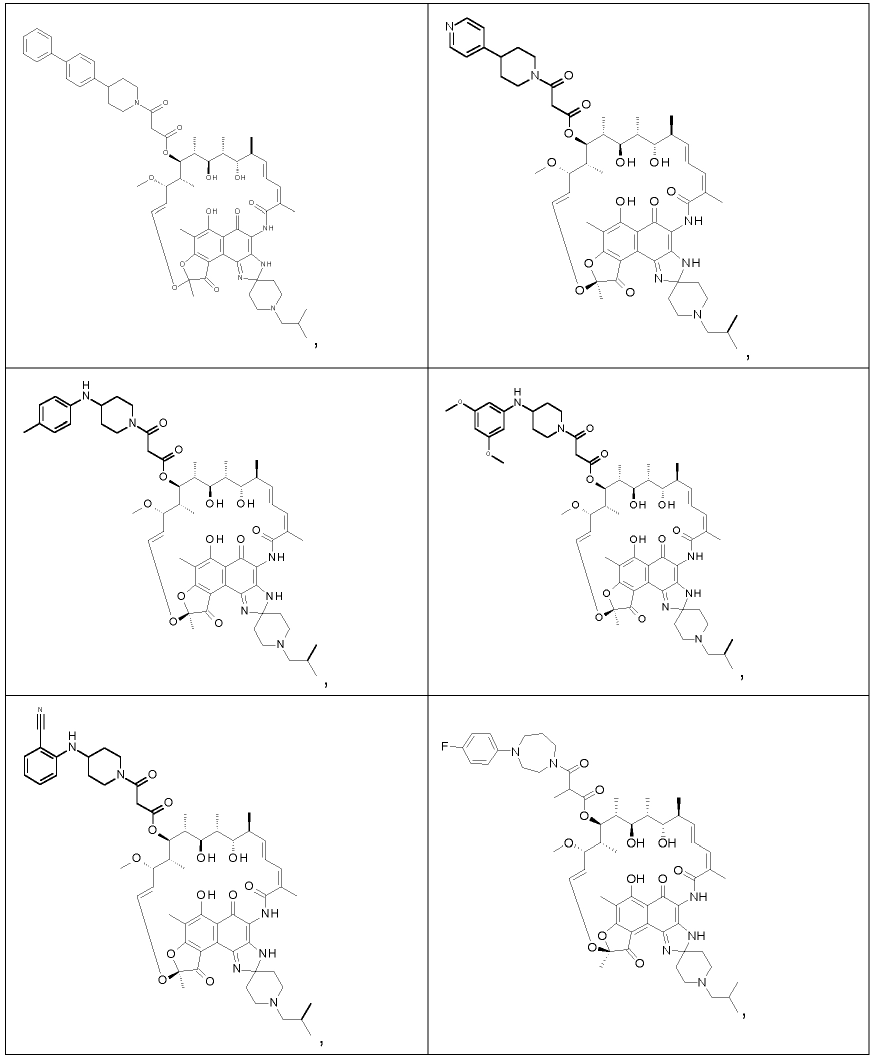

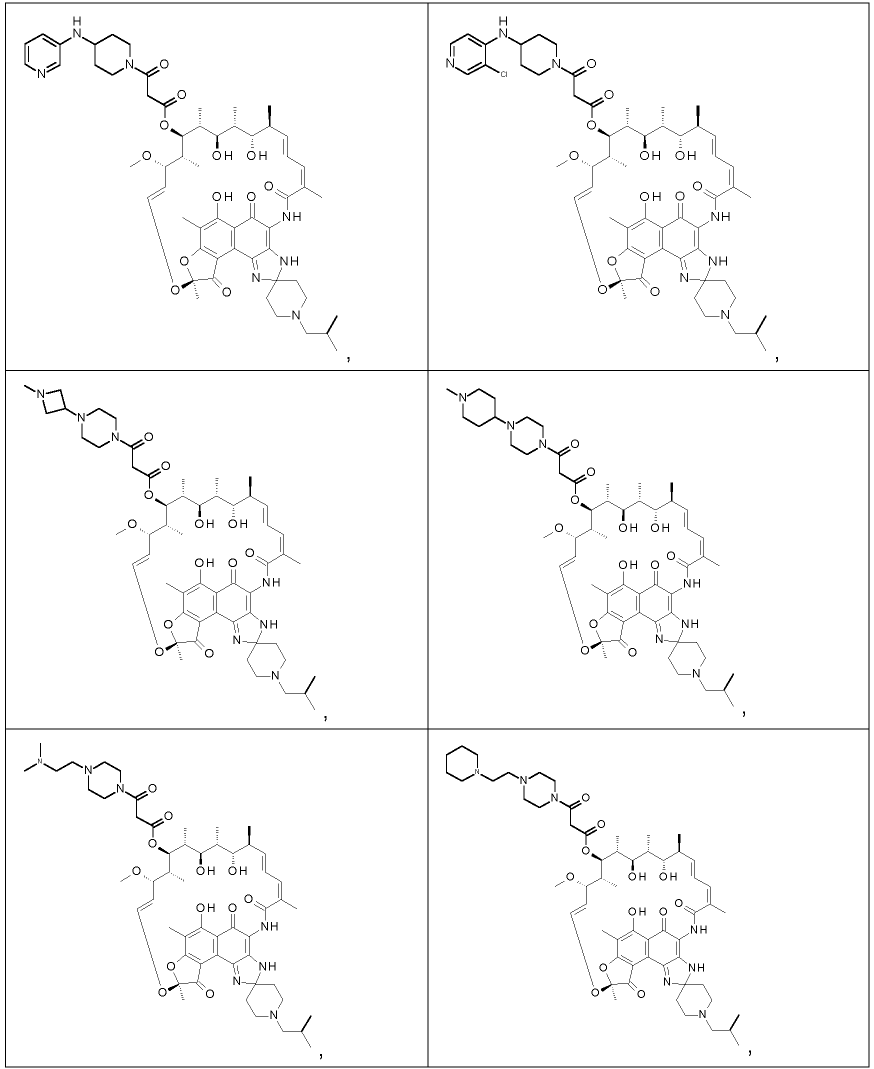

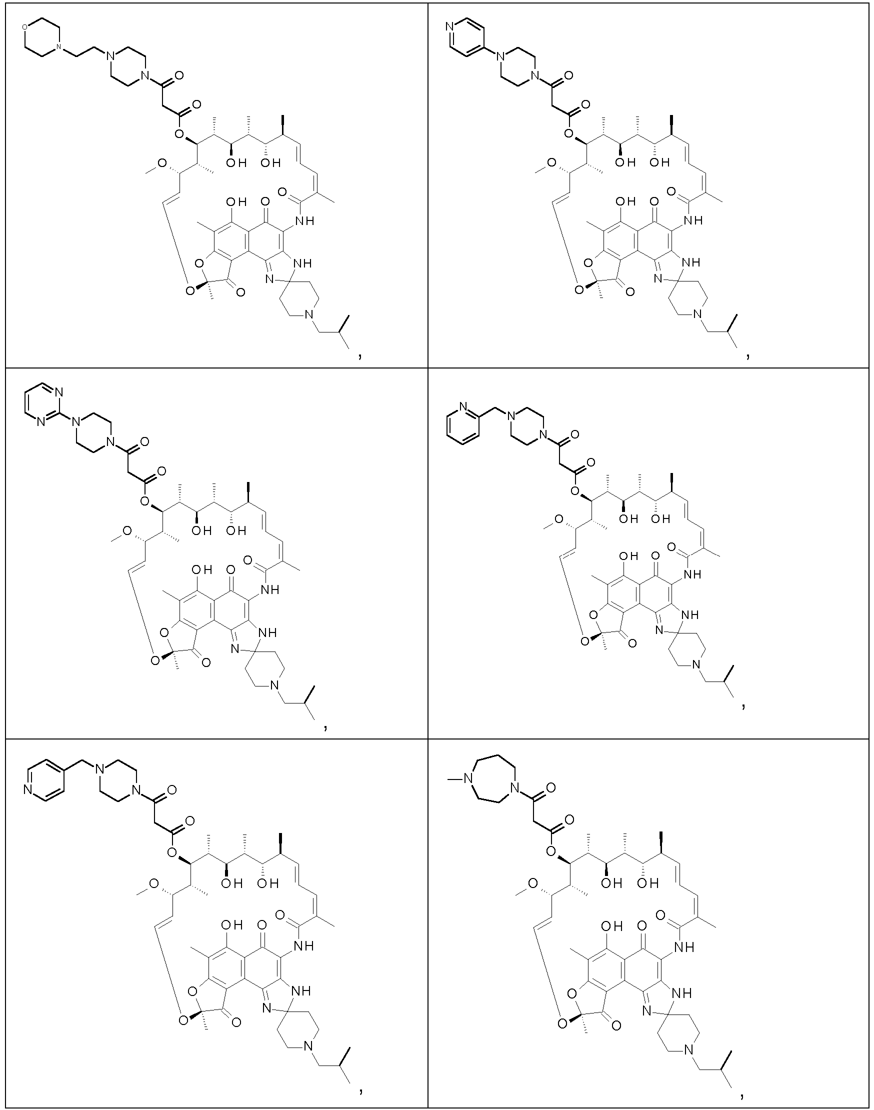

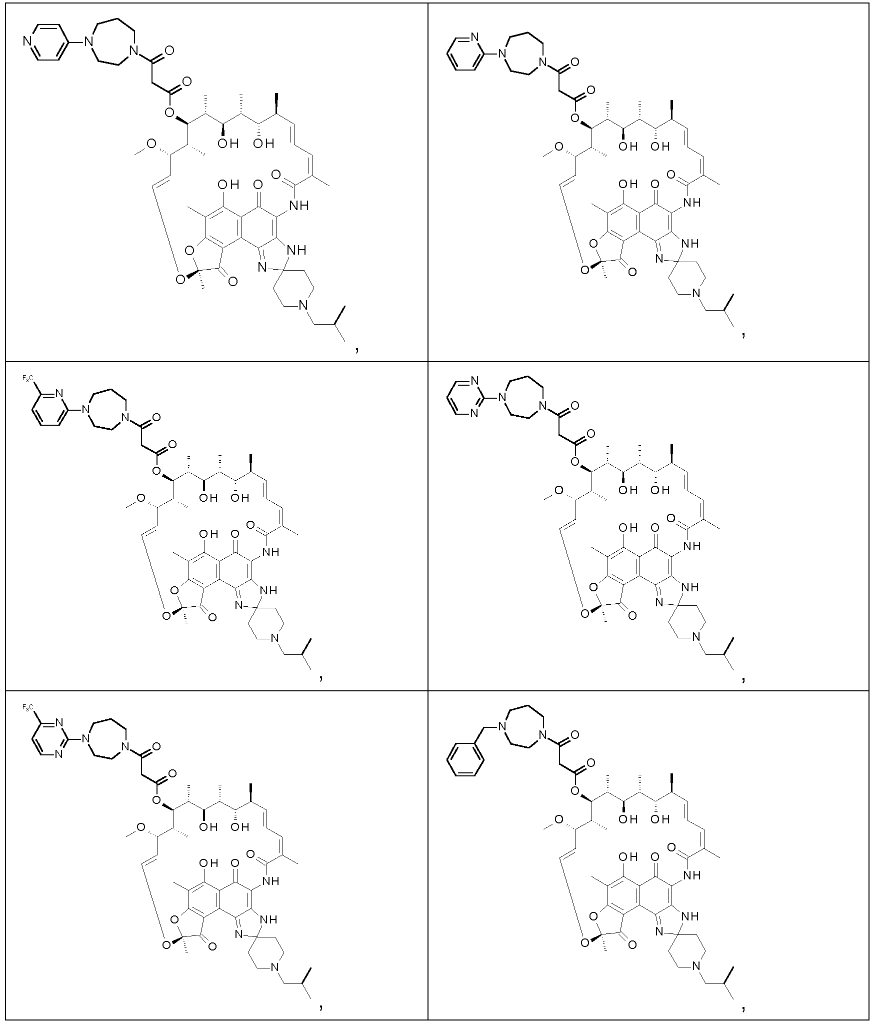

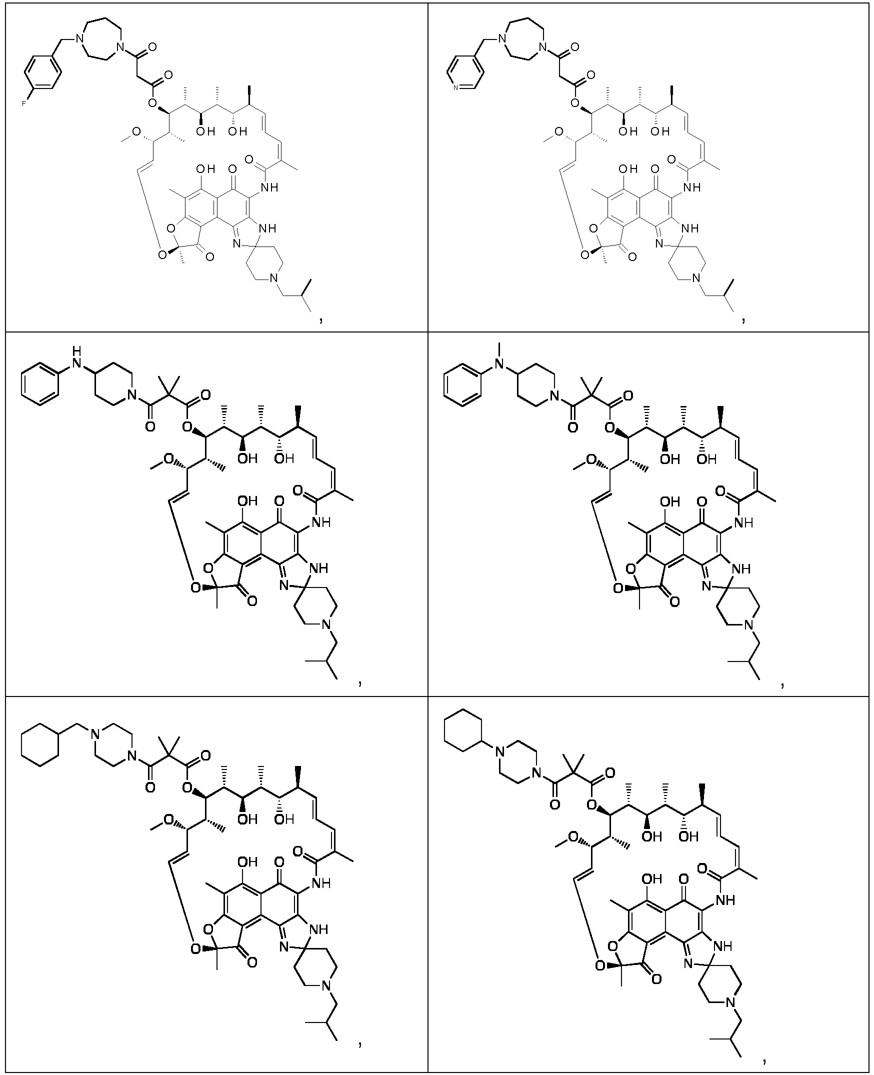

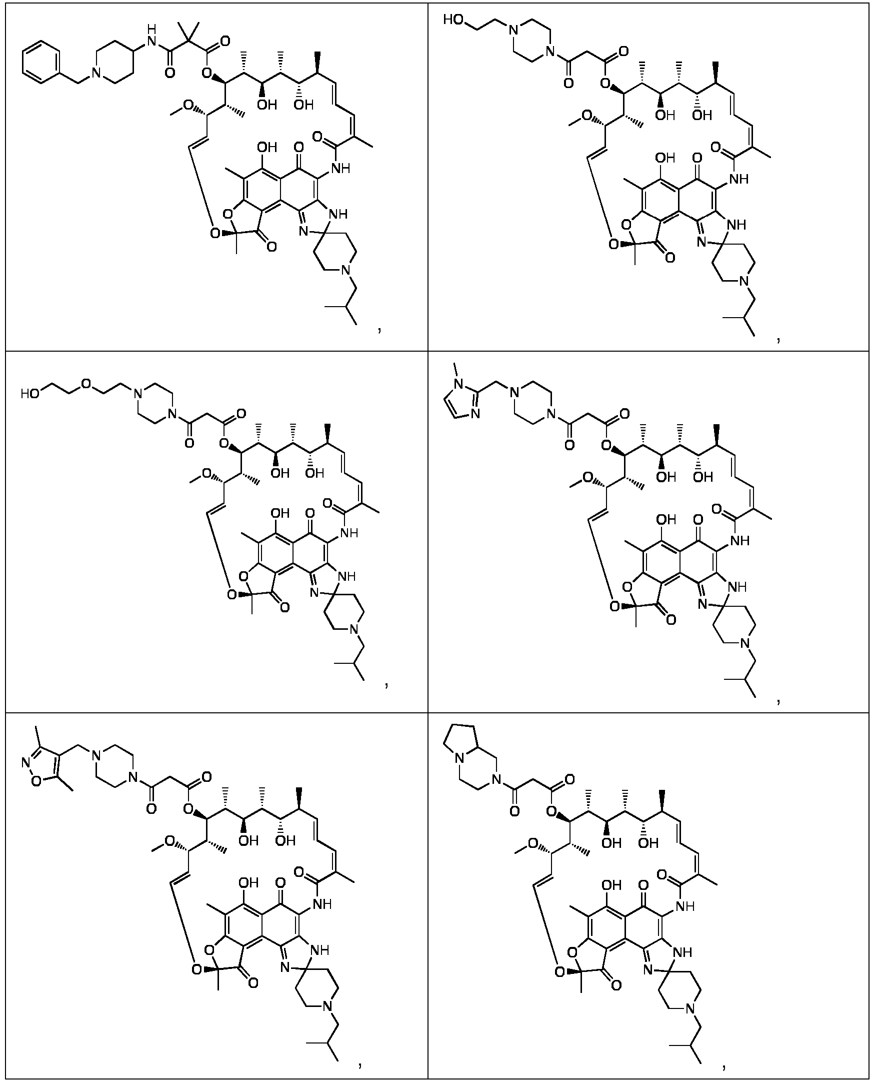

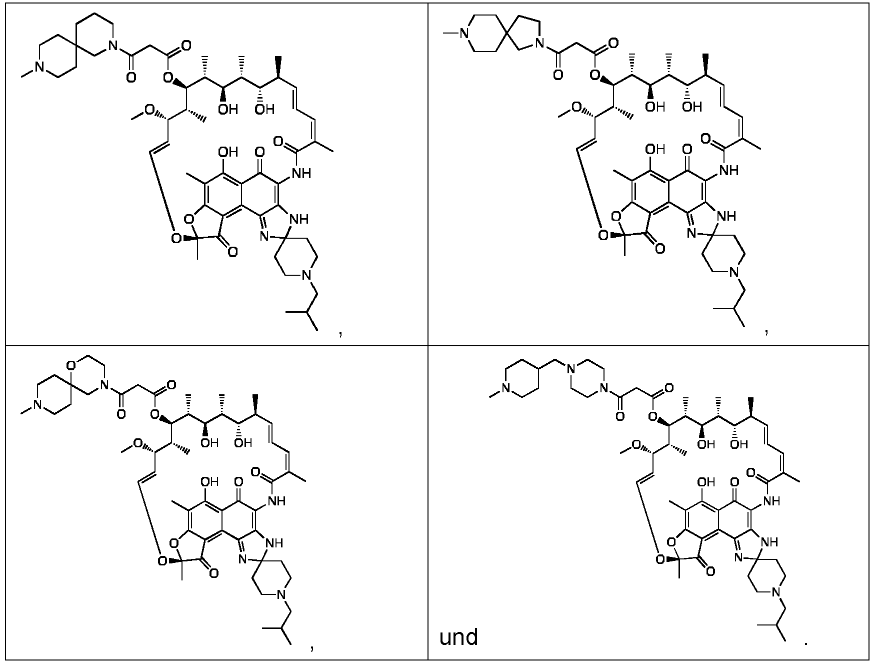

14. Pharmazeutische Zusammensetzung, umfassend mindestens eine Verbindung nach einem der vorstehenden Ansprüche oder ein pharmazeutisch verträgliches Salz, Tautomer, Solvat oder Hydrat davon und einen pharmazeutisch verträglichen Arzneistoffträger.

15. Verbindung nach einem der Ansprüche 1 bis 13 oder ein pharmazeutisch verträgliches Salz, Tautomer, Solvat oder Hydrat davon oder eine pharmazeutische Zusammensetzung nach Anspruch 14 zur Verwendung als ein Arzneimittel, vorzugsweise zur Verwendung in einem Verfahren zum Behandeln einer bakteriellen Infektion.

## Revendications

1. Composé de formule I ou sel, tautomère, solvate, hydrate ou énantiomère pharmaceutiquement acceptable de celui-ci : 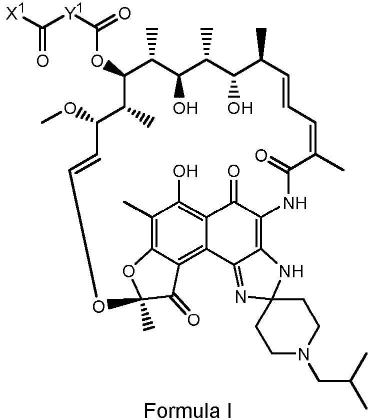 dans lequel :
Y¹ est (CR¹R²)ₙ, dans lequel n est un nombre entier de 1 à 6 et R¹ et R² sont indépendamment, à chaque occurrence, choisis dans le groupe constitué d'hydrogène, -C₁-C₆ alkyle, et -C₁-C₆ alkoxy, dans lequel chaque alkyle est éventuellement substitué par un ou plusieurs halogènes ou -C₃-C₆ cycloalkyle, et dans lequel chaque cycloalkyle est en outre éventuellement substitué par un ou plusieurs halogènes ;
X¹ est -OR¹¹ ou -NR⁵¹R⁵² ;
R¹¹ est indépendamment -C₁-C₆ alkyle, -C₁-C₆ alkylène-R^{12A}, -C₂-C₆ alcényle, -C₂-C₆ alcynyle, -C₃-C₆ cycloalkyle, -C₅-C₈ cycloalcényle, hétérocycloalkyle de 3 à 10 chaînons, -C₆-C₁₀ aryle, ou hétéroaryle de 5 à 10 chaînons, dans lequel chaque alkyle, alkylène, alcényle, alcynyle, cycloalkyle, cycloalcényle, hétérocycloalkyle, aryle ou hétéroaryle est éventuellement substitué par un ou plusieurs R¹² ;
R¹² et R^{12A} sont chacun indépendamment -C₁-C₆ alkyle, -C₁-C₆ alkylène-R^{16A}, -C₂-C₆ alcényle, -C₂-C₆ alcynyle, -C₃-C₆ cycloalkyle, -C₅-C₈ cycloalcényle, hétérocycloalkyle de 3 à 10 chaînons, -C₆-C₁₀ aryle, hétéroaryle de 5 à 10 chaînons, -C₁-C₆ alkoxy, -C₁-C₆ haloalkyle, halogène, cyano, oxo, -OR¹³, - CO₂R¹³ ou -NR¹⁴R¹⁵, dans lequel chaque alkyle, alkylène, alcényle, alcynyle, cycloalkyle, cycloalcényle, hétérocycloalkyle, aryle, hétéroaryle et alkoxy est éventuellement substitué par un ou plusieurs R¹⁶ ;
R¹³, R¹⁴ et R¹⁵ sont chacun indépendamment hydrogène, -C₁-C₆ alkyle, - C₁-C₆ alkylène-R^{20A}, -C₂-C₆ alcényle, -C₂-C₆ alcynyle, -C₃-C₆ cycloalkyle, -C₅-C₈ cycloalcényle, hétérocycloalkyle de 3 à 10 chaînons, -C₆-C₁₀ aryle, hétéroaryle de 5 à 10 chaînons, -C₁-C₆ alkoxy, -C₁-C₆ haloalkyle, -S(O)₂(C₁-C₆ alkyle) ou - S(O)₂NR¹⁸R¹⁹, dans lequel chaque alkyle, alkylène, alcényle, alcynyle, cycloalkyle, cycloalcényle, hétérocycloalkyle, aryle, hétéroaryle et alkoxy est éventuellement substitué par un ou plusieurs R²⁰ ;
R¹⁶ et R^{16A} sont chacun indépendamment -C₁-C₆ alkyle, -C₁-C₆ alkylène-R^{20A}, -C₂-C₆ alcényle, -C₂-C₆ alcynyle, -C₃-C₆ cycloalkyle, -C₅-C₈ cycloalcényle, hétérocycloalkyle de 3 à 10 chaînons, -C₆-C₁₀ aryle, hétéroaryle de 5 à 10 chaînons, -C₁-C₆ alkoxy, -C₁-C₆ haloalkyle, halogène, cyano, oxo, -OR¹⁷, - S(O)₂(C₁-C₆ alkyle) ou -S(O)₂NR¹⁸R¹⁹, dans lequel chaque alkyle, alkylène, alcényle, alcynyle, cycloalkyle, cycloalcényle, hétérocycloalkyle, aryle, hétéroaryle et alkoxy est éventuellement substitué par un ou plusieurs R²⁰ ;
R¹⁷, R¹⁸ et R¹⁹ sont chacun indépendamment hydrogène, halogène, -OH, - C₁-C₆ alkyle, -C₁-C₆ alkylène-R^{23A}, -C₂-C₆ alcényle, -C₂-C₆ alcynyle, -C₃-C₆ cycloalkyle, -C₅-C₈ cycloalcényle, hétérocycloalkyle de 3 à 10 chaînons, -C₆-C₁₀ aryle, hétéroaryle de 5 à 10 chaînons, -C₁-C₆ alkoxy, -C₁-C₆ haloalkyle, -S(O)₂(C₁-C₆ alkyle) ou -S(O)₂NR²¹R²², dans lequel chaque alkyle, alkylène, alcényle, alcynyle, cycloalkyle, cycloalcényle, hétérocycloalkyle, aryle, hétéroaryle et alkoxy est éventuellement substitué par un ou plusieurs R²³ ;
R²⁰ et R^{20A} sont chacun indépendamment halogène, -OH, -C₁-C₆ alkyle, - C₁-C₆ alkylène-R^{23A}, -C₂-C₆ alcényle, -C₂-C₆ alcynyle, -C₃-C₆ cycloalkyle, -C₅-C₈ cycloalcényle, hétérocycloalkyle de 3 à 10 chaînons, -C₆-C₁₀ aryle, hétéroaryle de 5 à 10 chaînons, -C₁-C₆ alkoxy, -C₁-C₆ haloalkyle, halogène, cyano, oxo, - S(O)₂(C₁-C₆ alkyle) ou -S(O)₂NR²¹R²², dans lequel chaque alkyle, alkylène, alcényle, alcynyle, cycloalkyle, cycloalcényle, hétérocycloalkyle, aryle, hétéroaryle et alkoxy est éventuellement substitué par un ou plusieurs R²³ ;
R²¹ et R²² sont chacun indépendamment hydrogène ou -C₁-C₆ alkyle ; et
R²³ et R^{23A} sont chacun indépendamment -C₁-C₆ alkyle, -C₁-C₆ alkoxy, - C₁-C₆ haloalkyle, halogène ou cyano ;
R⁵¹ et R⁵² sont chacun indépendamment hydrogène, -C₁-C₆ alkyle, -C₁-C₆ alkylène-R^{53A}, -C₂-C₆ alcényle, -C₂-C₆ alcynyle, -C₃-C₆ cycloalkyle, -C₅-C₈ cycloalcényle, hétérocycloalkyle de 3 à 10 chaînons, -C₆-C₁₀ aryle, hétéroaryle de 5 à 10 chaînons, dans lequel chaque alkyle, alkylène, alcényle, alcynyle, cycloalkyle, cycloalcényle, hétérocycloalkyle, aryle ou hétéroaryle est éventuellement substitué par un ou plusieurs R⁵³ ; ou R⁵¹ et R⁵², ainsi que l'atome d'azote auquel ils sont attachés, se combinent pour former un hétérocycloalkyle de 3 à 15 chaînons ou un hétéroaryle de 5 à 15 chaînons, dans lequel l'hétérocycloalkyle ou l'hétéroaryle sont éventuellement substitués par un ou plusieurs R⁵³ ;
R⁵³ et R^{53A} sont chacun indépendamment -C₁-C₆ alkyle, -C₁-C₆ alkylène-R^{57A}, -C₂-C₆ alcényle, -C₂-C₆ alcynyle, -C₃-C₆ cycloalkyle, -C₅-C₈ cycloalcényle, hétérocycloalkyle de 3 à 10 chaînons, -C₆-C₁₀ aryle, hétéroaryle de 5 à 10 chaînons, -C₁-C₆ alkoxy, -C₁-C₆ haloalkyle, halogène, cyano, oxo, -OR⁵⁴, - C(O)R⁵⁴, -CO₂R⁵⁴ ou -NR⁵⁵R⁵⁶, dans lequel chaque alkyle, alkylène, alcényle, alcynyle, cycloalkyle, cycloalcényle, hétérocycloalkyle, aryle, hétéroaryle et alkoxy est éventuellement substitué par un ou plusieurs R⁵⁷ ;
R⁵⁴, R⁵⁵ et R⁵⁶ sont chacun indépendamment hydrogène, -C₁-C₆ alkyle, - C₁-C₆ alkylène-R^{61A}, -C₂-C₆ alcényle, -C₂-C₆ alcynyle, -C₃-C₆ cycloalkyle, -C₅-C₈ cycloalcényle, hétérocycloalkyle de 3 à 10 chaînons, -C₆-C₁₀ aryle, hétéroaryle de 5 à 10 chaînons, -C₁-C₆ alkoxy, -C₁-C₆ haloalkyle, -CO₂R⁵⁸, -S(O)₂(C₁-C₆ alkyle), - S(O)₂(C₆-C₁₀ aryle) ou -S(O)₂NR⁵⁹R⁶⁰, dans lequel chaque alkyle, alkylène, alcényle, alcynyle, cycloalkyle, cycloalcényle, hétérocycloalkyle, aryle, hétéroaryle et alkoxy est éventuellement substitué par un ou plusieurs R⁶¹ ;
R⁵⁷ et R^{57A} sont chacun indépendamment -C₁-C₆ alkyle, -C₁-C₆ alkylène-R^{61A}, -C₂-C₆ alcényle, -C₂-C₆ alcynyle, -C₃-C₆ cycloalkyle, -C₅-C₈ cycloalcényle, hétérocycloalkyle de 3 à 10 chaînons, -C₆-C₁₀ aryle, hétéroaryle de 5 à 10 chaînons, -C₁-C₆ alkoxy, -C₁-C₆ haloalkyle, halogène, cyano, oxo, -OR⁵⁸, NR⁵⁶R⁶⁰, -S(O)₂(C₁-C₆ alkyle) ou -S(O)₂NR⁵⁹R⁶⁰, dans lequel chaque alkyle, alkylène, alcényle, alcynyle, cycloalkyle, cycloalcényle, hétérocycloalkyle, aryle, hétéroaryle et alkoxy est éventuellement substitué par un ou plusieurs R⁶¹ ;
R⁵⁸, R⁵⁹ et R⁶⁰ sont chacun indépendamment hydrogène, -C₁-C₆ alkyle, - C₁-C₆ alkylène-R⁶⁴, -C₂-C₆ alcényle, -C₂-C₆ alcynyle, -C₃-C₆ cycloalkyle, -C₅-C₈ cycloalcényle, hétérocycloalkyle de 3 à 10 chaînons, -C₆-C₁₀ aryle, hétéroaryle de 5 à 10 chaînons, -C₁-C₆ alkoxy, -C₁-C₆ haloalkyle, -S(O)₂(C₁-C₆ alkyle) ou - S(O)₂NR⁶²R⁶³, dans lequel chaque alkyle, alkylène, alcényle, alcynyle, cycloalkyle, cycloalcényle, hétérocycloalkyle, aryle, hétéroaryle et alkoxy est éventuellement substitué par un ou plusieurs R⁶⁴ ;
R⁶¹ et R^{61A} sont chacun indépendamment halogène, -OH, -C₁-C₆ alkyle, - C₁-C₆ alkylène-R^{64A}, -C₂-C₆ alcényle, -C₂-C₆ alcynyle, -C₃-C₆ cycloalkyle, -C₅-C₈ cycloalcényle, hétérocycloalkyle de 3 à 10 chaînons, -C₆-C₁₀ aryle, hétéroaryle de 5 à 10 chaînons, -C₁-C₆ alkoxy, -C₁-C₆ haloalkyle, halogène, cyano, oxo, - S(O)₂(C₁-C₆ alkyle) ou -S(O)₂NR⁶²R⁶³, dans lequel chaque alkyle, alkylène, alcényle, alcynyle, cycloalkyle, cycloalcényle, hétérocycloalkyle, aryle, hétéroaryle et alkoxy est éventuellement substitué par un ou plusieurs R⁶⁴ ; et
R⁶² et R⁶³ sont chacun indépendamment hydrogène et -C₁-C₆ alkyle ; et
R⁶⁴ et R^{64A} sont chacun indépendamment -C₁-C₆ alkyle, -C₁-C₆ alkoxy, - C₁-C₆ haloalkyle, phényle, halogène ou cyano.

2. Composé selon la revendication 1, dans lequel le composé est de formule I-A : 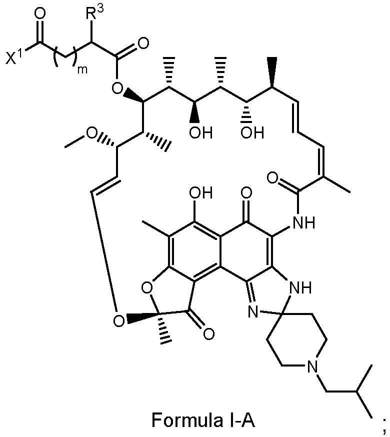 dans lequel
m est un nombre entier entre 0 et 4 ; et
R³ est choisi parmi -H, méthyle et éthyle, et dans lequel de préférence le composé est de formule I-B, formule I-C, formule I-D, formule I-E, formule I-F, ou formule I-G :

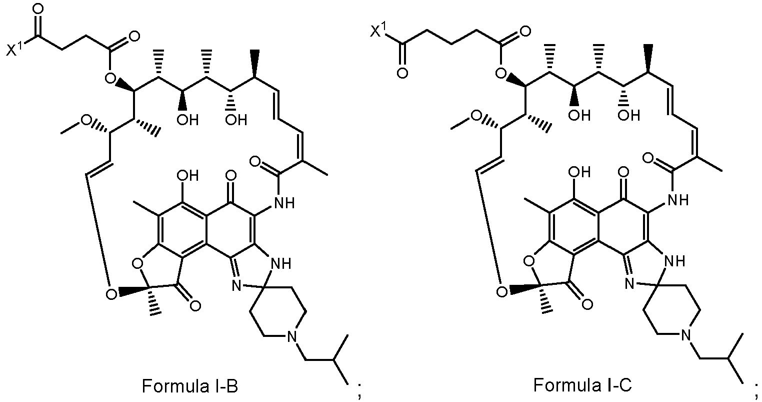

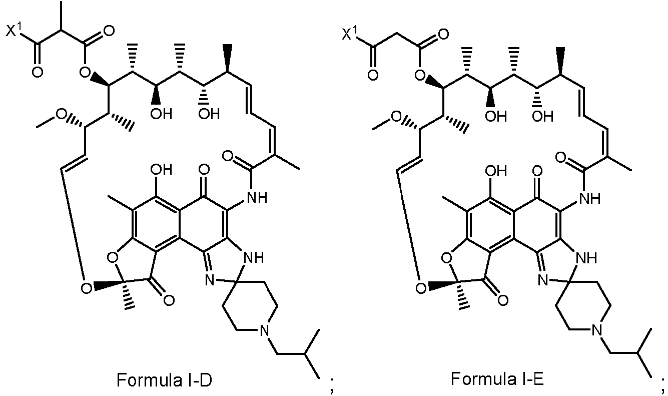

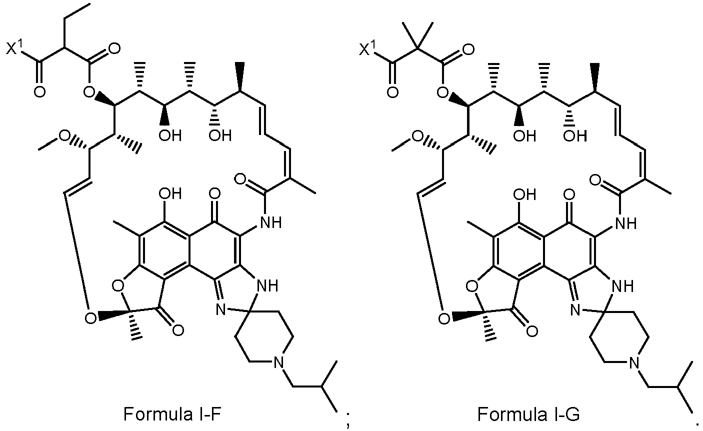

3. Composé selon la revendication 1 ou la revendication 2, dans lequel X¹ est -OR¹¹.

4. Composé selon la revendication 1 ou la revendication 2, dans lequel X¹ est -NR⁵¹NR⁵².

5. Composé selon la revendication 4, dans lequel R⁵¹ et R⁵² sont chacun indépendamment hydrogène, -C₁-C₆ alkyle, -C₁-C₆ alkylène-R^{53A}, -C₅-C₆ cycloalkyle, hétérocycloalkyle de 5 à 6 chaînons ou phényle, dans lequel de préférence au moins l'un parmi R⁵¹ et R⁵² est hydrogène, dans lequel chaque alkyle, alkylène, cycloalkyle, hétérocycloalkyle ou phényle est éventuellement substitué par un ou plusieurs, de préférence un ou deux, R⁵³ ; ou R⁵¹ et R⁵², ainsi que l'atome d'azote auquel ils sont attachés, se combinent pour former un hétérocycloalkyle de 3 à 15 chaînons, dans lequel ledit hétérocycloalkyle est éventuellement substitué par un ou plusieurs, de préférence un ou deux, R⁵³.

6. Composé selon la revendication 4, dans lequel R⁵¹ et R⁵², ainsi que l'atome d'azote auquel ils sont attachés, se combinent pour former un hétérocycloalkyle de 3 à 15 chaînons, dans lequel ledit hétérocycloalkyle est éventuellement substitué par un ou plusieurs, de préférence un ou deux, R⁵³ ; ou dans lequel ledit hétérocycle est fusionné à un ou plusieurs cycles aryle ou hétéroaryle supplémentaires pour former un hétéroaryle de 10 à 15 chaînons, dans lequel ledit hétéroaryle de 10 à 15 chaînons est éventuellement substitué par un ou plusieurs, de préférence un ou deux, R⁵³ ; ou dans lequel ledit hétérocycle est lié au niveau d'un seul atome à un autre cycloalkyle ou hétérocycloalkyle pour former un hétérocycle spirocyclique de 10 à 15 chaînons ; et dans lequel ledit spirocyclique est éventuellement substitué par un ou plusieurs, de préférence un ou deux, R⁵³.

7. Composé selon la revendication 6, dans lequel ledit hétérocycloalkyle de 3 à 15 chaînons est pipéridine, morpholine, 1,4-diazépane, pipérazine ou azétidine.

8. Composé selon la revendication 6, dans lequel ledit hétérocycloalkyle est un pipéridine.

9. Composé selon l'une quelconque des revendications 4 à 8, dans lequel :
R⁵³ et R^{53A} sont indépendamment -C₁-C₄ alkyle, -C₁-C₃ alkylène-R^{57A}, hétérocycloalkyle de 5 à 15 chaînons, hétéroaryle de 5 à 15 chaînons ou phényle,-OR⁵⁴, -C(O)R⁵⁴, -CO₂R⁵⁴, oxo ou -NR⁵⁵R⁵⁶, dans lequel chaque alkylène, hétérocycloalkyle, hétéroaryle et phényle est éventuellement substitué par un ou plusieurs R⁵⁷ ;
dans lequel R⁵⁴ est indépendamment hydrogène, -C₁-C₄ alkyle, -C₅-C₆ cycloalkyle ou phényle ; dans lequel chaque alkyle, cycloalkyle ou phényle est éventuellement substitué par un ou plusieurs R⁶¹ ;
R⁵⁵ et R⁵⁶ sont chacun indépendamment hydrogène, -C₁-C₄ alkyle, -C₁-C₄ alkylène-R^{61A}, phényle, -CO₂C₁-C₆ alkyle, -S(O)₂(phényle), dans lequel le phényle ou le cycloalkyle est éventuellement substitué par un ou plusieurs R⁶¹.

10. Composé selon l'une quelconque des revendications 4 à 9, dans lequel R⁵⁷ et R^{57A} sont indépendamment -C₁-C₆ alkyle, hétérocycloalkyle de 3 à 10 chaînons, phényle, hétéroaryle de 5 à 10 chaînons, oxo, halogène, -OH, ou - SO₂NH₂, dans lequel chaque alkyle, hétérocycloalkyle, phényle, et hétéroaryle est éventuellement substitué par un ou plusieurs R⁶¹.

11. Composé selon l'une quelconque des revendications 4 à 10, dans lequel R⁶¹ et R^{61A} sont indépendamment -C₁-C₂ alkyle, phényle, -C₁-C₂ alkoxy, -C₁-C₂ haloalkyle, ou halogène.

12. Composé selon la revendication 1, choisi X¹ est choisi dans le groupe constitué de :

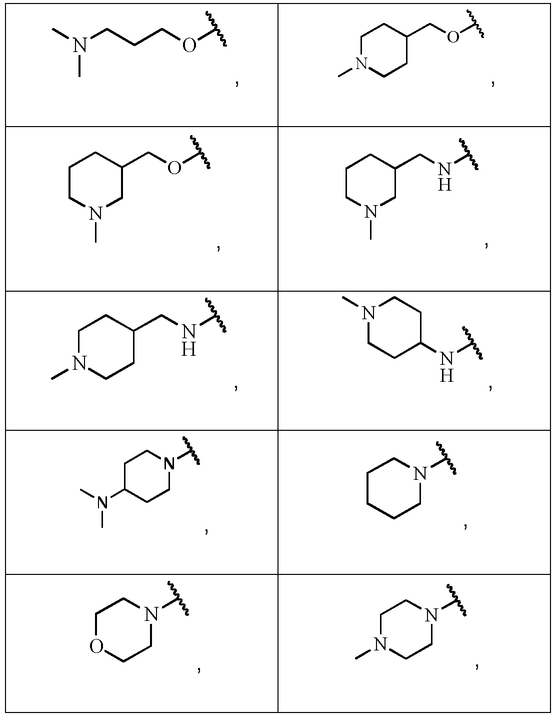

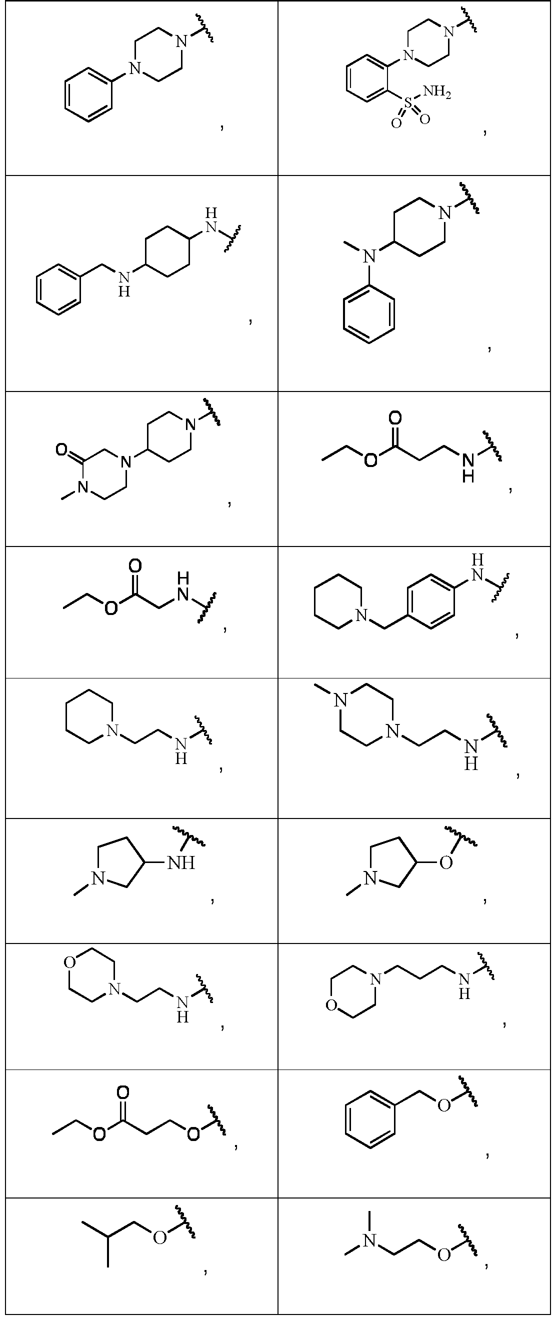

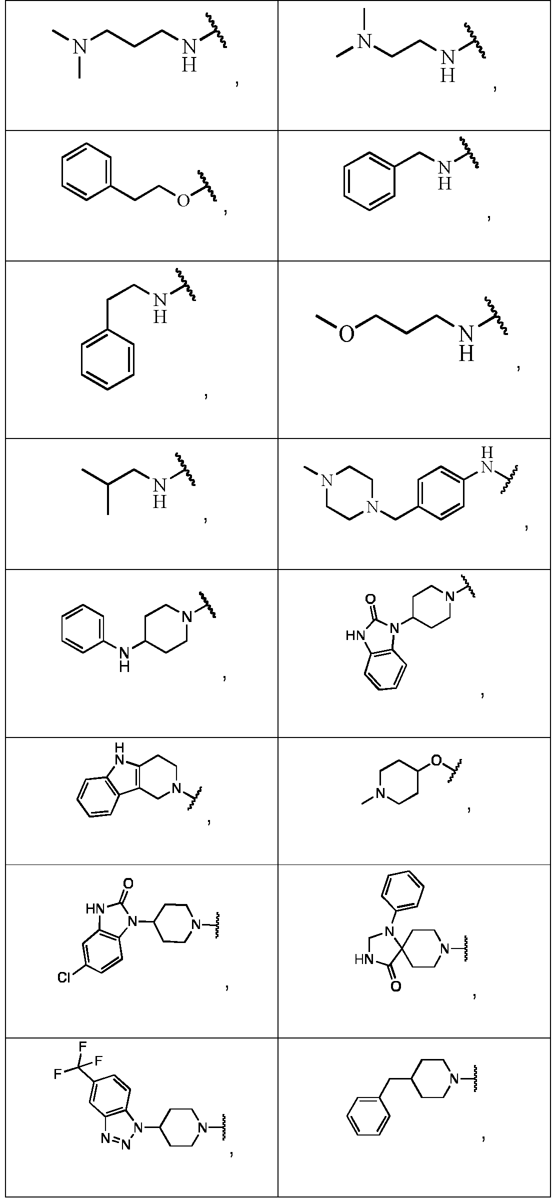

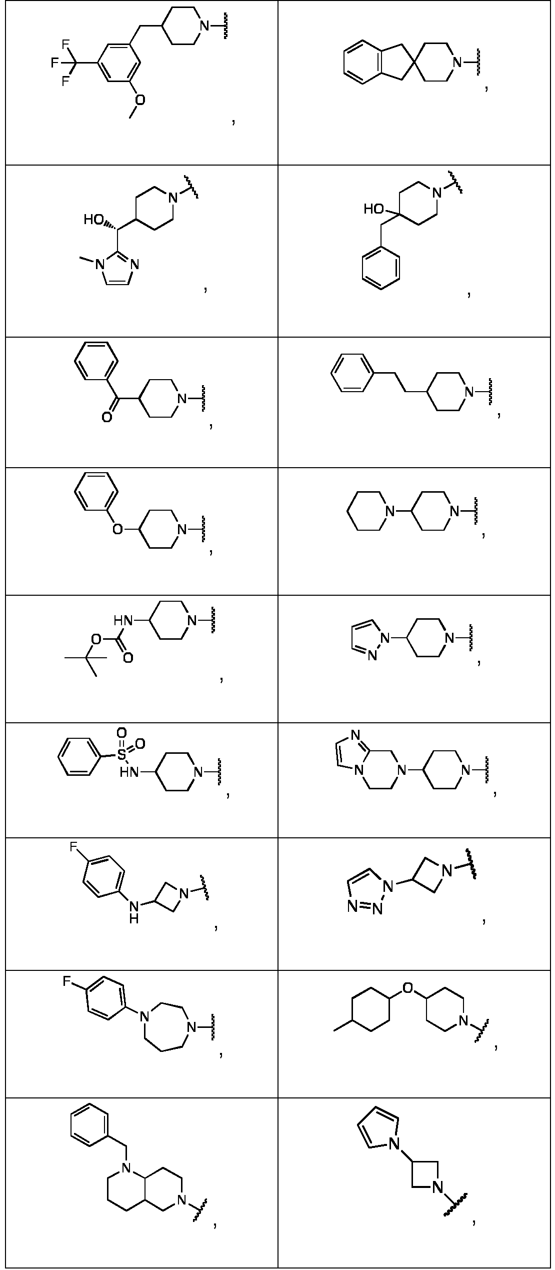

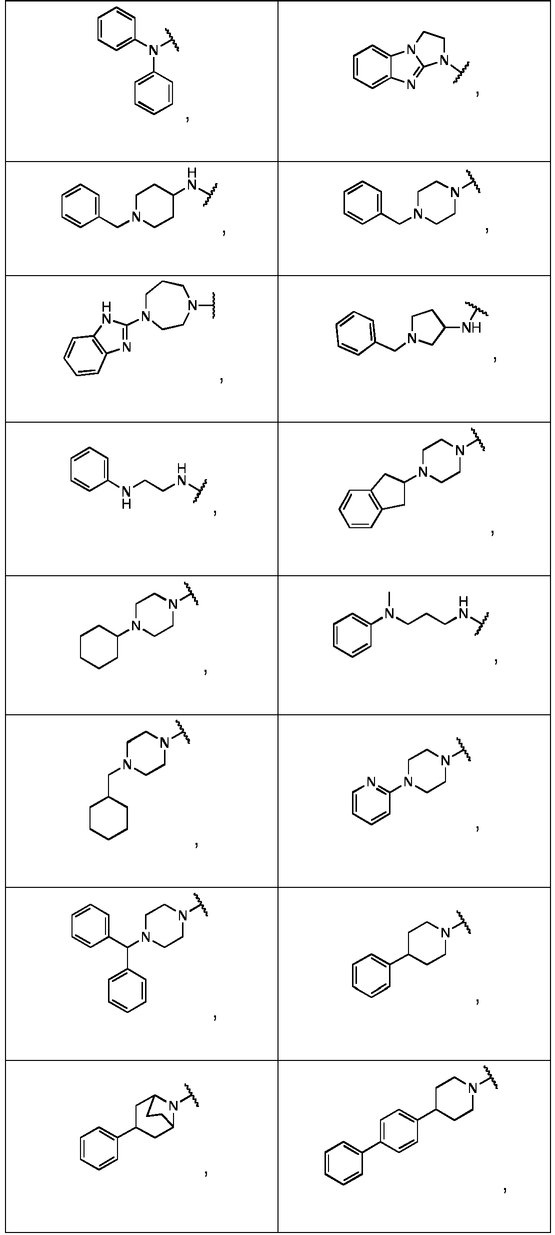

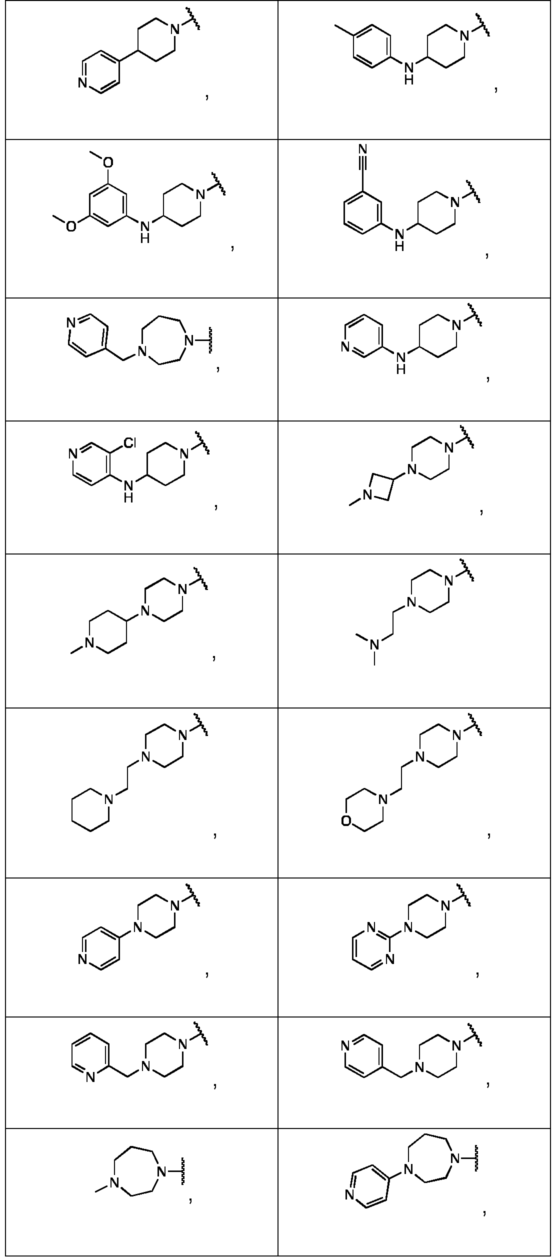

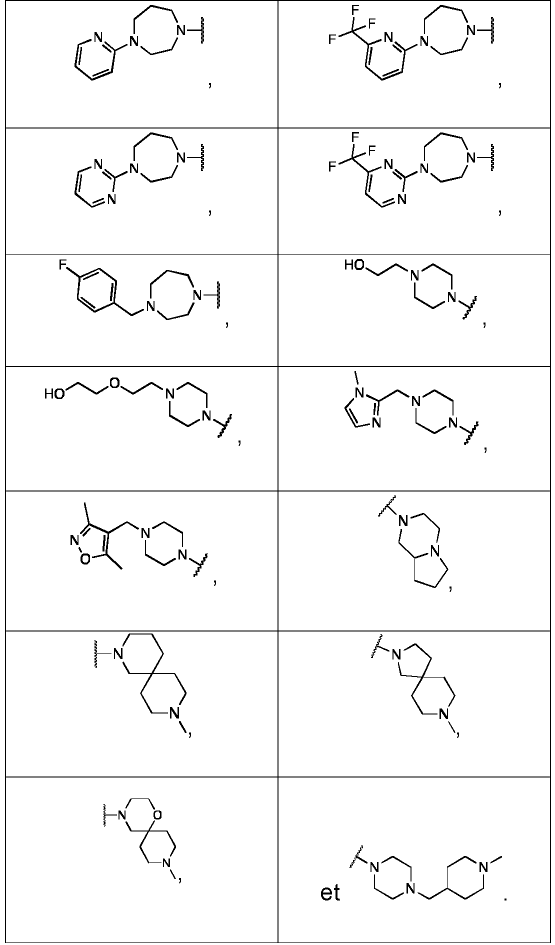

13. Composé selon la revendication 1, dans lequel le composé est choisi dans le groupe constitué de :

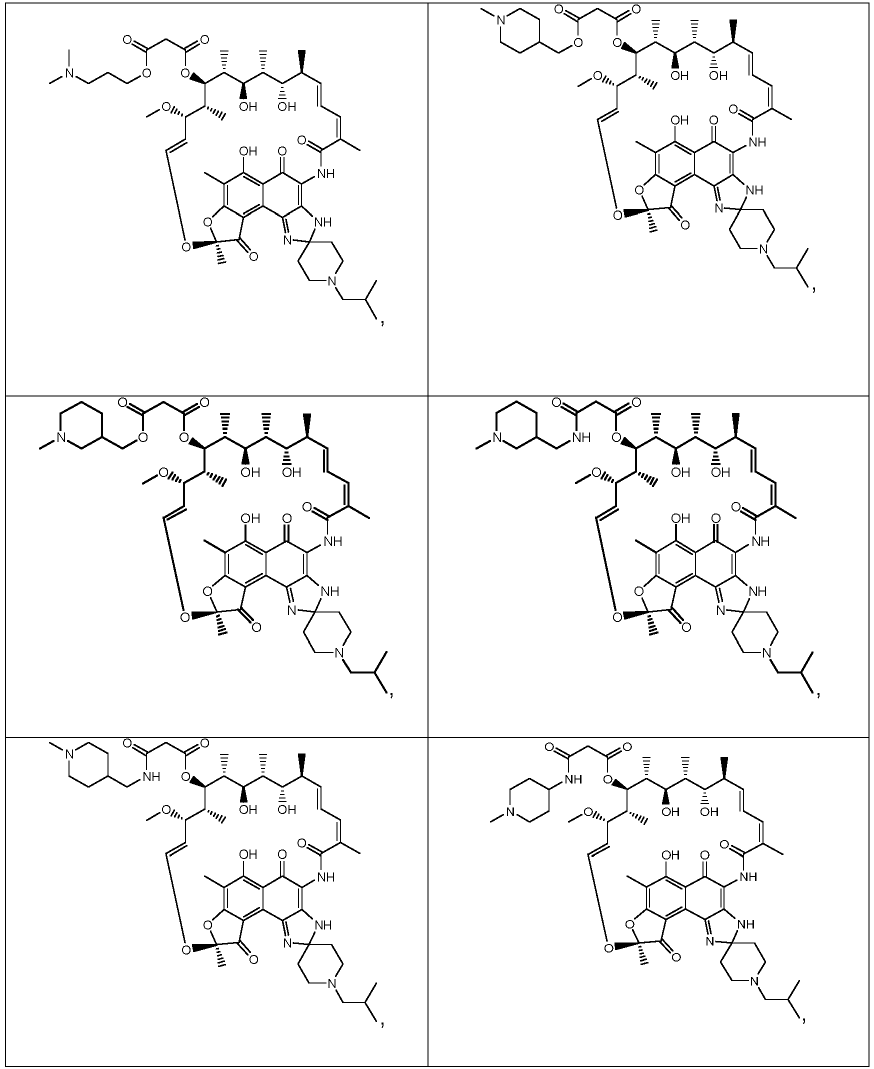

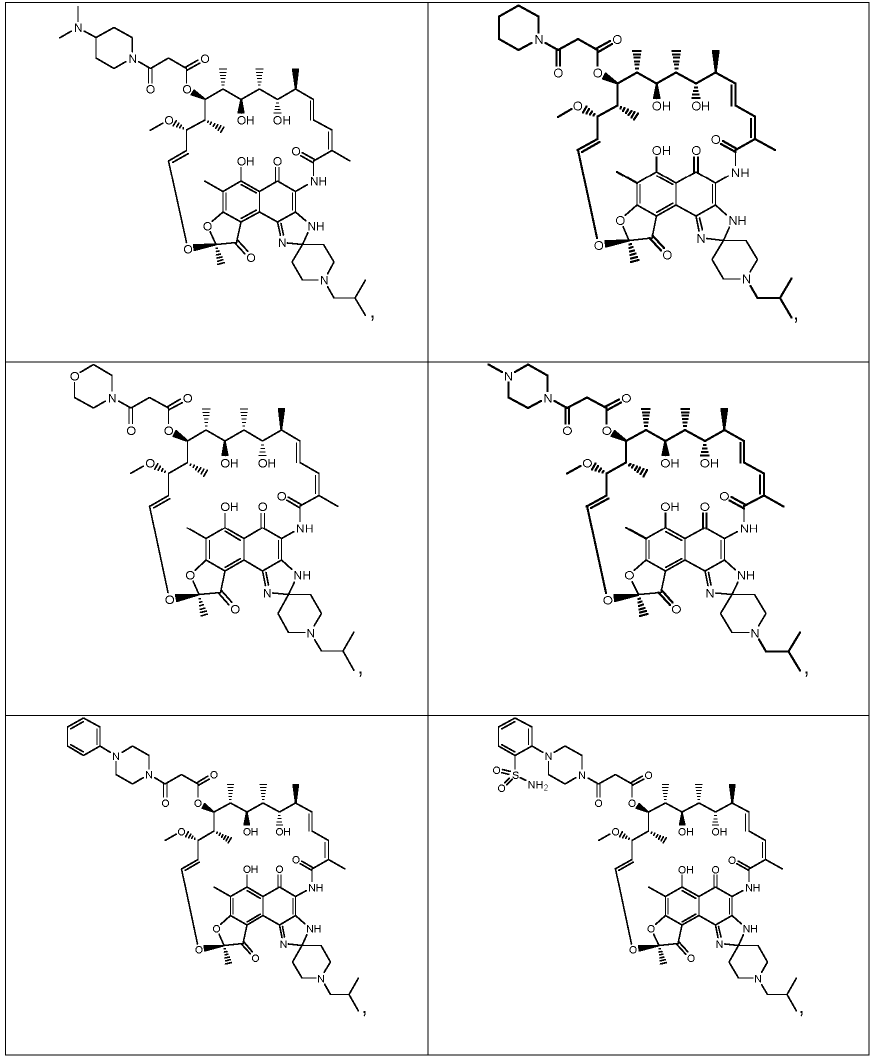

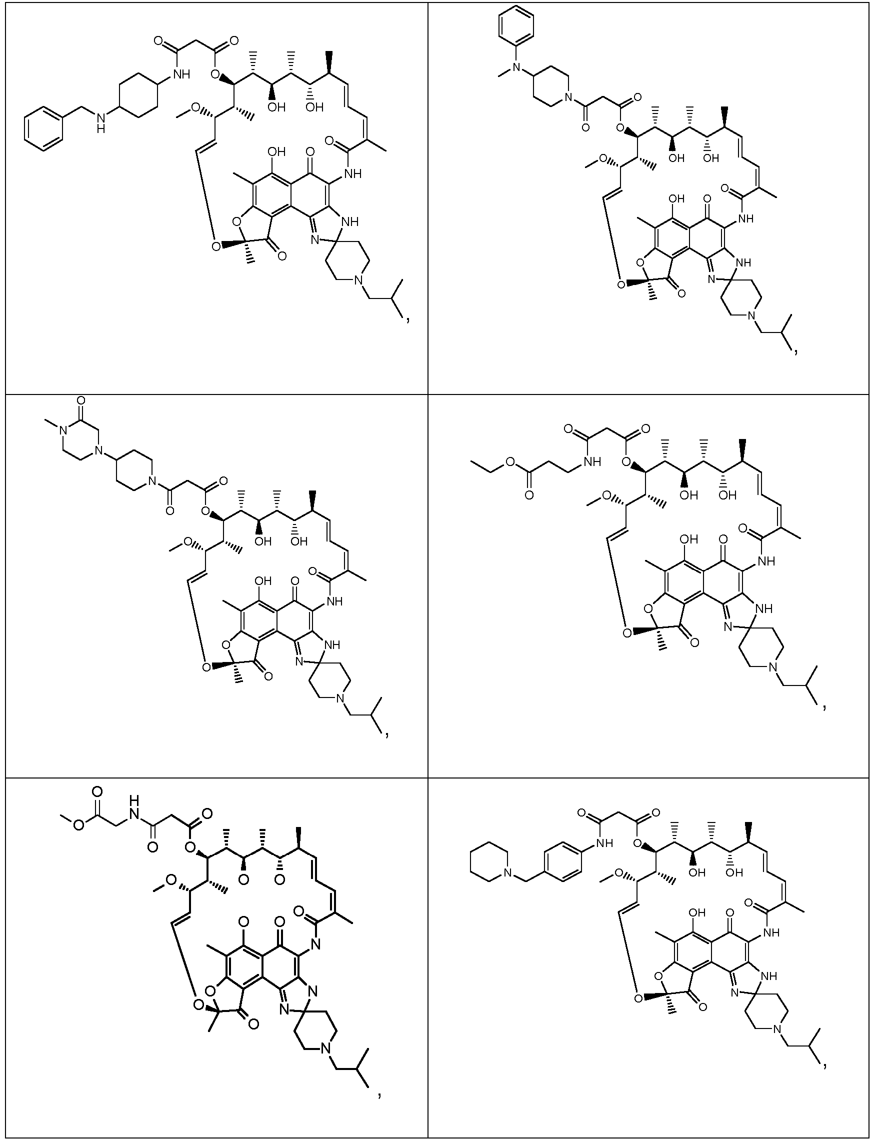

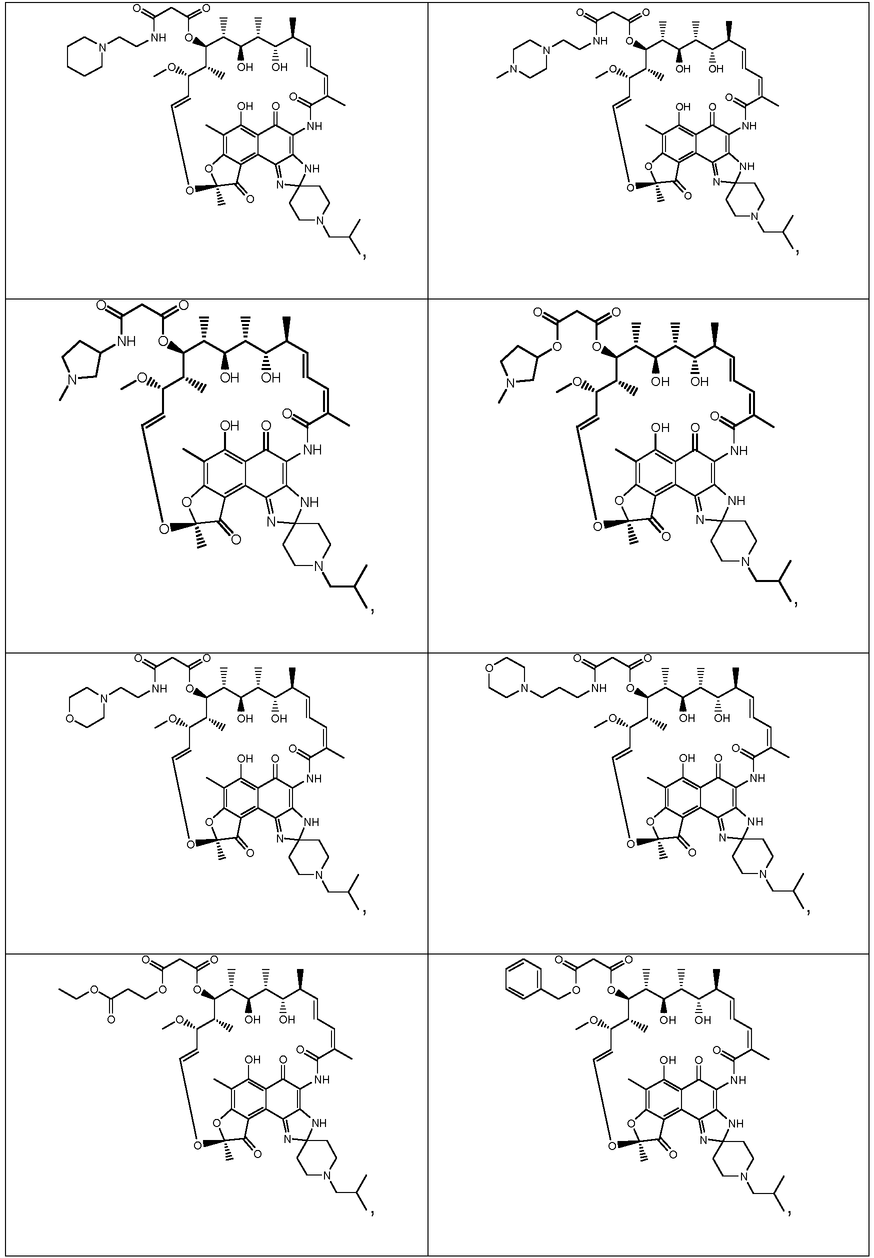

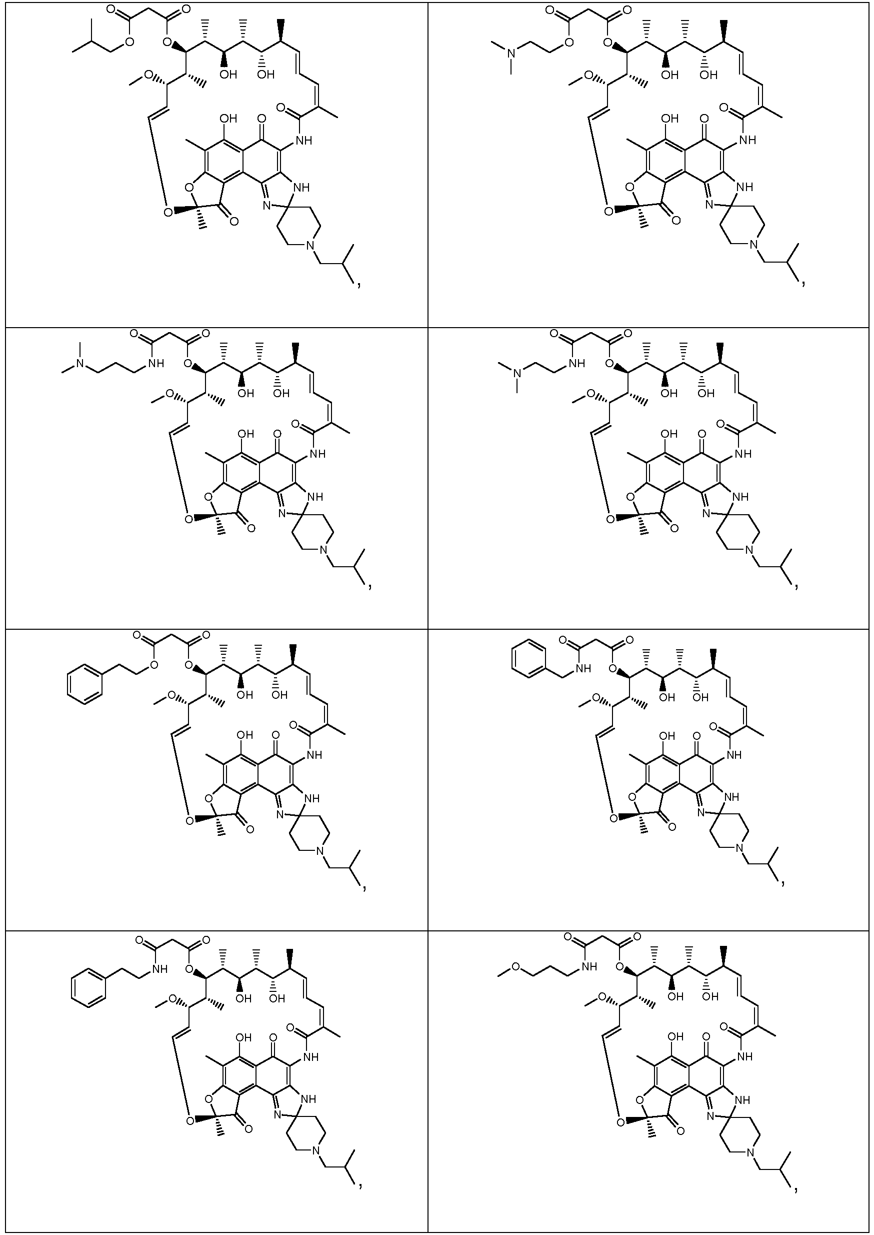

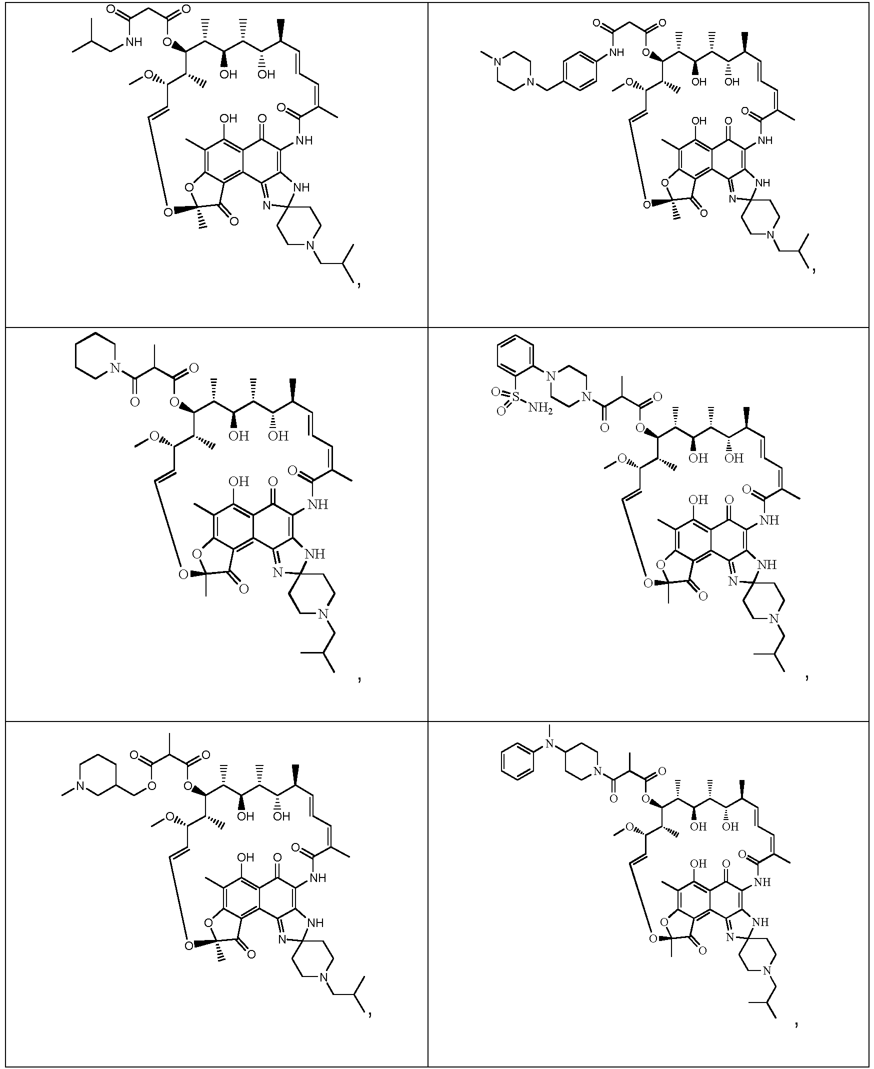

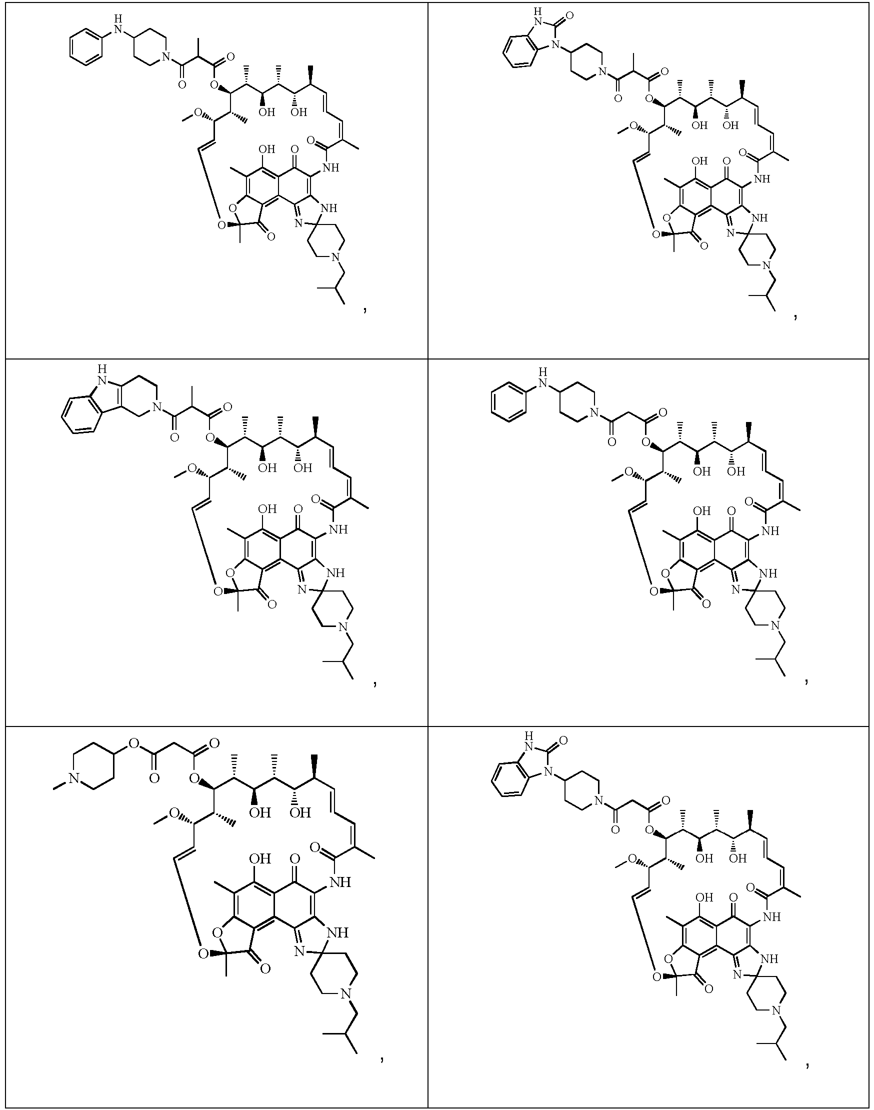

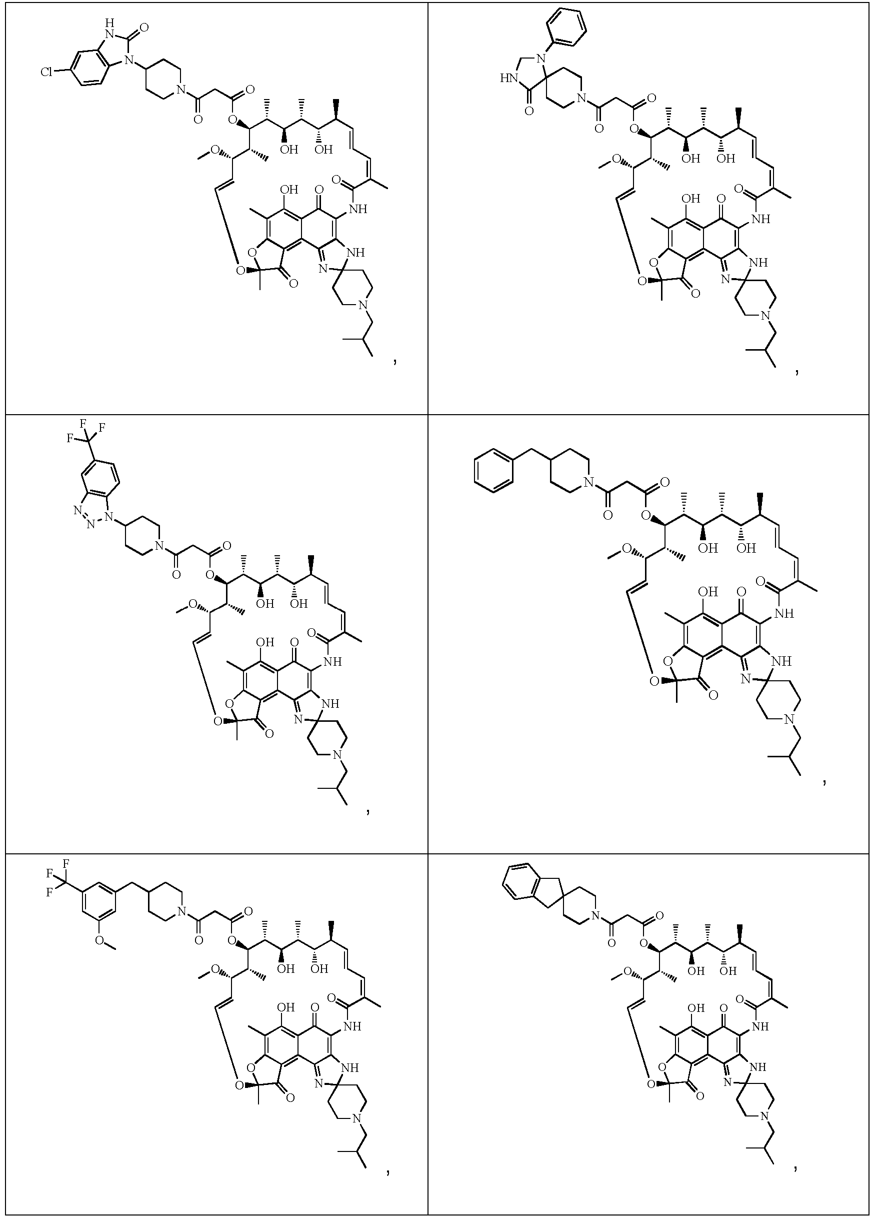

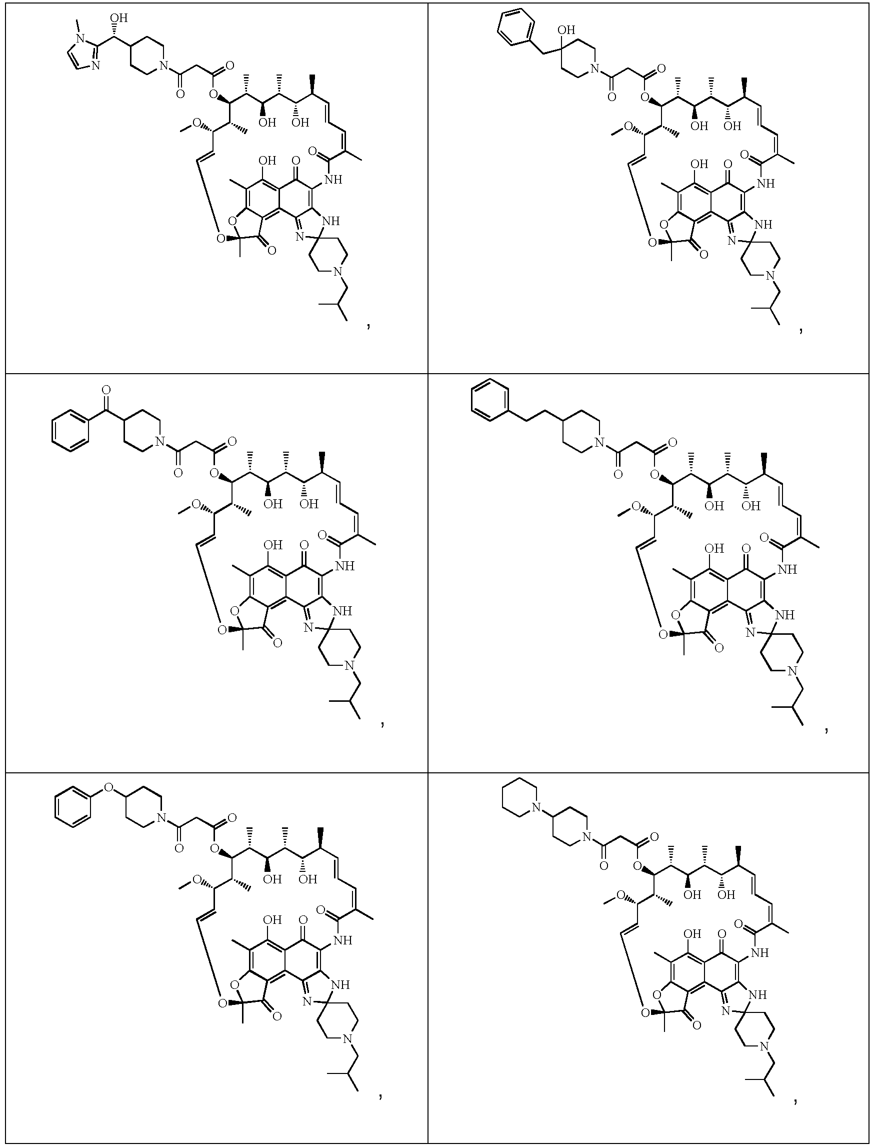

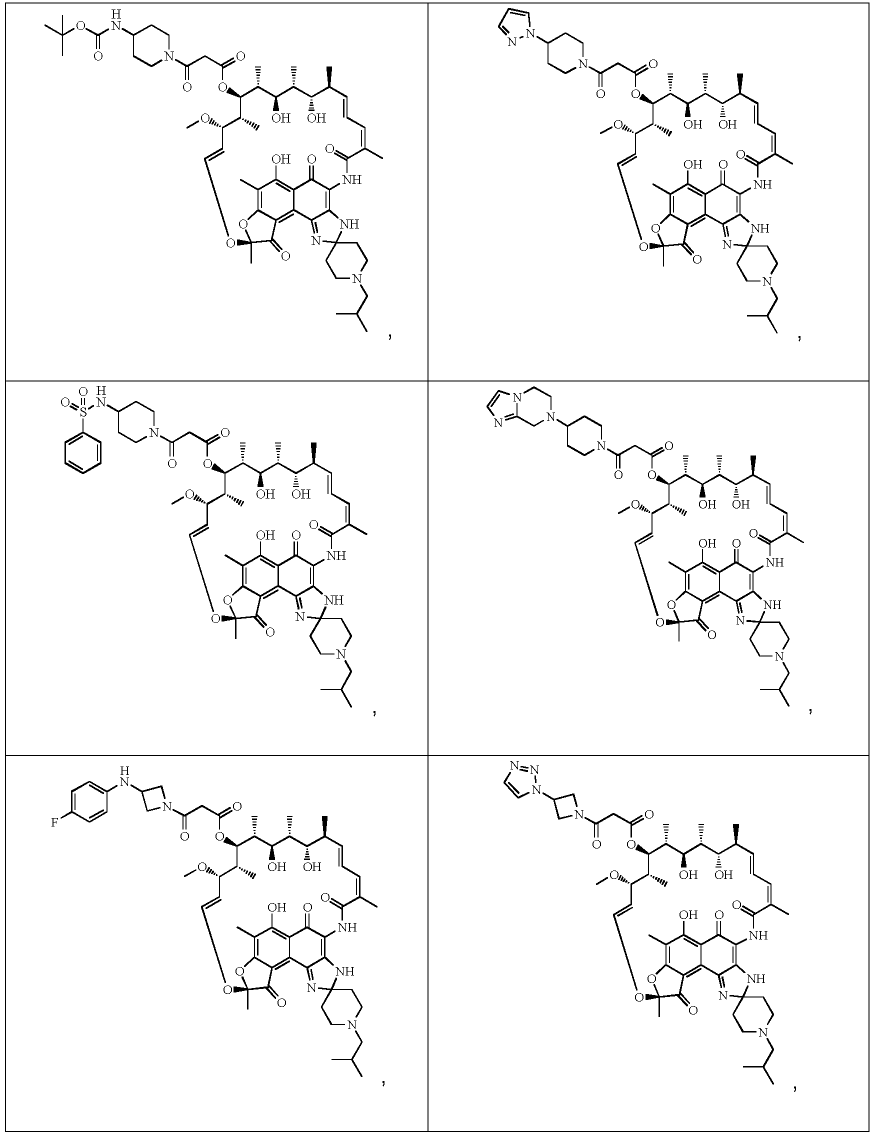

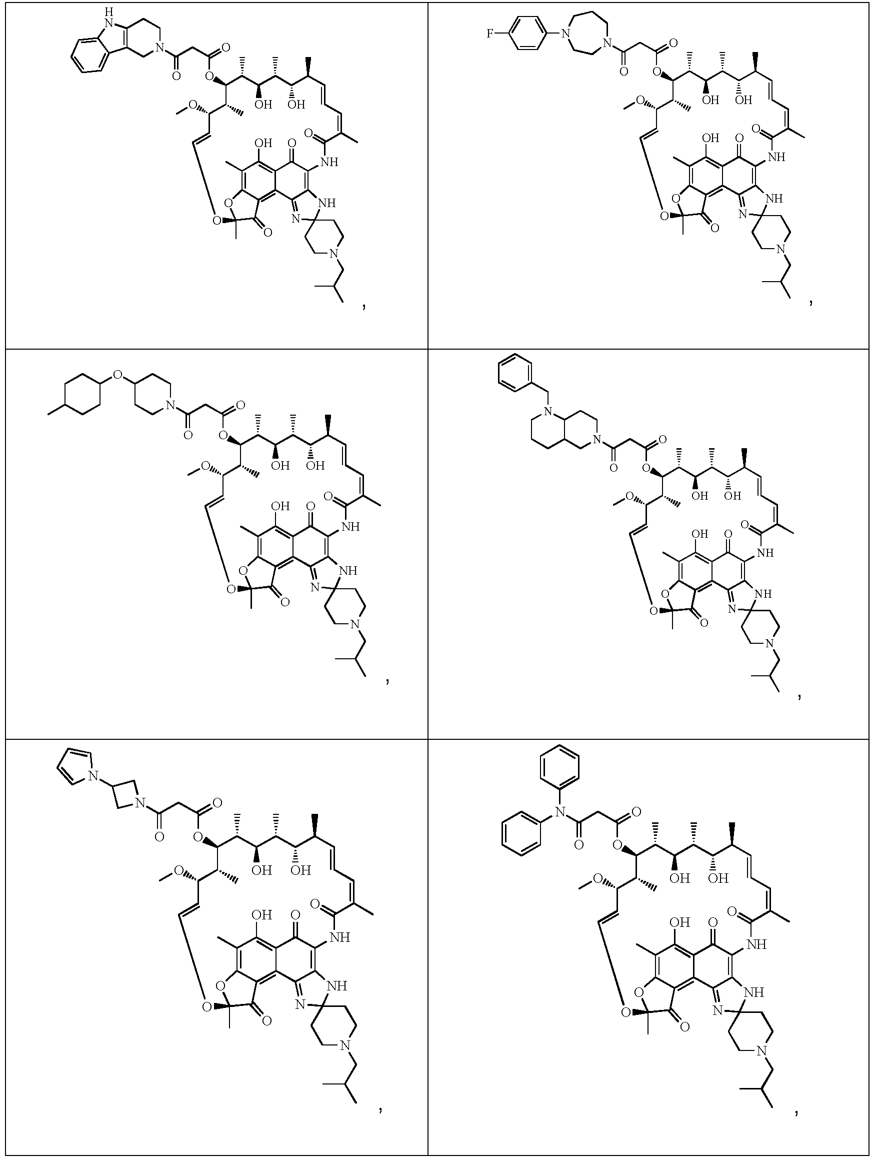

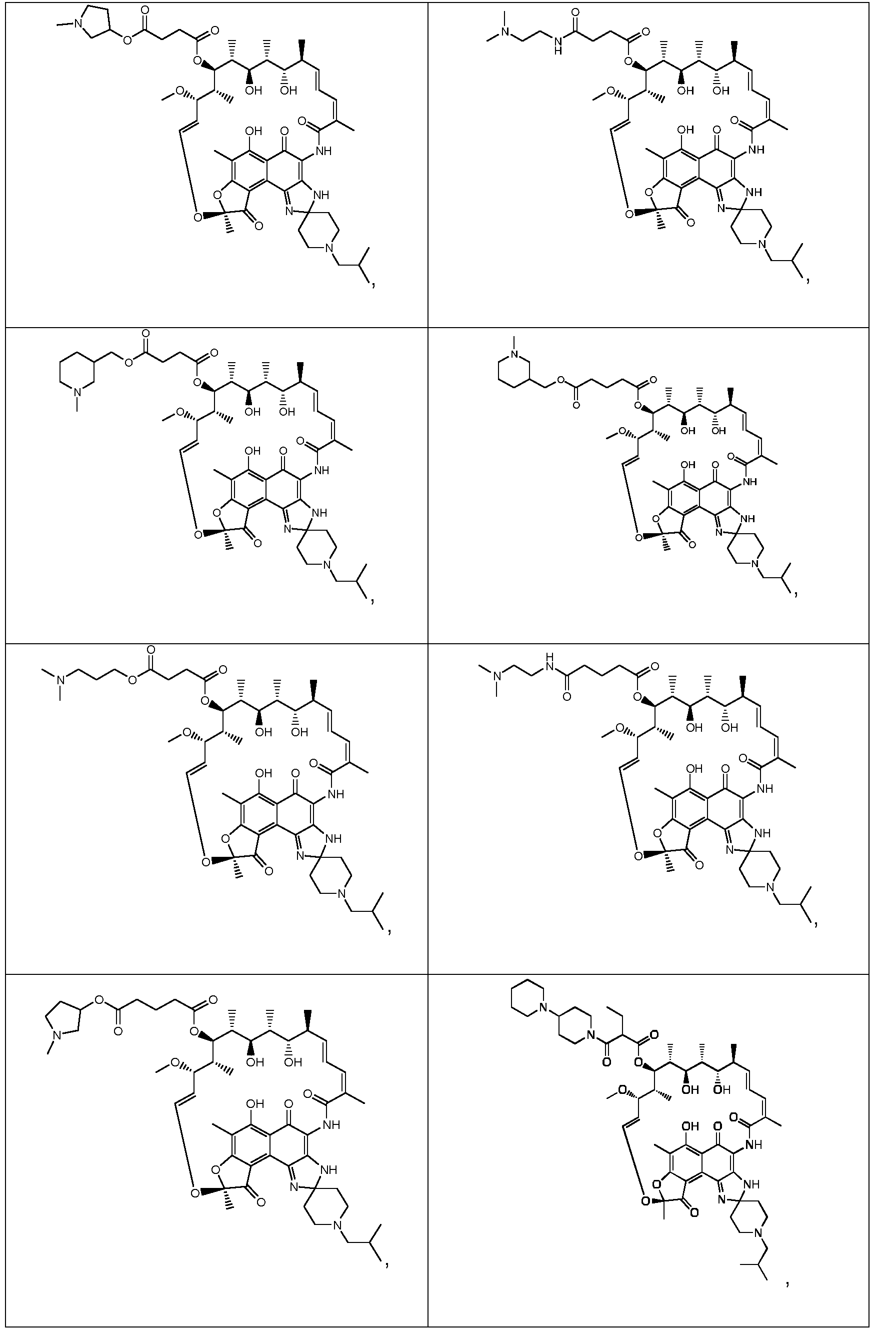

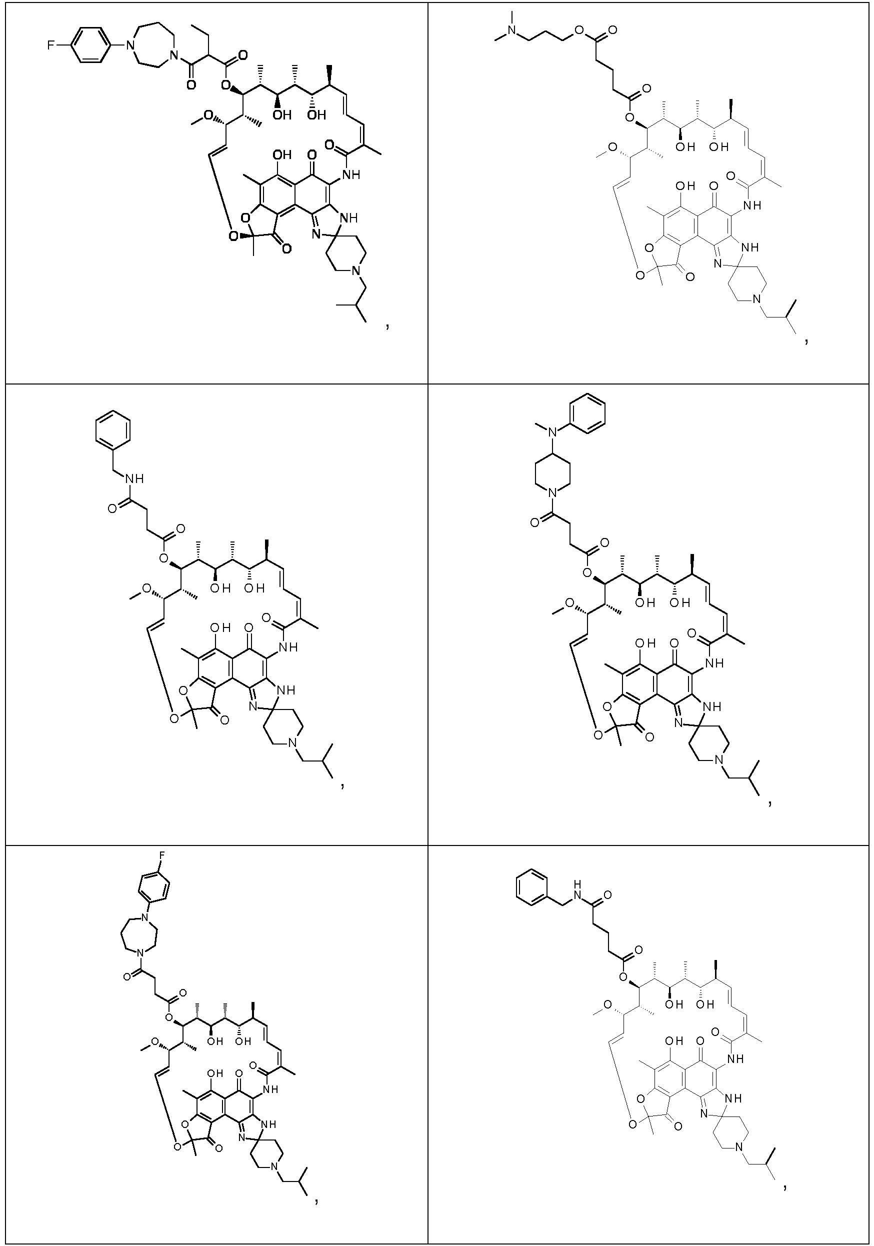

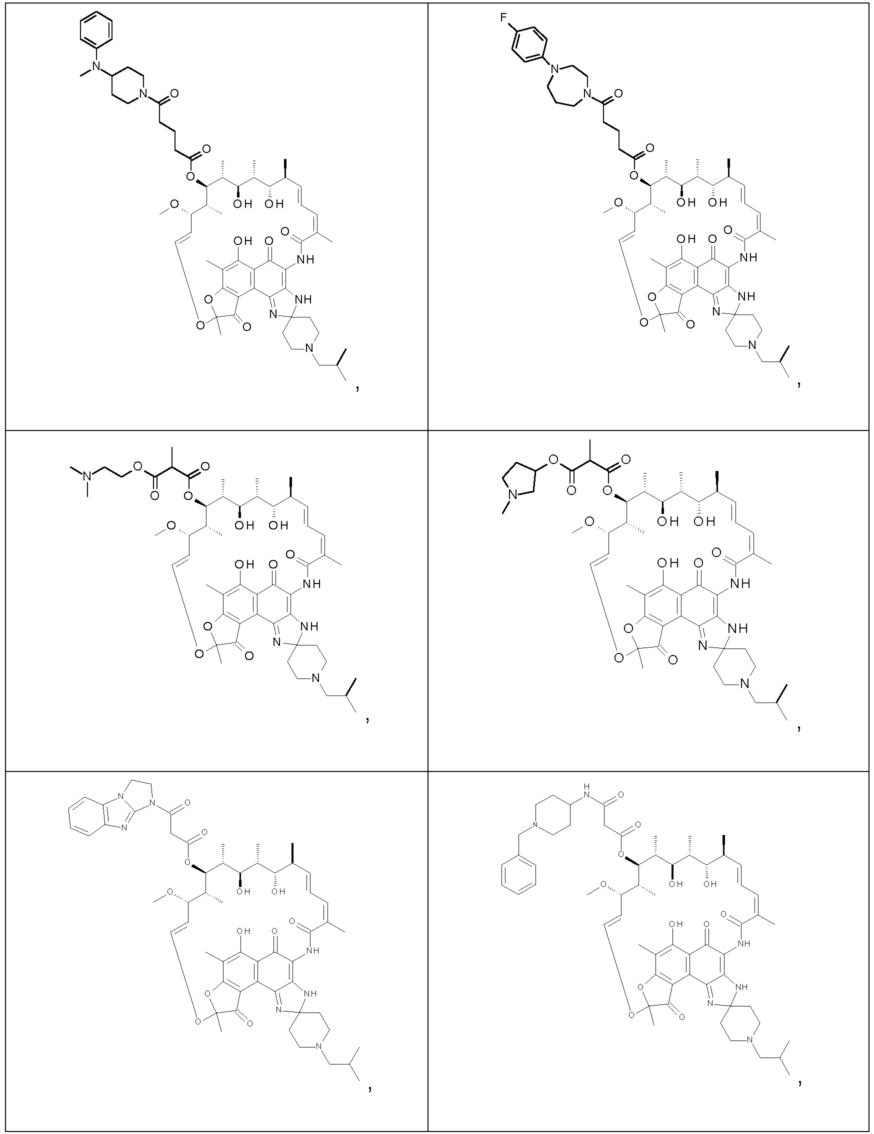

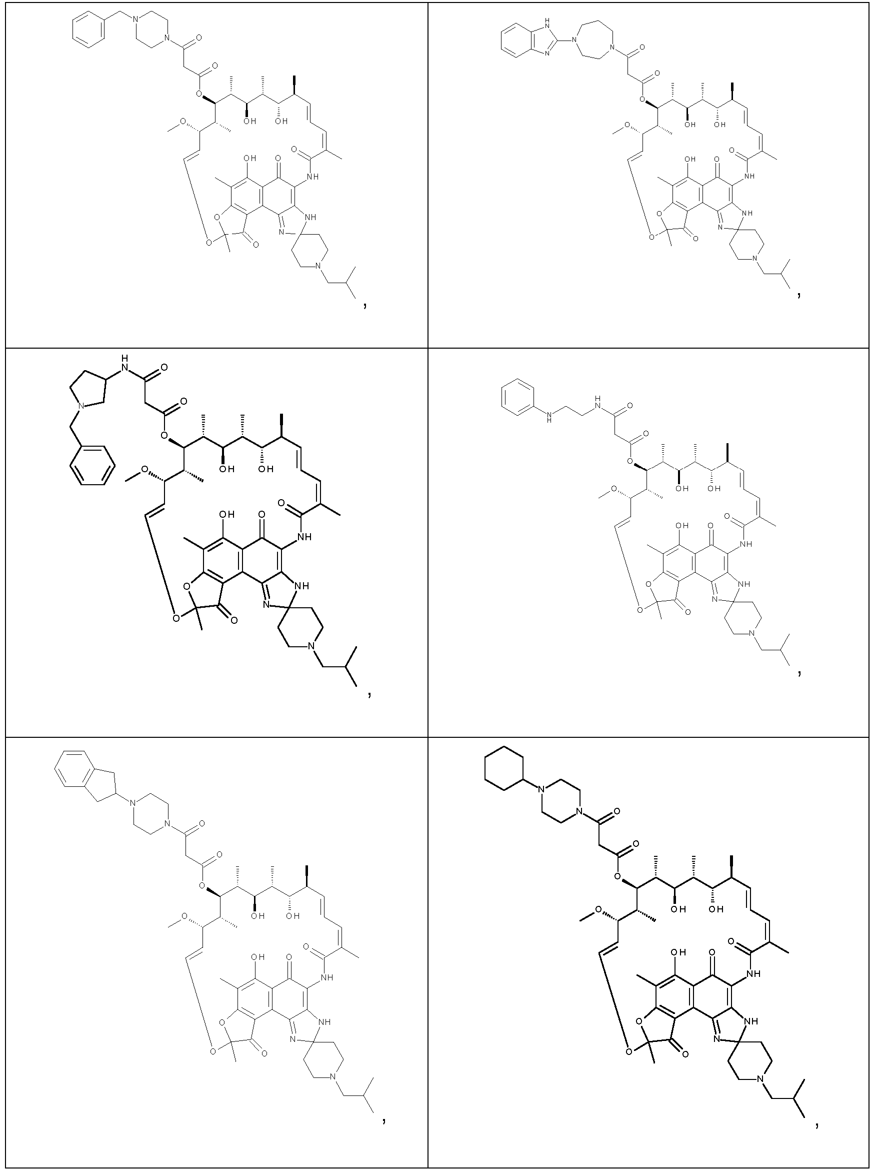

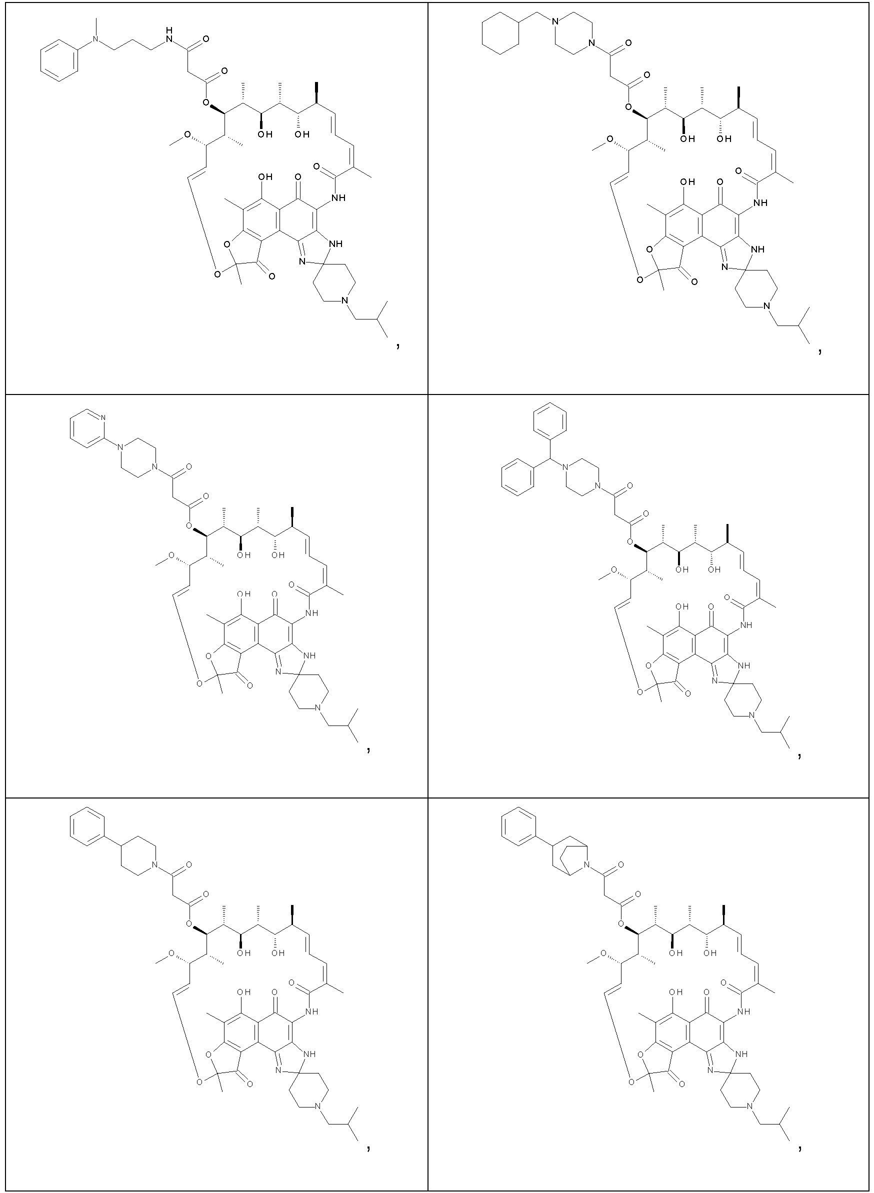

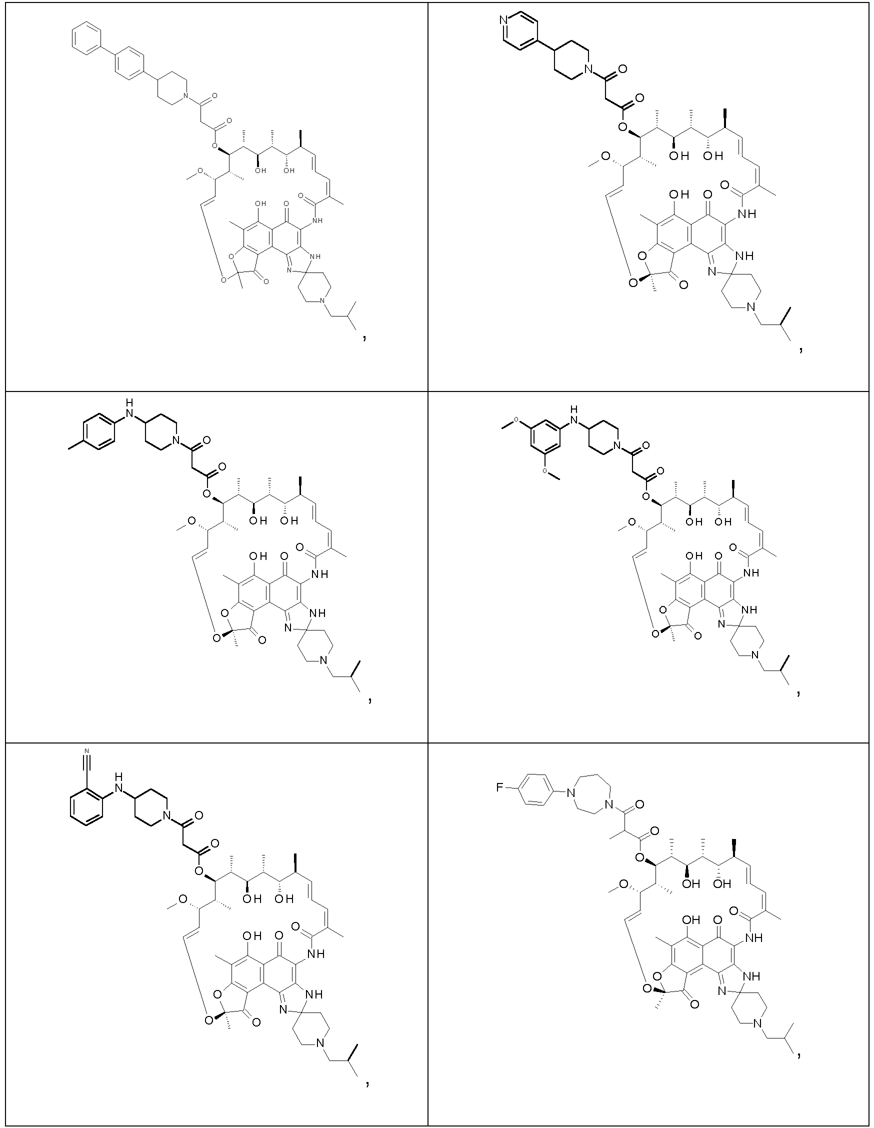

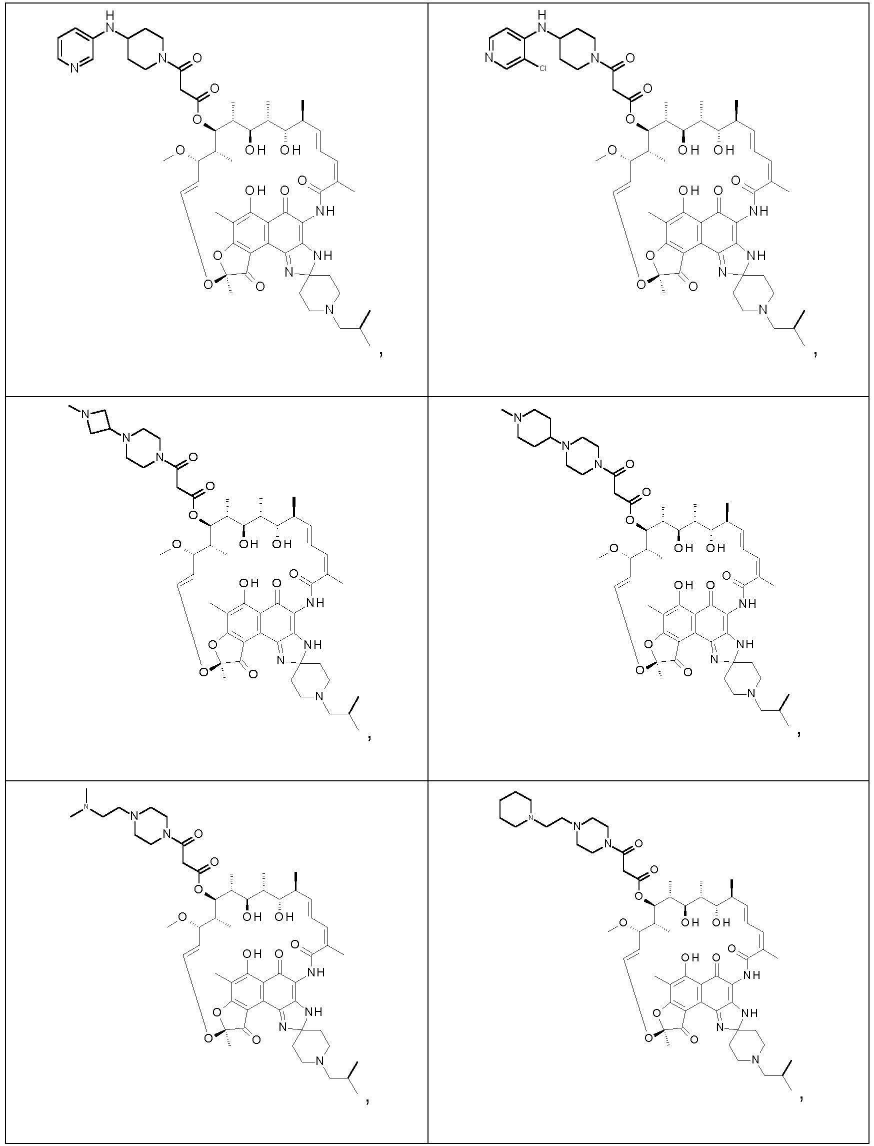

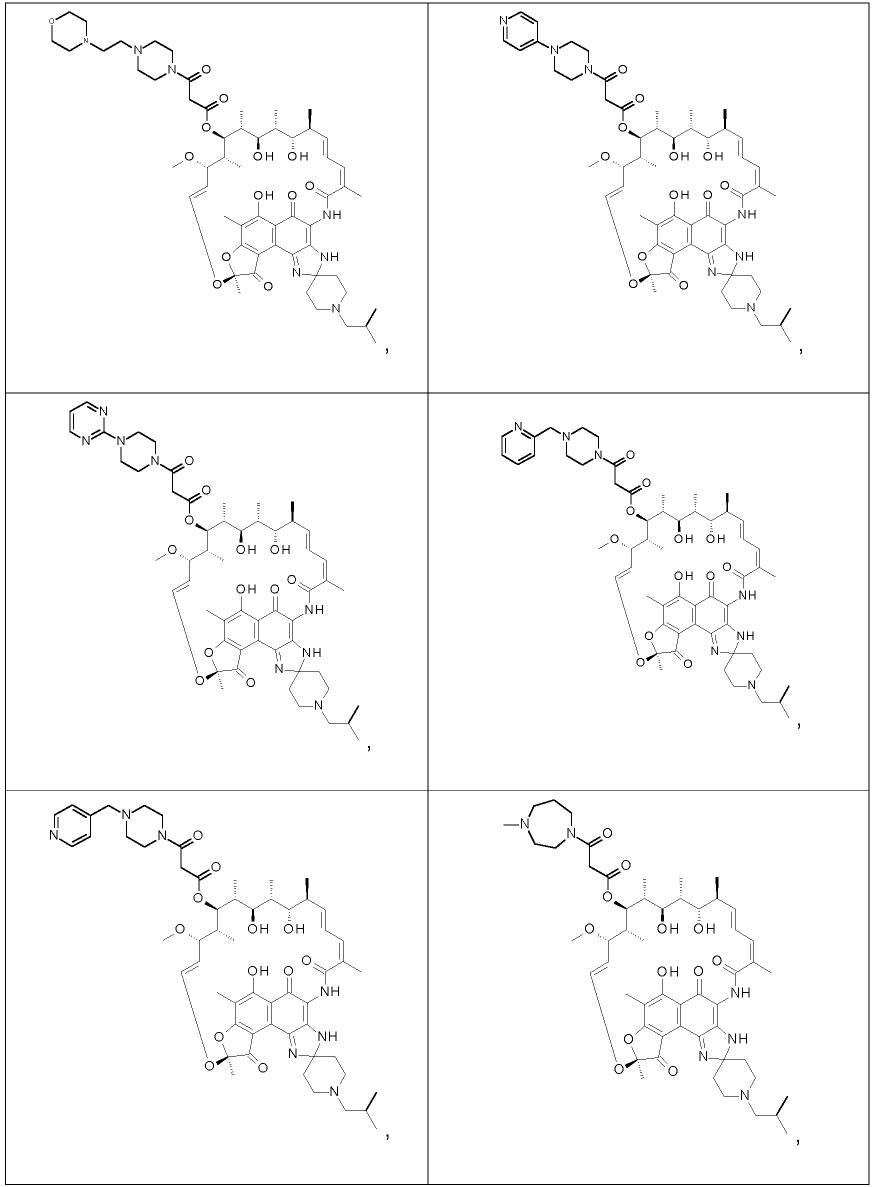

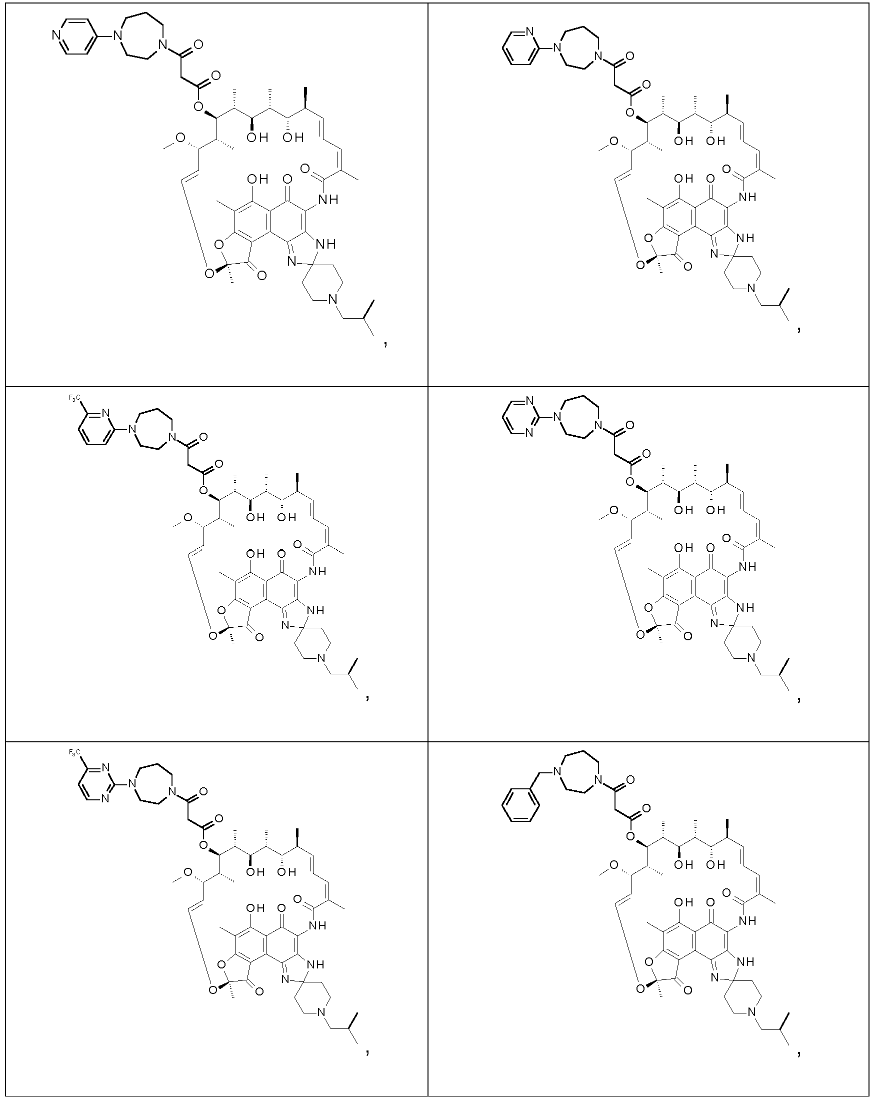

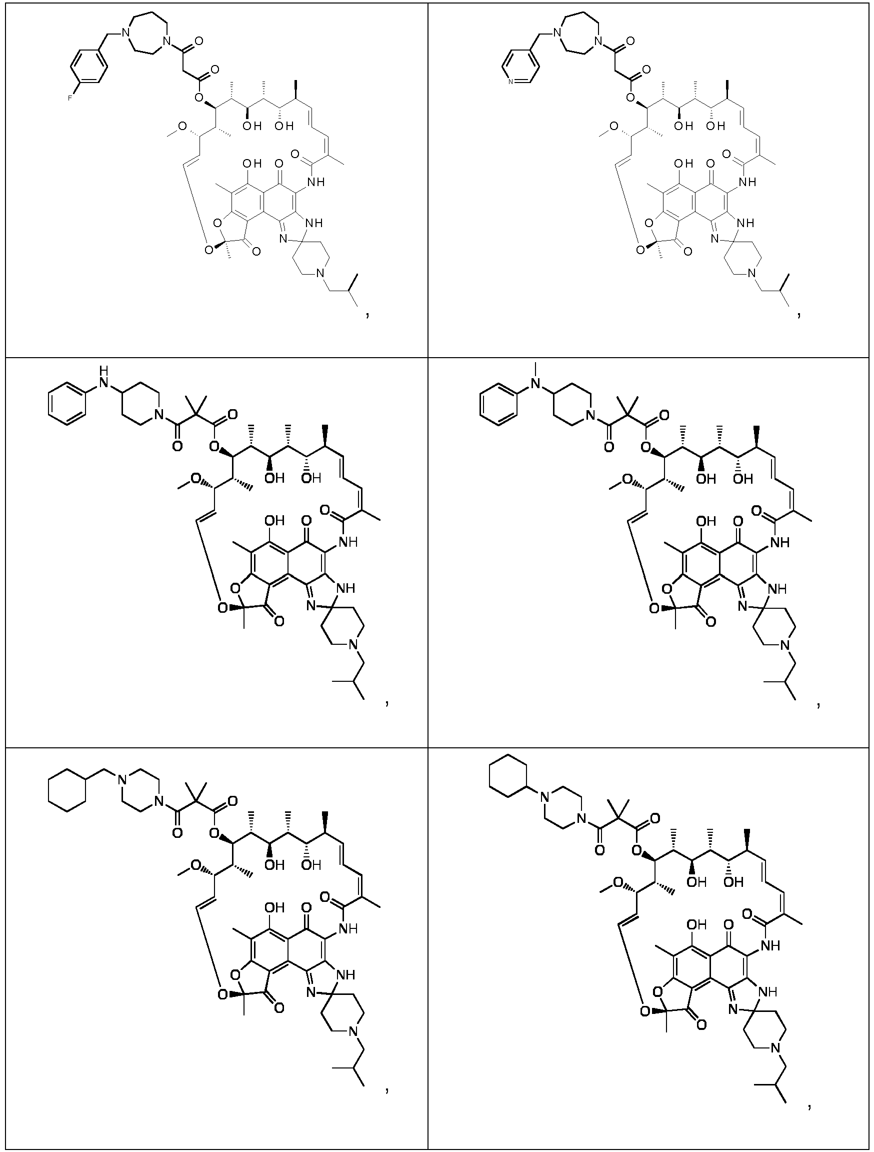

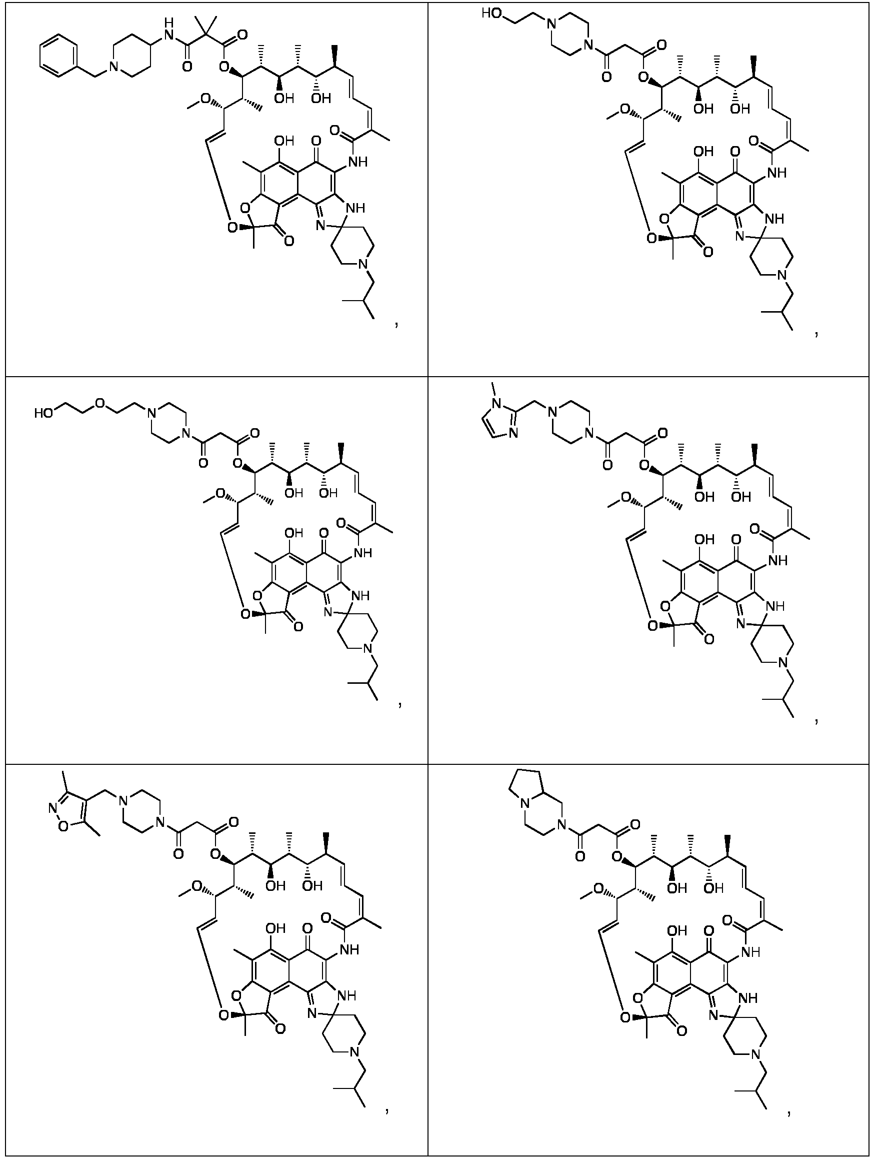

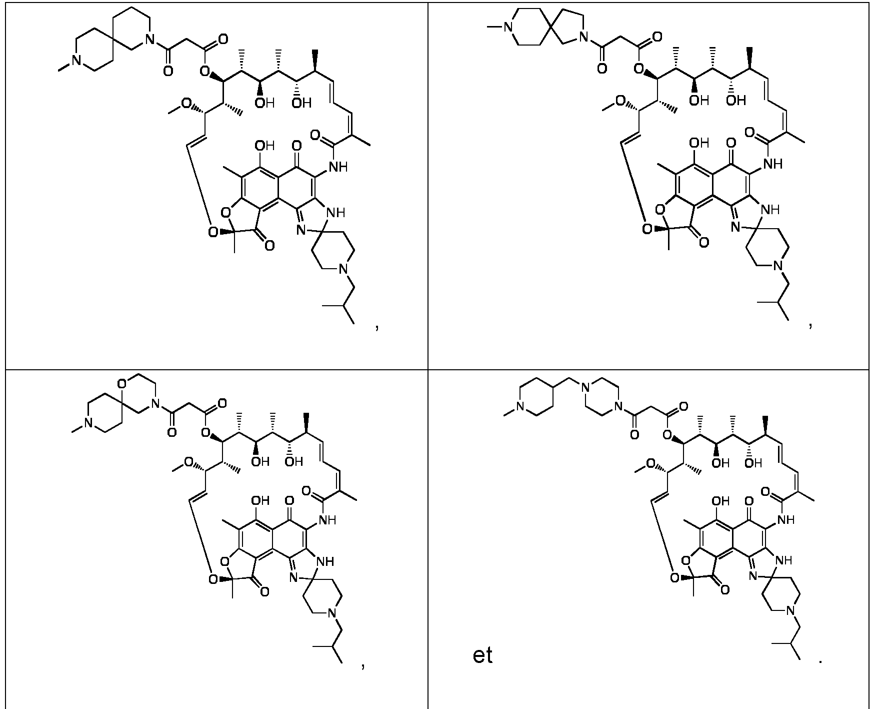

14. Composition pharmaceutique comprenant au moins un composé selon l'une quelconque des revendications précédentes, ou un sel, tautomère, solvate ou hydrate pharmaceutiquement acceptable de celui-ci, et un excipient pharmaceutiquement acceptable.

15. Composé selon l'une quelconque des revendications 1 à 13 ou sel, tautomère, solvate ou hydrate pharmaceutiquement acceptable de celui-ci, ou composition pharmaceutique selon la revendication 14, pour une utilisation en tant que médicament, de préférence pour une utilisation dans une méthode de traitement d'une infection bactérienne.
